# EUROPÄISCHE PATENTSCHRIFT

(11) **EP 3 197 891 B1**
(45) Veröffentlichungstag und Bekanntmachung des Hinweises auf die Patenterteilung: **21.11.2018**
(21) Anmeldenummer: 15766510.0
(22) Anmeldetag: 22.09.2015
(51) Int. Cl.: C07D 471/04, A61K 31/55, A61P 7/02, A61P 27/02

(54) **FAKTOR XIA HEMMENDE PYRIDOBENZAZEPIN- UND PYRIDOBENZAZOCIN-DERIVATE**
FACTOR XIA-INHIBITING PYRIDOBENZAZEPINE AND PYRIDOBENZAZOCINE DERIVATIVES
DÉRIVÉS DE PYRIDOBENZAZÉPINE ET DE PYRIDOBENZAZOCINE INHIBANT LE FACTEUR XIA

(30) Priorität: 24.09.2014 EP 14186080
(43) Veröffentlichungstag der Anmeldung: 02.08.2017
(73) Patentinhaber: Bayer Pharma Aktiengesellschaft, 13353 Berlin (DE)
(72) Erfinder: ROEHRIG, Susanne, 40724 Hilden (DE); HILLISCH, Alexander, 42651 Solingen (DE); HEITMEIER, Stefan, 42489 Wülfrath (DE); SCHMIDT, Martina Victoria, 51063 Köln (DE); SCHLEMMER, Karl-Heinz, 42113 Wuppertal (DE); TERSTEEGEN, Adrian, 42111 Wuppertal (DE); SCHÄFER, Martina, 13465 Berlin (DE); TELLER, Henrik, 18258 Schwaan (DE); JIMÈNEZ NÙNEZ, Eloisa, 42117 Wuppertal (DE)
(74) Vertreter: BIP Patents
(86) Internationale Anmeldenummer: PCT/EP2015/071645
(87) Internationale Veröffentlichungsnummer: WO 2016/046157

(56) Entgegenhaltungen:
- WO-A2-2008/079787
- GB-A- 2 497 806

## Beschreibung

Die Erfindung betrifft substituierte Pyridobenzazepin- und Pyridobenzazocin-Derivate und Verfahren zu ihrer Herstellung sowie ihre Verwendung zur Herstellung von Arzneimitteln zur Behandlung und/oder Prophylaxe von Krankheiten, insbesondere von Herz-Kreislauf-Erkrankungen, vorzugsweise von thrombotischen beziehungsweise thromboembolischen Erkrankungen sowie von Ödemen, als auch von ophthalmologischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden beziehungsweise minimiert werden. Die Blutstillung nach Gefäßverletzung erfolgt im Wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommen den Faktoren Xa und IIa (Thrombin) Schlüsselrollen zu: Faktor Xa bündelt die Signale der beiden Gerinnungswege, da er sowohl über Faktor VIIa/Tissue Factor (extrinsischer Weg) wie auch den Tenase Komplex (intrisischer Weg) durch Umsetzung von Faktor X entsteht. Die aktivierte Serinprotease Xa spaltet Prothrombin zu Thrombin, das über eine Reihe von Umsetzungen die Impulse aus der Kaskade auf den Gerinnungsstatus des Blutes überträgt.

In der jüngeren Vergangenheit ist die traditionelle Theorie der zwei getrennten Bereiche der Koagulationskaskade (extrinsischer beziehungsweise intrinsischer Pfad) aufgrund neuer Erkenntnisse modifiziert worden: In diesen Modellen wird die Koagulation durch Bindung von aktiviertem Faktor VIIa an Tissue Faktor (TF) initiiert. Der entstandene Komplex aktiviert Faktor X, was wiederum zur Thrombin-Generierung mit anschließender Herstellung von Fibrin und Thrombozyten-Aktivierung (via PAR-1) als verletzungsverschließende Endprodukte der Hämostase führt. Im Vergleich zur anschließenden Amplifikations-/Propagationsphase ist die Geschwindigkeit der Thrombinherstellung in dieser ersten Phase klein und durch das Auftreten von TFPI als Hemmer des TF-FVIIa-FX-Komplexes zeitlich begrenzt.

Ein zentraler Bestandteil des Übergangs von der Initiation zur Amplifikation und Propagation der Koagulation ist Faktor XIa: Thrombin aktiviert in positiven Rückkopplungsschleifen neben Faktor V und Faktor VIII auch Faktor XI zu Faktor XIa, der Faktor IX zu Faktor IXa umsetzt und über den so generierten Faktor IXa/Faktor VIIIa-Komplex die Faktor X-Aktivierung und damit wiederum die Thrombinbildung stark stimuliert, was zu starkem Thrombuswachstum führt und den Thrombus stabilisiert.

Darüber hinaus ist in den Blickpunkt gerückt, dass neben der Stimulation über Tissue Faktor die Aktivierung des Gerinnungssystems an insbesondere negativ geladenen Oberflächen erfolgen kann, zu denen neben Oberflächenstrukturen körperfremder Zellen (z.B. Bakterien) auch artifizielle Oberflächen wie Gefäßprothesen, Stents und extrakorporale Kreisläufe gehören. Auf der Oberfläche findet zunächst die Aktivierung von Faktor XII (FXII) zu Faktor XIIa statt, der im Folgenden an Zelloberflächen gebundenen Faktor XI zu Faktor XIa aktiviert. Dieser führt wie zuvor beschrieben zur weiteren Aktivierung der Gerinnungskaskade. Daneben aktiviert Faktor XIIa ebenfalls gebundenes Plasmaprokallikrein zu Plasmakallikrein (PK), das zum einen zu weiterer Faktor XII-Aktivierung im Rahmen einer Potentierungsschleife führt, was insgesamt eine Verstärkung der Initiation der Gerinnungskaskade zur Folge hat. Zusätzlich stellt PK eine wichtige Bradikinin-freisetzende Protease dar, die somit unter anderem zum Anstieg der endothelialen Permeabilität führt. Als weitere Substrate wurden Prorenin und Prourokinase beschrieben, deren Aktivierung die regulatorischen Prozesse des Renin-Angiotensin-Systems und der Fibrinolyse beeinflussen kann. Damit stellt die Aktivierung von PK ein wichtiges Bindeglied zwischen koagulativen und inflammatorischen Prozessen dar.

Eine unkontrollierte Aktivierung des Gerinnungssystems oder eine defekte Hemmung der Aktivierungsprozesse kann die Bildung von lokalen Thrombosen oder Embolien in Gefäßen (Arterien, Venen, Lymphgefäßen) oder Herzhöhlen bewirken. Darüber hinaus kann eine systemische Hyperkoagulabilität zur systemweiten Bildung von Thromben und schließlich zu einer Verbrauchskoagulopathie im Rahmen einer disseminierten intravasalen Gerinnung führen. Thromboembolische Komplikationen können ferner in extrakorporalen Blutkreisläufen, wie z. B. während einer Hämodialyse, sowie in Gefäß- beziehungsweise Herzklappenprothesen und Stents auftreten.

Im Verlauf vieler Herzkreislauf- und Stoffwechselerkrankungen kommt es infolge systemischer Faktoren, wie z.B. Hyperlipidämie, Diabetes oder Rauchen, infolge von Blutflußveränderungen mit Stase, wie z.B. beim Vorhofflimmern, oder infolge pathologischer Gefäßwandveränderungen, z.B. endothelialer Dysfunktionen oder Atherosklerose, zu einer erhöhten Neigung von Gerinnungs- und Thrombozytenaktivierung. Diese unerwünschte und überschießende Aktivierung der Gerinnung kann durch Bildung fibrin- und plättchenreicher Thromben zu thromboembolischen Erkrankungen und thrombotischen Komplikationen mit lebensbedrohlichen Zuständen führen. Hierbei können auch entzündliche Prozesse involviert sein. Thromboembolische Erkrankungen gehören daher nach wie vor zu den häufigsten Ursachen von Morbidität und Mortalität in den meisten industrialisierten Ländern.

Die aus dem Stand der Technik bekannten Antikoagulantien, d.h. Stoffe zur Hemmung oder Verhinderung der Blutgerinnung, weisen verschiedene, Nachteile auf. Eine effiziente Behandlungsmethode beziehungsweise Prophylaxe von thrombotischen/thromboembolischen Erkrankungen erweist sich in der Praxis deshalb als sehr schwierig und unbefriedigend.

In der Therapie und Prophylaxe von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im Folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen. Niedermolekulare Heparine besitzen zwar eine geringere Wahrscheinlichkeit zur Ausbildung einer Heparin-induzierten Thrombocytopenie, sind aber auch nur subkutan applizierbar. Dies gilt auch für Fondaparinux, einem synthetisch hergestellten, selektiven Faktor Xa Inhibitor mit einer langen Halbwertszeit.

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig. Darüber hinaus sind weitere Nebenwirkungen wie gastrointestinale Störungen, Haarausfall und Hautnekrosen beschrieben.

Neuere Ansätze für orale Antikoagulantien befinden sich in verschiedenen Phasen der klinischen Erprobung beziehungsweise im klinischen Einsatz und haben ihre Wirksamkeit in verschiedenen Studien unter Beweis gestellt. Allerdings kann es auch unter der Einnahme dieser Arzneimittel insbesondere bei prädisponierten Patienten zu Blutungskomplikationen kommen. Daher ist bei antithrombotischen Arzneimitteln ist die therapeutische Breite von zentraler Bedeutung: Der Abstand zwischen der therapeutisch wirksamen Dosis zur Gerinnungshemmung und der Dosis, bei der Blutungen auftreten können, sollte möglichst groß sein, so dass eine maximale therapeutische Wirksamkeit bei minimalem Risikoprofil erreicht wird.

In verschiedenen in-vitro und in-vivo Modellen mit beispielsweise Antikörpern als Faktor XIa Inhibitoren, aber auch in Faktor XIa-Knock-out-Modellen, wurde der anti-thrombotische Effekt bei geringer/keiner Verlängerung der Blutungszeit oder Vergrößerung des Blutvolumens belegt. In klinischen Studien waren erhöhte Faktor XIa-Spiegel mit einer gesteigerten Ereignisrate assoziiert. Dagegen führte Faktor XI-Defizienz (Hämophilie C) nicht zu spontanen Blutungen und fiel nur im Rahmen von Operationen und Traumen auf, zeigte aber eine Protektion gegenüber bestimmten thromboembolischen Ereignissen.

Daneben ist Plasmakallikrein (PK) mit weiteren Erkrankungen assoziiert, die mit erhöhten Gefäßpermeabilitäten oder chronisch-entzündlichen Erkrankungen einhergehen, wie es z.B. bei der diabetischen Retinopathie, dem Makulaödem und dem hereditären Angioödem beziehungsweise chronisch-entzündlichen Darmerkrankungen der Fall ist. Der diabetischen Retinopathie liegt in erster Linie eine Mikrogefäßschwäche zu Grunde, in deren Folge es zu einer Basalmembranverdickung der Gefäße und zum Verlust von gefäßummantelnden Perizyten, später zum Gefäßverschluss mit retinaler Ischämie kommt, welche aufgrund der hervorgerufenen retinalen Hypoxie zu verstärkter Gefäßpermeabilität mit nachfolgender Ausbildung eines Makulaödems und aufgrund aller vorliegender Prozesse zur Erblindung des Patienten führen kann. Beim Hereditären Angioödem (HAE) kommt es durch verminderte Bildung des physiologischen Kallikrein-Inhibitors C1-Esterase-Inhibitors zur unkontrollierten Plasmakallikrein-Aktivierung und damit zu Entzündungen mit fulminanter Ödembildung und starken Schmerzen. Aus tierexperimentellen Ansätzen gibt es Hinweise, dass die Inhibition von Plasmakallikrein die erhöhte Gefäßpermeabilität inhibiert und somit die Ausbildung eines Makulaödems beziehungsweise der diabetischen Retinopathie verhindern beziehungsweise die akute Symptomatik des HAE verbessern kann. Orale Plasmakallikrein-Inhibitoren könnten ebenfalls zur Prophylaxe des HAE eingesetzt werden.

Bei der Progression von chronisch-entzündlichen Darmerkrankungen (CED) haben vor allem die mittels Plasmakallikrein generierten Kinine eine tragende Rolle. Deren pro-inflammatorische Wirkung über Aktivierung von Bradykinin-Rezeptoren induziert und potenziert den Krankheitsverlauf. Studien an Morbus Crohn-Patienten zeigen eine Korrelation zwischen der Kallikrein-Konzentration im Darmepithel und dem Grad der Darmentzündung. Eine Aktivierung des Kallikrein-Kinin-Systems wurde ebenfalls in tierexperimentellen Studien beobachtet. Eine Hemmung der Bradykinin-Synthese durch Kallikrein-Inhibitoren könnte demnach auch zur Prophylaxe und/oder Therapie von chronisch-entzündlichen Darmerkrankungen eingesetzt werden.

Weiterhin kann auch die Kombination von antithrombotischen und antiinflammtorischen Prinzipien für viele Erkrankungen besonders attraktiv sein, um die wechselseitige Verstärkung von Koagulation und Inflammation zu unterbinden.

Eine Aufgabe der vorliegenden Erfindung ist daher die Bereitstellung neuer Verbindungen zur Behandlung von Herz-Kreislauf-Erkrankungen, insbesondere von thrombotischen beziehungsweise thromboembolischen Erkrankungen, und/oder ödematösen Erkrankungen, und/oder ophthalmologischen Erkrankungen, insbesondere von diabetischer Retinopathie beziehungsweise des Makulaödems, bei Menschen und Tieren, die eine große therapeutische Bandbreite aufweisen.

WO 2006/030032 beschreibt unter anderem substituierte Pyridinone als allosterische Modulatoren des mGluR2 Rezeptors und WO 2008/079787 beschreibt substituierte Pyridin-2-one und ihre Verwendung als Glucokinase Aktivatoren. GB 2497806, WO 2014/154794, WO 2014/160592, WO 2015/011087 und WO 2015/063093 beschreiben substituierte Pyridin-2-one und ihre Verwendung als Faktor XIa Inhibitoren.

Gegenstand der Erfindung sind Verbindungen der Formel 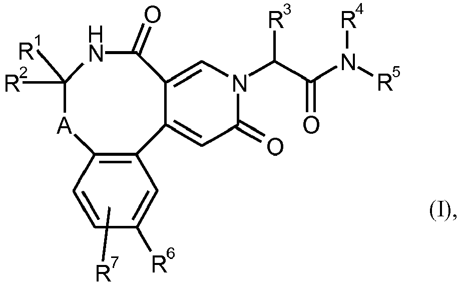 in welcher
- A: für eine Bindung oder -CH₂- steht,
- R¹: für Wasserstoff oder Methyl steht,
wobei Methyl substituiert sein kann mit einem Substituenten Fluor,
- R²: für Wasserstoff oder Methyl steht,
oder
- R¹ und R²: bilden zusammen mit dem Kohlenstoffatom, an das sie gebunden sind, einen Cyclopropyl-Ring,
- R³: für Wasserstoff, C₁-C₅-Alkyl, C₁-C₄-Alkoxy, Difluormethyl, Trifluormethyl, 1,1-Difluorethyl, 3,3,3-Trifluor-2-hydroxyprop-1-yl, 3,3,3-Trifluor-2-methoxyprop-1-yl, 3,3,3-Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, C₃-C₆-Cycloalkyl, 4- bis 6-gliedriges Oxo-Heterocyclyl, 1,4-Dioxanyl, Oxazolyl, Phenyl und Pyridyl,
worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy,
- R⁴: für Wasserstoff steht,
- R⁵: für eine Gruppe der Formel 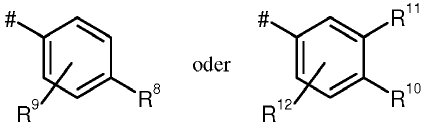 oder 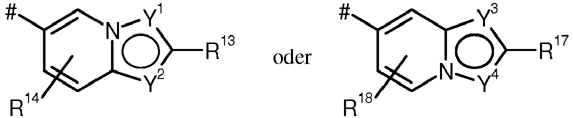 oder 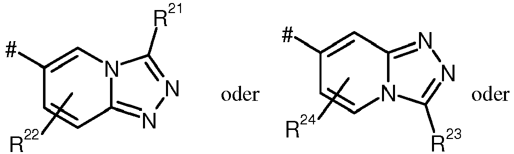 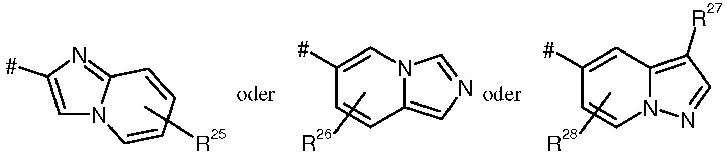 oder 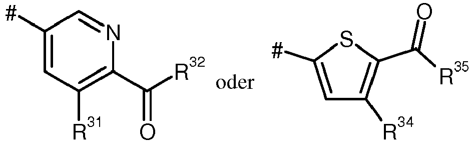 oder 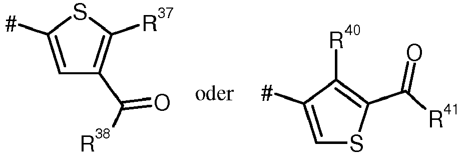
steht,
wobei # die Anknüpfstelle an das Stickstoffatom ist,
- R⁸: für Hydroxycarbonyl, Aminocarbonyl oder 5-gliedriges Heterocyclyl steht,
wobei Heterocyclyl substituiert sein kann mit 1 bis 2 Substituenten unabhängig voneinander ausgewählt aus der Gruppe bestehend aus Oxo, Hydroxy, Thioxo, Sulfanyl, Methyl, Difluormethyl, Trifluormethyl, 2-Hydroxycarbonyl-1,1,2,2-tetrafluorethyl und 2-Methoxycarbonyl-1,1,2,2-tetrafluorethyl,
worin Methyl substituiert sein kann mit einem Substituenten Methoxy,
- R⁹: für Wasserstoff, Chlor, Fluor oder Methyl steht,
- R¹⁰ und R¹¹: zusammen mit den Kohlenstoffatomen an die sie gebunden sind einen 5-gliedrigen Heterocyclus bilden,
wobei der Heterocyclus substituiert sein kann mit 1 bis 2 Substituenten unabhängig voneinander ausgewählt aus der Gruppe bestehend aus Oxo, Chlor, Hydroxy, Hydroxycarbonyl, Methyl, Difluormethyl, Trifluormethyl, 1,1,2,2,2-Pentafluorethyl, 2-Hydroxycarbonyl-1,1,2,2-tetrafluorethyl und 2-Methoxy-carbonyl-1,1,2,2-tetrafluorethyl,
- R¹²: für Wasserstoff, Chlor, Fluor, Methyl oder Methoxy steht,
- Y¹: für ein Stickstoffatom oder C-R¹⁵ steht,
worin
R¹⁵ für Wasserstoff, Chlor, Hydroxy, Methoxy oder C₁-C₃-Alkoxycarbonyl steht,
- Y²: für ein Stickstoffatom oder C-R¹⁶ steht,
worin
R¹⁶ für Wasserstoff, Chlor, Hydroxy oder Methoxy steht,
- R¹³: für Wasserstoff, Hydroxycarbonyl, Hydroxycarbonylmethyl oder Phenyl steht, worin Phenyl substituiert sein kann mit 1 bis 2 Substituenten Fluor,
- R¹⁴: für Wasserstoff, Chlor, Fluor oder Methyl steht,
- Y³: für ein Stickstoffatom oder C-R¹⁹ steht,
worin
R¹⁹ für Wasserstoff, Chlor, Hydroxy oder Methoxy steht,
- Y⁴: für ein Stickstoffatom oder C-R²⁰ steht,
worin
R²⁰ für Wasserstoff, Chlor, Hydroxy oder Methoxy steht,
- R¹⁷: für Wasserstoff, Hydroxycarbonyl, Hydroxycarbonylmethyl, C₁-C₃-Alkoxycarbonyl oder Aminocarbonyl steht,
- R¹⁸: für Wasserstoff, Chlor, Fluor oder Methyl steht,
- R²¹: für Wasserstoff, Chlor, Hydroxy, C₁-C₄-Alkyl, Methoxy, C₁-C₃-Alkylaminomethyl oder Morpholinylmethyl steht,
- R²²: für Wasserstoff, Chlor, Fluor oder Methyl steht,
- R²³: für Wasserstoff, Chlor, Hydroxy oder Methoxy steht,
- R²⁴: für Wasserstoff, Chlor, Fluor oder Methyl steht,
- R²⁵: für Wasserstoff, Hydroxycarbonyl oder Hydroxycarbonylmethyl steht,
- R²⁶: für Wasserstoff, Chlor, Fluor oder Methyl steht,
- R²⁷: für Hydroxycarbonyl, Aminocarbonyl, C₁-C₃-Alkoxycarbonyl oder C₁-C₃-Alkylaminocarbonyl steht,
wobei Alkylaminocarbonyl substituiert sein kann mit einem Substituenten ausgewählt aus der Gruppe bestehend aus Hydroxy, Trifluormethyl, Methoxy und Trifluormethoxy,
- R²⁸: für Wasserstoff, Chlor, Fluor oder Methyl steht,
- R³¹: für Wasserstoff oder Fluor steht,
- R³²: für Hydroxy oder -NHR³³ steht,
worin
R³³ für Wasserstoff, Methyl oder Ethyl steht,
- R³⁴: für Wasserstoff oder Fluor steht,
- R³⁵: für Hydroxy oder -NHR³⁶ steht,
worin
R³⁶ für Wasserstoff, Methyl oder Ethyl steht,
- R³⁷: für Wasserstoff oder Fluor steht,
- R³⁸: für Hydroxy oder -NHR³⁹ steht,
worin
R³⁹ für Wasserstoff, Methyl oder Ethyl steht,
- R⁴⁰: für Wasserstoff oder Fluor steht,
- R⁴¹: für Hydroxy oder -NHR⁴² steht,
worin
R⁴² für Wasserstoff, Methyl oder Ethyl steht,
- R⁶: für Brom, Chlor, Fluor, Methyl, Difluormethyl, Trifluormethyl, Methoxy, Difluormethoxy oder Trifluormethoxy steht,
- R⁷: für Wasserstoff, Chlor oder Fluor steht,
und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler beziehungsweise chiraler Phase.

Sofern die erfindungsgemäßen Verbindungen in tautomeren Formen vorkommen können, umfasst die vorliegenden Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagenzien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin, *N*-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Im Rahmen der vorliegenden Erfindung haben die Substituenten, soweit nicht anders spezifiziert, die folgende Bedeutung:
Alkyl steht für einen linearen oder verzweigten Alkylrest mit 1 bis 5 Kohlenstoffatomen, bevorzugt 1 bis 3 Kohlenstoffatomen, beispielhaft und vorzugsweise für Methyl, Ethyl, n-Propyl, iso-Propyl, 2-Methyl-prop-1-yl, n-Butyl, *tert*.-Butyl und 2,2-Dimethylprop-1-yl.

Alkoxy steht für einen linearen oder verzweigten Alkoxyrest mit 1 bis 4 Kohlenstoffatomen, bevorzugt 1 bis 3 Kohlenstoffatomen, beispielhaft und vorzugsweise für Methoxy, Ethoxy, n-Propoxy, iso-Propoxy, 2-Methyl-prop-1-oxy, n-Butoxy und *tert*.-Butoxy.

Alkoxycarbonyl steht für einen linearen oder verzweigten Alkoxyrest, der über eine Carbonylgruppe gebunden ist, mit 1 bis 3 Kohlenstoffatomen, bevorzugt 1 bis 2 Kohlenstoffatomen, beispielhaft und vorzugsweise für Methoxycarbonyl, Ethoxycarbonyl, n-Propoxycarbonyl und iso-Propoxycarbonyl.

Alkylaminocarbonyl steht für eine Amino-Gruppe mit einem oder zwei unabhängig voneinander gewählten gleichen oder verschiedenen geradkettigen oder verzweigten Alkylsubstituenten, die jeweils 1 bis 3 Kohlenstoffatome aufweisen, und die über eine Carbonylgruppe gebunden ist, beispielhaft und vorzugsweise für Methylaminocarbonyl, Ethylaminocarbonyl, n-Propylaminocarbonyl, iso-Propylaminocarbonyl, *N*,*N*-Dimethylaminocarbonyl, *N*,*N*-Diethylaminocarbonyl, *N-*Ethyl-*N*-methylaminocarbonyl, *N*-Methyl-*N*-n-propylaminocarbonyl, *N*-iso-Propyl-*N*-n-propylaminocarbonyl und *N*,*N*-Diisopropylaminocarbonyl. C₁-C₃-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent.

Alkylaminomethyl steht für eine Amino-Gruppe mit einem oder zwei unabhängig voneinander gewählten gleichen oder verschiedenen geradkettigen oder verzweigten Alkylsubstituenten, die jeweils 1 bis 3 Kohlenstoffatome aufweisen, und die über eine Methylgruppe gebunden ist, beispielhaft und vorzugsweise für Methylaminomethyl, Ethylaminomethyl, n-Propylaminomethyl, iso-Propylaminomethyl, *N*,*N*-Dimethylaminomethyl, *N*,*N*-Diethylaminomethyl, *N*-Ethyl-*N-*methylaminomethyl, *N*-Methyl-*N*-n-propylaminomethyl, *N*-iso-Propyl-*N*-n-propylaminomethyl und *N*,*N*-Diisopropylaminomethyl. C₁-C₃-Alkylaminomethyl steht beispielsweise für einen Monoalkylaminomethylrest mit 1 bis 3 Kohlenstoffatomen oder für einen Dialkylaminomethylrest mit jeweils 1 bis 3 Kohlenstoffatomen pro Alkylsubstituent.

Cycloalkyl steht für eine monocyclische Cycloalkylgruppe mit 3 bis 6 Kohlenstoffatomen, beispielhaft und vorzugsweise für Cycloalkyl seien genannt Cyclopropyl, Cyclobutyl, Cyclopentyl und Cyclohexyl.

5-gliedriges Heterocyclyl in der Definition des Restes R⁸ steht für einen gesättigten, teilweise ungesättigten oder aromatischen monocyclischen Rest mit 5 Ringatomen und bis zu 4 Heteroatomen aus der Reihe S, O und N, wobei ein Stickstoffatom auch ein N-Oxid bilden kann, beispielhaft und vorzugsweise für Thienyl, Furyl, Pyrrolyl, Thiazolyl, Oxazolyl, Isoxazolyl, Oxadiazolyl, Thiadiazolyl, Pyrazolyl, Imidazolyl, Triazolyl, Tetrazolyl, Dihydrooxazolyl und Dihydroimidazolyl.

5-gliedriger Heterocyclus in der Definition der Reste R¹⁰ und R¹¹ steht für einen gesättigten, teilweise ungesättigten oder aromatischen monocyclischen Rest mit 5 Ringatomen und bis zu 2 Heteroatomen aus der Reihe S, O und N, wobei ein Stickstoffatom auch ein N-Oxid bilden kann. Dieser 5-gliedrige Heterocyclus steht zusammen mit dem Phenylring, an den er gebunden ist, beispielhaft und vorzugsweise für 2,3-Dihydro-1-benzothiophen-5-yl, 1,3-Dihydro-2-benzothiophen-5-yl, 2,3-Dihydro-1-benzofuran-5-yl, 1,3-Dihydro-2-benzofuran-5-yl, Indolin-5-yl, Isoindolin-5-yl, 2,3-Dihydro-1H-indazol-5-yl, 2,3-Dihydro-1H-benzimidazol-5-yl, 1,3-Dihydro-2,1-benzoxazol-5-yl, 2,3-Dihydro-1,3-benzoxazol-5-yl, 1,3-Dihydro-2,1-benzothiazol-5-yl, 2,3-Dihydro-1,3-benzothiazol-5-yl, 1H-Benzimidazol-5-yl, 1H-Indazol-5-yl, 2H-Indazol-5-yl, 1,2-Benzoxazol-5-yl, Indol-5-yl, Isoindol-5-yl, Benzofuran-5-yl, Benzothiophen-5-yl, 2,3-Dihydro-1-benzothiophen-6-yl, 1,3-Dihydro-2-benzothiophen-6-yl, 2,3-Dihydro-1-benzofuran-6-yl, 1,3-Dihydro-2-benzofuran-6-yl, Indolin-6-yl, Isoindolin-6-yl, 2,3-Dihydro-1H-indazol-6-yl, 2,3-Dihydro-1H-benzimidazol-6-yl, 1,3-Dihydro-2,1-benzoxazol-6-yl, 2,3-Dihydro-1,3-benzoxazol-6-yl, 1,3-Dihydro-2,1-benzothiazol-6-yl, 2,3-Dihydro-1,3-benzothiazol-6-yl, 1H-Benzimidazol-6-yl, 1H-Indazol-6-yl, 2H-Indazol-6-yl, 1,2-Benzoxazol-6-yl, Indol-6-yl, Isoindol-6-yl, Benzofuran-6-yl und Benzothiophen-6-yl.

4- bis 6-gliedriges Oxo-Heterocyclyl in der Definition des Restes R³ steht für einen gesättigten monocyclischen Rest mit 4 bis 6 Ringatomen, in dem ein Ringatom ein Sauerstoffatom ist, beispielhaft und vorzugsweise für Oxetanyl, Tetrahydrofuranyl und Tetrahydro-2H-pyranyl.

In den Formeln der Gruppe, die für R⁵ stehen kann, steht der Endpunkt der Linie, neben der jeweils ein # steht, nicht für ein Kohlenstoffatom beziehungsweise eine CH₂-Gruppe sondern ist Bestandteil der Bindung zu dem Atom, an das R⁵ gebunden ist.

Bevorzugt sind Verbindungen der Formel (I), in welcher
- A: für eine Bindung oder -CH₂- steht,
- R¹: für Wasserstoff oder Methyl steht,
- R²: für Wasserstoff oder Methyl steht,
oder
- R¹ und R²: bilden zusammen mit dem Kohlenstoffatom, an das sie gebunden sind, einen Cyclopropyl-Ring,
- R³: für Wasserstoff, C₁-C₅-Alkyl, C₁-C₄-Alkoxy, Difluormethyl, Trifluormethyl, 1,1-Difluorethyl, 3,3,3-Trifluor-2-hydroxyprop-1-yl, 3,3,3-Trifluor-2-methoxyprop-1-yl, 3,3,3-Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, C₃-C₆-Cycloalkyl, 4- bis 6-gliedriges Oxo-Heterocyclyl, 1,4-Dioxanyl, Oxazolyl, Phenyl und Pyridyl,
worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy,
- R⁴: für Wasserstoff steht,
- R⁵: für eine Gruppe der Formel 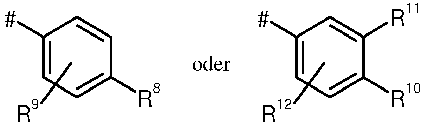 oder 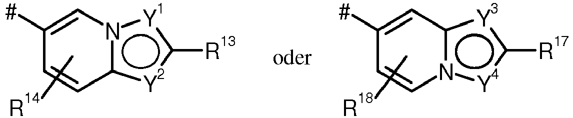 oder 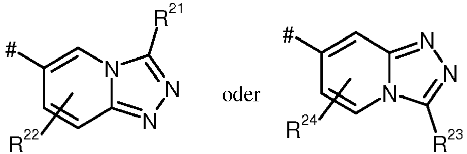 oder 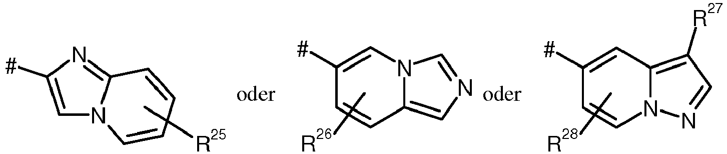 oder 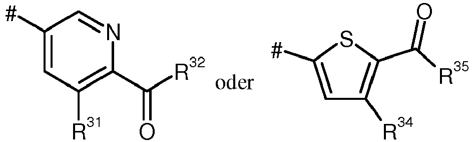
steht,
wobei # die Anknüpfstelle an das Stickstoffatom ist,
- R⁸: für Hydroxycarbonyl, Aminocarbonyl oder 5-gliedriges Heterocyclyl steht,
wobei Heterocyclyl substituiert sein kann mit 1 bis 2 Substituenten unabhängig voneinander ausgewählt aus der Gruppe bestehend aus Oxo, Hydroxy, Thioxo, Sulfanyl, Methyl, Difluormethyl, Trifluormethyl, 2-Hydroxycarbonyl-1,1,2,2-tetrafluorethyl und 2-Methoxycarbonyl-1,1,2,2-tetrafluorethyl,
worin Methyl substituiert sein kann mit einem Substituenten Methoxy,
- R⁹: für Wasserstoff, Chlor, Fluor oder Methyl steht,
- R¹⁰ und R¹¹: zusammen mit den Kohlenstoffatomen an die sie gebunden sind einen 5-gliedrigen Heterocyclus bilden,
wobei der Heterocyclus substituiert sein kann mit 1 bis 2 Substituenten unabhängig voneinander ausgewählt aus der Gruppe bestehend aus Oxo, Chlor, Hydroxy, Hydroxycarbonyl, Methyl, Difluormethyl, Trifluormethyl, 1,1,2,2,2-Pentafluorethyl, 2-Hydroxycarbonyl-1,1,2,2-tetrafluorethyl und 2-Methoxy-carbonyl-1,1,2,2-tetrafluorethyl,
- R¹²: für Wasserstoff, Chlor, Fluor, Methyl oder Methoxy steht,
- Y¹: für ein Stickstoffatom oder C-R¹⁵ steht,
worin
R¹⁵ für Wasserstoff, Chlor, Hydroxy, Methoxy oder C₁-C₃-Alkoxycarbonyl steht,
- Y²: für ein Stickstoffatom oder C-R¹⁶ steht,
worin
R¹⁶ für Wasserstoff, Chlor, Hydroxy oder Methoxy steht,
- R¹³: für Wasserstoff, Hydroxycarbonyl, Hydroxycarbonylmethyl oder Phenyl steht,
worin Phenyl substituiert sein kann mit 1 bis 2 Substituenten Fluor,
- R¹⁴: für Wasserstoff, Chlor, Fluor oder Methyl steht,
- Y³: für ein Stickstoffatom oder C-R¹⁹ steht,
worin
R¹⁹ für Wasserstoff, Chlor, Hydroxy oder Methoxy steht,
- Y⁴: für ein Stickstoffatom oder C-R²⁰ steht,
worin
R²⁰ für Wasserstoff, Chlor, Hydroxy oder Methoxy steht,
- R¹⁷: für Wasserstoff, Hydroxycarbonyl, Hydroxycarbonylmethyl, C₁-C₃-Alkoxycarbonyl oder Aminocarbonyl steht,
- R¹⁸: für Wasserstoff, Chlor, Fluor oder Methyl steht,
- R²¹: für Wasserstoff, Chlor, Hydroxy, C₁-C₄-Alkyl, Methoxy, C₁-C₃-Alkylaminomethyl oder Morpholinylmethyl steht,
- R²²: für Wasserstoff, Chlor, Fluor oder Methyl steht,
- R²³: für Wasserstoff, Chlor, Hydroxy oder Methoxy steht,
- R²⁴: für Wasserstoff, Chlor, Fluor oder Methyl steht,
- R²⁵: für Wasserstoff, Hydroxycarbonyl oder Hydroxycarbonylmethyl steht,
- R²⁶: für Wasserstoff, Chlor, Fluor oder Methyl steht,
- R²⁷: für Hydroxycarbonyl, Aminocarbonyl, C₁-C₃-Alkoxycarbonyl oder C₁-C₃-Alkylaminocarbonyl steht,
wobei Alkylaminocarbonyl substituiert sein kann mit einem Substituenten ausgewählt aus der Gruppe bestehend aus Hydroxy, Trifluormethyl, Methoxy und Trifluormethoxy,
- R²⁸: für Wasserstoff, Chlor, Fluor oder Methyl steht,
- R³¹: für Wasserstoff oder Fluor steht,
- R³²: für Hydroxy oder -NHR³³ steht,
worin
R³³ für Wasserstoff, Methyl oder Ethyl steht,
- R³⁴: für Wasserstoff oder Fluor steht,
R³⁵ für Hydroxy oder -NHR³⁶ steht,
worin
R³⁶ für Wasserstoff, Methyl oder Ethyl steht,
- R⁶: für Brom, Chlor, Fluor, Methyl, Difluormethyl, Trifluormethyl, Methoxy, Difluormethoxy oder Trifluormethoxy steht,
- R⁷: für Wasserstoff, Chlor oder Fluor steht,
und ihre Salze, ihre Solvate und die Solvate ihrer Salze.

Bevorzugt sind auch Verbindungen der Formel (I), in welcher
- A: für eine Bindung oder -CH₂- steht,
- R¹: für Wasserstoff oder Methyl steht,
- R²: für Wasserstoff oder Methyl steht,
oder
- R¹ und R²: bilden zusammen mit dem Kohlenstoffatom, an das sie gebunden sind, einen Cyclopropyl-Ring,
- R³: für Wasserstoff, Methyl, Ethyl, n-Propyl, 2-Methyl-prop-1-yl, n-Butyl oder Ethoxy steht,
wobei Methyl substituiert sein kann mit einem Substituenten ausgewählt aus der Gruppe bestehend aus Difluormethyl, Trifluormethyl, Cyclopropyl, Cyclobutyl, Cyclohexyl, Oxetanyl, Tetrahydrofuranyl, Tetrahydro-2H-pyranyl und 1,4-Dioxanyl,
worin Cyclopropyl, Cyclobutyl, Cyclohexyl und Oxetanyl substituiert sein können mit 1 bis 2 Substituenten unabhängig voneinander ausgewählt aus der Gruppe bestehend aus Fluor, Hydroxy, Methyl, Ethyl und Methoxy,
und
wobei Ethyl, n-Propyl und n-Butyl substituiert sein können mit einem Substituenten ausgewählt aus der Gruppe bestehend aus Fluor, Methoxy und Trifluormethoxy,
- R⁴: für Wasserstoff steht,
- R⁵: für eine Gruppe der Formel 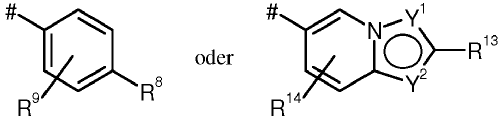 oder 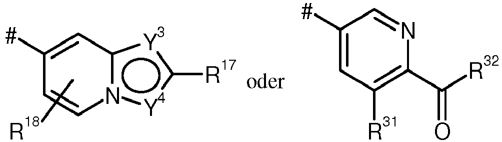 oder 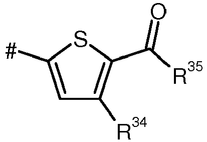 steht,
wobei # die Anknüpfstelle an das Stickstoffatom ist,
R⁸ für Hydroxycarbonyl, Aminocarbonyl, Oxazolyl, Oxadiazolyl, Thiadiazolyl, Pyrazolyl, Imidazolyl, Triazolyl, Tetrazolyl oder Dihydrooxazolyl steht,
wobei Oxazolyl, Oxadiazolyl, Thiadiazolyl, Pyrazolyl, Imidazolyl, Triazolyl und Dihydrooxazolyl substituiert sein können mit 1 bis 2 Substituenten unabhängig voneinander ausgewählt aus der Gruppe bestehend aus Oxo, Hydroxy, Thioxo, Sulfanyl, Methyl, Trifluormethyl und 2-Hydroxycarbonyl-1,1,2,2-tetrafluorethyl,
worin Methyl substituiert sein kann mit einem Substituenten Methoxy,
R⁹ für Wasserstoff, Chlor, Fluor oder Methyl steht,
Y¹ für ein Stickstoffatom oder C-R¹⁵ steht,
worin
R¹⁵ für Wasserstoff, Chlor, Hydroxy oder Methoxy steht,
Y² für ein Stickstoffatom oder C-R¹⁶ steht,
worin
R¹⁶ für Wasserstoff, Chlor, Hydroxy oder Methoxy steht,
R¹³ für Wasserstoff oder Hydroxycarbonyl steht,
R¹⁴ für Wasserstoff oder Fluor steht,
Y³ für ein Stickstoffatom oder C-R¹⁹ steht,
worin
R¹⁹ für Wasserstoff, Chlor, Hydroxy oder Methoxy steht,
Y⁴ für ein Stickstoffatom oder C-R²⁰ steht,
worin
R²⁰ für Wasserstoff, Chlor, Hydroxy oder Methoxy steht,
R¹⁷ für Wasserstoff oder Hydroxycarbonyl steht,
R¹⁸ für Wasserstoff oder Fluor steht,
R³¹ für Wasserstoff steht,
R³² für Hydroxy oder -NHR³³ steht,
worin
R³³ für Wasserstoff steht,
R³⁴ für Wasserstoff steht,
R³⁵ für Hydroxy steht, oder
- R⁵: für 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl, 1H-Indazol-5-yl oder 2H-Indazol-5-yl steht,
wobei der 5-gliedrige Heterocyclus in 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl, 1H-Indazol-5-yl und 2H-Indazol-5-yl substituiert sein kann mit 1 bis 2 Substituenten unabhängig voneinander ausgewählt aus der Gruppe bestehend aus Oxo, Chlor, Hydroxycarbonyl, Methyl und Trifluormethyl,
und
wobei der Benzylring in 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl und 1H-Indazol-5-yl substituiert sein kann mit einem Substituenten ausgewählt aus der Gruppe bestehend aus Fluor und Methoxy,
- R⁶: für Chlor steht,
- R⁷: für Wasserstoff steht,
und ihre Salze, ihre Solvate und die Solvate ihrer Salze.

Bevorzugt sind auch Verbindungen der Formel (I), in welcher
- A: für eine Bindung oder -CH₂- steht,
- R¹: für Wasserstoff steht,
- R²: für Wasserstoff steht,
oder
- R¹ und R²: bilden zusammen mit dem Kohlenstoffatom, an das sie gebunden sind, einen Cyclopropyl-Ring,
- R³: für Wasserstoff, Ethyl oder 2-Methyl-prop-1-yl steht, wobei Ethyl substituiert sein kann mit einem Substituenten Methoxy,
- R⁴: für Wasserstoff steht,
- R⁵: für eine Gruppe der Formel 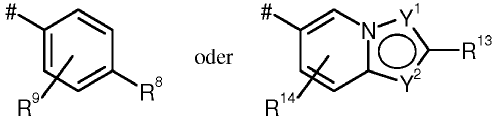 oder 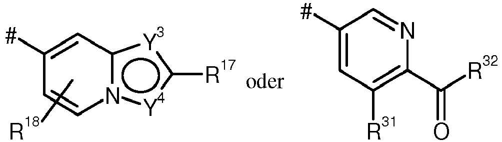 oder 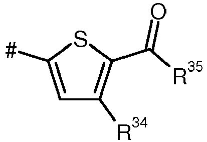 steht,
wobei # die Anknüpfstelle an das Stickstoffatom ist,
R⁸ für Hydroxycarbonyl, Aminocarbonyl oder Oxadiazolyl steht,
wobei Oxadiazolyl substituiert sein kann mit einem Substituenten Oxo,
R⁹ für Wasserstoff oder Fluor steht,
Y¹ für C-R¹⁵ steht,
worin
R¹⁵ für Wasserstoff steht,
Y² für ein Stickstoffatom steht,
R¹³ für Wasserstoff steht,
R¹⁴ für Wasserstoff steht,
Y³ für C-R¹⁹ steht,
worin
R¹⁹ für Wasserstoff steht,
Y⁴ für ein Stickstoffatom steht,
R¹⁷ für Wasserstoff steht,
R¹⁸ für Wasserstoff steht,
R³¹ für Wasserstoff steht,
R³² für Hydroxy oder -NHR³³ steht,
worin
R³³ für Wasserstoff steht,
R³⁴ für Wasserstoff steht,
R³⁵ für Hydroxy steht, oder
- R⁵: für 2,3-Dihydro-1H-indazol-6-yl, 2,3-Dihydro-1H-benzimidazol-5-yl, 1H-Indazol-5-yl oder 2H-Indazol-5-yl steht,
wobei der 5-gliedrige Heterocyclus in 2,3-Dihydro-1H-indazol-6-yl, 2,3-Dihydro-1H-benzimidazol-5-yl, 1H-Indazol-5-yl und 2H-Indazol-5-yl substituiert sein kann mit 1 bis 2 Substituenten unabhängig voneinander ausgewählt aus der Gruppe bestehend aus Oxo und Methyl,
- R⁶: für Chlor steht,
- R⁷: für Wasserstoff steht,
und ihre Salze, ihre Solvate und die Solvate ihrer Salze.

Bevorzugt sind auch Verbindungen der Formel (I), in welcher R¹ und R² für Wasserstoff stehen.

Bevorzugt sind auch Verbindungen der Formel (I), in welcher R¹ und R² zusammen mit dem Kohlenstoffatom, an das sie gebunden sind, einen Cyclopropyl-Ring bilden.

Bevorzugt sind auch Verbindungen der Formel (I), in welcher
- R³: für Wasserstoff, Ethyl oder 2-Methyl-prop-1-yl steht,
wobei Ethyl substituiert sein kann mit einem Substituenten Methoxy.

Bevorzugt sind auch Verbindungen der Formel (I), in welcher
- R⁵: für eine Gruppe der Formel 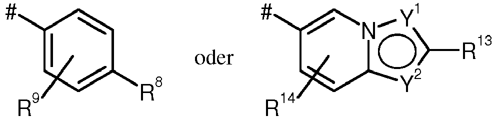 oder 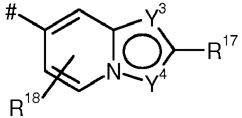 steht,
wobei # die Anknüpfstelle an das Stickstoffatom ist,
R⁸ für Hydroxycarbonyl, Aminocarbonyl oder Oxadiazolyl steht,
wobei Oxadiazolyl substituiert sein kann mit einem Substituenten Oxo,
R⁹ für Wasserstoff oder Fluor steht,
Y¹ für C-R¹⁵ steht,
worin
R¹⁵ für Wasserstoff steht,
Y² für ein Stickstoffatom steht,
R¹³ für Wasserstoff steht,
R¹⁴ für Wasserstoff steht,
Y³ für C-R¹⁹ steht,
worin
R¹⁹ für Wasserstoff steht,
Y⁴ für ein Stickstoffatom steht,
R¹⁷ für Wasserstoff steht,
R¹⁸ für Wasserstoff steht.

Bevorzugt sind auch Verbindungen der Formel (I), in welcher
- R⁵: für eine Gruppe der Formel 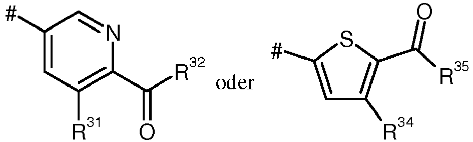 steht,
wobei # die Anknüpfstelle an das Stickstoffatom ist,
R³¹ für Wasserstoff steht,
R³² für Hydroxy oder -NHR³³ steht,
worin
R³³ für Wasserstoff steht,
- R³⁴: für Wasserstoff steht,
- R³⁵: für Hydroxy steht.

Bevorzugt sind auch Verbindungen der Formel (I), in welcher
- R⁵: für 2,3-Dihydro-1H-indazol-6-yl, 2,3-Dihydro-1H-benzimidazol-5-yl, 1H-Indazol-5-yl oder 2H-Indazol-5-yl steht,
wobei der 5-gliedrige Heterocyclus in 2,3-Dihydro-1H-indazol-6-yl, 2,3-Dihydro-1H-benzimidazol-5-yl, 1H-Indazol-5-yl und 2H-Indazol-5-yl substituiert sein kann mit 1 bis 2 Substituenten unabhängig voneinander ausgewählt aus der Gruppe bestehend aus Oxo und Methyl.

Bevorzugt sind auch Verbindungen der Formel (I), in welcher R⁶ für Chlor steht.

Bevorzugt sind auch Verbindungen der Formel (I), in welcher R⁷ für Wasserstoff steht.

Bevorzugt sind auch Verbindungen, welche die Formel (Ia) aufweisen 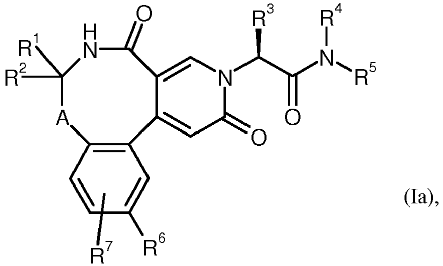 worin A, R¹, R², R³, R⁴, R⁵, R⁶ und R⁷ wie oben definiert sind.

Gegenstand der Erfindung ist weiterhin ein Verfahren zur Herstellung der Verbindungen der Formel (I), oder ihrer Salze, ihrer Solvate oder der Solvate ihrer Salze, wobei
[A] die Verbindungen der Formel 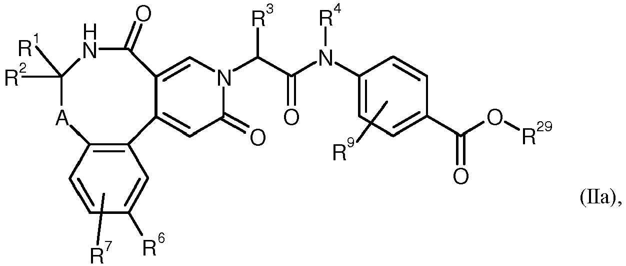 in welcher
   A, R¹, R², R³, R⁴, R⁶, R⁷ und R⁹ die oben angegebene Bedeutung haben, und
   R²⁹ für tert-Butyl steht,
   mit einer Säure zu Verbindungen der Formel 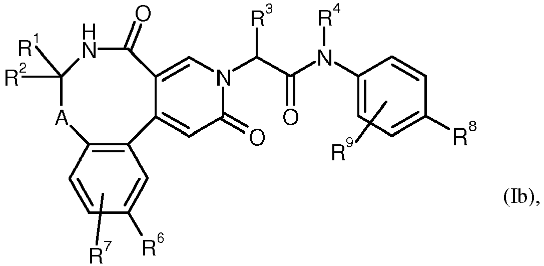 in welcher
      A, R¹, R², R³, R⁴, R⁶, R⁷ und R⁹ die oben angegebene Bedeutung haben, und
      R⁸ für Hydroxycarbonyl steht,
      umgesetzt werden,
      oder
[B] die Verbindungen der Formel 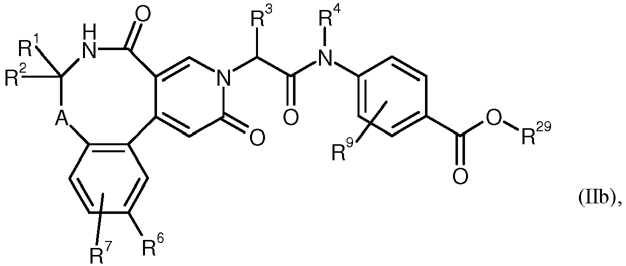 in welcher
   A, R¹, R², R³, R⁴, R⁶, R⁷ und R⁹ die oben angegebene Bedeutung haben, und
   R²⁹ für Methyl oder Ethyl steht,
   mit einer Base zu Verbindungen der Formel 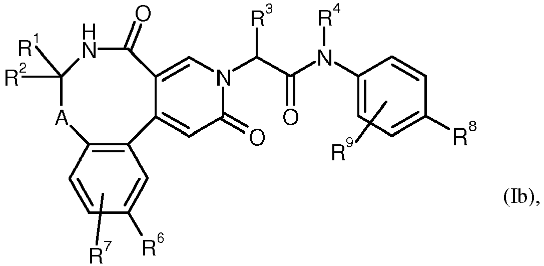 in welcher
      A, R¹, R², R³, R⁴, R⁶, R⁷ und R⁹ die oben angegebene Bedeutung haben, und
      R⁸ für Hydroxycarbonyl steht,
      umgesetzt werden,
      oder
[C] die Verbindungen der Formel 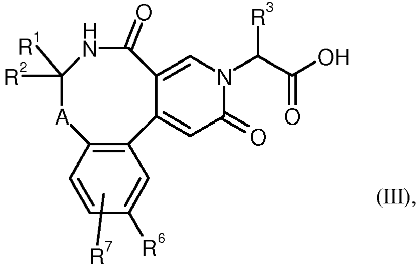 in welcher
   A, R¹, R², R³, R⁶ und R⁷ die oben angegebene Bedeutung haben,
   mit Verbindungen der Formel 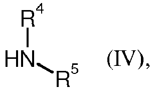 in welcher
   R⁴ und R⁵ die oben angegebene Bedeutung haben,
   in Gegenwart eines Dehydratisierungsreagenzes zu Verbindungen der Formel (I) umgesetzt werden.

Die Verbindungen der Formel (Ib) sind eine Teilmenge der Verbindungen der Formel (I).

Die Verbindungen der Formeln (IIa) und (IIb) bilden zusammen die Menge der Verbindungen der Formel (II).

Die Umsetzung nach Verfahren [A] erfolgt im Allgemeinen in inerten Lösungsmitteln, bevorzugt in einem Temperaturbereich von Raumtemperatur bis 60°C bei Normaldruck.

Inerte Lösungsmittel sind beispielsweise Halogenkohlenwasserstoffe wie Dichlormethan, Trichlormethan, Tetrachlormethan oder 1,2-Dichlorethan, oder Ether wie Tetrahydrofuran oder Dioxan, bevorzugt ist Dichlormethan.

Säuren sind beispielsweise Trifluoressigsäure oder Chlorwasserstoff in Dioxan, bevorzugt ist Trifluoressigsäure.

Die Umsetzung nach Verfahren [B] erfolgt im Allgemeinen in inerten Lösungsmitteln, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist ein Gemisch aus Tetrahydrofuran und Wasser, ein Gemisch aus Methanol und Wasser oder ein Gemisch aus Ethanol und Wasser.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Alkoholate wie Kalium- oder Natrium-tert-butylat, bevorzugt ist Lithiumhydroxid oder Cäsiumcarbonat.

Die Umsetzung nach Verfahren [C] erfolgt im Allgemeinen in inerten Lösungsmitteln, gegebenenfalls in Gegenwart einer Base, bevorzugt in einem Temperaturbereich von 0°C bis Raumtemperatur bei Normaldruck.

Als Dehydratisierungsreagenzien eignen sich hierbei beispielsweise Carbodiimide wie z.B. *N,N'-*Diethyl-, *N*,*N*'-Dipropyl-, *N*,*N*'-Diisopropyl-, *N*,*N*'-Dicyclohexylcarbodiimid, *N*-(3-Dimethylaminoisopropyl)-*N*'-ethylcarbodiimid-Hydrochlorid (EDC) (gegebenenfalls in Gegenwart von Pentafluorphenol (PFP)), *N*-Cyclohexylcarbodiimid-*N*'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N*,*N*,*N*',*N*'-tetra-methyluronium-hexafluorophosphat (HBTU), 2-(2-Oxo-1-(2H)-pyridyl)-1,1,3,3-tetramethyluroniumtetrafluoroborat (TPTU), (Benzotriazol-1-yloxy)bisdimethylaminomethyliumfluoroborat (TBTU) oder *O*-(7-Azabenzotriazol-1-yl)-*N*,*N*,*N'*,*N'*-tetramethyl-uroniumhexafluorophosphat (HATU), oder 1-Hydroxybenztriazol (HOBt), oder Benzotriazol-1-yloxy-tris(dimethylamino)-phosphoniumhexafluorophosphat (BOP), oder Ethyl-cyan(hydroxyimino)acetat (Oxyma), oder (1-Cyano-2-ethoxy-2-oxoethylidenaminooxy)dimethylaminomorpholino-carbenium hexafluorophosphat (COMU), oder N-[(Dimethylamino)(3*H-*[1,2,3]triazolo[4,5-b]pyridin-3-yloxy)methyliden]-N-methylmethanaminium-hexafluorophosphat, oder 2,4,6-Tripropyl-1,3,5,2,4,6-trioxatriphosphinan-2,4,6-trioxid (T3P), oder Mischungen aus diesen, mit Basen. Vorzugsweise wird die Kondensation mit HATU oder mit T3P durchgeführt.

Basen sind beispielsweise Alkalicarbonate, wie z.B. Natrium- oder Kaliumcarbonat, oder -hydrogencarbonat, oder organische Basen wie Trialkylamine, z.B. Triethylamin, *N*-Methylmorpholin, *N*-Methylpiperidin, 4-Dimethylaminopyridin oder Diisopropylethylamin, oder Pyridin. Vorzugsweise wird die Kondensation mit Diisopropylethylamin oder Pyridin durchgeführt.

Inerte Lösungsmittel sind beispielsweise Halogenkohlenwasserstoffe wie Dichlormethan oder Trichlormethan, Kohlenwasserstoffe wie Benzol, oder andere Lösungsmittel wie Nitromethan, Dioxan, Dimethylformamid, Dimethylsulfoxid oder Acetonitril. Ebenso ist es möglich, Gemische der Lösemittel einzusetzen. Besonders bevorzugt ist Dimethylformamid.

Die Verbindungen der Formel (IV) sind bekannt, lassen sich nach bekannten Verfahren aus den entsprechenden Ausgangsverbindungen synthetisieren oder können analog den im Beispielteil beschriebenen Verfahren hergestellt werden.

Die Verbindungen der Formel (II) sind bekannt oder können hergestellt werden, indem Verbindungen der Formel 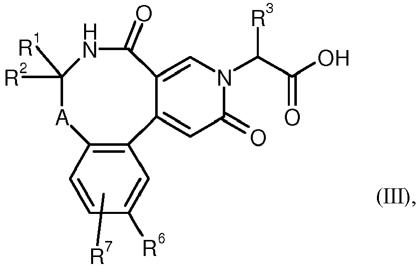 in welcher
A, R¹, R², R³, R⁶ und R⁷ die oben angegebene Bedeutung haben,
mit Verbindungen der Formel 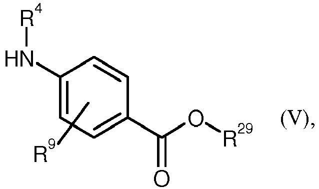 in welcher
R⁴ und R⁹ die oben angegebene Bedeutung haben, und
R²⁹ für Methyl, Ethyl oder tert-Butyl steht,
in Gegenwart eines Dehydratisierungsreagenzes umgesetzt werden.

Die Umsetzung erfolgt wie für Verfahren [C] beschrieben.

Die Verbindungen der Formel (V) sind bekannt, lassen sich nach bekannten Verfahren aus den entsprechenden Ausgangsverbindungen synthetisieren oder können analog den im Beispielteil beschriebenen Verfahren hergestellt werden.

Die Verbindungen der Formel (III) sind bekannt oder können hergestellt werden, indem Verbindungen der Formel 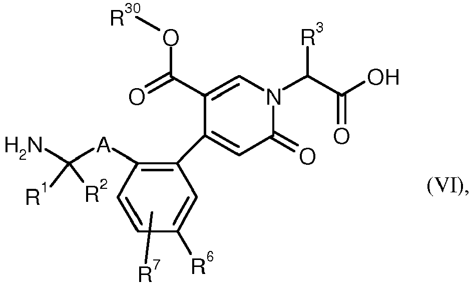 in welcher
A, R¹, R², R³, R⁶ und R⁷ die oben angegebene Bedeutung haben, und
R³⁰ für Methyl oder Ethyl steht,
mit einer Base umgesetzt werden.

Die Umsetzung erfolgt im Allgemeinen in inerten Lösungsmitteln, gegebenenfalls in Gegenwart von Molsieb, bevorzugt in einem Temperaturbereich von Raumtemperatur bis 50°C bei Normaldruck.

Inerte Lösungsmittel sind beispielsweise Alkohole wie Methanol oder Ethanol, Ether wie Dioxan oder Tetrahydrofuran, oder Gemische von Lösungsmitteln, bevorzugt ist Tetrahydrofuran.

Basen sind beispielsweise Natriumhydrid oder Alkoholate wie Kalium- oder Natriumethylat oder Kalium- oder Natriummethylat, bevorzugt ist Natriumethylat.

Die Verbindungen der Formel (VI) sind bekannt oder können hergestellt werden, indem Verbindungen der Formel 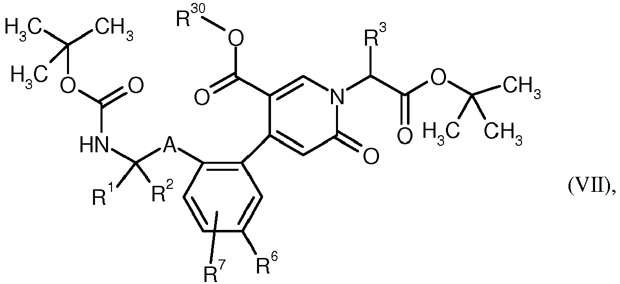 in welcher
A, R¹, R², R³, R⁶ und R⁷ die oben angegebene Bedeutung haben, und
R³⁰ für Methyl oder Ethyl steht,
mit einer Säure umgesetzt werden.

Die Umsetzung erfolgt wie für Verfahren [A] beschrieben.

Die Verbindungen der Formel (VII) sind bekannt oder können hergestellt werden, indem Verbindungen der Formel 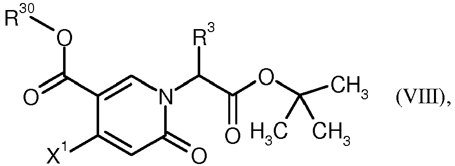 in welcher
R³ die oben angegebene Bedeutung hat,
R³⁰ für Methyl oder Ethyl steht, und
X¹ für Chlor, Brom, Iod oder Trifluormethansulfonyloxy steht,
mit Verbindungen der Formel 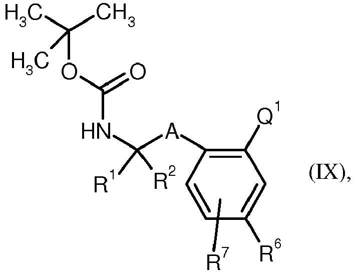 in welcher
   A, R¹, R², R⁶ und R⁷ die oben angegebene Bedeutung haben, und
   Q¹ für -B(OH)₂, einen Boronsäure-Ester, bevorzugt Boronsäurepinakolester, oder -BF₃⁻K⁺ steht,
   unter Suzuki-Kupplungsbedingungen zu Verbindungen der Formel (VII) umgesetzt werden.

Die Umsetzung nach erfolgt im Allgemeinen in inerten Lösungsmitteln, in Gegenwart eines Katalysators, gegebenenfalls in Gegenwart eines Zusatzreagenzes, gegebenenfalls in einer Mikrowelle, bevorzugt in einem Temperaturbereich von Raumtemperatur bis 150°C bei Normaldruck bis 3 bar.

Katalysatoren sind beispielsweise für Suzuki-Reaktionsbedingungen übliche Palladium-Katalysatoren, bevorzugt sind Katalysatoren wie z.B. Dichlorbis(triphenylphosphin)-palladium, Tetrakistriphenylphosphinpalladium(0), Palladium(II)acetat/Triscyclohexylphosphin, Tris(dibenzylidenaceton)dipalladium, Bis-(diphenylphosphanferrocenyl)-palladium-(II)-chlorid, 1,3-Bis(2,6-diisopropylphenyl)imidazol-2-yliden(1,4-napthtochinon)palladiumdimer, Allyl(chlor)-(1,3-dimesityl-1,3-dihydro-2H-imidazol-2-yliden)palladium, Palladium(II)acetat/Dicyclohexyl-(2',4',6'-triisopropyl-biphenyl-2-yl)-phosphin, [1,1-Bis-(diphenylphosphino)-ferrocen]-palladium(II)chlorid-Monodichlormethan-addukt oder XPhos Prekatalysator [(2'-Aminobiphenyl-2-yl)(chlor)palladium-Dicyclohexyl(2',4',6'-triisopropylbiphenyl-2-yl)phosphan (1:1)], bevorzugt ist Tetrakistriphenylphosphin-palladium(0), [1,1-Bis-(diphenylphosphino)-ferrocen]-palladium(II)chlorid-Monodichlormethan-addukt oder XPhos Prekatalysator [(2'-Aminobiphenyl-2-yl)(chlor)palladium-Dicyclohexyl(2',4',6'-triisopropylbiphenyl-2-yl)phosphan (1:1)].

Zusatzreagenzien sind beispielsweise Kaliumacetat, Cäsium-, Kalium- oder Natriumcarbonat, Kalium-tert.-butylat, Cäsiumfluorid oder Kaliumphosphat, wobei diese in wässriger Lösung vorliegen können, bevorzugt sind Zusatzreagenzien wie Kaliumcarbonat oder wässrige Kaliumphosphat-Lösung.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder Carbonsäureamide wie Dimethylformamid oder Dimethylacetamid, Alkylsulfoxide wie Dimethylsulfoxid, oder N-Methylpyrrolidon oder Acetonitril, oder Gemische der Lösungsmittel mit Alkoholen wie Methanol oder Ethanol und/oder Wasser, bevorzugt ist Tetrahydrofuran, Dioxan oder Acetonitril.

Die Verbindungen der Formeln (VIII) und (IX) sind bekannt, lassen sich nach bekannten Verfahren aus den entsprechenden Ausgangsverbindungen synthetisieren oder können analog den im Beispielteil beschriebenen Verfahren hergestellt werden.

Die Herstellung der Ausgangsverbindungen und der Verbindungen der Formel (I) kann durch das folgende Syntheseschema verdeutlicht werden.

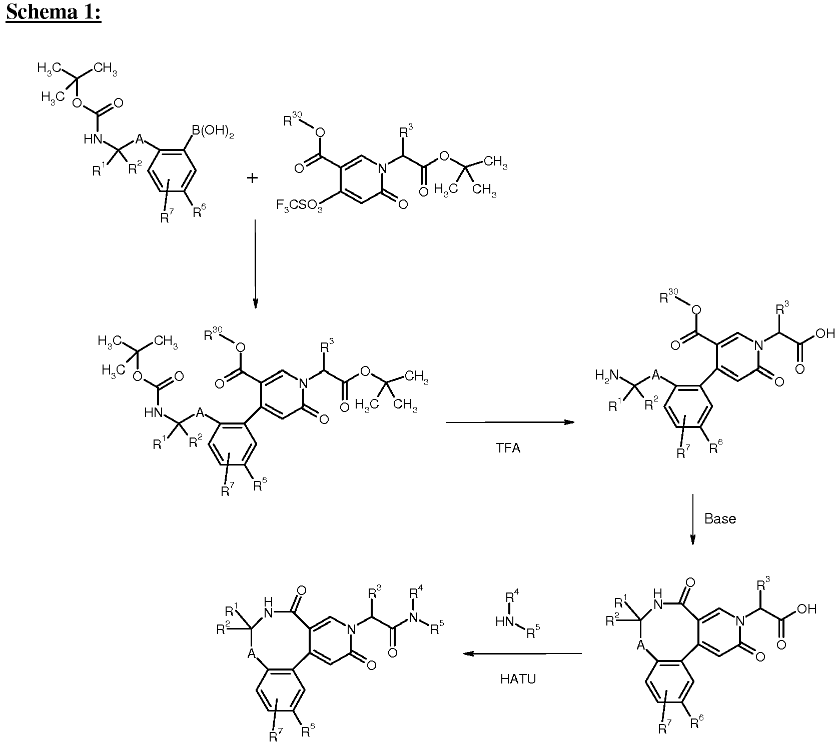

Die erfindungsgemäßen Verbindungen zeigen ein nicht vorhersehbares, wertvolles pharmakologisches Wirkspektrum und ein gutes pharmakokinetisches Verhalten. Es handelt sich dabei um Verbindungen, die die proteolytische Aktivität der Serinprotease Faktor XIa (FXIa) und/oder der Serinprotease Plasmakallikrein (PK) beeinflussen. Die erfindungsgemäßen Verbindungen hemmen die von FXIa und/oder PK katalysierte enzymatische Spaltung von Substraten, die wesentliche Rollen in der Aktivierung der Blutgerinnung, in der Aggregation von Blutplättchen via Reduktion des für die PAR-1-Aktivierung der Plättchen notwendigen Thrombins, und in inflammatorischen Prozessen einnehmen, die insbesondere eine Steigerung der Gefäßpermeabilität miteinschließen.

Sie eigenen sich daher zur Verwendung als Arzneimittel zur Behandlung und/oder Prophylaxe von Krankheiten bei Menschen und Tieren.

Weiterer Gegenstand der vorliegenden Erfindung ist der Einsatz der erfindungsgemäßen Verbindungen zur Behandlung und/oder Prophylaxe von Erkrankungen, insbesondere von Herz-Kreislauf-Erkrankungen, vorzugsweise von thrombotischen beziehungsweise thromboembolischen Erkrankungen und/oder thrombotischen beziehungsweise thromboembolischen Komplikationen, und/oder ophthalmologischen Erkrankungen, insbesondere von diabetischer Retinopathie beziehungsweise des Makulaödems, und/oder entzündlichen Erkrankungen, insbesondere diejenigen, die mit übermäßiger Plasmakallikrein-Aktivität in Zusammenhang stehen, wie z.B. das hereditäre Angioödem (HAE) oder chronisch-entzündliche Erkrankungen, insbesondere des Darms, wie z. B. Morbus Crohn.

Faktor XIa (FXIa) ist ein wichtiges Enzym im Rahmen der Koagulation, das sowohl durch Thrombin als auch Faktor XIIa (FXIIa) aktiviert werden kann und somit in zwei wesentlichen Prozessen der Koagulation involviert ist: Es ist ein zentraler Bestandteil des Übergangs von der Initiation zur Amplifikation und Propagation der Koagulation: Thrombin aktiviert in positiven Rückkopplungsschleifen neben Faktor V und Faktor VIII auch Faktor XI zu Faktor XIa, der Faktor IX zu Faktor IXa umsetzt und über den so generierten Faktor IXa/ Faktor VIIIa-Komplex die Faktor X-Aktivierung und damit wiederum die Thrombinbildung stark stimuliert, was zu starkem Thrombuswachstum führt und den Thrombus stabilisiert.

Darüber hinaus ist Faktor XIa wichtiger Bestandteil der intrinsischen Initiation der Gerinnung: Neben der Stimulation über Gewebefaktor (tissue factor, TF) kann die Aktivierung des Gerinnungssystems auch auf insbesondere negativ geladenen Oberflächen erfolgen, zu denen neben Oberflächenstrukturen körperfremder Zellen (z.B. Bakterien) auch artifizielle Oberflächen wie Gefäßprothesen, Stents und extrakorporale Kreisläufe gehören. Auf der Oberfläche findet zunächst die Aktivierung von Faktor XII (FXII) zu Faktor XIIa (FXIIa) statt, der im Folgenden an Zelloberflächen gebundenen FXI zu FXIa aktiviert. Dieser führt wie zuvor beschrieben zur weiteren Aktivierung der Gerinnungskaskade.

Dagegen bleibt die Thrombingenerierung in der Initiationsphase über TF/Faktor VIIa und Faktor X-Aktivierung und letztlich Thrombinbildung, die physiologische Reaktion auf Gefäßverletzungen, unbeeinflußt. Dies könnte erklären, warum keine Verlängerungen der Blutungszeiten in FXIa-Knock- out-Mäusen, wie auch in Kaninchen und anderen Spezies unter FXIa-Inhibitor-Gabe gefunden wurde. Diese niedrige durch die Substanz hervorgerufene Blutungsneigung ist für die Anwendung am Menschen insbesondere bei Patienten mit erhöhtem Blutungsrisiko von großem Vorteil.

Daneben aktiviert Faktor XIIa im Rahmen der intrinsischen Aktivierung ebenfalls Plasmaprokallikrein zu Plasmakallikrein (PK), das unter anderem zu weiterer Faktor XII-Aktivierung im Rahmen einer Potentierungsschleife führt, was insgesamt eine Verstärkung der Initiation der Gerinnungskaskade an Oberflächen zur Folge hat. Eine PK-hemmende Aktivität einer erfindungsgemäßen Verbindung wird also die Gerinnung via Oberflächenaktivierung reduzieren und somit antikoagulatorisch wirken. Ein Vorteil könnte in der Kombination von Faktor XIa- und PK-inhibitorischer Aktivität liegen, die eine balancierte antithrombotische Wirkung zuläßt.

Die erfindungsgemäßen Verbindungen eignen sich daher zur Behandlung und/oder Prophylaxe von Erkrankungen oder Komplikationen, die durch Gerinnselbildung entstehen können.

Zu den "thrombotischen beziehungsweise thromboembolischen Erkrankungen" im Sinne der vorliegenden Erfindung zählen Erkrankungen, die sowohl im arteriellen als auch im venösen Gefäßbett auftreten und mit den erfindungsgemäßen Verbindungen behandelt werden können, insbesondere Erkrankungen in den Koronararterien des Herzens, wie das akute Koronarsyndrom (ACS), Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie, Stentimplantation oder aortokoronarem Bypass, aber auch thrombotische beziehungsweise thromboembolische Erkrankungen in weiteren Gefäßen, die zu peripheren arteriellen Verschlusskrankheiten, Lungenembolien, venösen Thromboembolien, venösen Thrombosen, insbesondere in tiefen Beinvenen und Nierenvenen, transitorische ischämische Attacken sowie thrombotischer Hirnschlag und thromboembolischer Hirnschlag führen.

Die Stimulation des Gerinnungssystems kann durch verschiedene Ursachen beziehungsweise Begleiterkrankungen erfolgen. Unter anderem kann im Rahmen von chirurgischen Eingriffen, Immobilität, Bettlägerigkeit, Infektionen, Inflammation oder einer Krebserkrankung beziehungsweise Krebstherapie das Gerinnungssystem stark angeregt sein und es zu thrombotischen Komplikationen, insbesondere venösen Thrombosen, kommen. Die erfindungsgemäßen Verbindungen eignen sich daher zur Thromboseprophylaxe im Rahmen von chirurgischen Eingriffen bei Patienten, die eine Krebserkrankung haben. Die erfindungsgemäßen Verbindungen eignen sich daher auch zur Thromboseprophylaxe in Patienten mit aktiviertem Gerinnungssystem, beispielsweise unter den beschriebenen Stimulationssituationen.

Die erfindungsgemäßen Verbindungen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und bei Patienten, die sich einer Kardioversion unterziehen, ferner bei Patienten mit Herzklappen-Erkrankungen oder mit künstlichen Herzklappen.

Darüber hinaus sind die erfindungsgemäßen Verbindungen zur Behandlung und Prävention einer disseminierten intravasalen Gerinnung (DIC) geeignet, die unter anderem im Rahmen einer Sepsis, aber auch infolge von Operationen, Tumorerkrankungen, Verbrennungen oder anderen Verletzungen auftreten und durch Mikrothrombosierungen zu schweren Organschäden führen kann.

Thromboembolische Komplikationen treten ferner auf bei mikroangiopathischen hämolytischen Anämien und durch Kontakt des Blutes mit körperfremden Oberflächen im Rahmen von extrakorporalen Blutkreisläufen, wie zum Beispiel Hämodialyse, ECMO ("extracorporal membrane oxygenation"), LVAD ("left ventricular assist device") und ähnlichen Verfahren, AV-Fisteln, Gefäß- sowie Herzklappenprothesen.

Außerdem kommen die erfindungsgemäßen Verbindungen zur Behandlung und/oder Prophylaxe von Erkrankungen in Betracht, bei denen Mikrogerinnselbildungen beziehungsweise Fibrinablagerungen in Gehirngefäßen auftreten, die zu Demenzerkrankungen, wie beispielsweise vaskulärer Demenz oder Morbus Alzheimer führen können. Hierbei kann das Gerinnsel sowohl über Okklusionen als auch über die Bindung weiterer krankheitsrelevanter Faktoren zur Erkrankung beitragen.

Außerdem kommen die erfindungsgemäßen Verbindungen insbesondere zur Behandlung und/oder Prophylaxe von Erkrankungen in Betracht, bei denen neben der prokoagulanten auch die proinflammatorische Komponente eine wesentliche Rolle spielt. Insbesondere die gegenseitige Verstärkung von Koagulation und Inflammation kann durch die erfindungsgemäßen Verbindungen unterbunden und daher die Wahrscheinlichkeit für eine thrombotische Komplikation entscheidend gesenkt werden. Dabei können sowohl die Faktor XIa-inhibitorische Komponente (über Hemmung der Thrombinproduktion) als auch die PK-inhibitorische Komponente zur antikoagulanten und antiinflammatorischen Wirkung (z.B. via Bradikinin) beitragen. Daher kommen unter anderem die Behandlung und/oder Prophylaxe im Rahmen von atherosklerotischen Gefäßerkrankungen, Entzündungen im Rahmen von rheumatischen Erkrankungen des Bewegungsapparates, entzündlichen Erkrankungen der Lunge, wie beispielsweise Lungenfibrosen, entzündliche Erkrankungen der Niere, wie beispielsweise Glomerulonephritiden, entzündliche Erkrankungen des Darms, wie beispielsweise Morbus Crohn oder Colitis ulcerosa, oder Erkrankungen, die im Rahmen einer diabetischen Grunderkrankung vorliegen können, wie beispielsweise diabetische Retinopathie beziehungsweise Nephropathie in Betracht.

Bei der Progression von chronisch-entzündlichen Darmerkrankungen (CED) haben unter anderem mittels Plasmakallikrein generierten Kinine eine tragende Rolle. Deren pro-inflammatorische Wirkung über Aktivierung von Bradykinin-Rezeptoren induziert und potenziert den Krankheitsverlauf. Studien an Morbus Crohn-Patienten zeigen eine Korrelation zwischen der Kallikrein-Konzentration im Darmepithel und dem Grad der Darmentzündung. Eine Aktivierung des Kallikrein-Kinin-Systems wurde ebenfalls in tierexperimentellen Studien beobachtet. Eine Hemmung der Bradykinin-Synthese durch Kallikrein-Inhibitoren könnte demnach auch zur Prophylaxe und/oder Therapie von chronisch-entzündlichen Darmerkrankungen eingesetzt werden.

Außerdem können die erfindungsgemäßen Verbindungen zur Inhibition des Tumorwachstums und der Metastasenbildung, sowie zur Prophylaxe und/oder Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Außerdem kommen die erfindungsgemäßen Verbindungen auch für die Prophylaxe und/oder Behandlung von pulmonaler Hypertonie in Betracht.

Der Begriff "pulmonale Hypertonie" umfasst im Rahmen der vorliegenden Erfindung die pulmonale arterielle Hypertonie, die pulmonale Hypertonie bei Erkrankungen des linken Herzens, die pulmonale Hypertonie bei Lungenerkrankung und/oder Hypoxie und die Pulmonale Hypertonie aufgrund chronischer Thrombembolien (CTEPH).

Die "pulmonale arterielle Hypertonie" beinhaltet die Idiopathische Pulmonale Arterielle Hypertonie (IPAH, früher auch als primäre pulmonale Hypertonie bezeichnet), die Familiär bedingte Pulmonale Arterielle Hypertonie (FPAH) und die Assoziierte Pulmonal-Arterielle Hypertonie (APAH), die assoziiert ist mit Kollagenosen, kongenitalen systemisch-pulmonalen Shuntvitien, portaler Hypertension, HIV-Infektionen, der Einnahme bestimmter Drogen und Medikamente, mit anderen Erkrankungen (Schilddrüsenerkrankungen, Glykogenspeicherkrankheiten, Morbus Gaucher, hereditäre Teleangiektasie, Hämoglobinopathien, myeloproliferative Erkrankungen, Splenektomie), mit Erkrankungen mit einer signifikanten venösen/kapillären Beteiligung wie der pulmonal-venookklusiven Erkrankung und der pulmonal-kapillären Hämangiomatose, sowie die persistierende pulmonale Hypertonie der Neugeborenen.

Die pulmonale Hypertonie bei Erkrankungen des linken Herzens beinhaltet die Erkrankung des linken Vorhofes oder Ventrikels und Mitral- oder Aortenklappenfehler.

Die pulmonale Hypertonie bei Lungenerkrankung und/oder Hypoxie beinhaltet chronisch obstruktive Lungenerkrankungen, interstitielle Lungenerkrankung, Schlafapnoe-Syndrom, alveolärer Hypoventilation, chronische Höhenkrankheit und anlagebedingte Fehlbildungen.

Die Pulmonale Hypertonie aufgrund chronischer Thrombembolien (CTEPH) beinhaltet den thrombembolischen Verschluss proximaler Lungenarterien, den thrombembolischen Verschluss distaler Lungenarterien und nicht-thrombotische Lungenembolien (Tumor, Parasiten, Fremdkörper).

Weiterer Gegenstand der vorliegenden Erfindung ist die Verwendung der erfindungsgemäßen Verbindungen zur Herstellung von Arzneimitteln zur Behandlung und/oder Prophylaxe von pulmonaler Hypertonie bei Sarkoidose, Histiozytose X und Lymphangiomatosis.

Außerdem kommen die erfindungsgemäßen Substanzen auch zur Behandlung von pulmonalen und hepatischen Fibrosen in Betracht.

Außerdem kommen die erfindungsgemäßen Verbindungen auch für die Behandlung und/oder Prophylaxe einer Disseminierten intravasalen Koagulation im Rahmen einer Infektionserkrankung und/oder von Systemic Inflammatory Syndrome (SIRS), septischer Organdysfunktion, septischem Organversagen und Multiorganversagen, Acute Respiratory Distress Syndrome (ARDS), Acute Lung Injury (ALI), Septischer Schock und/oder des septischen Organversagens in Betracht.

Im Verlauf einer Infektion kann es zur generalisierten Aktivierung des Gerinnungssystems kommen ("Disseminated Intravascular coagulation", oder "Verbrauchskoagulopathie", nachfolgend als "DIC" bezeichnet) mit Mikrothrombosierung in verschiedenen Organen und sekundärer Blutungskomplikationen. Außerdem kann es zur endothelialen Schädigung mit Erhöhung der Gefäßpermeabilität und Austritt von Flüssigkeit und Proteinen in den Extravasalraum kommen. Im weiteren Verlauf kann ein Versagen eines Organs (z.B. Nierenversagen, Leberversagen, Atemversagen, zentralnervöse Defizite und Herz-/Kreislaufversagen) oder zum Multiorganversagen kommen.

Bei der DIC kommt es an der Oberfläche von geschädigten Endothelzellen, Fremdkörperoberflächen oder vernetztem extravaskulärem Gewebe zur massiven Aktivierung des Gerinnungssystems. Als Folge kommt es zur Gerinnung in kleinen Gefäßen verschiedener Organe mit Hypoxie und anschließender Organdysfunktion. Sekundär kommt es zum Verbrauch von Gerinnungsfaktoren (z.B. Faktor X, Prothrombin und Fibrinogen) und Plättchen, wodurch die Gerinnungsfähigkeit des Blutes herabgesetzt wird und schwere Blutungen auftreten können.

Erfindungsgemäße Verbindungen, die Plasmakallikrein alleine oder in Kombination mit Faktor XIa hemmen, kommen zusätzlich zur Behandlung und/oder Prophylaxe von Erkrankungen in Betracht, in deren Verlauf Plasmakallikrein involviert ist. Zusätzlich zur antikoagulanten Aktivität stellt Plasmakallikrein eine wichtige Bradikinin-freisetzende Protease dar, die somit unter anderem zum Anstieg der endothelialen Permeabilität führt. Die Verbindungen können somit zur Behandlung und/oder Prophylaxe von Erkrankungen eingesetzt werden, die mit Ödembildungen einhergehen, wie zum Beispiel ophthalmologische Erkrankungen, insbesondere die diabetische Retinopathie oder das Makulaödem, oder das hereditäre Angioödem.

Zu den "ophthalmologischen Erkrankungen" im Sinne der vorliegenden Erfindung zählen insbesondere Erkrankungen wie diabetische Retinopathie, diabetisches Makulaödem (diabetic macular edema, DME), Makulaödem, Makulaödem assoziiert mit retinalem Venenverschluss, altersbedingte Makula-Degeneration (AMD), choroidale Neovaskularisierung (CNV), choroidale neovaskuläre Membranen (CNVM), zystoides Makulaödem (cystoid macula edema, CME), epiretinale Membranen (ERM) und Makula-Perforationen, Myopie-assoziierte choroidale Neovaskularisierung, angioide beziehungsweise vaskuläre Streifen (angioid streaks, vascular streaks), Retina-Ablösung, atrophische Veränderungen des retinalen Pigmentepithels, hypertrophische Veränderungen des retinalen Pigmentepithels, retinaler Venenverschluss, choroidaler retinaler Venenverschluss, Retinitis pigmentosa, Stargardt'sche Erkrankung, Frühgeborenen-Retinopathie, Glaukom, entzündliche Erkrankungen des Auges wie z.B. Uveitis, Skleritis oder Endophthalmitis, Katarakt, Refraktionsanomalien wie z.B. Myopie, Hyperopie oder Astigmatismus und Keratokonus, Erkrankungen des vorderen Auges wie z.B. korneale Angiogenese als Folge von z.B. Keratitis, Hornhaut-Transplantation oder Keratoplastie, korneale Angiogenese als Folge von Hypoxie (z.B. durch zu langes Tragen von Kontaktlinsen), Pterygium conjunctivae, subkorneales Ödem und intrakorneales Ödem.

Weiterhin kommen die erfindungsgemäßen Verbindungen zur Primärprophylaxe thrombotischer oder thromboembolischer Erkrankungen und/oder inflammatorischer Erkrankungen und/oder Erkrankungen mit erhöhter Gefäßpermeabilität in Patienten in Frage, in denen Genmutationen zu verstärkter Aktivität der Enzyme oder erhöhten Spiegeln der Zymogene führen und diese durch entsprechende Tests/Messungen der Enzymaktivität bzw. Zymogenkonzentrationen festgestellt werden.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind die erfindungsgemäßen Verbindungen zur Verwendung in einem Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind Arzneimittel, enthaltend eine erfindungsgemäße Verbindung und einen oder mehrere weitere Wirkstoffe.

Die erfindungsgemäßen Verbindungen können darüber hinaus auch zur Verhinderung von Koagulation *ex vivo* eingesetzt werden, z.B. zum Schutz von zu transplantierenden Organen vor durch Gerinnselbildung verursachten Organschäden und zum Schutz des Organ-Empfängers vor Thromboemboli aus dem transplantierten Organ, zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor XIa oder Plasmakallikrein enthalten könnten.

Weiterer Gegenstand der vorliegenden Erfindung ist ein Verfahren zur Verhinderung der Blutkoagulation *in vitro*, insbesondere bei Blutkonserven oder biologischen Proben, die Faktor XIa oder Plasmakallikrein oder beide Enzyme enthalten könnten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren wie beispielsweise Lovastatin (Mevacor), Simvastatin (Zocor), Pravastatin (Pravachol), Fluvastatin (Lescol) und Atorvastatin (Lipitor);
- Koronartherapeutika/Vasodilatatoren, insbesondere ACE-(Angiotensin-Converting-Enzyme)-Inhibitoren wie beispielsweise Captopril, Lisinopril, Enalapril, Ramipril, Cilazapril, Benazepril, Fosinopril, Quinapril und Perindopril, oder AII-(Angiotensin II)-Rezeptor-Antagonisten wie beispielsweise Embusartan, Losartan, Valsartan, Irbesartan, Candesartan, Eprosartan und Temisarta, oder β-Adrenozeptor-Antagonisten wie beispielsweise Carvedilol, Alprenolol, Bisoprolol, Acebutolol, Atenolol, Betaxolol, Carteolol, Metoprolol, Nadolol, Penbutolol, Pindolol, Propanolol und Timolol, oder alpha-1-Adrenozeptor-Antagonisten wie beispielsweise Prazosin, Bunazosin, Doxazosin und Terazosin, oder Diuretika wie beispielsweise Hydrochlorothiazid, Furosemid, Bumetanid, Piretanid, Torasemid, Amilorid und Dihydralazin, oder Calciumkanal-Blocker wie beispielsweise Verapamil und Diltiazem, oder Dihydropyridin-Derivate wie beispielsweise Nifedipin (Adalat) und Nitrendipin (Bayotensin), oder Nitropräparate wie beispielsweise Isosorbid-5-mononitrat, Isosorbiddinitrat und Glyceroltrinitrat, oder Substanzen, die eine Erhöhung von cyclischem Guanosinmonophosphat (cGMP) bewirken, wie beispielsweise Stimulatoren der löslichen Guanylatcyclase, wie beispielsweise Riociguat;

- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren) wie beispielsweise Gewebsplasminogen-Aktivator (t-PA, beispielsweise Actilyse®), Streptokinase, Reteplase und Urokinase oder Plasminogen-modulierende Substanzen, die zu verstärkter Plasmin-Bildung führen;
- antikoagulatorisch wirksame Substanzen (Antikoagulantien) wie beispielsweise Heparin (UFH), niedermolekulare Heparine (NMH) wie beispielsweise Tinzaparin, Certoparin, Parnaparin, Nadroparin, Ardeparin, Enoxaparin, Reviparin, Dalteparin, Danaparoid, Semuloparin (AVE 5026), Adomiparin (M118) und EP-42675/ORG42675;
- direkte Thrombin Inhibitoren (DTI) wie beispielsweise Pradaxa (Dabigatran), Atecegatran (AZD-0837), DP-4088, SSR-182289A, Argatroban, Bivalirudin und Tanogitran (BIBT-986 und prodrug BIBT-1011), Hirudin;
- direkte Faktor Xa-Inhibitoren wie beispielsweise Rivaroxaban, Apixaban, Edoxaban (DU-176b), Betrixaban (PRT-54021), R-1663, Darexaban (YM-150), Otamixaban (FXV-673/RPR-130673), Letaxaban (TAK-442), Razaxaban (DPC-906), DX-9065a, LY-517717, Tanogitran (BIBT-986, prodrug: BIBT-1011), Idraparinux und Fondaparinux,
- plättchenaggregationshemmende Substanzen (Plättchenaggregationshemmer, Thrombozytenaggregationshemmer) wie beispielsweise Acetylsalicylsäure (wie beispielsweise Aspirin), P2Y12-Antagonisten wie beispielsweise Ticlopidin (Ticlid), Clopidogrel (Plavix), Prasugrel, Ticagrelor, Cangrelor, Elinogrel, PAR-1-Antagonisten wie beispielsweise Vorapaxar, PAR-4 Antagonisten, EP3-Antagonisten wie beispielsweise DG041;
- Plättchenadhäsionshemmern wie GPVI- und/oder GPIb-Antagonisten wie beispielsweise Revacept oder Caplacizumab;
- Fibrinogen-Rezeptor-Antagonisten (Glycoprotein-IIb/IIIa-Antagonisten) wie beispielsweise Abciximab, Eptifibatide, Tirofiban, Lamifiban, Lefradafiban und Fradafiban;
- rekombinantes humanes aktiviertes Protein C wie beispielsweise Xigris oder rekombinantes Thrombomodulin;
- sowie Antiarrhythmika;
- Inhibitoren der VEGF- und/oder PDGF Signalwege wie beispielsweise Aflibercept, Ranibizumab, Bevacizumab, KH-902, Pegaptanib, Ramucirumab, Squalamin oder Bevasiranib, Apatinib, Axitinib, Brivanib, Cediranib, Dovitinib, Lenvatinib, Linifanib, Motesanib, Pazopanib, Regorafenib, Sorafenib, Sunitinib, Tivozanib, Vandetanib, Vatalanib, Vargatef und E-10030;
- Hemmer der Angiopoietin-Tie Signalwege wie beispielsweise AMG386;
- Inhibitoren der Tie2 Rezeptortyrosinkinase;
- Hemmer der Integrin-Signalwege wie beispielsweise Volociximab, Cilengitid und ALG1001;
- Hemmer der PI3K-Akt-mTor Signalwege wie beispielsweise XL-147, Perifosin, MK2206, Sirolimus, Temsirolimus und Everolimus;
- Corticosteroid wie beispielsweise Anecortave, Betamethason, Dexamethason, Triamcinolon, Fluocinolon und Fluocinolonacetonid;
- Inhibitoren des ALK1-Smad1/5 Signalweges wie beispielsweise ACE041;
- Cyclooxygenaseinhibitoren wie beispielsweise Bromfenac und Nepafenac;
- Hemmer des Kallikrein-Kinin-Systems wie beispielsweise Safotibant und Ecallantid;
- Inhibitoren der Sphingosin-1-phosphat-Signalwege wie beispielsweise Sonepcizumab;
- Inhibitoren des Komplement-C5a Rezeptors wie beispielsweise Eculizumab;
- Inhibitoren des 5HTla Rezeptors wie beispielsweise Tandospiron;
- Hemmer des Ras-Raf-Mek-Erk Signalwegs; Hemmer der MAPK Signalwege; Hemmer der FGF-Signalwege; Hemmer der Endothelzellproliferation; Apoptose-induzierende Wirkstoffe;
- Photodynamische Therapie, bestehend aus einem Wirkstoff und der Einwirkung von Licht, wobei der Wirkstoff beispielsweise Verteporfin ist.

Unter "Kombinationen" im Sinne der Erfindung werden nicht nur Darreichungsformen, die alle Komponenten enthalten (sog. Fixkombinationen) und Kombinationspackungen, die die Komponenten voneinander getrennt enthalten, verstanden, sondern auch gleichzeitig oder zeitlich versetzt applizierte Komponenten, sofern sie zur Prophylaxe und/oder Behandlung derselben Krankheit eingesetzt werden. Ebenso ist es möglich, zwei oder mehr Wirkstoffe miteinander zu kombinieren, es handelt sich dabei also jeweils um zwei- oder mehrfach-Kombinationen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat beziehungsweise Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die extraoculare (topische) Applikation eignen sich nach dem Stand der Technik funktionierende schnell und/oder modifiziert beziehungsweise kontrolliert den Wirkstoff abgebende Applikationsformen, die den Wirkstoff in kristalliner und/oder amorphisierter und/oder gelöster Form enthalten, wie z.B. Augentropfen, Sprays und Lotionen (z.B. Lösungen, Suspensionen, vesikuläre/kolloidale Systeme, Emulsionen, Aerosole), Pulver für Augentropfen, Sprays und Lotionen (z.B. gemahlener Wirkstoff, Mischungen, Lyophilisate, gefällter Wirkstoff), halbfeste Augenzubereitungen (z.B. Hydrogele, in-situ Hydrogele, Cremes und Salben), Augeninserte (feste und halbfeste Zubereitungen, z.B. Bioadhesives, Filme/Wafer, Tabletten, Kontaktlinsen).

Die intraoculare Applikation umfasst z.B. die intravitreale subretinale, subsclerale, intrachoroidale, subconjunctivale, retrobulbare und subtenone Applikation. Für die intraoculare Applikation eignen sich nach dem Stand der Technik funktionierende schnell und/oder modifiziert beziehungsweise kontrolliert den Wirkstoff abgebende Applikationsformen, die den Wirkstoff in kristalliner und/oder amorphisierter und/oder gelöster Form enthalten, wie z.B. Injektionen und Konzentrate für Injektionen (z.B. Lösungen, Suspensionen, vesikuläre/kolloidale Systeme, Emulsionen, Pulver für Injektionen (z.B. gemahlener Wirkstoff, Mischungen, Lyophilisate, gefällter Wirkstoff), Gele für Injektionen (halbfeste Zubereitungen, z.B. Hydrogele, in-situ Hydrogele) und Implantate (feste Zubereitungen, z.B. bioabbaubare und nicht-bioabbaubare Implantate, implantierbare Pumpen).

Bevorzugt ist die orale Applikation beziehungsweise bei ophthalmologischen Erkrankungen die extraokulare und die intraokulare Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich als vorteilhaft erwiesen, bei parenteraler Applikation Mengen von etwa 5 bis 250 mg je 24 Stunden zur Erzielung wirksamer Ergebnisse zu verabreichen. Bei oraler Applikation beträgt die Menge etwa 5 bis 500 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt beziehungsweise Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

### A) Beispiele

### Abkürzungen:

- Boc: *tert.* -Butyloxycarbonyl
- ca.: circa
- d: Tag(e), Dublett (bei NMR)
- DC: Dünnschicht-Chromatographie
- DCM: Dichlormethan
- DCI: direkte chemische Ionisation (bei MS)
- dd: Doppeltes Dublett (bei NMR)
- DIC: *N*,*N'*-Diisopropylcarbodiimid
- DIEA: *N*,*N*-Diisopropylethylamin
- DMAP: 4-Dimethylaminopyridin
- DMF: *N*,*N*-Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- h: Stunde(n)
- HATU: *O*-(7-Azabenzotriazol-1-yl)-*N*,*N*,*N'*,*N'*-tetramethyluronium-Hexafluorphosphat
- HPLC: Hochdruck-, Hochleistungsflüssigchromatographie
- HV: Hochvakuum
- LC-MS: Flüssigchromatographie-gekoppelte Massenspektroskopie
- LDA: Lithiumdiisopropylamid
- m: Multiplett (bei NMR)
- min: Minute(n)
- MS: Massenspektroskopie
- NMR: Kernresonanzspektroskopie
- Oxyma: Cyano(hydroxyimino)essigsäureethylester
- q: Quartett oder Quadruplett (bei NMR)
- quant.: quantitativ
- quin: Quintett (bei NMR)
- RP: reverse phase (bei HPLC)
- RT: Raumtemperatur
- Rₜ: Retentionszeit (bei HPLC)
- s: Singulett (bei NMR)
- sxt: Sextett (bei NMR)
- SFC: superkritische Flüssigkeitschromatographie (mit überkritischem Kohlendioxid als mobile Phase)
- t: Triplett (bei NMR)
- THF: Tetrahydrofuran
- TFA: Trifluoressigsäure
- T3P: 2,4,6-Tripropyl-1,3,5,2,4,6-trioxatriphosphinan-2,4,6-trioxid

### HPLC-. LC/MS- und GC-Methoden:

Methode 1: Instrument: Waters ACQUITY SQD UPLC System; Säule: Waters Acquity UPLC HSS T3 1.8 µ 50 mm x 1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 208-400 nm.

Methode 2: Instrument: Waters ACQUITY SQD UPLC System; Säule: Waters Acquity UPLC HSS T3 1.8 µ 50 mm x 1 mm; Eluent A: 1 l Wasser + 0.25 ml 99%ige Ameisensäure, Eluent B: 1 l Acetonitril + 0.25 ml 99%ige Ameisensäure; Gradient: 0.0 min 95% A → 6.0 min 5% A → 7.5 min 5% A; Ofen: 50°C; Fluss: 0.35 ml/min; UV-Detektion: 210-400 nm.

Methode 3: Instrument: Micromass Quattro Premier mit Waters UPLC Acquity; Säule: Thermo Hypersil GOLD 1.9 µ 50 mm x 1 mm; Eluent A: 1 l Wasser + 0.5 ml 50%ige Ameisensäure, Eluent B: 1 l Acetonitril + 0.5 ml 50%ige Ameisensäure; Gradient: 0.0 min 97% A → 0.5 min 97% A → 3.2 min 5% A → 4.0 min 5% A; Ofen: 50°C; Fluss: 0.3 ml/min; UV-Detektion: 210 nm.

Methode 4: Instrument MS: Waters (Micromass) Quattro Micro; Instrument HPLC: Agilent 1100 Serie; Säule: YMC-Triart C18 3µ 50 mm x 3 mm; Eluent A: 1 l Wasser + 0.01 mol Ammoniumcarbonat, Eluent B: 1 l Acetonitril; Gradient: 0.0 min 100% A → 2.75 min 5% A → 4.5 min 5% A; Ofen: 40°C; Fluss: 1.25 ml/min; UV-Detektion: 210 nm.

Methode 5: Instrument MS: Waters (Micromass) QM; Instrument HPLC: Agilent 1100 Serie; Säule: Agient ZORBAX Extend-C18 3.0 mm x 50 mm 3.5-Micron; Eluent A: 1 l Wasser + 0.01 mol Ammoniumcarbonat, Eluent B: 1 l Acetonitril; Gradient: 0.0 min 98% A → 0.2 min 98% A → 3.0 min 5% A → 4.5 min 5% A; Ofen: 40°C; Fluss: 1.75 ml/min; UV-Detektion: 210 nm.

Methode 6: Instrument MS: Waters (Micromass) ZQ; Instrument HPLC: Agilent 1100 Serie; Säule: Agient ZORBAX Extend-C18 3.0 mm x 50 mm 3.5-Micron; Eluent A: 1 l Wasser + 0.01 mol Ammoniumcarbonat, Eluent B: 1 l Acetonitril; Gradient: 0.0 min 98% A → 0.2 min 98% A → 3.0 min 5% A → 4.5 min 5% A; Ofen: 40°C; Fluss: 1.75 ml/min; UV-Detektion: 210 nm.

Methode 7: Instrument: Thermo DFS, Trace GC Ultra; Säule: Restek RTX-35, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

Methode 8: Instrument: Agilent MS Quad 6150; HPLC: Agilent 1290; Säule: Waters Acquity UPLC HSS T3 1.8 µ 50 mm x 2.1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 0.3 min 90% A → 1.7 min 5% A → 3.0 min 5% A; Ofen: 50°C; Fluss: 1.20 ml/min; UV-Detektion: 205-305 nm.

Methode 9: Instrument: Thermo Scientific DSQII, Thermo Scientific Trace GC Ultra; Säule: Restek RTX-35MS, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

Methode 10: Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril + 0.025% Ameisensäure; Gradient: 0.0 min 98%A → 0.9 min 25%A → 1.0 min 5%A → 1.4 min 5%A → 1.41 min 98%A → 1.5 min 98%A; Ofen: 40°C; Fluss: 0.60 ml/min; UV-Detektion: DAD; 210 nm.

Methode 11: Instrument MS: Waters (Micromass) Quattro Micro; Instrument Waters UPLC Acquity; Säule: Waters BEH C18 1.7 µ 50 mm x 2.1 mm; Eluent A: 1 l Wasser + 0.01 mol Ammoniumformiat, Eluent B: 1 l Acetonitril; Gradient: 0.0 min 95% A → 0.1 min 95% A → 2.0 min 15% A → 2.5 min 15% A → 2.51 min 10% A → 3.0 min 10% A; Ofen: 40°C; Fluss: 0.5 ml/min; UV-Detektion: 210 nm.

Methode 12: Gerätetyp MS: Thermo Scientific FT-MS; Gerätetyp UHPLC+: Thermo Scientific UltiMate 3000; Säule: Waters HSST3, 2.1 mm x 75 mm, C18 1.8 µm; Eluent A: 1 l Wasser + 0.01% Ameisensäure; Eluent B: 1 l Acetonitril + 0.01% Ameisensäure; Gradient: 0.0 min 10% B → 2.5 min 95% B → 3.5 min 95% B; Ofen: 50°C; Fluss: 0.90 ml/min; UV-Detektion: 210 nm/ Optimum Integration Path 210-300 nm.

Methode 13: GC/MS-Messungen: OV-1 Säule, 50 m Säulenlänge, 0.25 mm Innendurchmesser, 0.25 µm Filmdicke; Temperatur zunächst für 1 min bei 50°C gehalten, dann mit einer Rate von 10°C/min auf 280°C erhöht und dort 6 min isotherm gehalten; Trägergas: Helium mit einer Flußrate von 2 ml/min.

Methode 14: HPLC/ESI-Messungen: RP-C18 Säule (150 mm x 4.6 mm) mit 1 cm Vorsäule, Partikelgröße 5 µm, Porengröße 300 Ä; Eluent: Gemische von Acetonitril und Wasser mit 1% Ameisensäure, Mischungsverhältnis über 15 min linear von initial 77% Wasser zu 77% Acetonitril geändert und dann 5 min isokratisch gehalten; Flußrate: 1 ml/min.

Mikrowelle: Als Mikrowellenreaktor wurde ein "single mode" Gerät vom Typ Emrys™ Optimizer verwendet.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen eine ausreichend basische beziehungsweise saure Funktionalität enthalten. Ein solches Salz kann durch verschiedene dem Fachmann bekannte Methoden in die entsprechende freie Base beziehungsweise Säure überführt werden.

Wenn bei den im Folgenden beschriebenen Synthese-Intermediaten und Ausführungsbeispielen der Erfindung eine Verbindung in der Form eines Salzes der korrespondierenden Base beziehungsweise Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃COOH", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

### Ausgangsverbindungen

### Allgemeine Methode 1A: Amid-Kupplung mit HATU/DIEA

Eine Lösung der entsprechenden Carbonsäure (1.0 eq.) in Dimethylformamid (7-15 ml/mmol) wurde unter Argon bei RT mit dem Amin (1.1-1.2 eq.), mit *N*,*N*-Diisopropylethylamin (2.2 eq.) und einer Lösung von HATU (1.2 eq.) in etwas Dimethylformamid versetzt. Das Reaktionsgemisch wurde bei RT gerührt. Nach Zugabe von Wasser/Essigsäureethylester und Phasentrennung wurde die organische Phase mit Wasser und mit gesättigter, wässriger Natriumchlorid-Lösung gewaschen, getrocknet (Natriumsulfat oder Magnesiumsulfat), filtriert und im Vakuum eingeengt. Das Rohprodukt wurde anschließend entweder mittels Normalphasen-Chromatographie (Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 2A: Amid-Kupplung mit T3P/DIEA

Eine Lösung der Carbonsäure und des entsprechenden Amins (1.1-1.5 eq.) in Dimethylformamid (0.15-0.05 mmol) wurde unter Argon bei 0°C oder RT tropfenweise mit *N*,*N*-Diisopropylethylamin (3 eq.) und Propylphosphonsäureanhydrid (T3P, 50%ig in Dimethylformamid oder in Essigsäureethylester, 3 eq.) versetzt. Das Reaktionsgemisch wurde bei RT gerührt und anschließend im Vakuum eingeengt. Nach Zugabe von Wasser/Essigsäureethylester und Phasentrennung wurde die wässrige Phase zweimal mit Essigsäureethylester extrahiert. Die vereinigten, organischen Phasen wurden getrocknet (Natriumsulfat oder Magnesiumsulfat), filtriert und im Vakuum eingeengt. Das Rohprodukt wurde anschließend entweder mittels Flash-Chromatographie (Cyclohexan-Essigsäureethylester-Gemische oder Dichlormethan-Methanol-Gemische) oder präparativer HPLC (Wasser-Acetonitril-Gradient oder Wasser-Methanol-Gradient) aufgereinigt.

### Allgemeine Methode 3A: Amid-Kupplung mit T3P/Pyridin

Eine Lösung der entsprechenden Carbonsäure (1 eq.) und des entsprechenden Amins (1.1-1.5 eq.) in Pyridin (ca. 0.1-0.2M) wurde auf 60 bis 90°C erwärmt und tropfenweise mit T3P (50%ig in Dimethylformamid oder Essigsäureethylester, 1.5-4 eq.) versetzt. Alternativ wurde T3P (50%ig in Dimethylformamid oder Essigsäureethylester, 1.5-4 eq.) tropfenweise bei RT hinzugefügt und danach bei RT gerührt oder auf 50 bis 90°C erhitzt. Nach 1 bis 20 h wurde das Reaktionsgemisch auf RT gekühlt und mit Wasser und Essigsäureethylester versetzt. Die wässrige Phase wurde mit Essigsäureethylester extrahiert. Die vereinigten organischen Phasen wurden mit wässriger Puffer-Lösung (pH=5), mit gesättigter, wässriger Natriumhydrogencarbonat-Lösung und mit gesättigter, wässriger Natriumchlorid-Lösung gewaschen, über Natriumsulfat getrocknet und im Vakuum eingeengt. Das Rohprodukt wurde gegebenenfalls anschließend entweder mittels Normalphasen-Chromatographie (Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Beispiel 1.1A

### 2-Fluor-4-nitrobenzamid

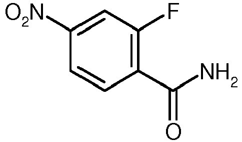

Eine Lösung von 5.00 g (27.0 mmol) 2-Fluor-4-nitrobenzoesäure und 2.29 g (29.7 mmol, 1.1 eq.) Ammoniumacetat in 50 ml Dimethylformamid wurde unter Argon bei 0°C mit 14.1 ml (81.0 mmol, 3 eq.) *N*,*N*-Diisopropylethylamin und 47.3 ml (81.0 mmol, 3 eq.) einer T3P-Lösung (50%ig in Dimethylformamid) versetzt und anschließend 3.5 h bei RT und 3 h bei 60°C gerührt. Nach weiterer Zugabe von 7.1 ml (40.5 mmol, 1.5 eq.) *N*,*N*-Diisopropylethylamin und 23.7 ml (40.5 mmol, 1.5 eq.) einer T3P-Lösung (50%ig in Dimethylformamid) wurde das Reaktionsgemisch über Nacht bei RT gerührt und im Vakuum eingeengt. Der Rückstand wurde mit Wasser und Essigsäureethylester versetzt und die wässrige Phase zweimal mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt. Der Rückstand wurde mittels Flash-Chromatographie (Kieselgel-50, Cyclohexan-Essigsäureethylester-Gemisch) aufgereinigt. Ausbeute: 1.06 g (21% d. Th.)
LC/MS [Methode 3]: Rₜ = 1.21 min; MS (ESIpos): m/z = 185 (M+H)⁺.

### Beispiel 1.1B

### 4-Amino-2-fluorbenzamid

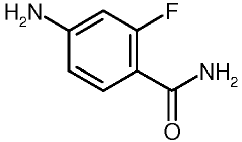

Eine Lösung von 1.11 g (5.88 mmol) 2-Fluor-4-nitrobenzamid in 60 ml Ethanol wurde in Gegenwart von 111 mg Palladium (10%ig auf Aktivkohle) 4 h bei RT und Normaldruck hydriert. Anschließend wurde das Reaktionsgemisch über Celite filtriert und der Rückstand mit Ethanol gewaschen. Die vereinigten Filtrate wurden im Vakuum eingeengt. Der Rückstand wurde ohne weitere Reinigung eingesetzt. Ausbeute: 860 mg (95% d. Th.)
LC/MS [Methode 5]: Rₜ = 0.85 min; MS (ESIpos): m/z = 155 (M+H)⁺.

### Beispiel 1.2A

### Imidazo[1,2-a]pyridin-6-amin

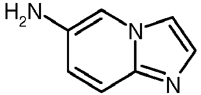

Eine Lösung von 600 mg (3.68 mmol) 6-Nitroimidazo[1,2-*a*]pyridin in 30 ml Ethanol wurde in Gegenwart von 60 mg Palladium (10%ig auf Aktivkohle) über Nacht bei RT und Normaldruck hydriert. Anschließend wurde das Reaktionsgemisch über Celite filtriert und der Rückstand mit Ethanol gewaschen. Die vereinigten Filtrate wurden im Vakuum eingeengt und getrocknet. Das Rohprodukt wurde ohne weitere Reinigung in der nächsten Stufe eingesetzt. Ausbeute: 512 mg (quant.)
LC/MS [Methode 5]: Rₜ = 0.89 min; MS (ESIpos): m/z = 134 (M+H)⁺,
¹H-NMR (400 MHz, DMSO-d₆): δ [ppm] = 7.72-7.62 (m, 2H), 7.33 (d, 1H), 7.30 (d, 1H), 6.80 (dd, 1H), 4.83 (s, 2H).

### Beispiel 1.3A

### 1-Methylhydrazin-1,2-dicarbonsäure-1-benzyl-2-tert.-butylester

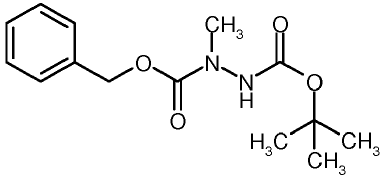

Eine Lösung von 14.2 g (78.8 mmol) 1-Methylhydrazincarbonsäurebenzylester in 100 ml Propan-2-ol wurde bei RT mit 20.6 g (94.6 mmol, 1.2 eq.) Di-*tert*.-butyldicarbonat in 42 ml Dichlormethan versetzt und 24 h bei RT gerührt. Das Reaktionsgemisch wurde mit Dichlormethan und Wasser verdünnt. Nach Phasentrennung wurde die organische Phase getrocknet (Natriumsulfat), filtriert und im Vakuum eingeengt. Das Rohprodukt wurde mittels Flash-Chromatographie (Kieselgel-60, Cyclohexan-Essigsäureethylester-Gemische) aufgereinigt. Ausbeute: 24.8 g (80% Reinheit, 90% d. Th.)
LC/MS [Methode 1]: Rₜ = 1.00 min; MS (ESIneg): m/z = 279 (M-H)⁻.

### Beispiel 1.3B

### 2-Methylhydrazincarbonsäure-tert.-butylester

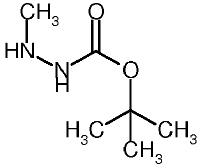

Eine Lösung von 24.8 g (80% Reinheit, 70.8 mmol) 1-Methylhydrazin-1,2-dicarbonsäure-1-benzyl-2-*tert*.-butylester in 500 ml Ethanol wurde in Gegenwart von 1.24 g Palladium (10%ig auf Aktivkohle) bei RT und Normaldruck hydriert. Anschließend wurde das Reaktionsgemisch über Celite filtriert und das Filtrat im Vakuum eingeengt und getrocknet. Ausbeute: 12.3 g (48% Reinheit, 57% d. Th.)

### Beispiel 1.3C

### 2-(2-Fluor-4-nitrobenzoyl)-2-methylhydrazincarbonsäure-tert.-butylester

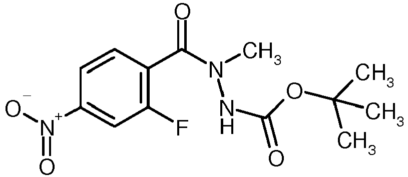

Eine Lösung von 9.1 g (49.3 mmol, 1.2 eq.) 2-Fluor-4-nitrobenzoesäure in 200 ml Dimethylformamid wurde unter Argon bei RT mit 17.1 g (53.4 mmol, 1.3 eq.) (Benzotriazol-1-yloxy)bisdimethyl-aminomethylium-fluorborat und 21.4 ml (123.1 mol, 3.0 eq.) *N,N-*Diisopropylethylamin versetzt und 20 min bei RT gerührt. Eine Lösung von 12.5 g (48% Reinheit, 41 mmol) 2-Methylhydrazincarbonsäure-*tert*.-butylester in 50 ml Dimethylformamid wurde dazugegeben und das Reaktionsgemisch 6 h bei RT gerührt. Dimethylformamid wurde im Vakuum entfernt. Nach Zugabe von Wasser/Essigsäureethylester und Phasentrennung wurde die organische Phase mit 10%iger wässriger Zitronensäure und mit gesättigter, wässriger Natriumchlorid-Lösung gewaschen, getrocknet (Natriumsulfat), filtriert und im Vakuum eingeengt. Das Rohprodukt wurde mittels Flash-Chromatographie (Kieselgel-60, Cyclohexan-Essigsäureethylester-Gemische) aufgereinigt. Ausbeute: 8.35 g (65% d. Th.)
LC/MS [Methode 1]: Rₜ = 0.91 min; MS (ESIneg): m/z = 312 (M-H)⁻,
¹H-NMR (400 MHz, DMSO-d₆): δ [ppm] = 9.78 (s, 1H), 8.20 (d, 1H), 8.11 (d, 1H), 7.59 (t, 1H), 3.13 (s, 3H), 1.24 (s, 9H).

### Beispiel 1.3D

### 2-Fluor-N-methyl-4-nitrobenzohydrazid

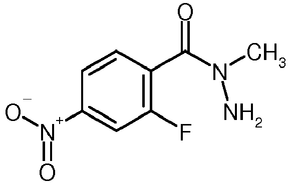

Eine Lösung von 3.6 g (11.5 mmol) 2-(2-Fluor-4-nitrobenzoyl)-2-methylhydrazincarbonsäure-*tert-*butylester in 57 ml Chlorwasserstoff in Dioxan (4M) wurde 3 h bei RT gerührt. Das Reaktionsgemisch wurde im Vakuum eingeengt, der Rückstand in Essigsäureethylester aufgenommen und mit gesättigter, wässriger Natriumhydrogencarbonat-Lösung gewaschen. Die organische Phase wurde getrocknet (Natriumsulfat), filtriert und im Vakuum eingeengt. Ausbeute: 2.0 g (81% d. Th.)
LC/MS [Methode 1]: Rₜ = 0.50 min; MS (ESIpos): m/z = 214 (M+H)⁺,
¹H-NMR (400 MHz, DMSO-d₆): δ [ppm] = 8.09 (m, 2H), 7.61 (dd, 1H), 3.2 (s, 3H).

### Beispiel 1.3E

### 2-Methyl-6-nitro-1,2-dihydro-3H-indazol-3-on

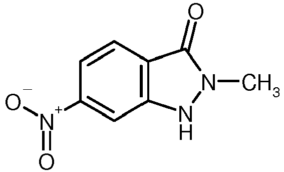

Eine Lösung von 2.4 g (10.9 mmol) 2-Fluor-*N*-methyl-4-nitrobenzohydrazid in 25 ml Dimethylformamid wurde bei RT mit 6.6 ml (37.9 mmol, 3.5 eq.) *N*,*N*-Diisopropylethylamin versetzt und über Nacht bei 80°C gerührt. Das Reaktionsgemisch wurde im Vakuum eingeengt und der Rückstand in Essigsäureethylester aufgenommen. Der ausfallende Feststoff wurde filtriert und im Vakuum getrocknet. Ausbeute: 595 mg (28% d. Th.)
LC/MS [Methode 1]: Rₜ = 0.49 min; MS (ESIpos): m/z = 194 (M+H)⁺,
¹H-NMR (400 MHz, DMSO-d₆): δ [ppm] = 8.14 (s, 1H), 7.85 (d, 1H), 7.78 (dd, 1H), 3.48 (s. 3H).

### Beispiel 1.3F

### 2-Methyl-6-nitro-3-oxo-2,3-dihydro-1H-indazol-1-carbonsäure-tert.-butylester

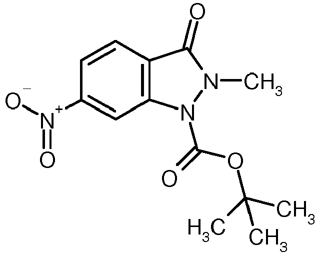

Eine Lösung von 595 mg (3.0 mmol) 2-Methyl-6-nitro-1,2-dihydro-3*H*-indazol-3-on in 25 ml Propan-2-ol wurde bei RT mit einer Lösung von 0.8 g (3.7 mmol, 1.2 eq.) Di-*tert*.-butyldicarbonat in 6 ml Dichlormethan versetzt und 12 h bei RT gerührt. Zur besseren Löslichkeit des Reaktionsgemisches wurden 6 ml Dimethylformamid zugegeben. Das Reaktionsgemisch wurde mit weiteren 4 eq. Di-*tert*.-butyldicarbonat versetzt, 24 h bei RT gerührt und anschließend mit Dichlormethan und Wasser verdünnt. Nach Phasentrennung wurde die organische Phase getrocknet (Natriumsulfat), filtriert und im Vakuum eingeengt. Das Rohprodukt wurde mittels Flash-Chromatographie (Kieselgel-60, Cyclohexan-Essigsäureethylester-Gemische) aufgereinigt. Ausbeute: 720 mg (80% d. Th.)
LC/MS [Methode 1]: Rₜ = 1.04 min; MS (ESIpos): m/z = 194 (M+H-Boc)⁺,
¹H-NMR (400 MHz, DMSO-d₆): δ [ppm] = 8.62 (d, 1H), 8.17 (dd, 1H), 8.05 (d, 1H), 3.58 (s. 3H), 1.63 (s, 9H).

### Beispiel 1.3G

### 6-Amino-2-methyl-3-oxo-2,3-dihydro-1H-indazol-1-carbonsäure-tert.-butylester

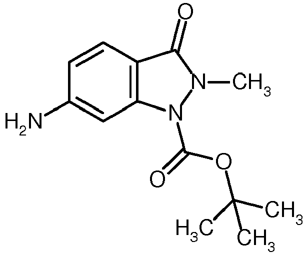

Eine Lösung von 715 mg (2.4 mmol) 2-Methyl-6-nitro-3-oxo-2,3-dihydro-1*H*-indazol-1-carbonsäure-*tert*.-butylester in 30 ml Ethanol wurde in Gegenwart von 52 mg Palladium (10%ig auf Aktivkohle) bei RT und Normaldruck hydriert. Anschließend wurde das Reaktionsgemisch über Celite filtriert und das Filtrat im Vakuum eingeengt und getrocknet. Ausbeute: 668 mg (quant.)
LC/MS [Methode 1]: Rₜ = 0.79 min; MS (ESIpos): m/z = 264 (M+H)⁺,
¹H-NMR (400 MHz, DMSO-d₆): δ [ppm] = 7.36 (d, 1H), 6.94 (d, 1H), 6.53 (dd, 1H), 6.21 (s, 2H), 1.58 (s, 9H).

### Beispiel 2.1A

### 1-(2-Brom-4-chlorphenyl)methanamin

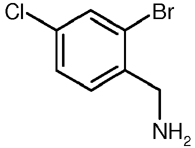

Eine Lösung von 5.00 g (23.1 mmol) 2-Brom-4-chlorbenzonitril in 200 ml THF wurde unter Eisbadkühlung mit 23.1 ml (46.2 mmol) Lithiumaluminiumhydrid (2M in THF) tropfenweise versetzt und für 2 h bei 0 bis 4°C nachgerührt. Anschließend wurden nacheinander 2 ml Wasser, 2 ml wässrige 20%ige Natriumhydroxid-Lösung und 6 ml Wasser vorsichtig zugetropft. Es wurde auf Raumtemperatur erwärmt und für 1 h nachgerührt. Der Niederschlag wurde über Celite abfiltriert und das Filtrat unter reduziertem Druck eingeengt. Ausbeute: 3.52 g (53% Reinheit, 37% d. Th.)
LC/MS [Methode 1]: Rₜ = 0.42 min; MS (ESIpos): m/z = 222 (M+H)⁺.

### Beispiel 2.1B

### tert.-Butyl-(2-brom-4-chlorbenzyl)carbamat

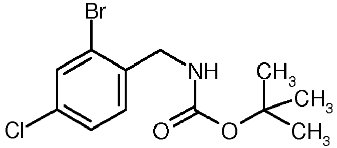

Zu einer Lösung von 3.52 g (53% Reinheit, 8.46 mmol) 1-(2-Brom-4-chlorphenyl)methanamin in 17 ml THF wurden 1.98 g (9.05 mmol) Di-*tert*.-butyldicarbonat gelöst in 17 ml Tetrahydrofuran zugegeben und die resultierende Reaktionslösung über Nacht unter Rückfluss erhitzt. Es wurden weitere 3.69 g (16.9 mmol) Di-*tert*.-butyldicarbonat zugegeben und für 48 h unter Rückfluss erhitzt. Die Reaktion wurde durch Zugabe von gesättigter, wässriger Natriumchlorid-Lösung beendet, und es wurde dreimal mit Methyl-*tert*.-butylether extrahiert. Die vereinigten, organischen Phasen wurden über Magnesiumsulfat getrocknet, filtriert und das Lösungsmittel unter reduziertem Druck entfernt. Der Rückstand wurde mittels Flash-Chromatographie (Kieselgel-50, Cyclohexan-Essigsäureethylester-Gemisch) aufgereinigt. Ausbeute: 1.63 g (60% d. Th.)
¹H-NMR (400 MHz, DMSO-d₆): δ [ppm] = 7.73 (d, 1H), 7.51-7.45 (m, 2H), 7.28 (d, 1H), 4.13 (d, 2H), 1.40 (s, 9H).

### Beispiel 2.1C

### (2-{[(tert.-Butoxycarbonyl)amino]methyl}-5-chlorphenyl)borsäure

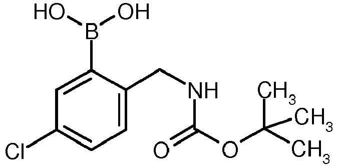

1.63 g (5.08 mmol) *tert*.-Butyl-(2-brom-4-chlorbenzyl)carbamat wurden dreimal mit jeweils 30 ml Toluol kodestilliert und anschließend im Hochvakuum für 1 h getrocknet. Anschließend wurde eine Lösung dieses *tert*.-Butyl-(2-brom-4-chlorbenzyl)carbamats in 50 ml Tetrahydrofuran unter Argon bei -78°C tropfenweise mit 3.18 ml (5.08 mmol, 1.0 eq.) einer Methyllithium-Lösung (1.6M in Diethylether) versetzt, 30 min gerührt, mit 2.03 ml (5.08 mmol, 1.0 eq.) einer *n*-Butyllithium-Lösung (2.5M in *n*-Hexan) versetzt, 30 min gerührt und zuletzt mit 570 µl (5.08 mmol, 1.0 eq.) Trimethylborat versetzt. Nach beendeter Zugabe wurde das Reaktionsgemisch auf RT kommen gelassen, 1 h bei RT nachgerührt und anschließend unter Eisbadkühlung tropfenweise mit 50 ml einer gesättigten, wässrigen Ammoniumchlorid-Lösung versetzt. Es wurde dreimal mit Essigsäureethylester extrahiert. Die vereinigten, organischen Phasen wurden mit gesättigter, wässriger Natriumchlorid-Lösung gewaschen, über Magnesiumsulfat getrocknet, filtriert und das Lösungsmittel unter reduziertem Druck entfernt. Ausbeute: 1.27 g (50% Reinheit, 44% d. Th.)
LC/MS [Methode 1]: Rₜ = 0.90 min; MS (ESIneg): m/z = 284 (M-H)⁻.

### Beispiel 2.2A

### 1-(2-Brom-4-chlorphenyl)cyclopropanamin

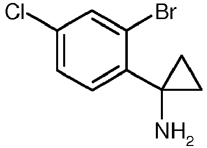

4.00 g (18.5 mmol) 2-Brom-4-chlorbenzonitril wurden dreimal mit 10 ml Toluol koevaporiert und der Rückstand über Nacht im Hochvakuum getrocknet. Der Rückstand wurde in 90 ml Diethylether aufgenommen, mit 5.59 ml (20.3 mmol) Titan-tetra-*iso*-propylat versetzt und auf -65°C abgekühlt. Anschließend wurden 20.3 ml (40.7 mmol) Ethylmagnesiumchlorid-Lösung (2M in Diethylether) hinzugegeben und für 10 min bei -65°C und 1 h bei Raumtemperatur nachgerührt.

Abschließend wurden 4.68 ml (36.9 mmol) Bortrifluorid-Diethylether-Komplex zugetropft und die Reaktionsmischung für 1 h bei Raumtemperatur nachgerührt. Die Reaktionsmischung wurde auf 250 ml 10%ige, wässrige Natriumhydroxid-Lösung und 100 ml Diethylether gegossen und 1 h nachgerührt. Es wurde über Celite filtriert, die Phasen getrennt und die wässrige Phase zweimal mit 100 ml Diethylether extrahiert. Die vereinigten, organischen Phasen wurden über Magnesiumsulfat getrocknet, filtriert und das Lösungsmittel unter reduziertem Druck entfernt. Ausbeute: 2.78 g (64% Reinheit, 39% d. Th.)
LC/MS [Methode 3]: Rₜ = 1.44 min; MS (ESIpos): m/z = 248 (M+H)⁺.

### Beispiel 2.2B

### tert.-Butyl-[1-(2-brom-4-chlorphenyl)cyclopropyl]carbamat

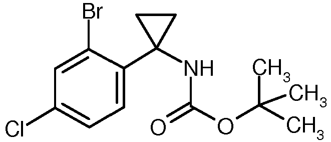

Zu einer Lösung von 2.78 g (64% Reinheit, 7.22 mmol) 1-(2-Brom-4-chlorphenyl)cyclopropanamin in 72 ml Dichlormethan wurden bei Raumtemperatur 2.64 ml (15.2 mmol) *N,N*-Diisopropylethylamin und 2.68 g (12.3 mmol) Di-*tert*.-butyldicarbonat gegeben und über Nacht nachgerührt. Die Reaktionslösung wurde mit 50 ml Wasser verdünnt und die Phasen getrennt. Die wässrige Phase wurde einmal mit 50 ml Dichlormethan extrahiert und die vereinigten, organischen Phasen mit 50 ml gesättigter, wässriger Natriumchlorid-Lösung gewaschen, über Magnesiumsulfat getrocknet, filtriert und das Lösungsmittel unter reduziertem Druck entfernt. Der Rückstand wurde mittels Flash-Chromatographie (Kieselgel-50, Cyclohexan-Essigsäureethylester-Gemisch) aufgereinigt. Ausbeute: 1.27 g (51% d. Th.)
¹H-NMR (400 MHz, DMSO-d₆): δ [ppm] = 7.70-7.37 (m, 3H), 1.31 (s, 9H), 1.14-0.96 (m, 4H).

### Beispiel 2.2C

### (2-{1-[(tert.-Butoxycarbonyl)amino]cyclopropyl}-5-chlorphenyl)borsäure

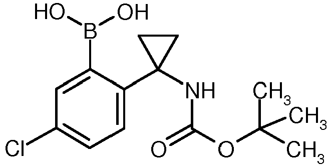

1.27 g (3.66 mmol) *tert*.-Butyl-[1-(2-brom-4-chlorphenyl)cyclopropyl]carbamat wurden dreimal mit jeweils 30 ml Toluol kodestilliert und anschließend im Hochvakuum für 1 h getrocknet. Anschließend wurde eine Lösung dieses *tert*.-Butyl-[1-(2-brom-4-chlorphenyl)cyclopropyl]-carbamats in 40 ml Tetrahydrofuran unter Argon bei -78°C tropfenweise mit 2.29 ml (3.66 mmol, 1.0 eq.) einer Methyllithium-Lösung (1.6M in Diethylether) versetzt, 30 min gerührt, mit 1.47 ml (3.66 mmol, 1.0 eq.) einer *n*-Butyllithium-Lösung (2.5M in *n*-Hexan) versetzt, 30 min gerührt und zuletzt mit 411 µl (3.66 mmol, 1.0 eq.) Trimethylborat versetzt. Nach beendeter Zugabe wurde das Reaktionsgemisch auf RT kommen gelassen, 1 h bei RT nachgerührt und anschließend unter Eisbadkühlung tropfenweise mit 50 ml einer gesättigten, wässrigen Ammoniumchlorid-Lösung versetzt. Es wurde dreimal mit 50 ml Essigsäureethylester extrahiert. Die vereinigten, organischen Phasen wurden mit gesättigter, wässriger Natriumchlorid-Lösung gewaschen, über Magnesiumsulfat getrocknet, filtriert und das Lösungsmittel unter reduziertem Druck entfernt. Ausbeute: 0.99 g (68% Reinheit, 59% d. Th.)
LC/MS [Methode 1]: Rₜ = 0.98 min; MS (ESIneg): m/z = 310 (M-H)⁻.

### Beispiel 2.3A

### 2-Brom-4-chlor-1-[2-nitroethenyl]benzol

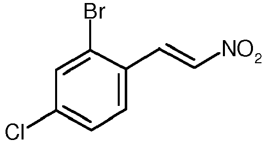

6.83 g (31.1 mmol) 2-Brom-4-chlorbenzaldehyd und 5.52 g (71.6 mmol) Ammoniumacetat wurden in 78 ml Essigsäure angelöst. Anschließend wurden 4.80 ml (88.7 mmol) Nitromethan zugegeben und die Reaktionsmischung für 90 min unter Rückfluss erhitzt. Es wurde auf Raumtemperatur abgekühlt, mit 80 ml Wasser versetzt und mit Dichlormethan extrahiert. Die vereinigten, organischen Phasen wurden mit Wasser und gesättigter, wässriger Natriumchlorid-Lösung gewaschen, über Natriumsulfat getrocknet, filtriert und das Lösungsmittel unter reduziertem Druck entfernt. Ausbeute: 6.86 g (82% d. Th.)
¹H-NMR (400 MHz, DMSO-d₆): δ [ppm] = 8.29 (d, 1H), 8.17 (d, 1H), 8.06 (d, 1H), 7.99 (d, 1H), 7.62 (dd, 1H).

### Beispiel 2.3B

### 2-(2-Brom-4-chlorphenyl)ethanamin

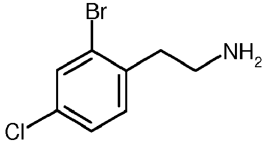

Zu einer Lösung von 38.1 ml (76.2 mmol) Lithiumborhydrid (2M in THF) in 200 ml THF wurden bei Raumtemperatur 19.3 ml (152 mmol) Chlortrimethylsilan zügig zugetropft und anschließend 20 min nachgerührt. Die resultierende Reaktionsmischung wurde entgast, eine Lösung von 5.00 g (19.0 mmol) 2-Brom-4-chlor-1-[2-nitroethenyl]benzol in THF zugetropft und für 2 h unter Rückfluss erhitzt. Es wurde auf Raumtemperatur abgekühlt und die Reaktion durch Zugabe von 50 ml Methanol beendet. Das Lösungsmittel wurde unter reduziertem Druck entfernt, der Rückstand mit 50 ml 20%iger, wässriger Kaliumhydroxid-Lösung versetzt und mit Essigsäureethylester extrahiert. Die vereinigten, organischen Phasen wurden mit gesättigter, wässriger Natriumchlorid-Lösung gewaschen, über Magnesiumsulfat getrocknet, filtriert und das Lösungsmittel unter reduziertem Druck entfernt. Ausbeute: 4.06 g (72% Reinheit, 65% d. Th.)
LC/MS [Methode 1]: Rₜ = 0.53 min; MS (ESIpos): m/z = 236 (M+H)⁺.

### Beispiel 2.3C

### tert.-Butyl-[2-(2-brom-4-chlorphenyl)ethyl]carbamat

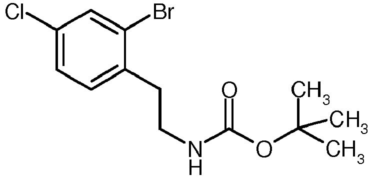

Zu einer Lösung von 4.05 g (72% Reinheit, 12.4 mmol) 2-(2-Brom-4-chlorphenyl)ethanamin in 100 ml THF wurden unter Eisbadkühlung 2.85 g (13.1 mmol) Di-*tert*.-butyldicarbonat und 152 mg (1.24 mmol) 4-Dimethylaminopyridin hinzugegeben. Es wurde spontan auf Raumtemperatur erwärmt und für 1 h nachgerührt. Anschließend wurde das Lösungsmittel unter reduziertem Druck entfernt, der Rückstand in Wasser aufgenommen und mit Essigsäureethylester extrahiert. Die vereinigten, organischen Phasen wurden mit gesättigter, wässriger Natriumchlorid-Lösung gewaschen, über Magnesiumsulfat getrocknet, filtriert und das Lösungsmittel unter reduziertem Druck entfernt. Der Rückstand wurde mittels Flash-Chromatographie (Kieselgel-50, Cyclohexan-Essigsäureethylester-Gradient) gereinigt. Ausbeute: 2.53 g (61% d. Th.)
LC/MS [Methode 1]: Rₜ = 1.23 min; MS (ESIpos): m/z = 334 (M+H)⁺,
¹H-NMR (400 MHz, DMSO-d₆): δ [ppm] = 7.70 (d, 1H), 7.41 (dd, 1H), 7.30 (d, 1H), 6.95-6.88 (m, 1H), 3.15 (dt, 2H), 2.80 (t, 2H), 1.35 (s, 9H).

### Beispiel 2.3D

### tert.-Butyl-{2-[4-chlor-2-(4,4,5,5-tetramethyl-1,3,2-dioxaborolan-2-yl)phenyl]ethyl}carbamat

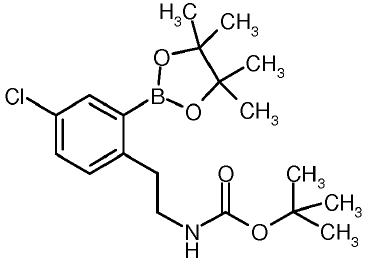

1.04 g (3.11 mmol) *tert*.-Butyl-[2-(2-brom-4-chlorphenyl)ethyl]carbamat wurden in 15.6 ml 1,2-Dimethoxyethan vorgelegt und die resultierende Lösung entgast. Anschließend wurden 868 mg (3.42 mmol) 4,4,4',4',5,5,5',5'-Octamethyl-2,2'-bi-1,3,2-dioxaborolan, 610 mg (6.22 mmol) Kaliumacetat und 254 mg (311 µmol) 1,1'-Bis(diphenylphosphin)ferrocendichlorpalladium(II) hinzugegeben und das Reaktionsgemisch für 8 h bei 80°C gerührt. Die Reaktionsmischung wurde auf Raumtemperatur abgekühlt, über Celite filtriert, der Filterkuchen mit Dichlormethan nachgewaschen und das Lösungsmittel unter reduziertem Druck entfernt. Der Rückstand wurde mittels Flash-Chromatographie (Kieselgel-50, Cyclohexan-Essigsäureethylester-Gemisch) aufgereinigt. Ausbeute: 363 mg (82% Reinheit, 25% d. Th.) und 580 mg (78% Reinheit, 38% d. Th.)
LC/MS [Methode 1]: Rₜ = 1.41 min; MS (ESIneg): m/z = 380 (M-H)⁻.

### Beispiel 2.4A

### (2-Brom-4-chlorphenyl)methanol

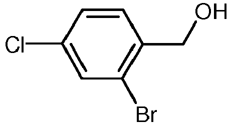

In einem ausgeheizten Kolben wurde eine Lösung von 10.0 g (44.2 mmol) 2-Brom-4-chlorbenzaldehyd in 90 ml Methanol unter Argon und Eisbadkühlung portionsweise mit 836 mg (22.1 mmol) Natriumborhydrid versetzt und 1 h unter Eisbadkühlung gerührt. Nach Zugabe von 200 ml Wasser wurde das Methanol im Vakuum entfernt. Die wässrige Phase wurde dreimal mit jeweils 80 ml Essigsäureethylester extrahiert. Die vereinigten, organischen Phasen wurden getrocknet (Magnesiumsulfat), filtriert und im Vakuum eingeengt. Ausbeute: 7.75 g (79% d. Th.)
GC/MS [Methode 9]: Rₜ = 4.91 min; MS: m/z = 220 (M)⁺,
¹H-NMR (400 MHz, DMSO-d₆): δ [ppm] = 7.70 (d, 1H), 7.54 (d, 1H), 7.48 (dd, 1H), 5.51 (t, 1H), 4.48 (d, 2H).

### Beispiel 2.4B

### 2-Brom-4-chlorbenzyl-4-methylbenzolsulfonat

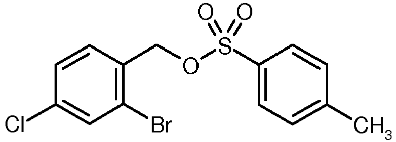

Eine Lösung von 7.75 g (35.0 mmol) (2-Brom-4-chlorphenyl)methanol in 100 ml Tetrahydrofuran wurde unter Argon und Eisbadkühlung mit 9.82 g (175.0 mmol, 5.0 eq.) Kaliumhydroxid (zuvor getrocknet und zerrieben) und anschließend tropfenweise mit einer Lösung von 8.17 g (42.0 mmol, 1.2 eq.) *para*-Toluolsulfonsäurechlorid in 65 ml Tetrahydrofuran versetzt. Das Reaktionsgemisch wurde weitere 30 min unter Eisbadkühlung gerührt, nach Entfernen des Kältebades anschließend auf RT kommen gelassen und über Celite filtriert. Das Filtrat wurde am Rotationsverdampfer im Vakuum (bei <35°C Wasserbadtemperatur) eingeengt. Der Rückstand wurde dreimal mit Hexan (10 ml und zweimal 15 ml) koevaporiert, im Hochvakuum getrocknet und ohne weitere Reinigung eingesetzt (Lagerung im Kühlschrank). Ausbeute: 14.20 g (75% Reinheit, 81% d. Th.)

### Beispiel 2.4C

### (2-Brom-4-chlorphenyl)acetonitril

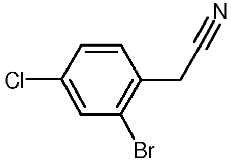

Eine Lösung von 14.20 g (75% Reinheit, 28.3 mmol) 2-Brom-4-chlorbenzyl-4-methylbenzolsulfonat in 142 ml Acetonitril wurde unter Argon bei RT portionsweise mit 6.83 g (104.9 mmol, 3.7 eq.) Kaliumcyanid versetzt und 60 h bei RT gerührt. Das Reaktionsgemisch wurde über Celite filtriert und das Filtrat im Vakuum eingeengt. Der Rückstand wurde mehrmals mit Hexan koevaporiert und anschließend mittels Flash-Chromatographie (120 g Kieselgel, Cyclohexan-Essigsäureethylester-Gemisch) aufgereinigt. Ausbeute: 3.10 g (47% d. Th.)
GC/MS [Methode 9]: Rₜ = 5.29 min; MS: m/z = 230 (M)⁺,
¹H-NMR (400 MHz, DMSO-d₆): δ [ppm] = 7.87 (d, 1H), 7.61-7.53 (m, 2H), 4.09 (s, 2H).

### Beispiel 2.4D

### 1-(2-Brom-4-chlorbenzyl)cyclopropanamin

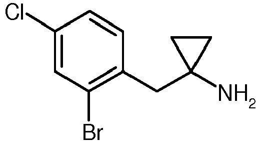

3.49 g (15.1 mmol) (2-Brom-4-chlorphenyl)acetonitril wurden dreimal mit Toluol kodestilliert und anschließend im Hochvakuum über Nacht getrocknet. Dieser Rückstand wurde in 100 ml Diethylether aufgenommen und die Lösung unter Argon bei RT mit 4.4 ml (15.9 mmol, 1.05 eq.) Titan(IV)-*iso*-proylat versetzt und 5 min bei RT gerührt. Anschließend wurde das Reaktionsgemisch tropfenweise mit 10.1 ml (30.3 mmol, 2.0 eq.) einer Ethylmagnesiumbromid-Lösung (3M in Diethylether) versetzt, 30 min bei RT gerührt, zügig mit 3.8 ml (30.3 mmol, 2.0 eq.) Bortrifluorid-Diethylether-Komplex versetzt und weitere 60 min bei RT gerührt. Die Reaktionsmischung wurde auf ein Gemisch aus 250 ml einer 10%igen, wässrigen Natriumhydroxid-Lösung und 100 ml Diethylether gegossen und über Celite filtriert. Nach Phasentrennung wurde die wässrige Phase zweimal mit jeweils 50 ml Diethylether extrahiert. Die vereinigten, organischen Phasen wurden getrocknet (Magnesiumsulfat), filtriert, im Vakuum eingeengt und ohne weitere Reinigung umgesetzt (Lagerung im Kühlschrank). Ausbeute: 3.88 g (45% Reinheit, 44% d. Th.)
LC/MS [Methode 3]: Rₜ = 1.56 min; MS (ESIpos): m/z = 260 (M+H)⁺.

### Beispiel 2.4E

### tert.-Butyl-[1-(2-brom-4-chlorbenzyl)cyclopropyl]carbamat

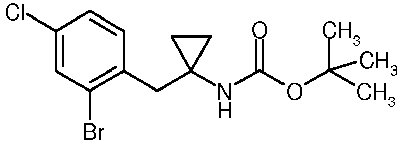

Eine Lösung von 3.88 g (45% Reinheit, 6.7 mmol) 1-(2-Brom-4-chlorbenzyl)cyclopropanamin in 94 ml Dichlormethan wurde mit 2.5 ml (14.1 mmol, 2.1 eq.) *N*,*N*-Diisopropylethylamin und 2.5 g (11.4 mmol, 1.7 eq.) Di-*tert*.-butyldicarbonat versetzt und über Nacht bei RT gerührt. Das Reaktionsgemisch wurde dreimal mit jeweils 100 ml Wasser extrahiert. Die wässrige Phase wurde einmal mit 100 ml Dichlormethan reextrahiert. Die vereinigten, organischen Phasen wurden mit gesättigter, wässriger Natriumchlorid-Lösung gewaschen, getrocknet (Magnesiumsulfat), filtriert und im Vakuum eingeengt. Der Rückstand wurde mittels Flash-Chromatographie (Kieselgel-50, Cyclohexan-Essigsäureethylester-Gemisch) gereinigt. Ausbeute: 1.03 g (43% d. Th.)
LC/MS [Methode 1]: Rₜ = 1.32 min; MS (ESIpos): m/z = 360 (M+H)⁺,
¹H-NMR (400 MHz, DMSO-d₆): δ [ppm] = 7.70 (s, 1H), 7.45-7.38 (m, 2H), 7.09 (s, 1H), 2.94 (s, 2H), 1.34 (s, 9H), 0.80-0.60 (m, 4H).

### Beispiel 2.4F

### [2-({1-[(tert.-Butoxycarbonyl)amino]cyclopropyl}methyl)-5-chlorphenyl]borsäure

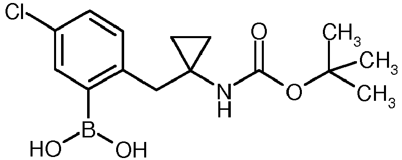

7.21 g (20.0 mmol) *tert*.-Butyl-[1-(2-brom-4-chlorbenzyl)cyclopropyl]carbamat wurden dreimal mit jeweils 60 ml Toluol kodestilliert und im Hochvakuum über Nacht getrocknet. Anschließend wurde eine Lösung dieses *tert*.-Butyl-[1-(2-brom-4-chlorbenzyl)cyclopropyl]-carbamats in 200 ml Tetrahydrofuran unter Argon bei -78°C tropfenweise mit 12.5 ml (20.0 mmol, 1.0 eq.) einer Methyllithium-Lösung (1.6M in Diethylether) versetzt, 30 min gerührt, mit 8.0 ml (20.0 mmol, 1.0 eq.) einer *n*-Butyllithium-Lösung (2.5M in *n*-Hexan) versetzt, 30 min gerührt und zuletzt mit 2.2 ml (20 mmol, 1.0 eq.) Trimethylborat versetzt. Nach beendeter Zugabe wurde das Reaktionsgemisch auf RT kommen gelassen, 1 h bei RT nachgerührt und anschließend unter Eisbadkühlung tropfenweise mit 210 ml einer gesättigten, wässrigen Ammoniumchlorid-Lösung versetzt. Die ausgefallenen Salze wurden filtriert und mit Essigsäureethylester gewaschen. Die vereinigten Filtrate wurden dreimal mit Essigsäureethylester extrahiert. Die vereinigten, organischen Phasen wurden mit gesättigter, wässriger Natriumchlorid-Lösung gewaschen und im Vakuum eingeengt. Der Rückstand wurde im Hochvakuum getrocknet und unter Argon gelagert. Ausbeute: 6.5 g (81% Reinheit, 81% d. Th.)
LC/MS [Methode 1]: Rₜ = 0.91 min; MS (ESIneg): m/z = 324 (M-H)⁻.

### Beispiel 2.5A

### Methyl-2-brom-N-(tert.-butoxycarbonyl)-4-chlorphenylalaninat (Racemat)

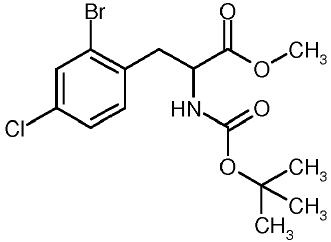

Eine Lösung von 47.8 ml (318 mmol, 2 eq.) *N,N,N',N'*-Tetramethylethan-1,2-diamin und 44.5 ml (318 mmol, 2 eq.) *N*-Isopropylpropan-2-amin (32.0 g, 318 mmol, 2 eq.) in 600 ml Tetrahydrofuran wurde unter Argon bei -78°C tropfenweise mit 127 ml (318 mmol, 2 eq.) einer Lösung von *n*-Butyllithium in Hexan (2.5M) versetzt und 20 min bei -78°C gerührt. Das Reaktionsgemisch wurde dann tropfenweise mit einer Lösung aus 30 g (159 mmol, 1 eq.) Methyl-*N*-(*tert*.-butoxycarbonyl)glycinat in 25 ml Tetrahydrofuran versetzt, wobei die Innentemperatur unter -70°C gehalten wurde, und weitere 60 min bei dieser Temperatur gerührt. Anschließend wurde eine Lösung aus 45 g (158 mmol, 1 eq.) 2-Brom-1-(brommethyl)-4-chlorbenzol in 40 ml Tetrahydrofuran tropfenweise zugegeben, wobei die Innentemperatur unter -69°C gehalten wurde. Das Reaktionsgemisch wurde weitere 2 h bei unter -78°C gerührt, anschließend auf -20°C kommen gelassen und mit 600 ml gesättigter, wässriger Ammoniumchlorid-Lösung gequencht. Nach Zugabe von Essigsäureethylester und Phasentrennung wurde die wässrige Phase mit Essigsäureethylester extrahiert. Die vereinigten, organischen Phasen wurden zweimal mit gesättigter, wässriger Natriumchlorid-Lösung gewaschen, getrocknet (Natriumsulfat), filtriert und im Vakuum bis zu einem Druck von 100 mbar weitestgehend eingeengt. Das gewünschte Produkt kristallisierte aus dem Rückstand bei Stehenlassen über Nacht aus, es wurde mit 80 ml Ethanol verrührt, filtriert und im Vakuum getrocknet. Ausbeute: 38.1 g (61% d. Th.)
GC/MS [Methode 13]: Rₜ = 23.23 min; MS: m/z = 391 (M)⁺,
¹H-NMR (500 MHz, CDCl₃): δ [ppm] = 7.54 (d, 1H), 7.21 (dd, 1H), 7.12 (d, 1H), 5.06 (d, 1H), 4.60 (q, 1H), 3.26 (dd, 1H), 3.24 (s, 3H), 3.05 (dd, 1 H), 1.37 (s, 9H).

### Beispiel 2.5B

### tert.-Butyl-[1-(2-brom-4-chlorphenyl)-3-hydroxypropan-2-yl]carbamat (Racemat)

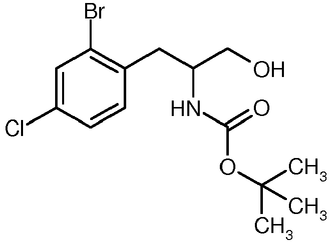

Eine Suspension von 28.3 g (72.1 mmol, 1 eq.) Methyl-2-brom-*N*-(*tert*.-butoxycarbonyl)-4-chlorphenylalaninat (Racemat) in 470 ml Tetrahydrofuran wurde bei 0°C mit 3.6 g (93.7 mmol, 1.3 eq.) Lithiumaluminiumhydrid versetzt, wobei die Innentemperatur des Reaktionsgemisches unter 10°C gehalten wurde. Nach Beendigung der Zugabe wurde das Reaktionsgemisch auf RT kommen gelassen. Nach Zugabe von 300 ml Tetrahydrofuran zur besseren Rührbarkeit wurde das Reaktionsgemisch 2.5 h bei RT gerührt. Anschließend wurde das Reaktionsgemisch durch langsame Zugabe von 280 ml gesättigter, wässriger Kalium-Natrium-Tartrat-Lösung gequencht und 20 min bei RT gerührt. Nach Phasentrennung wurde die wässrige Phase zweimal mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt. Der Rückstand wurde in 2-Methoxy-2-methylpropan aufgenommen, das Gemisch zweimal mit Wasser und einmal mit gesättigter, wässriger Natriumchlorid-Lösung gewaschen und im Vakuum eingeengt. Ausbeute: 19.8 g (75% d. Th.). Der Rückstand wurde ohne weitere Reinigung in die nächste Stufe eingesetzt.

### Beispiel 2.5C

### tert.-Butyl-4-(2-brom-4-chlorbenzyl)-1,2,3-oxathiazolidin-3-carboxylat-2-oxid (Diastereomerengemisch)

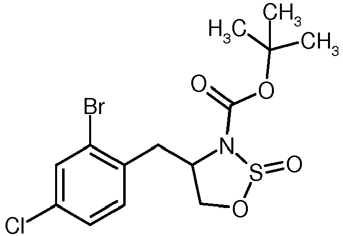

Eine Lösung von 8.0 g (22.0 mmol, 1 eq.) *tert*.-Butyl-[1-(2-brom-4-chlorphenyl)-3-hydroxypropan-2-yl]carbamat (Racemat) in 150 ml Dichlormethan wurde unter Argon bei -60°C tropfenweise mit 2.08 ml (28.7 mmol, 1.3 eq.) Thionylchlorid versetzt. Anschließend wurden 8.9 ml Pyridin (110 mmol, 5 eq.) zugetropft, wobei nach Zugabe weniger Tropfen Pyridin eine exotherme Reaktion auftrat, die Reaktionstemperatur auf -40°C stieg und das Reaktionsgemisch erneut auf -60°C gekühlt wurde. Nach beendeter Zugabe wurde das Reaktionsgemisch über Nacht auf RT kommen gelassen und mit Wasser gequencht. Nach Zugabe von Essigsäureethylester und Phasentrennung wurde die wässrige Phase zweimal mit Essigsäureethylester extrahiert. Die vereinigten, organischen Phasen wurden mit einer Mischung (1:1) von gesättigter, wässriger Natriumchlorid-Lösung und 20%iger wässriger Zitronensäure-Lösung und zweimal mit gesättigter, wässriger Natriumchlorid-Lösung gewaschen, getrocknet (Natriumsulfat), filtriert und im Vakuum eingeengt. Ausbeute: 7.8 g (87% d. Th.). Der Rückstand wurde ohne weitere Reinigung in die nächste Stufe eingesetzt.
HPLC/MS [Methode 14]: Rₜ = 17.3/17.6 min; MS (ESIpos): m/z = 434 (M+Na)⁺.

### Beispiel 2.5D

### tert.-Butyl-4-(2-brom-4-chlorbenzyl)-1,2,3-oxathiazolidin-3-carboxylat-2,2-dioxid (Racemat)

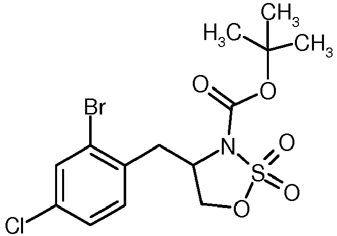

Eine Lösung von 20 g (48.9 mmol, 1 eq.) *tert*.-Butyl-4-(2-brom-4-chlorbenzyl)-1,2,3-oxathiazolidin-3-carboxylat-2-oxid (Diastereomerengemisch) in 500 ml Acetonitril wurde bei 0°C mit 16.75 g (78.3 mmol, 1.6 eq.) Natriumperiodat, 114 mg Rutheniumtrichlorid und 125 ml Wasser versetzt. Das Reaktionsgemisch wurde 2.5 h gerührt, wobei sich ein Niederschlag bildete, der anschließend filtriert wurde. Das Filtrat wurde mit 150 ml Wasser versetzt, und nach Zugabe von 2-Methoxy-2-methylpropan und Phasentrennung wurde die wässrige Phase dreimal mit 2-Methoxy-2-methylpropan extrahiert. Die vereinigten, organischen Phasen wurden mit gesättigter, wässriger Natriumsulfit-Lösung und mit gesättigter, wässriger Natriumchlorid-Lösung gewaschen, getrocknet (Natriumsulfat), filtriert und im Vakuum eingeengt, wobei 7.5 g Rohmaterial erhalten wurden. Der Feststoff aus der Filtration wurde dreimal mit heißem 2-Methoxy-2-methylpropan extrahiert. Die vereinigten, organischen Phasen wurden mit gesättigter, wässriger Natriumsulfit-Lösung und mit gesättigter, wässriger Natriumchlorid-Lösung gewaschen, getrocknet (Natriumsulfat), filtriert und im Vakuum eingeengt, wobei 9.6 g Rohmaterial erhalten wurden. Die vereinigten Rohprodukte wurden mit 30 ml Essigsäureethylester verrührt, filtriert und im Vakuum getrocknet. Ausbeute: 9.4 g (45% d. Th.)
HPLC/MS [Methode 14]: Rₜ = 16.6 min; MS (ESIpos): m/z = 875 (2M+Na)⁺.

### Beispiel 2.5E

### tert.-Butyl-[1-(2-brom-4-chlorphenyl)-3-fluorpropan-2-yl]carbamat (Racemat)

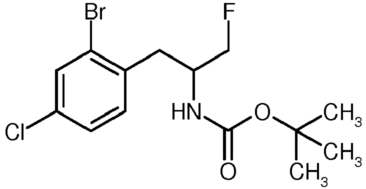

Eine Suspension von 9.3 g (21.8 mmol, 1 eq.) *tert*.-Butyl-4-(2-brom-4-chlorbenzyl)-1,2,3-oxathiazolidin-3-carboxylat-2,2-dioxid (Racemat) in 250 ml Tetrahydrofuran wurde mit 1.8 ml (10.9 mmol, 0.5 eq.) Triethylamin-trihydrofluorid und 6.65 ml (47.9 mmol, 2.2 eq.) Triethylamin versetzt. Das Reaktionsgemisch wurde 16 h bei 55°C gerührt und nach Abkühlen auf RT mit 380 ml Wasser gequencht. Nach Zugabe von Essigsäureethylester und Phasentrennung wurde die wässrige Phase dreimal mit Essigsäureethylester extrahiert. Die vereinigten, organischen Phasen wurden zweimal mit gesättigter, wässriger Natriumchlorid-Lösung gewaschen, getrocknet (Natriumsulfat), filtriert und im Vakuum eingeengt. Es wurden 7.8 g Rohprodukt erhalten. Zur säulenchromatographischen Aufreinigung wurde das Rohprodukt mit Material aus anderen Ansätzen vereinigt und so erhaltene 8.2 g mittels Flash-Chromatographie (250 g Kieselgel, Dichlormethan-Methanol-Gemische von 95/5 → 85/15) aufgereinigt. Ausbeute: 3.3 g. Weitere Flash-Chromatographie (150 g Kieselgel, Dichlormethan-Methanol-Gemische von 95/5 bis 85/15) der Mischfraktion ergab nochmals 1.3 g gewünschtes Produkt.
HPLC/MS [Methode 14]: Rₜ = 16.0 min; MS (ESIpos): m/z = 388 (M+Na)⁺,
¹H-NMR (500 MHz, CDCl₃): δ [ppm] = 7.51-7.49 (m, 1H), 7.46-7.41 (m, 1H), 7.22-7.16 (m, 1H), 4.97-4.51 (m, 3H), 3.33-3.05 (m, 2H), 1.27-1.07 (br. s, 9H),
¹⁹F-NMR (470 MHz, CDCl₃): δ [ppm] = -216.6 ppm.

### Beispiel 3.1A

### 4-(2-{[(tert.-Butoxycarbonyl)amino]methyl}-5-chlorphenyl)-1-(1-tert.-butoxy-1-oxobutan-2-yl)-6-oxo-1,6-dihydropyridin-3-carbonsäureethylester (Stereoisomerengemisch)

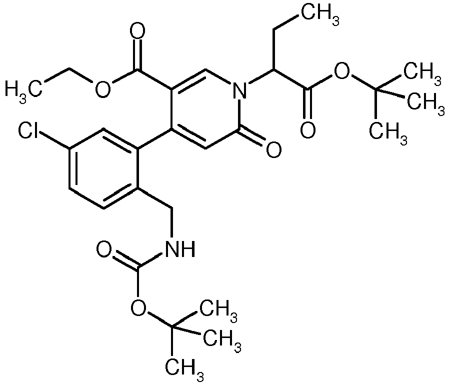

796 mg (5.76 mmol) Kaliumcarbonat wurden im Reaktionsgefäß getrocknet und anschließend 850 mg (1.86 mmol) 1-(1-*tert*.-Butoxy-1-oxobutan-2-yl)-6-oxo-4-{[(trifluormethyl)sulfonyl]oxy}-1,6-dihydropyridin-3-carbonsäureethylester (Racemat), 1.27 g (2.23 mmol) (2-{[(*tert*.-Butoxycarbonyl)amino]methyl}-5-chlorphenyl)borsäure (50% Reinheit) und 18 ml 1,4-Dioxan zugegeben. Das Reaktionsgemisch wurde entgast, mit 215 mg (186 µmol) Tetrakis(triphenylphosphin)palladium(0) versetzt und für 1.5 h bei 110°C geschüttelt. Es wurde auf Raumtemperatur abgekühlt und das Lösungsmittel unter reduziertem Druck entfernt. Der Rückstand mittels Flash-Chromatographie (Kieselgel-50, Cyclohexan-Essigsäureethylester-Gemisch) gereinigt. Ausbeute: 685 mg (65% d. Th.)
LC/MS [Methode 1]: Rₜ = 1.34 min; MS (ESIpos): m/z = 549 (M+H)⁺.

### Beispiel 3.1B

### 2-{4-[2-(Aminomethyl)-5-chlorphenyl]-5-(ethoxycarbonyl)-2-oxopyridin-1(2H)-yl}butansäure (Stereoisomerengemisch)

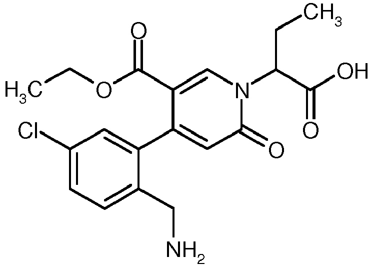

680 mg (1.24 mmol) 4-(2-{[(*tert*.-Butoxycarbonyl)amino]methyl}-5-chlorphenyl)-1-(1-*tert*.-butoxy-1-oxobutan-2-yl)-6-oxo-1,6-dihydropyridin-3-carbonsäureethylester (Stereoisomerengemisch) wurden in 5 ml Dichlormethan vorgelegt und mit 954 µl (12.4 mmol) Trifluoressigsäure versetzt. Es wurde bis zum vollständigen Umsatz bei Raumtemperatur nachgerührt. Anschließend wurde das Lösungsmittel unter reduziertem Druck entfernt und der Rückstand mehrmals mit Dichlormethan und Toluol koevaporiert. Ausbeute: 751 mg (89% Reinheit, quantitativ)
LC/MS [Methode 1]: Rₜ = 0.65 min; MS (ESIpos): m/z = 393 (M+H)⁺.

### Beispiel 3.1C

### 2-(10-Chlor-2,5-dioxo-2,5,6,7-tetrahydro-3H-pyrido[3,4-d][2]benzazepin-3-yl)butansäure (Stereoisomerengemisch)

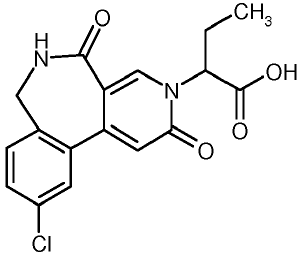

Eine Lösung von 486 mg (1.10 mmol) 2-{4-[2-(Aminomethyl)-5-chlorphenyl]-5-(ethoxycarbonyl)-2-oxopyridin-1(2*H*)-yl}butansäure (Stereoisomerengemisch) in 11 ml Tetrahydrofuran wurde unter Argon zu aktiviertem Molsieb 3Å (über Nacht bei 200°C im Trockenschrank getrocknet) gegeben und 2 h bei RT gerührt. Anschließend wurde das Reaktionsgemisch unter Argon bei RT mit 8.22 ml (22.0 mmol, 20 eq.) einer Natriumethylat-Lösung (21%ig in Ethanol, zuvor 2 h über aktiviertem Molsieb 3Å getrocknet) versetzt und 45 min bei Raumtemperatur gerührt. Nach Verdünnen mit Essigsäureethylester wurde das Reaktionsgemisch mit gesättigter, wässriger Ammoniumchlorid-Lösung versetzt und mit wässriger Salzsäure-Lösung (1N) auf pH 3 gebracht und erneut mit Essigsäureethylester extrahiert. Die vereinigten, organischen Phasen wurden dreimal mit 50 ml wässriger 0.5M Salzsäure und 50 ml wässriger, gesättigter Natriumchlorid-Lösung gewaschen, über Magnesiumsulfat getrocknet, filtriert und das Lösungsmittel unter reduziertem Druck entfernt. Ausbeute: 364 mg (95% d. Th.)
LC/MS [Methode 1]: Rₜ = 0.70 min; MS (ESIpos): m/z = 347 (M+H)⁺.

### Beispiel 3.1D

### 4-{[2-(10-Chlor-2,5-dioxo-2,5,6,7-tetrahydro-3H-pyrido[3,4-d][2]benzazepin-3-yl)butanoyl]-amino}benzoesäure-tert.-butylester (Stereoisomerengemisch)

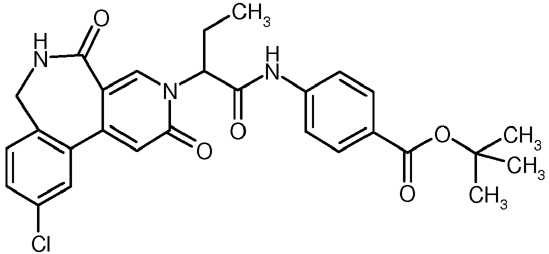

75.0 mg (216 µmol) 2-(10-Chlor-2,5-dioxo-2,5,6,7-tetrahydro-3*H*-pyrido[3,4-*d*][2]benzazepin-3-yl)butansäure (Stereoisomerengemisch) und 62.7 mg (324 µmol, 1.5 eq.) 4-Aminobenzoesäure-*tert*.-butylester wurden in 5 ml Pyridin vorgelegt. Anschließend wurden 505 µl (865 µmol) Propylphosphonsäureanhydrid (T3P, 50%ig in Essigsäureethylester, 4 eq.) zugetropft und die Reaktionslösung über Nacht bei 60°C gerührt. Die Reaktion wurde auf Raumtemperatur abgekühlt und mit 50 ml Wasser versetzt. Es wurde dreimal mit 30 ml Essigsäureethylester extrahiert. Die vereinigten, organischen Phasen wurden dreimal mit pH 5 Puffer gewaschen, und das Lösungsmittel wurde unter reduziertem Druck entfernt. Der Rückstand wurde mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 44.2 mg (39% d. Th.)
LC/MS [Methode 1]: Rₜ = 1.05 min; MS (ESIpos): m/z = 522 (M+H)⁺,
¹H-NMR (400 MHz, DMSO-d₆): δ [ppm] = 10.9 (s, 1H), 8.55 (br. s, 1H), 8.27 (s, 1H), 7.87 (d, 2H), 7.76-7.72 (m, 3H), 7.54 (dd, 1H), 7.43 (d, 1H), 6.65 (s, 1H), 5.67-5.60 (m, 1H), 4.29-3.89 (2x br. s, 2H), 2.26-2.15 (m, 1H), 2.11-1.97 (m, 1H), 1.54 (s, 9H), 0.92 (t, 3H).

### Beispiel 4.1A

### 4-(2-{1-[(tert.-Butoxycarbonyl)amino]cyclopropyl}-5-chlorphenyl)-1-(1-tert.-butoxy-1-oxobutan-2-yl)-6-oxo-1,6-dihydropyridin-3-carbonsäureethylester (Stereoisomerengemisch)

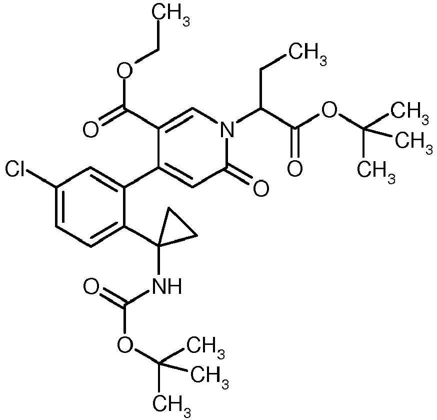

860 mg (6.22 mmol) Kaliumcarbonat wurden im Reaktionsgefäß getrocknet und anschließend 918 mg (2.01 mmol) 1-(1-*tert*.-Butoxy-1-oxobutan-2-yl)-6-oxo-4-{[(trifluormethyl)sulfonyl]oxy}-1,6-dihydropyridin-3-carbonsäureethylester (Racemat), 920 mg (2.08 mmol) (2-{1-[(*t*e*rt*.-Butoxycarbonyl)amino]cyclopropyl}-5-chlorphenyl)borsäure (68% Reinheit) und 20 ml 1,4-Dioxan zugegeben. Das Reaktionsgemisch wurde entgast, mit 232 mg (201 µmol) Tetrakis(triphenylphosphin)palladium(0) versetzt und für 7 h bei 80°C geschüttelt. Es wurde auf Raumtemperatur abgekühlt, filtriert und das Lösungsmittel unter reduziertem Druck entfernt. Der Rückstand wurde mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 149 mg (12% d. Th.)
LC/MS [Methode 1]: Rₜ = 1.39 min; MS (ESIpos): m/z = 575 (M+H)⁺.

### Beispiel 4.1B

### 2-{4-[2-(1-Aminocyclopropyl)-5-chlorphenyl]-5-(ethoxycarbonyl)-2-oxopyridin-1(2H)-yl}butansäure (Stereoisomerengemisch)

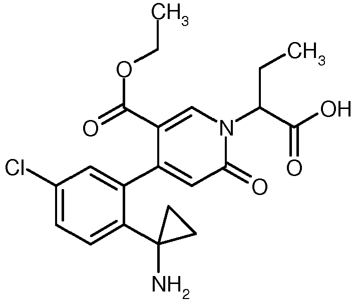

165 mg (275 µmol) 4-(2-{1-[(*tert*.-Butoxycarbonyl)amino]cyclopropyl}-5-chlorphenyl)-1-(1-*tert*.-butoxy-1-oxobutan-2-yl)-6-oxo-1,6-dihydropyridin-3-carbonsäureethylester (Stereoisomerengemisch) wurden in 3 ml Dichlormethan vorgelegt und mit 530 µl (6.89 mmol) Trifluoressigsäure portionsweise unter Eisbadkühlung versetzt. Es wurde bis zum vollständigen Umsatz bei Raumtemperatur nachgerührt. Anschließend wurde das Lösungsmittel unter reduziertem Druck entfernt und der Rückstand dreimal mit 10 ml Toluol koevaporiert. Der Rückstand wurde mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 60 mg (94% Reinheit, 49% d. Th.)
LC/MS [Methode 1]: Rₜ = 0.66 min; MS (ESIpos): m/z = 419 (M+H)⁺.

### Beispiel 4.1C

### 2-(10'-Chlor-2',5'-dioxo-5',6'-dihydrospiro[cyclopropan-1,7'-pyrido[3,4-d][2]benzazepin]-3'(2'H)-yl)butansäure (Stereoisomerengemisch)

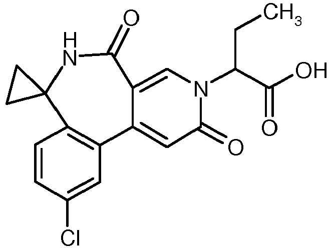

Eine Lösung von 60.0 mg (143 µmol) 2-{4-[2-(1-Aminocyclopropyl)-5-chlorphenyl]-5-(ethoxycarbonyl)-2-oxopyridin-1(2*H*)-yl}butansäure (Stereoisomerengemisch) in 3 ml Tetrahydrofuran wurde unter Argon zu aktiviertem Molsieb 3Å (über Nacht bei 200°C im Trockenschrank getrocknet) gegeben und 2 h bei RT gerührt. Anschließend wurde das Reaktionsgemisch unter Argon bei RT mit 670 µl (2.87 mmol, 20 eq.) einer Natriumethylat-Lösung (21%ig in Ethanol, zuvor 2 h über aktiviertem Molsieb 3Å getrocknet) versetzt und 45 min bei Raumtemperatur gerührt. Nach Verdünnen mit Essigsäureethylester wurde das Reaktionsgemisch mit gesättigter, wässriger Ammoniumchlorid-Lösung versetzt und mit wässriger Salzsäure-Lösung (1N) auf pH 3 gebracht und erneut mit Essigsäureethylester extrahiert. Die vereinigten, organischen Phasen wurden mit 50 ml wässriger, gesättigter Natriumchlorid-Lösung gewaschen, über Magnesiumsulfat getrocknet, filtriert und das Lösungsmittel unter reduziertem Druck entfernt. Ausbeute: 51.8 mg (97% d. Th.)
LC/MS [Methode 1]: Rₜ = 0.69 min; MS (ESIpos): m/z = 373 (M+H)⁺.

### Beispiel 4.1D

### 4-{[2-(10'-Chlor-2',5'-dioxo-5',6'-dihydrospiro[cyclopropan-1,7'-pyrido[3,4-d][2]benzazepin]-3'(2'H)-yl)butanoyl]amino}benzoesäure-tert.-butylester (Stereoisomerengemisch)

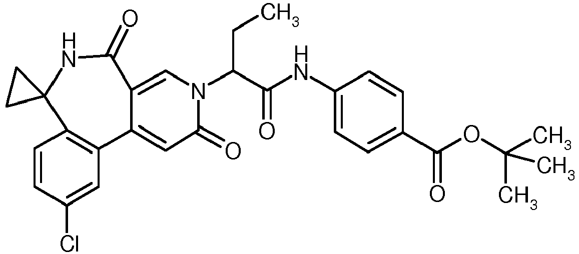

52.0 mg (139 µmol) 2-(10'-Chlor-2',5'-dioxo-5',6'-dihydrospiro[cyclopropan-1,7'-pyrido[3,4-*d*][2]benzazepin]-3'(2'*H*)-yl)butansäure (Stereoisomerengemisch) und 40.4 mg (209 µmol, 1.5 eq.) 4-Aminobenzoesäure-*tert*.-butylester wurden in 3 ml Pyridin vorgelegt. Anschließend wurden 163 µl (558 µmol) Propylphosphonsäureanhydrid (T3P, 50%ig in Essigsäureethylester, 4 eq.) zugetropft und die Reaktionslösung über Nacht bei 60°C und 2 h bei 80°C gerührt. Das Reaktionsgemisch wurde direkt mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 46.9 mg (76% d. Th.)
LC/MS [Methode 1]: Rₜ = 1.13 min; MS (ESIpos): m/z = 548 (M+H)⁺,
¹H-NMR (400 MHz, DMSO-d₆): δ [ppm] = 10.9 (2x s, 1H), 8.88/8.85 (2x s, 1H), 8.20 (s, 1H), 7.89-7.85 (m, 2H), 7.76-7.71 (m, 3H), 7.52 (dd, 1H), 7.38 (d, 1H), 6.66/6.64 (2x s, 1H), 5.68-5.61 (m, 1H), 2.28-1.99 (m, 2H), 1.54 (s, 9H), 1.52-1.45 (m, 1H), 1.21-1.12 (m, 1H), 0.98-0.81 (m, 4H), 0.63-0.54 (m, 1H).

### Beispiel 5.1A

### 4-Chlor-6-oxo-1,6-dihydropyridin-3-carbonsäureethylester

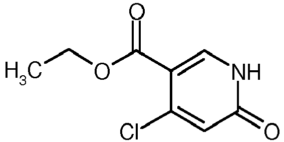

9.82 g (44.6 mmol) 4,6-Dichlorpyridin-3-carbonsäureethylester wurden in 98 ml Essigsäure vorgelegt und mit 4.03 g (49.1 mmol) Natriumacetat versetzt. Die Reaktionsmischung wurde über Nacht unter Rückfluss erhitzt und anschließend auf 100 ml Wasser gegossen. Der Niederschlag wurde abgesaugt, mit Wasser gewaschen und im Hochvakuum getrocknet. Ausbeute: 6.35 g (91% Reinheit, 64% d. Th.)
LC/MS [Methode 1]: Rₜ = 0.61 min; MS (ESIpos): m/z = 202 (M+H)⁺,
¹H-NMR (400 MHz, DMSO-d₆): δ [ppm] = 12.4 (br. s, 1H), 8.11 (s, 1H), 6.55 (s, 1H), 4.22 (q, 2H), 1.28 (t, 3H).

### Beispiel 5.1B

### 1-(2-tert.-Butoxy-2-oxoethyl)-4-chlor-6-oxo-1,6-dlhydropyridin-3-carbonsäureethylester

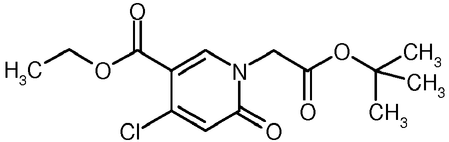

2.00 g (9.92 mmol) 4-Chlor-6-oxo-1,6-dihydropyridin-3-carbonsäureethylester, 2.32 g (11.9 mmol) Bromessigsäure-tert.-butylester und 2.06 g (14.9 mmol) Kaliumcarbonat wurden in 35 ml Dimethylformamid für 45 min bei 100°C gerührt. Anschließend wurde das Lösungsmittel unter reduziertem Druck entfernt, der Rückstand in Wasser aufgenommen und mit Essigsäureethylester extrahiert. Die vereinigten, organischen Phasen wurden mit wässriger, gesättigter Natriumchlorid-Lösung gewaschen, mit Magnesiumsulfat getrocknet, filtriert und das Lösungsmittel unter reduziertem Druck entfernt. Ausbeute: 3.07 g (94% Reinheit, 92% d. Th.)
LC/MS [Methode 1]: Rₜ = 0.96 min; MS (ESIpos): m/z = 316 (M+H)⁺,
¹H-NMR (400 MHz, DMSO-d₆): δ [ppm] = 8.62 (s, 1H), 6.60 (s, 1H), 4.71 (s, 2H), 4.26 (q, 2H), 1.42 (s, 9H), 1.30 (t, 3H).

### Beispiel 5.1C

### [1-(2-tert.-Butoxy-2-oxoethyl)-5-(ethoxycarbonyl)-2-oxo-1,2-dihydropyridin-4-yl]borsäure

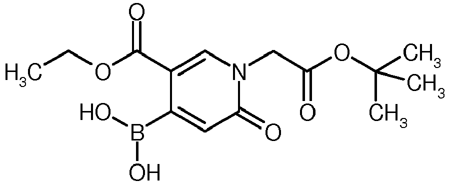

3.07 g (9.72 mmol) 1-(2-*tert*.-Butoxy-2-oxoethyl)-4-chlor-6-oxo-1,6-dihydropyridin-3-carbonsäureethylester wurden in 35 ml 1,4-Dioxan vorgelegt und die Lösung wurde entgast. Anschließend wurden 1.91 g (19.4 mmol) Kaliumacetat, 0.71 g (0.97 mmol) 1,1'-Bis(diphenylphosphin)ferrocenpalladium(II)chlorid, 2.72 g (10.7 mmol) 4,4,4',4',5,5,5',5'-Octamethyl-2,2'-bi-1,3,2-dioxaborolan hinzugegeben und die Reaktionsmischung für 2.5 h bei 130°C gerührt. Es wurde auf Raumtemperatur abgekühlt, mit Essigsäureethylester verdünnt, über Kieselgel filtriert und das Lösungsmittel unter reduziertem Druck entfernt. Der Rückstand wurde mittels Flash-Chromatographie (Kieselgel-50, Cyclohexan-Essigsäureethylester-Gemisch, dann Dichlormethan-Methanol-Gemisch) gereinigt. Ausbeute: 431 mg (69% Reinheit, 9% d. Th.)
LC/MS [Methode 1]: Rₜ = 0.64 min; MS (ESIpos): m/z = 326 (M+H)⁺.

### Beispiel 5.1D

### 4-(2-{2-[(tert.-Butoxycarbonyl)amino]ethyl}-5-chlorphenyl)-1-(2-tert.-butoxy-2-oxoethyl)-6-oxo-1,6-dihydropyridin-3-carbonsäureethylester

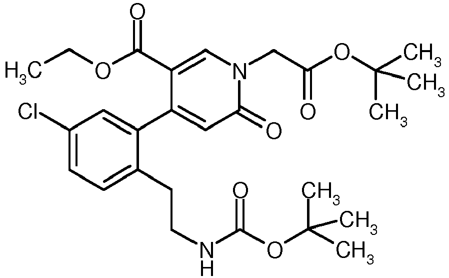

386 mg (1.15 mmol) *tert*.-Butyl-[2-(2-brom-4-chlorphenyl)ethyl]carbamat wurden in 11 ml THF vorgelegt und die resultierende Lösung entgast. Anschließend wurden 1.00 g (75% Reinheit, 2.31 mmol) [1-(2-*tert*.-Butoxy-2-oxoethyl)-5-(ethoxycarbonyl)-2-oxo-1,2-dihydropyridin-4-yl]boronsäure, 67 mg (58 µmol) Tetrakis(triphenylphosphin)palladium(0) und 350 mg (2.31 mmol) Cäsiumfluorid hinzugegeben und die Reaktionsmischung über Nacht bei 110°C gerührt. Das Lösungsmittel wurde unter reduziertem Druck entfernt, der Rückstand in Acetonitril aufgenommen, durch einen Feinfilter filtriert und mittels präparativer HPLC [Säule: Chromatorex C18,10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 303 mg (49% d. Th.)
LC/MS [Methode 1]: Rₜ = 1.27 min; MS (ESIpos): m/z = 535 (M+H)⁺,
¹H-NMR (400 MHz, DMSO-d₆): δ [ppm] = 8.61 (s, 1H), 7.37 (dd, 1H), 7.26 (d, 1H), 7.12 (d, 1H), 6.75-6.69 (m, 1H), 6.28 (s, 1H), 4.77 (s, 2H), 4.00-3.92 (m, 2H), 3.04-2.95 (m, 2H), 2.57-2.38 (m, 2H), 1.44 (s, 9H), 1.32 (s, 9H), 0.93 (t, 3H).

### Beispiel 5.1E

### {4-[2-(2-Aminoethyl)-5-chlorphenyl]-5-(ethoxycarbonyl)-2-oxopyridin-1(2H)-}essigsäure

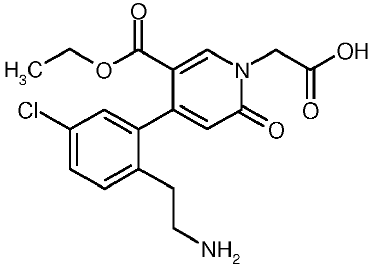

307 mg (574 µmol) 4-(2-{2-[(*tert*.-Butoxycarbonyl)amino]ethyl}-5-chlorphenyl)-1-(2-*tert*.-butoxy-2-oxoethyl)-6-oxo-1,6-dihydropyridin-3-carbonsäureethylester wurden in 5 ml Dichlormethan vorlegt und mit 633 µl (8.61 mmol) Trifluoressigsäure versetzt. Es wurde 3 h nachgerührt. Das Lösungsmittel wurde unter reduziertem Druck entfernt und der Rückstand dreimal mit 2 ml Dichlormethan kodestilliert und abschließend lyophilisiert. Das Lyophilisat wurde mittels präparativer HPLC [Säule: Chromatorex C18,10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 74 mg (30% d. Th.)
LC/MS [Methode 1]: Rₜ = 0.63 min; MS (ESIpos): m/z = 379 (M+H)⁺,
¹H-NMR (400 MHz, DMSO-d₆): δ [ppm] = 8.42 (s, 1H), 7.41 (dd, 1H), 7.33 (d, 1H), 7.14 (d, 1H), 6.18 (s, 1H), 4.42-4.30 (m, 2H), 3.96 (q, 2H), 2.93-2.82 (m, 2H), 2.76-2.59 (m, 2H), 0.97 (t, 3H).

### Beispiel 5.1F

### (11-Chlor-2,5-dioxo-5,6,7,8-tetrahydropyrido[3,4-e][3]benzazocin-3(2H)-yl)essigsäure

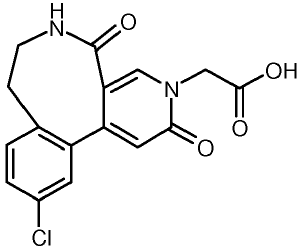

74.0 mg (195 µmol) {4-[2-(2-Aminoethyl)-5-chlorphenyl]-5-(ethoxycarbonyl)-2-oxopyridin-1(2*H*)-yl}essigsäure wurden in 3.9 ml THF vorgelegt und mit 15.6 mg (391 µmol) Natriumhydrid (60% in Mineralöl) versetzt. Es wurde für 30 min bei Raumtemperatur nachgerührt und anschließend 7.8 mg (0.20 mmol) Natriumhydrid (60% in Mineralöl) hinzugegeben. Es wurde für 4 h nachgerührt und die Reaktion durch Zugabe von Wasser beendet. Die Phasen wurden getrennt und die wässrige Phase dreimal mit 10 ml Essigsäureethylester extrahiert. Die vereinigten, organischen Phasen wurden über Magnesiumsulfat getrocknet, filtriert und das Lösungsmittel unter reduziertem Druck entfernt. Der Rückstand wurde mittels präparativer HPLC [Säule: Chromatorex C18,10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 4.6 mg (95% Reinheit, 7% d. Th.) und 6.4 mg (85% Reinheit, 8% d. Th.)
LC/MS [Methode 1]: Rₜ = 0.56 min; MS (ESIpos): m/z = 333 (M+H)⁺,
¹H-NMR (400 MHz, DMSO-d₆): δ [ppm] = 7.86 (s, 1H), 7.72 (t, 1H), 7.45 (dd, 1H), 7.31 (d, 1H), 7.23 (d, 1H), 6.28 (s, 1H), 4.64 (s, 2H), 3.47-3.21 (m, 2H), 2.98-2.89 (m, 2H).

Alternativ kann die Titelverbindung auch in Analogie zu der in Beispiel 9.1G beschriebenen Methode dargestellt werden. Die Umsetzung von 36 mg (61 µmol) {4-[2-(2-Aminoethyl)-5-chlorphenyl]-5-(ethoxycarbonyl)-2-oxopyridin-1(2*H*)-yl}essigsäure (64% Reinheit) ergab 15.4 mg (44% Reinheit, 33% d. Th.) der Titelverbindung.
LC/MS [Methode 3]: Rₜ = 1.49 min; MS (ESIpos): m/z = 333 (M+H)⁺.

### Beispiel 5.1G

### 4-{[(11-Chlor-2,5-dioxo-5,6,7,8-tetrahydropyrido[3,4-e][3]benzazocin-3(2H-yl)acetyl]amino}-benzoesäure-tert.-butylester

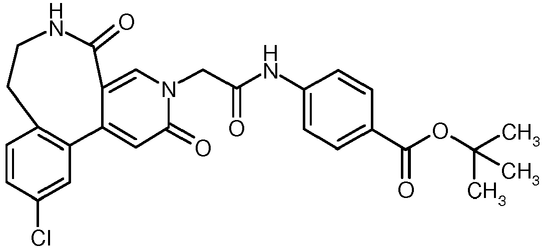

15 mg (45 µmol) (11-Chlor-2,5-dioxo-5,6,7,8-tetrahydropyrido[3,4-*e*][3]benzazocin-3(2*H*)-yl)essigsäure wurden in 0.5 ml Dimethylformamid gelöst und mit 8.7 mg (45 µmol) 4-Aminobenzoesäure-*tert*.-butylester und 0.64 mg (4.5 µmol) Oxyma versetzt. Anschließend wurden 7.0 µl (45 µmol) Di-*iso*-propylcarbodiimid zugetropft und die Reaktionslösung über das Wochenende bei 40°C geschüttelt. Das Lösungsmittel wurde unter reduziertem Druck entfernt und der Rückstand mittels präparativer HPLC [Säule: Chromatorex C18,10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 5.7 mg (21% Reinheit, 5% d. Th.)
LC/MS [Methode 2]: Rₜ = 2.94 min; MS (ESIpos): m/z = 508 (M+H)⁺.

### Beispiel 6.1A

### 2-Aminobutansäure-tert.-butylester (Racemat)

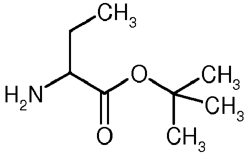

10.0 g (44.8 mmol) 2-Brombutansäure-*tert*.-butylester (Racemat) wurden in 30 ml THF vorlegt und bei Raumtemperatur mit 800 ml wässriger Ammoniumhydroxid-Lösung (25%ig) versetzt. Es wurde für 96 h nachgerührt und anschließend alle flüchtigen Bestandteile bei 100 mbar und 35 bis 38°C Badtemperatur am Rotationsverdampfer entfernt. Die wässrige Phase wurde dreimal mit 200 ml Dichlormethan extrahiert. Die vereinigten, organischen Phasen wurden getrocknet, filtriert und das Lösungsmittel unter reduziertem Druck bei 35°C Badtemperatur entfernt. Ausbeute: 5.40 g (74% d. Th.)
¹H-NMR (400 MHz, CDCl₃): δ [ppm] = 3.28-3.24 (m, 1H), 1.76-1.66 (m, 1H), 1.64-1.53 (m, 1H), 1.45 (s, 9H), 0.94 (t, 3H).

### Beispiel 6.1B

### 1-(1-tert.-Butoxy-1-oxobutan-2-yl)-4-hydroxy-6-oxo-1,6-dihydropyridin-3-carbonsäureethylester (Racemat)

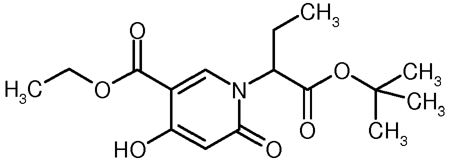

2.66 ml (14.7 mmol) 3-Oxopentandicarbonsäurediethylester und 3.35 ml (20.5 mmol, 1.4 eq.) Diethoxymethylacetat wurden 2.5 h bei 100°C gerührt, nach Abkühlen auf RT wurde dreimal mit Toluol koevaporiert und im Hochvakuum getrocknet. Der Rückstand wurde in 29.4 ml Ethanol aufgenommen, unter Eiskühlung mit einer Lösung von 2.50 g (15.4 mmol, 1.05 eq.) 2-Aminobutansäure-*tert*.-butylester (Racemat) in Ethanol versetzt und 2 h bei RT gerührt. Anschließend wurde das Reaktionsgemisch mit 5.47 ml (14.7 mmol, 1.0 eq.) einer Natriumethylat-Lösung (21%ig in Ethanol) versetzt und 1 h bei RT gerührt. Die Reaktion wurde durch Zugabe von 100 ml gesättigter, wässriger Ammoniumchlorid-Lösung und 50 ml Essigsäureethylester beendet. Die Phasen wurden getrennt und die wässrige Phase dreimal mit 50 ml Essigsäureethylester extrahiert. Die vereinigten, organischen Phasen wurden mit gesättigter, wässriger Natriumchlorid-Lösung gewaschen, über Magnesiumsulfat getrocknet, filtriert und das Lösungsmittel unter reduziertem Druck entfernt. Der Rückstand wurde mittels Flash-Chromatographie (Kieselgel-50, Cyclohexan-Essigsäureethylester-Gemisch) aufgereinigt. Ausbeute: 2.86 g (60% d. Th.)
LC/MS [Methode 1]: Rₜ = 1.00 min; MS (ESIpos): m/z = 326 (M+H)⁺,
¹H-NMR (400 MHz, DMSO-d₆): δ [ppm] = 10.9 (s, 1H), 8.33 (s, 1H), 5.70 (s, 1H), 4.99 (dd, 1H), 4.29 (q, 2H), 2.12-1.92 (m, 2H), 1.37 (s, 9H), 1.30 (t, 3H), 0.79 (t, 3H).

### Beispiel 6.1C

### 1-(1-tert.-Butoxy-1-oxobutan-2-yl)-6-oxo-4-{[(trifluormethyl)sulfonyl]oxy}-1,6-dihydropyridin-3-carbonsäureethylester (Racemat)

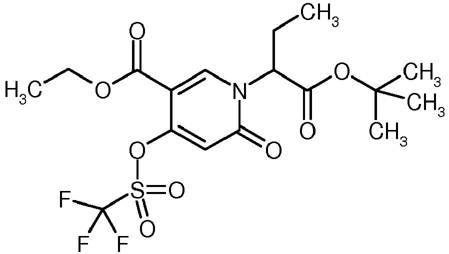

Eine Lösung von 2.86 g (8.79 mmol) 1-(1-*tert*.-Butoxy-1-oxobutan-2-yl)-4-hydroxy-6-oxo-1,6-dihydropyridin-3-carbonsäureethylester (Racemat) in 20 ml Dichlormethan wurde unter Argon bei -78°C mit 2.45 ml (17.6 mmol, 2.0 eq.) Triethylamin und anschließend portionsweise mit 5.99 g (14.1 mmol, 1.6 eq.) *N*-(4-*tert*.-Butylphenyl)-1,1,1-trifluor-*N-*[(trifluormethyl)sulfonyl]-methansulfonamid versetzt. Das Reaktionsgemisch wurde auf RT kommen gelassen, über Nacht bei RT gerührt und anschließend das Lösungsmittel unter reduziertem Druck entfernt. Der Rückstand wurde mittels Flash-Chromatographie (Kieselgel-50, Cyclohexan-Essigsäureethylester-Gemisch) aufgereinigt. Ausbeute: 2.76 g (69% d. Th.)
LC/MS [Methode 1]: Rₜ = 1.23 min; MS (ESIneg): m/z = 456 (M-H)⁻,
¹H-NMR (400 MHz, DMSO-d₆): δ [ppm] = 8.66 (s, 1H), 6.68 (s, 1H), 5.10 (dd, 1H), 4.31 (q, 2H), 2.19-1.99 (m, 2H), 1.37 (s, 9H), 1.31 (t, 3H), 0.83 (t, 3H).

### Beispiel 6.1D

### 4-(2-{2-[(tert.-Butoxycarbonyl)amino]ethyl}-5-chlorphenyl)-1-(1-tert.-butoxy-1-oxobutan-2-yl)-6-oxo-1,6-dihydropyridin-3-carbonsäureethylester (Stereoisomerengemisch)

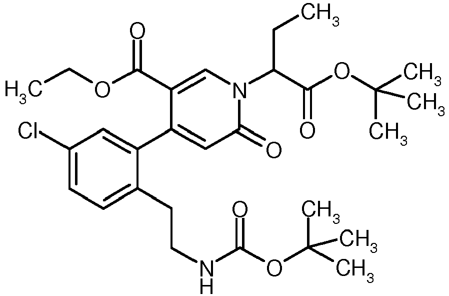

429 mg (3.10 mmol) Kaliumcarbonat wurden im Reaktionsgefäß getrocknet und anschließend 473 mg (1.03 mmol) 1-(1-*tert*.-Butoxy-1-oxobutan-2-yl)-6-oxo-4-{[(trifluormethyl)sulfonyl]oxy}-1,6-dihydropyridin-3-carbonsäureethylester (Racemat), 671 mg (1.76 mmol) *tert*.-Butyl-{2-[4-chlor-2-(4,4,5,5-tetramethyl-1,3,2-dioxaborolan-2-yl)phenyl]ethyl}carbamat und 10 ml 1,4-Dioxan zugegeben. Das Reaktionsgemisch wurde entgast, mit 119 mg (103 µmol) Tetrakis(triphenylphosphin)palladium(0) versetzt und über Nacht bei 110°C gerührt. Es wurde auf Raumtemperatur abgekühlt, über Celite filtriert und das Lösungsmittel unter reduziertem Druck entfernt. Der Rückstand wurde mittels Flash-Chromatographie (Kieselgel-50, Cyclohexan-Essigsäureethylester-Gemisch) aufgereinigt. Ausbeute: 330 mg (96% Reinheit, 54% d. Th.)
LC/MS [Methode 1]: Rₜ = 1.33 min; MS (ESIpos): m/z = 563 (M+H)⁺,
¹H-NMR (400 MHz, DMSO-d₆): δ [ppm] = 8.45/8.44 (2x s, 1H), 7.39-7.35 (m, 1H), 7.27/7.24 (2x d, 1H), 7.17/7.14 (2x d, 1H), 6.75-6.66 (m, 1H), 6.28/6.27 (2x s, 1H), 5.16-5.07 (m, 1H), 3.99-3.91 (m, 2H), 3.03-2.95 (m, 2H), 2.57-2.40 (m, 2H), 2.21-2.01 (m, 2H), 1.41/1.40 (2x s, 9H), 1.31 (s, 9H), 0.93-0.85 (m, 6H).

### Beispiel 6.1E

### 2-{4-[2-(2-Aminoethyl)-5-chlorphenyl]-5-(ethoxycarbonyl)-2-oxopyridin-1(2H)-yl}butansäure (Stereoisomerengemisch)

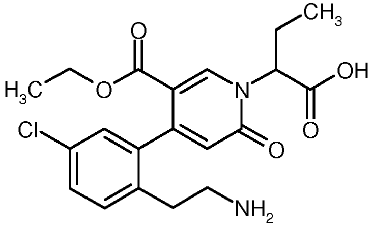

330 mg (586 µmol) 4-(2-{2-[(*tert*.-Butoxycarbonyl)amino]ethyl}-5-chlorphenyl)-1-(1-*tert*.-butoxy-1-oxobutan-2-yl)-6-oxo-1,6-dihydropyridin-3-carbonsäureethylester (Stereoisomerengemisch) wurden in 5.5 ml Dichlormethan vorgelegt und mit 1.36 ml (17.6 mmol) Trifluoressigsäure versetzt. Es wurde für 6 h bei Raumtemperatur nachgerührt. Anschließend wurde das Lösungsmittel unter reduziertem Druck entfernt und der Rückstand zweimal mit 20 ml Toluol koevaporiert. Ausbeute: 328 mg (95% Reinheit, quantitativ)
LC/MS [Methode 1]: Rₜ = 0.72 min; MS (ESIpos): m/z = 407 (M+H)⁺,
¹H-NMR (400 MHz, DMSO-d₆): δ [ppm] = 13.2 (br. s, 1H), 8.51/8.49 (2x s, 1H), 7.69 (br. s, 2H), 7.44 (dd, 1H), 7.35 (d, 1H), 7.23-7.21 (m, 1H), 6.31 (s, 1H), 5.23-5.17 (m, 1H), 4.00-3.94 (m, 2H), 3.00-2.85 (m, 2H), 2.77-2.58 (m, 2H), 2.25-2.03 (m, 2H), 0.94 (t, 3H), 0.88 (2x t, 3H).

### Beispiel 6.1F

### 2-(11-Chlor-2,5-dioxo-5,6,7,8-tetrahydropyrido[3,4-e][3]benzazocin-3(2H)-yl)butansäure (Stereoisomerengemisch)

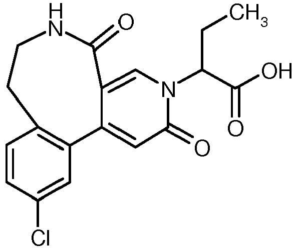

Eine Lösung von 238 mg (585 µmol) 2-{4-[2-(2-Aminoethyl)-5-chlorphenyl]-5-(ethoxycarbonyl)-2-oxopyridin-l(2H)-yl}butansäure (Stereoisomerengemisch) in 6 ml Tetrahydrofuran wurde unter Argon zu aktiviertem Molsieb 3Å (über Nacht bei 200°C im Trockenschrank getrocknet) gegeben und 2 h bei RT gerührt. Anschließend wurde das Reaktionsgemisch unter Argon bei RT mit 437 µl (1.17 mmol, 2 eq.) einer Natriumethylat-Lösung (21%ig in Ethanol, zuvor 2 h über aktiviertem Molsieb 3Å getrocknet) versetzt und 40 min bei Raumtemperatur gerührt. Nach Verdünnen mit Essigsäureethylester wurde das Reaktionsgemisch mit gesättigter, wässriger Ammoniumchlorid-Lösung versetzt. Die Phasen wurden getrennt und die wässrige Phase dreimal mit Essigsäureethylester extrahiert. Die wässrige Phase wurde anschließend mit wässriger Salzsäure-Lösung (1N) auf pH 3 gebracht und erneut mit Essigsäureethylester extrahiert. Die vereinigten, organischen Phasen wurden getrocknet (Natriumsulfat), filtriert und das Lösungsmittel unter reduziertem Druck entfernt. Ausbeute: 184 mg (87% d. Th.)
LC/MS [Methode 1]: Rₜ = 0.68 min; MS (ESIpos): m/z = 361 (M+H)⁺,
¹H-NMR (400 MHz, DMSO-d₆): δ [ppm] = 13.1 (br. s, 1H), 7.80/7.79 (2x s, 1H), 7.78-7.73 (m, 1H), 7.45 (dd, 1H), 7.31 (d, 1H), 7.28-7.26 (m, 1H), 6.30 (2x s, 1H), 5.13/5.03 (2x dd, 1H), 3.45-3.23 (m, 2H), 2.98-2.89 (m, 2H), 2.18-2.05 (m, 2H), 0.84/0.83 (2x t, 3H).

### Beispiel 6.1G

### 4-{[2-(11-Chlor-2,5-dioxo-5,6,7,8-tetrahydropyrido[3,4-e][3]benzazocin-3(2H)-yl)butanoyl]-amino}benzoesäure-tert.-butylester (Stereoisomerengemisch)

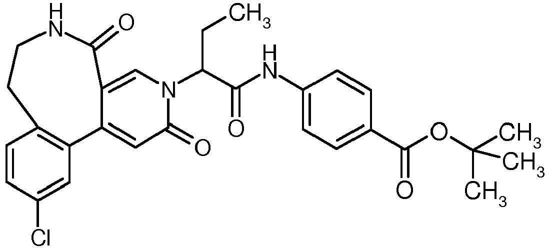

184 mg (510 µmol) 2-(11-Chlor-2,5-dioxo-5,6,7,8-tetrahydropyrido[3,4-*e*][3]benzazocin-3(2*H*)-yl)butansäure (Stereoisomerengemisch) wurden in 5 ml Dimethylformamid gelöst und mit 98.6 mg (510 µmol) 4-Aminobenzoesäure-*tert*.-butylester und 72.5 mg (510 µmol) Oxyma versetzt. Anschließend wurden 79.5 µl (510 µmol) Di-*iso*-propylcarbodiimid zugetropft und die Reaktionslösung über Nacht bei 40°C geschüttelt. Das Lösungsmittel wurde unter reduziertem Druck entfernt und der Rückstand mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 156 mg (97% Reinheit, 55% d. Th.)
LC/MS [Methode 1]: Rₜ = 1.07 min; MS (ESIpos): m/z = 536 (M+H)⁺,
¹H-NMR (400 MHz, DMSO-d₆): δ [ppm] = 10.8 (s, 1H), 7.90-7.71 (m, 6H), 7.48-7.43 (m, 1H), 7.31 (d, 1H), 7.29-7.26 (m, 1H), 6.34/6.33 (2x s, 1H), 5.63-5.55 (m, 1H), 3.48-3.23 (m, 2H), 2.99-2.90 (m, 2H), 2.24-2.03 (m, 2H), 1.54 (s, 9H), 0.91/0.90 (2x t, 3H).

### Beispiel 7.1A

### 2-(1,3-Dioxo-1,3-dihydro-2H-isoindol-2-yl)-4-methylpentansäure-tert.-butylester (Racemat)

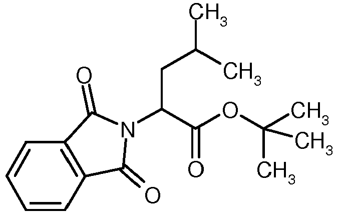

10.0 g (38.3 mmol) 2-(1,3-Dioxo-1,3-dihydro-2*H*-isoindol-2-yl)-4-methylpentansäure (Racemat) wurden in einer Mischung aus 6 ml THF und 6 ml *tert*.-Butanol gelöst. Anschließend wurden 8.77 g (40.2 mmol) Di-*tert*.-butyldicarbonat portionsweise und 1.40 g (11.5 mmol) 4-Dimethylaminopyridin zugegeben. Es wurde über Nacht bei Raumtemperatur nachgerührt. Die Reaktionsmischung wurde mit 150 ml Essigsäureethylester verdünnt, mit 100 ml gesättigter, wässriger Ammoniumchlorid-Lösung, 100 ml Wasser und 100 ml gesättigter, wässriger Natriumchlorid-Lösung gewaschen. Die organische Phase wurde über Natriumsulfat getrocknet, filtriert und das Lösungsmittel unter reduziertem Druck entfernt. Der Rückstand wurde mittels Flash-Chromatographie (Kieselgel-50, Cyclohexan-Essigsäureethylester-Gemisch) aufgereinigt. Ausbeute: 11.4 g (96% Reinheit, 90% d. Th.)
LC/MS [Methode 1]: Rₜ = 1.27 min; MS (ESIpos): m/z = 318 (M+H)⁺,
¹H-NMR (400 MHz, DMSO-d₆): δ [ppm] = 7.96-7.87 (m, 4H), 4.77 (dd, 1H), 2.18-2.08 (m, 1H), 1.87-1.78 (m, 1H), 1.50-1.39 (m, 1H), 1.34 (s, 9H), 0.89-0.85 (m, 6H).

### Beispiel 7.1B

### tert.-Butyl-leucinat (Racemat)

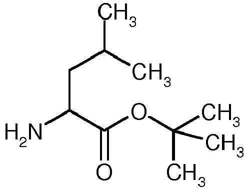

Zu einer Lösung von 11.3 g (35.6 mmol) 2-(1,3-Dioxo-1,3-dihydro-2*H*-isoindol-2-yl)-4-methylpentansäure-*tert*.-butylester (Racemat) in *tert*.-Butanol wurde 4.33 ml (71.2 mmol) Hydrazinhydrat (80%ig in Wasser) gegeben, und es wurde für 1 min zum Sieden erhitzt. Anschließend wurde mit Wasser und Natriumcarbonat solange gerührt, bis der gebildete Niederschlag in Lösung ging. Es wurde dreimal mit Diethylether extrahiert und anschließend mit Salzsäure (5M in Cyclopentylmethylether) auf pH 5 angesäuert. Es wurde noch einmal mit Diethylether extrahiert. Die vereinigten Diethylether-Phasen enthielten kein Produkt und wurden verworfen. Die wässrige Phase wurde mit Essigsäureethylester und Wasser verdünnt. Die Phasen wurden getrennt und die wässrige Phase mit Essigsäureethylester extrahiert. Die vereinigten, organischen Phasen wurden über Magnesiumsulfat getrocknet, filtriert und das Lösungsmittel unter reduziertem Druck (100 mbar, 35°C Wasserbadtemperatur) entfernt. Ausbeute: 5.65 g (39% Reinheit, 33% d. Th.)
GC/MS [Methode 9]: Rₜ = 2.81 min; MS: m/z = 187 (M)⁺.

### Beispiel 7.1C

### 1-(1-tert.-Butoxy-4-methyl-1-oxopentan-2-yl)-4-hydroxy-6-oxo-1,6-dihydropyridin-3-carbonsäureethylester (Racemat)

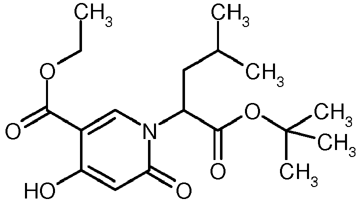

462 µl (2.54 mmol) 3-Oxopentandicarbonsäurediethylester und 581 µl (3.56 mmol, 1.4 eq.) Diethoxymethylacetat wurden 2.5 h bei 100°C gerührt. Nach Abkühlen auf Raumtemperatur wurde dreimal mit Toluol koevaporiert und im Hochvakuum getrocknet. Der Rückstand wurde in 2.5 ml Ethanol aufgenommen, unter Eiskühlung mit einer Lösung von 500 mg (2.67 mmol, 1.05 eq.) *tert.-*Butyl-leucinat (Racemat) in Ethanol versetzt und über Nacht bei Raumtemperatur gerührt. Anschließend wurde das Reaktionsgemisch mit 949 µl (2.54 mmol, 1.0 eq.) einer Natriumethylat-Lösung (21%ig in Ethanol) versetzt und über Nacht bei Raumtemperatur gerührt. Die Reaktion wurde durch Zugabe von gesättigter, wässriger Ammoniumchlorid-Lösung und Essigsäureethylester beendet. Die Phasen wurden getrennt und die wässrige Phase dreimal mit Essigsäureethylester extrahiert. Die vereinigten, organischen Phasen wurden mit gesättigter, wässriger Natriumchlorid-Lösung gewaschen, über Magnesiumsulfat getrocknet, filtriert und das Lösungsmittel unter reduziertem Druck entfernt. Der Rückstand wurde mittels Flash-Chromatographie (Kieselgel-50, Cyclohexan-Essigsäureethylester-Gemisch) und präparativer HPLC (*iso*-Hexan-*iso*-Propanol-Gemisch) aufgereinigt. Ausbeute: 154 mg (90% Reinheit, 15% d. Th.)
LC/MS [Methode 1]: Rₜ = 1.17 min; MS (ESIpos): m/z = 354 (M+H)⁺,
¹H-NMR (400 MHz, DMSO-d₆): δ [ppm] = 10.9 (s, 1H), 8.33 (s, 1H), 5.71 (s, 1H), 5.22 (dd, 1H), 4.29 (q, 2H), 2.02-1.93 (m, 1H), 1.88-1.79 (m, 1H), 1.38 (s, 9H), 1.38-1.27 (m, 1H), 1.30 (t, 3H), 0.88-0.83 (m, 6H).

### Beispiel 7.1D

### 1-(1-tert.-Butoxy-4-methyl-1-oxopentan-2-yl)-6-oxo-4-{[(trifluormethyl)sulfonyl]oxy}-1,6-dihydropyridin-3-carbonsäureethylester (Racemat)

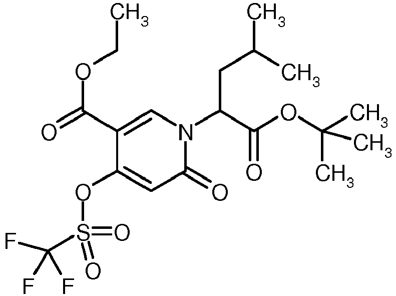

Eine Lösung von 140 mg (396 µmol) 1-(1-*tert*.-Butoxy-4-methyl-1-oxopentan-2-yl)-4-hydroxy-6-oxo-1,6-dihydropyridin-3-carbonsäureethylester (Racemat) in 1 ml Dichlormethan wurde unter Argon bei -78°C mit 110 µl (792 µmol, 2.0 eq.) Triethylamin und anschließend portionsweise mit 262 mg (634 µmol, 1.6 eq.) *N*-(4-*tert*.-Butylphenyl)-1,1,1-trifluor-*N*-[(trifluormethyl)sulfonyl]-methansulfonamid versetzt. Das Reaktionsgemisch wurde auf Raumtemperatur kommen gelassen, über Nacht bei Raumtemperatur gerührt und anschließend das Lösungsmittel unter reduziertem Druck entfernt. Der Rückstand wurde mittels Flash-Chromatographie (Kieselgel-50, Cyclohexan-Essigsäureethylester-Gemisch) aufgereinigt. Ausbeute: 169 mg (94% Reinheit, 82% d. Th.)
LC/MS [Methode 1]: Rₜ = 1.33 min; MS (ESIpos): m/z = 486 (M+H)⁺,
¹H-NMR (400 MHz, DMSO-d₆): δ [ppm] = 8.67 (s, 1H), 6.70 (s, 1H), 5.29 (dd, 1H), 4.32 (q, 2H), 2.08-2.00 (m, 1H), 1.96-1.88 (m, 1H), 1.37 (s, 9H), 1.43-1.33 (m, 1H), 1.31 (t, 3H), 0.89 (d, 3H), 0.87 (d, 3H).

### Beispiel 7.1E

### 4-(2-{2-[(tert.-Butoxycarbonyl)amino]ethyl}-5-chlorphenyl)-1-(1-tert.-butoxy-4-methyl-1-oxopentan-2-yl)-6-oxo-1,6-dihydropyridin-3-carbonsäureethylester (Stereoisomerengemisch)

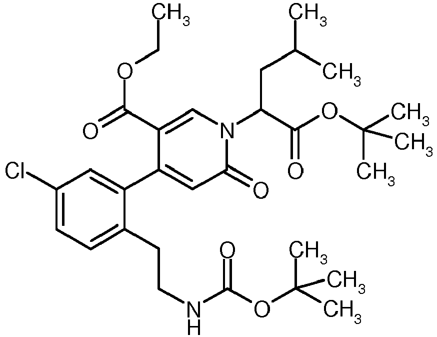

115 mg (834 µmol) Kaliumcarbonat wurden im Reaktionsgefäß getrocknet und anschließend 135 mg (278 µmol) 1-(1-*tert*.-Butoxy-4-methyl-1-oxopentan-2-yl)-6-oxo-4-{[(trifluormethyl)-sulfonyl]oxy}-1,6-dihydropyridin-3-carbonsäureethylester (Racemat), 122 mg (320 µmol) *tert.-*Butyl-{2-[4-chlor-2-(4,4,5,5-tetramethyl-1,3,2-dioxaborolan-2-yl)phenyl]ethyl}carbamat und 2.6 ml 1,4-Dioxan zugegeben. Das Reaktionsgemisch wurde entgast, mit 32 mg (28 µ mol) Tetrakis(triphenylphosphin)palladium(0) versetzt und 6 h bei 110°C, über Nacht bei Raumtemperatur und weitere 2 h bei 110°C gerührt. Es wurde auf Raumtemperatur abgekühlt, über Celite filtriert und das Lösungsmittel unter reduziertem Druck entfernt. Der Rückstand wurde mittels Flash-Chromatographie (Kieselgel-50, Cyclohexan-Essigsäureethylester-Gemisch) aufgereinigt. Ausbeute: 106 mg (93% Reinheit, 60% d. Th.)
LC/MS [Methode 1]: Rₜ = 1.46 min; MS (ESIpos): m/z = 591 (M+H)⁺.

### Beispiel 7.1F

### 2-{4-[2-(2-Aminoethyl)-5-chlorphenyl]-5-(ethoxycarbonyl)-2-oxopyridin-1(2H)-yl}-4-methylpentansäure (Stereoisomerengemisch)

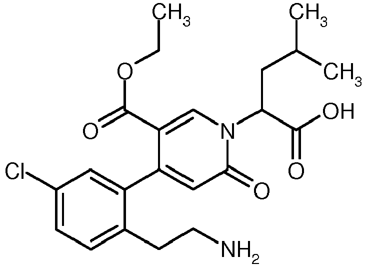

85.0 mg (144 µmol) 4-(2-{2-[(*tert*.-Butoxycarbonyl)amino]ethyl}-5-chlorphenyl)-1-(1-*tert*.-butoxy-4-methyl-1-oxopentan-2-yl)-6-oxo-1,6-dihydropyridin-3-carbonsäureethylester (Stereoisomerengemisch) wurden in 1.5 ml Dichlormethan vorgelegt und mit 415 µl (5.39 mmol) Trifluoressigsäure versetzt. Es wurde für 4 h bei Raumtemperatur nachgerührt. Anschließend wurde das Lösungsmittel unter reduziertem Druck entfernt und der Rückstand zweimal mit 20 ml Toluol koevaporiert. Ausbeute: 328 mg (95% Reinheit, quantitativ)
LC/MS [Methode 1]: Rₜ = 0.79 min; MS (ESIpos): m/z = 435 (M+H)⁺.

### Beispiel 7.1G

### 2-(11-Chlor-2,5-dioxo-5,6,7,8-tetrahydropyrido[3,4-e][3]benzazocin-3(2H)-yl)-4-methylpentansäure (Stereoisomerengemisch)

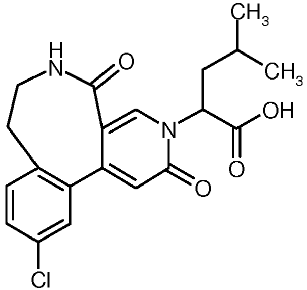

Eine Lösung von 66.0 mg (152 µmol) 2-{4-[2-(2-Aminoethyl)-5-chlorphenyl]-5-(ethoxycarbonyl)-2-oxopyridin-1(2*H*)-yl}-4-methylpentansäure (Stereoisomerengemisch) in 1.5 ml Tetrahydrofuran wurde unter Argon zu aktiviertem Molsieb 3Å (über Nacht bei 200°C im Trockenschrank getrocknet) gegeben und 2 h bei RT gerührt. Anschließend wurde das Reaktionsgemisch unter Argon bei RT mit 113 µl (304 µmol, 2 eq.) einer Natriumethylat-Lösung (21%ig in Ethanol, zuvor 2 h über aktiviertem Molsieb 3Å getrocknet) versetzt und 40 min bei Raumtemperatur gerührt. Es wurden noch einmal 55.0 µl (152 µmol) einer Natriumethylat-Lösung (21%ig in Ethanol, zuvor 2 h über aktiviertem Molsieb 3Å getrocknet) hinzugegeben und über Nacht bei Raumtemperatur gerührt. Nach Verdünnen mit Essigsäureethylester wurde das Reaktionsgemisch mit gesättigter, wässriger Ammoniumchlorid-Lösung versetzt. Die Phasen wurden getrennt und die wässrige Phase dreimal mit Essigsäureethylester extrahiert. Die wässrige Phase wurde anschließend mit wässriger Salzsäure-Lösung (1N) auf pH 3 gebracht und erneut mit Essigsäureethylester extrahiert. Die vereinigten, organischen Phasen wurden getrocknet (Natriumsulfat), filtriert und das Lösungsmittel unter reduziertem Druck entfernt. Ausbeute: 31.3 mg (96% Reinheit, 51% d. Th.)
LC/MS [Methode 1]: Rₜ = 0.78/0.80 min; MS (ESIpos): m/z = 389 (M+H)⁺,
¹H-NMR (400 MHz, DMSO-d₆): δ [ppm] = 13.2 (br. s, 1H), 7.82 (2x s, 1H), 7.80-7.74 (m, 1H), 7.45 (dd, 1H), 7.33-7.26 (m, 2H), 6.30 (s, 1H), 5.39-5.27 (m, 1H), 3.42-3.23 (m, 2H), 3.00-2.87 (m, 2H), 2.19-2.09 (m, 1H), 1.94-1.86 (m, 1H), 1.44-1.33 (m, 1H), 0.92-0.87 (m, 6H).

### Beispiel 7.1H

### 4-{[2-(11-Chlor-2,5-dioxo-5,6,7,8-tetrahydropyrido[3,4-e][3]benzazocin-3(2H)-yl)-4-methylpentanoyl]amino}benzoesäure-tert.-butylester (Stereoisomerengemisch)

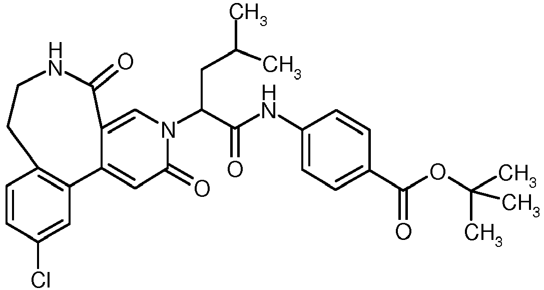

15 mg (39 µmol) 2-(11-Chlor-2,5-dioxo-5,6,7,8-tetrahydropyrido[3,4-*e*][3]benzazocin-3(2*H*)-yl)-4-methylpentansäure (Stereoisomerengemisch) wurden in 400 µl Dimethylformamid gelöst und mit 7.5 mg (39µmol) 4-Aminobenzoesäure-*tert*.-butylester und 5.5 mg (39µmol) Oxyma versetzt. 6.0 µl (39 µmol) Di-*iso*-propylcarbodiimid wurden zugetropft und die Reaktionslösung über Nacht bei 40°C geschüttelt. Anschließend wurden weitere 1.5 mg (8.0 µmol) 4-Aminobenzoesäure-*tert*.-butylester und 1.2 µl (8.0 µmol) Di-*iso*-propylcarbodiimid zugegeben und für weitere 3 h bei 40°C geschüttelt. Das Lösungsmittel wurde unter reduziertem Druck entfernt und der Rückstand mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 13.9 mg (64% d. Th.)
LC/MS [Methode 1]: Rₜ = 1.16 min; MS (ESIpos): m/z = 564 (M+H)⁺,
¹H-NMR (400 MHz, DMSO-d₆): δ [ppm] = 10.9 (2x s, 1H), 7.91-7.72 (m, 6H), 7.48-7.43 (m, 1H), 7.33-7.27 (m, 2H), 6.34/6.33 (2x s, 1H), 5.87-5.80 (m, 1H), 3.47-3.23 (m, 2H), 3.02-2.87 (m, 2H), 2.19-2.09 (m, 1H), 1.93-1.83 (m, 1H), 1.54 (s, 9H), 1.49-1.38 (m, 1H), 0.96-0.91 (m, 6H).

### Beispiel 8.1A

### 4-[2-(11-[(tert.-Butoxycarbonyl)aminolcyclopropyl}methyl)-5-chlorphenyl]-1-(1-tert.-butoxy-1-oxobutan-2-yl)-6-oxo-1,6-dihydropyridin-3-carbonsäureethylester (Stereoisomerengemisch)

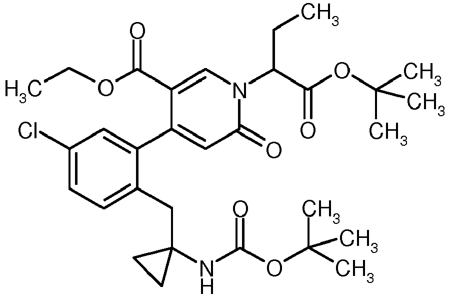

629 mg (4.55 mmol) Kaliumcarbonat wurden im Reaktionsgefäß getrocknet und anschließend 672mg (1.47 mmol) 1-(1-*tert*.-Butoxy-1-oxobutan-2-yl)-6-oxo-4-{[(trifluormethyl)sulfonyl]oxy}-1,6-dihydropyridin-3-carbonsäureethylester (Racemat), 800mg (1.47 mmol) [2-({1-[(*tert*.-Butoxycarbonyl)amino]cyclopropyl}methyl)-5-chlorphenyl]borsäure (60% Reinheit) und 14.7 ml 1,4-Dioxan zugegeben. Das Reaktionsgemisch wurde entgast, mit 170 mg (147 µmol) Tetrakis(triphenylphosphin)palladium(0) versetzt und über Nacht bei 110°C geschüttelt. Es wurde auf Raumtemperatur abgekühlt, durch einen Feinfilter filtriert und das Lösungsmittel unter reduziertem Druck entfernt. Der Rückstand mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 249 mg (29% d. Th.)
LC/MS [Methode 1]: Rₜ = 1.35 min; MS (ESIpos): m/z = 589 (M+H)⁺.

### Beispiel 8.1B

### 2-[4-{2-[(1-Aminocyclopropyl)methyl] -5-chlorpheny1}-5-(ethoxycarbonyl)-2-oxopyridin-1(2H)-yl]butansäure (Stereoisomerengemisch)

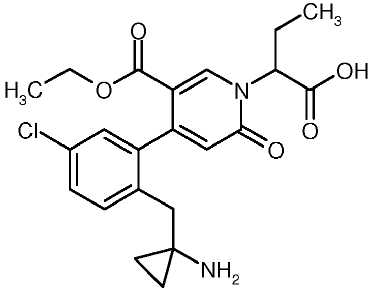

249 mg (423 µmol) 4-[2-({1-[(*tert*.-Butoxycarbonyl)amino]cyclopropyl}methyl)-5-chlorphenyl]-1-(1-*tert*.-butoxy-1-oxobutan-2-yl)-6-oxo-1,6-dihydropyridin-3-carbonsäureethylester (Stereoisomerengemisch) wurden in 4 ml Dichlormethan vorgelegt und mit 651 µl (8.45 mmol) Trifluoressigsäure versetzt. Es wurde für 2 h bei Raumtemperatur nachgerührt. Anschließend wurde das Lösungsmittel unter reduziertem Druck entfernt und der Rückstand mehrmals mit Toluol koevaporiert. Ausbeute: 263 mg (94% Reinheit, quantitativ)
LC/MS [Methode 1]: Rₜ = 0.68 min; MS (ESIpos): m/z = 433 (M+H)⁺.

### Beispiel 8.1C

### 2-(11'-Chlor-2',5'-dioxo-2',5',6',8'-tetrahydro-3'H-spiro[cyclopropan-1,7'-pyrido[3,4-e][3]benzazocin]-3'-yl)butansäure (Stereoisomerengemisch)

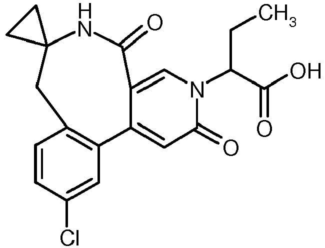

Eine Lösung von 263 mg (553 µmol) 2-[4-{2-[(1-Aminocyclopropyl)methyl]-5-chlorphenyl}-5-(ethoxycarbonyl)-2-oxopyridin-1(2*H*)-yl]butansäure (Stereoisomerengemisch) in 5 ml Tetrahydrofuran wurde unter Argon zu aktiviertem Molsieb 3Å (über Nacht bei 200°C im Trockenschrank getrocknet) gegeben und 2 h bei RT gerührt. Anschließend wurde das Reaktionsgemisch unter Argon bei RT mit 4.13 ml (11.1 mmol, 20 eq.) einer Natriumethylat-Lösung (21%ig in Ethanol, zuvor 2 h über aktiviertem Molsieb 3Å getrocknet) versetzt und 45 min bei Raumtemperatur gerührt. Nach Verdünnen mit Essigsäureethylester wurde das Reaktionsgemisch mit gesättigter, wässriger Ammoniumchlorid-Lösung versetzt und mit wässriger Salzsäure-Lösung (1N) auf pH 3 gebracht und erneut mit Essigsäureethylester extrahiert. Die vereinigten, organischen Phasen wurden getrocknet (Natriumsulfat), filtriert und das Lösungsmittel unter reduziertem Druck entfernt. Der Rückstand wurde nochmals in 50 ml Essigsäureethylester aufgenommen und über Celite filtriert. Das Filtrat wurde zweimal mit 25 ml wässriger Salzsäure (0.5M) und 20 ml wässriger, gesättigter Natriumchlorid-Lösung gewaschen, über Magnesiumsulfat getrocknet, filtriert und das Lösungsmittel unter reduziertem Druck entfernt. Ausbeute: 124 mg (94% Reinheit, 54% d. Th.)
LC/MS [Methode 1]: Rₜ = 0.72/0.74 min; MS (ESIpos): m/z = 387 (M+H)⁺.

### Beispiel 8.1D

### 4-{[2-(11'-Chlor-2',5'-dioxo-2',5',6',8'-tetrahydro-3'H-spiro[cyclopropan-1,7'-pyrido[3,4-e][3]benzazocin]-3'-yl)butanoyl]amino}benzoesäure-tert.-butylester (Stereoisomerengemisch)

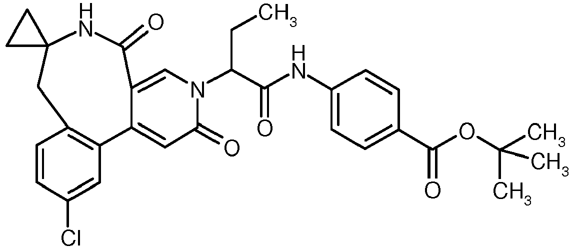

90.0 mg (219 µmol) 2-(11'-Chlor-2',5'-dioxo-2',5',6',8'-tetrahydro-3'*H*-spiro[cyclopropan-1,7'-pyrido[3,4-*e*][3]benzazocin]-3'-yl)butansäure (Stereoisomerengemisch) wurden in 2.2 ml Dimethylformamid gelöst und mit 42.3 mg (219 µmol) 4-Aminobenzoesäure-*tert*.-butylester und 21.8 mg (153 µmol) Oxyma versetzt. Anschließend wurden 34.1µl (219 µmol) Di-*iso-*propylcarbodiimid zugetropft und die Reaktionslösung über Nacht bei 40°C geschüttelt. Das Lösungsmittel wurde unter reduziertem Druck entfernt und der Rückstand mittels präparativer HPLC [Säule: Chromatorex C18, 10µm, 125mm x 30mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 87.2 mg (71% d. Th.)
LC/MS [Methode 1]: Rₜ = 1.08 min; MS (ESIpos): m/z = 562 (M+H)⁺,
¹H-NMR (400 MHz, DMSO-d₆): δ [ppm] = 10.8 (2x s, 1H), 8.15/8.09 (2x s, 1H), 7.90-7.68 (m, 5H), 7.45 (dd, 1H), 7.33-7.27 (m, 2H), 6.33/6.32 (2x s, 1H), 5.72/5.62 (2x dd, 1H), 3.28-3.16 (m, 1H), 2.74-2.63 (m, 1H), 2.23-2.05 (m, 2H), 1.54 (s, 9H), 1.04-0.87 (m, 4H), 0.80-0.65 (m, 3H).

### Beispiel 9.1A

### tert.-Butyl-N-(diphenylmethylen)-O-methylhomoserinat (Racemat)

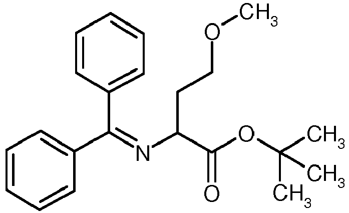

Eine Lösung von 4.43 g (15.0 mmol) *tert*.-Butyl-*N*-(diphenylmethylen)glycinat in 150 ml Tetrahydrofuran wurde unter Argon bei -78°C tropfenweise mit 19.5 ml (1.0M in THF, 19.5 mmol, 1.3 eq.) Bis-(trimethylsilyl)-lithiumamid versetzt, 15 min gerührt und anschließend tropfenweise mit 5.85 g (80% Reinheit, 22.5 mmol, 1.5 eq.) 2-Methoxyethyl-trifluormethansulfonat versetzt. Das Reaktionsgemisch wurde weitere 15 min bei -78°C gerührt, auf RT kommen gelassen, 30 min nachgerührt und anschließend mit Wasser gequencht. Nach Zugabe von Essigsäureethylester und Phasentrennung wurde die wässrige Phase zweimal mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt. Der Rückstand wurde mittels Flash-Chromatographie (Kieselgel-50, Cyclohexan-Essigsäureethylester-Gemisch) aufgereinigt. Ausbeute: 4.61 g (87% d. Th.)
LC/MS [Methode 1]: Rₜ = 1.17 min; MS (ESIpos): m/z = 354 (M+H)⁺,
¹H-NMR (400 MHz, DMSO-d₆): δ [ppm] = 7.56-7.35 (m, 8H), 7.18-7.11 (m, 2H), 3.92 (dd, 1H), 3.38-3.32 (m, 1H), 3.26-3.17 (m, 1H), 3.12 (s, 3H), 2.10-1.91 (m, 2H), 1.37 (s, 9H).

### Beispiel 9.1B

### tert.-Butyl-O-methylhomoserinat (Racemat)

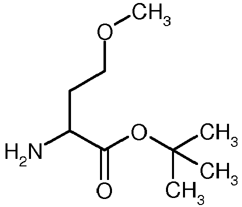

Eine Lösung von 4.61g (13.0 mmol) *tert*.-Butyl-*N*-(diphenylmethylen)-*O*-methylhomoserinat (Racemat) in 150 ml Tetrahydrofuran wurde mit 150 ml einer wässrigen Zitronensäure-Lösung (1M) versetzt und 2 h bei RT gerührt. Anschließend wurde Tetrahydrofuran im Vakuum entfernt. Die resultierende wässrige Lösung wurde vorsichtig mit festem Natriumbicarbonat neutralisiert und zweimal mit Dichlormethan extrahiert. Die vereinigten, organischen Phasen wurden getrocknet (Natriumsulfat), filtriert und am Rotationsverdampfer im Vakuum (>150 mbar bei <25°C Wasserbadtemperatur) eingeengt. Der Rückstand wurde mittels Flash-Chromatographie (Kieselgel-50, Dichlormethan-Methanol-Gemisch) aufgereinigt. Ausbeute: 2.58 g (quant.)
GC/MS [Methode 9]: Rₜ = 3.26 min; MS: m/z = 189 (M)⁺,
¹H-NMR (400 MHz, DMSO-d₆): δ [ppm] = 3.48-3.39 (m, 1H), 3.39-3.33 (m, 1H), 3.25-3.14 (m, 1H), 3.21 (s, 3H), 1.84-1.73 (m, 1H), 1.66 (br. s, 1H), 1.60-1.50 (m, 1H), 1.40 (s, 9H).

### Beispiel 9.1C

### 1-(1-tert.-Butoxy-4-methoxy-1-oxobutan-2-yl)-4-hydroxy-6-oxo-1,6-dihydropyridin-3-carbonsäureethylester (Racemat)

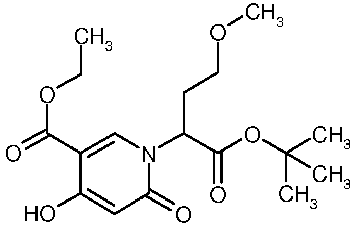

2.35 ml (90% Reinheit, 11.7 mmol) 3-Oxopentandicarbonsäurediethylester und 2.67 ml (16.3 mmol, 1.4 eq.) Diethoxymethylacetat wurden 2.5 h bei 100°C gerührt, nach Abkühlen auf RT dreimal mit Toluol koevaporiert und im Hochvakuum getrocknet. Der Rückstand wurde in 70 ml Ethanol aufgenommen, unter Eiskühlung mit einer Lösung von 2.32 g (12.2 mmol, 1.05 eq.) *tert*.-Butyl-*O*-methylhomoserinat (Racemat) in 20 ml Ethanol versetzt und 1h bei RT gerührt. Anschließend wurde das Reaktionsgemisch mit 4.36 ml (11.7 mmol, 1.0 eq.) einer Natriumethylat-Lösung (21%ig in Ethanol, zuvor über ca. 15 g aktiviertem Molsieb 3Å getrocknet) versetzt, 1h bei RT gerührt und mit gesättigter, wässriger Ammoniumchlorid-Lösung gequencht. Der ausfallende Niederschlag wurde filtriert, mit Essigsäureethylester gewaschen und verworfen. Nach Phasentrennung der vereinigten Filtrate wurde die wässrige Phase dreimal mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt. Der Rückstand wurde mittels Flash-Chromatographie (Kieselgel-50, Cyclohexan-Essigsäureethylester-Gemisch) aufgereinigt. Ausbeute: 2.37 g (56% d. Th.)
LC/MS [Methode 1]: Rₜ = 0.98 min; MS (ESIneg): m/z = 354 (M-H)⁻,
¹H-NMR (400 MHz, DMSO-d₆): δ [ppm] = 10.85 (s, 1H), 8.31 (s, 1H), 5.69 (s, 1H), 5.11 (dd, 1H), 4.36-4.23 (m, 2H), 3.38-3.32 (m, 1H), 3.15 (s, 3H), 3.14-3.07 (m, 1H), 2.31-2.14 (m, 2H), 1.37 (s, 9H), 1.29 (t, 3H).

### Beispiel 9.1D

### 1-(1-tert.-Butoxy-4-methoxy-1-oxobutan-2-yl)-6-oxo-4-{[(trifluormethyl)sulfonyl]oxy}-1,6-dihydropyridin-3-carbonsäureethylester (Racemat)

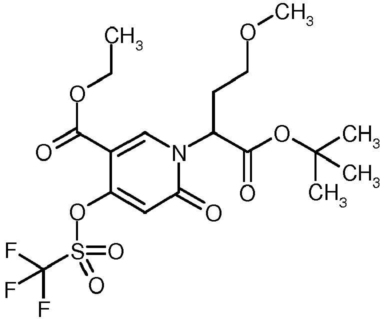

Eine Lösung von 4.49 g (12.4 mmol) 1-(1-*tert*.-Butoxy-4-methoxy-1-oxobutan-2-yl)-4-hydroxy-6-oxo-1,6-dihydropyridin-3-carbonsäureethylester (Racemat) in 50 ml Dichlormethan wurde unter Argon bei -78°C mit 3.45 ml (24.8 mmol, 2.0 eq.) Triethylamin und anschließend portionsweise mit 8.19 g (19.8 mmol, 1.6 eq.) *N*-(4-*tert*.-Butylphenyl)-1,1,1-trifluor-*N*-[(trifluormethyl)sulfonyl]-methansulfonamid versetzt. Das Reaktionsgemisch wurde auf RT kommen gelassen, 6 h bei RT gerührt und anschließend im Vakuum eingeengt. Der Rückstand wurde mittels Flash-Chromatographie (Kieselgel-50, Cyclohexan-Essigsäureethylester-Gemisch) aufgereinigt. Ausbeute: 4.85 g (76% d. Th.)
LC/MS [Methode 1]: Rₜ = 1.23 min; MS (ESIpos): m/z = 488 (M+H)⁺,
¹H-NMR (400 MHz, DMSO-d₆): δ [ppm] = 8.65 (s, 1H), 6.66 (s, 1H), 5.27 (t, 1H), 4.38-4.25 (m, 2H), 3.44-3.36 (m, 1H), 3.23-3.14 (m, 1H), 3.11 (s, 3H), 2.35-2.24 (m, 2H), 1.36 (s, 9H), 1.30 (t, 3H).

### Beispiel 9.1E

### 4-[2-({1-[(tert.-Butoxycarbonyl)amino]cyclopropyl}methyl)-5-chlorphenyl]-1-(1-tert.-butoxy-4-methoxy-1-oxobutan-2-yl)-6-oxo-1,6-dihydropyridin-3-carbonsäureethylester (Stereoisomerengemisch)

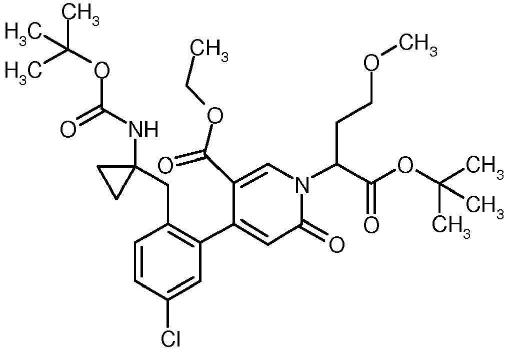

Ein Gemisch aus 4.60 g (9.0 mmol) 1-(1-*tert*.-Butoxy-4-methoxy-1-oxobutan-2-yl)-6-oxo-4-{[(trifluormethyl)sulfonyl]oxy}-1,6-dihydropyridin-3-carbonsäureethylester (Racemat), 4.14 g (10.3 mmol, 1.15 eq.) [2-({1-[(*tert*.-Butoxycarbonyl)amino]cyclopropyl}methyl)-5-chlorphenyl]borsäure und 3.72 g (26.9 mmol, 3.0 eq.) Kaliumcarbonat wurde im Hochvakuum getrocknet, unter Argon gesetzt, mit 100 ml Dioxan versetzt und 10 min mit Argon durchströmt. Anschließend wurde das Reaktionsgemisch mit 732mg (0.9 mmol, 0.1 eq.) [1,1-Bis-(diphenylphosphino)-ferrocen]-palladium(II)chlorid-Monodichlormethan-Addukt versetzt und 1h bei 80°C Ölbadtemperatur gerührt. Nach Abkühlen auf RT wurde das Reaktionsgemisch mit einem vorherigen, analog durchgeführten Testansatz von 200 mg (0.39 mmol) 1-(1-*tert*.-Butoxy-4-methoxy-1-oxobutan-2-yl)-6-oxo-4-{[(trifluormethyl)-sulfonyl]oxy}-1,6-dihydropyridin-3-carbonsäureethylester (Racemat) vereinigt und über Celite filtriert. Nach Waschen mit Dioxan wurden die vereinigten Filtrate im Vakuum eingeengt. Der Rückstand wurde in Wasser aufgenommen und zweimal mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt. Der Rückstand wurde mittels Flash-Chromatographie (Kieselgel-50, Cyclohexan-Essigsäureethylester-Gemisch) aufgereinigt. Ausbeute: 4.84 g (89% Reinheit, 74% d. Th. bezogen auf beide Ansätze)
LC/MS [Methode 1]: Rₜ = 1.29 min; MS (ESIpos): m/z = 619 (M+H)⁺.

### Beispiel 9.1F

### 2-[4-{2-[(1-Aminocyclopropyl)methyl] -5-chlorphenyl}-5-(ethoxycarbonyl)-2-oxopyridin-1(2H)-yl]-4-methoxybutansäure (Stereoisomerengemisch)

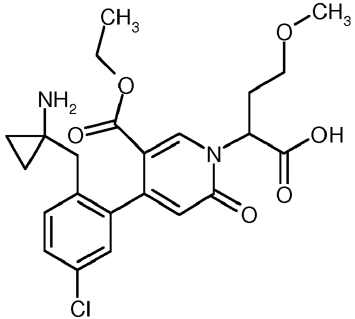

Eine Lösung von 4.84 g (89% Reinheit, 7.0 mmol) 4-[2-({1-[(*tert*.-Butoxycarbonyl)amino]-cyclopropyl}methyl)-5-chlorphenyl]-1-(1-*tert*.-butoxy-4-methoxy-1-oxobutan-2-yl)-6-oxo-1,6-dihydropyridin-3-carbonsäureethylester (Stereoisomerengemisch) in 50 ml Dichlormethan wurde unter Argon bei Eisbadkühlung tropfenweise mit 10.7 ml (139 mmol, 20 eq.) Trifluoressigsäure versetzt, 5 min gerührt, auf RT kommen gelassen, 6.5 h bei RT gerührt und anschließend im Vakuum eingeengt. Der Rückstand wurde zweimal mit Toluol und einmal mit Dichlormethan koevaporiert, im Hochvakuum getrocknet und ohne weitere Reinigung umgesetzt. Ausbeute: 4.45 g (80% Reinheit, quant.)
LC/MS [Methode 1]: Rₜ = 0.62 min; MS (ESIpos): m/z = 463 (M+H)⁺.

### Beispiel 9.1G

### 2-(11'-Chlor-2',5'-dioxo-2',5',6',8'-tetrahydro-3'H-spiro[cyclopropan-1,7'-pyrido[3,4-e][3]benzazocin]-3'-yl)-4-methoxybutansäure (Stereoisomerengemisch)

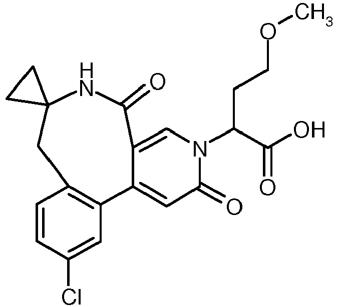

Eine Lösung von 4.37 g (80% Reinheit, 7.6 mmol) 2-[4-{2-[(1-Aminocyclopropyl)methyl]-5-chlorphenyl}-5-(ethoxycarbonyl)-2-oxopyridin-1(2*H*)-yl]-4-methoxybutansäure (Stereoisomerengemisch) in 100 ml Tetrahydrofuran wurde unter Argon zu ca. 15 g aktiviertem Molsieb 3Å (über Nacht bei 200°C im Trockenschrank getrocknet) gegeben und 2 h bei RT gerührt. Anschließend wurde das Reaktionsgemisch unter Argon bei RT mit 45 ml (151 mmol, 20 eq.) einer Natriumethylat-Lösung (21%ig in Ethanol, zuvor 2 h über ca. 15 g aktiviertem Molsieb 3Å getrocknet) versetzt und 75 min bei RT gerührt. Nach Verdünnen mit Essigsäureethylester wurde das Reaktionsgemisch vom Molsieb dekantiert, mit gesättigter, wässriger Ammoniumchlorid-Lösung versetzt und anschließend mit wässriger Salzsäure-Lösung (1N) auf pH 3 gebracht. Nach Phasentrennung wurde die wässrige Phase dreimal mit Essigsäureethylester extrahiert. Die vereinigten, organischen Phasen wurden getrocknet (Natriumsulfat), filtriert und im Vakuum eingeengt. Der Rückstand wurde mittels Flash-Chromatographie (Kieselgel-50, Dichlormethan-Methanol-Gemisch) aufgereinigt. Ausbeute: 1.05 g (33% d. Th.)
LC/MS [Methode 1]: Rₜ = 0.73 min; MS (ESIpos): m/z = 417 (M+H)⁺,
¹H-NMR (400 MHz, DMSO-d₆): δ [ppm] = 13.17-12.98 (m, 1H), 8.07/8.05 (2x s, 1H), 7.74/7.70 (2x s, 1H), 7.48-7.42 (m, 1H), 7.34-7.25 (m, 2H), 6.28 (s, 1H), 5.32/5.20 (2x t, 1H), 3.42-3.34 (m, 1H), 3.24-3.13 (m, 2H), 3.21/3.19 (2x s, 3H), 2.70 (dd, 1H), 2.41-2.30 (m, 2H), 1.01-0.92 (m, 1H), 0.80-0.72 (m, 1H), 0.72-0.63 (m, 2H).

### Beispiel 9.1H

### 4-{[2-(11'-Chlor-2',5'-dioxo-2',5',6',8'-tetrahydro-3'H-spiro[cyclopropan-1,7'-pyrido[3,4-e] [3]benzazocin]-3'-yl)-4-methoxybutanoyl]amino}benzoesäure-tert.-butylester (Stereoisomerengemisch)

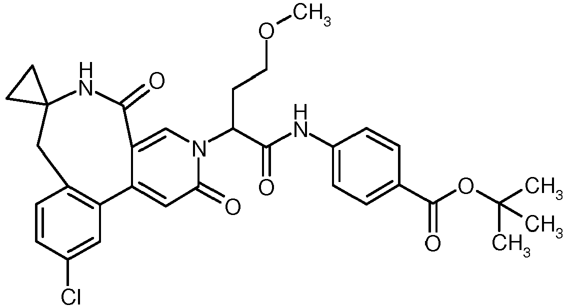

Gemäß der allgemeinen Methode 2A wurden 30 mg (71 µmol) 2-(11'-Chlor-2',5'-dioxo-2',5',6',8'-tetrahydro-3'*H*-spiro[cyclopropan-1,7'-pyrido[3,4-*e*][3]benzazocin]-3'-yl)-4-methoxybutansäure (Stereoisomerengemisch) mit 18 mg (93 µmol, 1.3 eq.) 4-Aminobenzoesäure-*tert*.-butylester umgesetzt. Nach wässriger Aufarbeitung wurde das Rohprodukt mittels präparativer RP-HPLC (Reprosil C18, Wasser-Acetonitril-Gradient) aufgereinigt. Ausbeute: 21 mg (49% d. Th.)
LC/MS [Methode 1]: Rₜ = 1.11 min; MS (ESIpos): m/z = 592 (M+H)⁺,
¹H-NMR (400 MHz, DMSO-d₆): δ [ppm] = 10.83/10.76 (2x s, 1H), 8.14/8.08 (2x s, 1H), 7.90-7.70 (2x m, 5H), 7.48-7.43 (m, 1H), 7.31 (d, 1H), 7.30-7.26 (m, 1H), 6.31 (s, 1H), 5.87/5.76 (t/dd, 1H), 3.45-3.36 (m, 1H), 3.3-3.15 (m, 2H), 3.23/3.20 (2x s, 3H), 2.75-2.63 (dd, 1H), 2.46-2.28 (m, 2H), 1.53 (s, 9H), 1.04-0.93 (m, 1H), 0.82-0.72 (m, 2H), 0.72-0.63 (m, 1H).

### Beispiel 9.2A

### 6-{[2-(11'-Chlor-2',5'-dioxo-2',5',6',8'-tetrahydro-3'H-spiro[cyclopropan-1,7'-pyrido[3,4-e][3]benzazocin]-3'-yl)-4-methoxybutanoyl]amino}-2-methyl-3-oxo-2,3-dihydro-1H-indazol-1-carbonsäure-tert.-butylester (Stereoisomerengemisch)

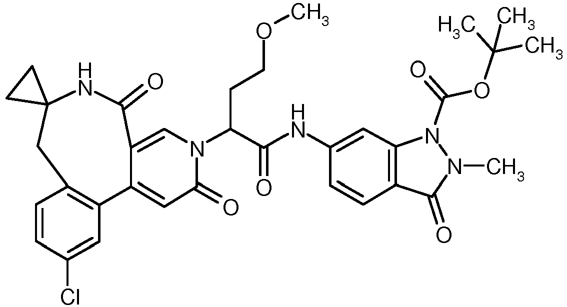

Gemäß der allgemeinen Methode 1A wurden 70 mg (168 µmοl) 2-(11'-Chlor-2',5'-dioxo-2',5',6',8'-tetrahydro-3'*H*-spiro[cyclopropan-1,1'-pyrido[3,4-*e*][3]benzazocin]-3'-yl)-4-methoxybutansäure (Stereoisomerengemisch) mit 51 mg (185 µmol, 1.1 eq.) 6-Amino-2-methyl-3-oxo-2,3-dihydro-1*H-*indazol-1-carbonsäure-*tert*.-butylester umgesetzt. Nach wässriger Aufarbeitung wurde das Rohprodukt mittels präparativer RP-HPLC (Reprosil C18, Wasser-Acetonitril-Gradient) aufgereinigt. Ausbeute: 81 mg (73% d. Th.)
LC/MS [Methode 1]: Rₜ = 0.95 min; MS (ESIpos): m/z = 662 (M+H)⁺.

### Beispiel 9.3A

### 5-{[2-(11'-Chlor-2',5'-dioxo-2',5',6',8'-tetrahydro-3'H-spiro[cyclopropan-1,7'-pyrido[3,4-e][3]benzazocin]-3'-yl)-4-methoxybutanoyl]amino}pyridin-2-carbonsäuremethylester (Stereoisomerengemisch)

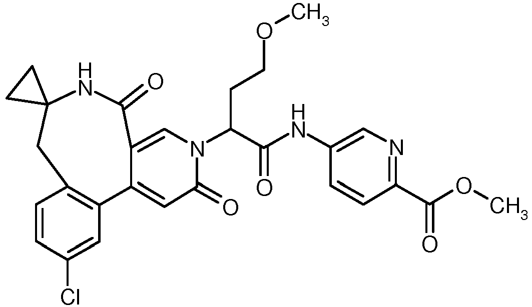

Gemäß der allgemeinen Methode 3A wurden 125 mg (300 µmol) 2-(11'-Chlor-2',5'-dioxo-2',5',6',8'-tetrahydro-3'*H*-spiro[cyclopropan-1,7'-pyrido[3,4-*e*][3]benzazocin]-3'-yl)-4-methoxybutansäure (Stereoisomerengemisch) mit 50 mg (330 µmol, 1.1 eq.) 5-Aminopyridin-2-carbonsäuremethylester umgesetzt. Pyridin wurde im Vakuum entfernt. Der Rückstand wurde mit Wasser verrührt, der entstandene Niederschlag filtriert, mit Wasser gewaschen und im Hochvakuum getrocknet. Ausbeute: 135 mg (89% Reinheit, 73% d. Th.)
LC/MS [Methode 1]: Rₜ = 0.80 min; MS (ESIpos): m/z = 551 (M+H)⁺.

### Beispiel 9.4A

### 5-{[2-(11'-Chlor-2',5'-dioxo-2',5',6',8'-tetrahydro-3'H-spiro[cyclopropan-1,7'-pyrido[3,4-e][3]benzazocin]-3'-yl)-4-methoxybutanoyl]amino}thiophen-2-carbonsäuremethylester (Stereoisomerengemisch)

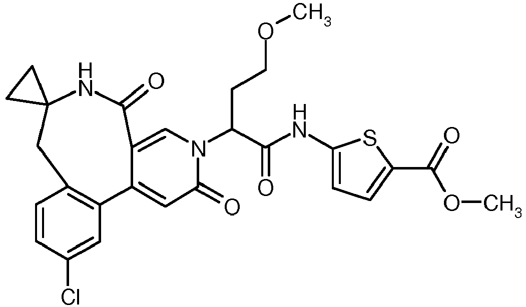

Gemäß der allgemeinen Methode 1A wurden 83 mg (200 µmol) 2-(11'-Chlor-2',5'-dioxo-2',5',6',8'-tetrahydro-3'*H*-spiro[cyclopropan-1,7'-pyrido[3,4-*e*][3]benzazocin]-3'-yl)-4-methoxybutansäure (Stereoisomerengemisch) mit 36 mg (220 µmol, 1.1 eq.) 5-Aminothiophen-2-carbonsäuremethylester umgesetzt. Das Reaktionsgemisch wurde im Vakuum eingeengt. Der Rückstand wurde mit Wasser verrührt, der entstandene Niederschlag filtriert, mit Wasser gewaschen und im Hochvakuum getrocknet. Der Niederschlag wurde mittels präparativer RP-HPLC (Reprosil C18, Wasser-Acetonitril-Gradient) weiter aufgereinigt. Ausbeute: 66 mg (58% d. Th.)
LC/MS [Methode 1]: Rₜ = 0.88 min; MS (ESIpos): m/z = 556 (M+H)⁺.

### Beispiel 9.5A

### 5-{[2-(11'-Chlor-2',5'-dioxo-2',5',6',8'-tetrahydro-3'H-spiro[cyclopropan-1,7'-pyrido[3,4-e][3]benzazocin]-3'-yl)-4-methoxybutanoyl]amino}thiophen-3-carbonsäuremethylester (Stereoisomerengemisch)

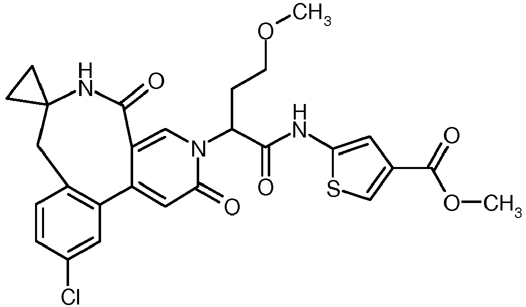

Gemäß der allgemeinen Methode 1A wurden 83 mg (200 µmol) 2-(11'-Chlor-2',5'-dioxo-2',5',6',8'-tetrahydro-3'*H*-spiro[cyclopropan-1,7'-pyrido[3,4-*e*][3]benzazocin]-3'-yl)-4-methoxybutansäure (Stereoisomerengemisch) mit 36 mg (220 µmol, 1.1 eq.) 5-Aminothiophen-3-carbonsäuremethylester umgesetzt. Das Reaktionsgemisch wurde im Vakuum eingeengt. Der Rückstand wurde mit Wasser verrührt, der entstandene Niederschlag filtriert, mit Wasser gewaschen und im Hochvakuum getrocknet. Ausbeute: 116 mg (quant.)
LC/MS [Methode 1]: Rₜ = 0.88 min; MS (ESIpos): m/z = 556 (M+H)⁺.

### Beispiel 9.6A

### 4-{[2-(11'-Chlor-2',5'-dioxo-2',5',6',8'-tetrahydro-3'H-spiro[cyclopropan-1,7'-pyrido[3,4-e][3]benzazocin]-3'-yl)-4-methoxybutanoyl]amino}thiophen-2-carbonsäuremethylester (Stereoisomerengemisch)

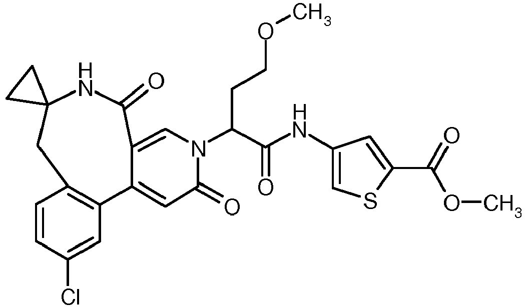

Gemäß der allgemeinen Methode 1A wurden 80 mg (192 µmοl) 2-(11'-Chlor-2',5'-dioxo-2',5',6',8'-tetrahydro-3'*H*-spiro[cyclopropan-1,7'-pyrido[3,4-*e*][3]benzazocin]-3'-yl)-4-methoxybutansäure (Stereoisomerengemisch) mit 33 mg (211 µmol, 1.1 eq.) 4-Aminothiophen-2-carbonsäuremethylester umgesetzt. Das Reaktionsgemisch wurde im Vakuum eingeengt. Der Rückstand wurde mit Wasser verrührt, der entstandene Niederschlag filtriert, mit Wasser gewaschen und im Hochvakuum getrocknet. Der Niederschlag wurde mittels präparativer RP-HPLC (Reprosil C18, Wasser-Acetonitril-Gradient) weiter aufgereinigt. Ausbeute: 83 mg (78% d. Th.)
LC/MS [Methode 1]: Rₜ = 0.93 min; MS (ESIpos): m/z = 556 (M+H)⁺.

### Beispiel 10.1A

### trans-4-Hydroxycyclohexancarbonsäuremethylester

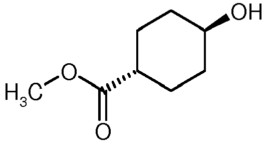

Zu einer Lösung von 25.0 g (165 mmol, 95% Reinheit) *trans*-4-Hydroxycyclohexancarbonsäure in 207 ml Methanol wurde bei RT 11.4 ml Schwefelsäure gegeben und die resultierende Reaktionslösung über Nacht unter Rückfluss erhitzt. Anschließend wurde mit gesättigter, wässriger Natriumhydrogencarbonat-Lösung neutralisiert und dreimal mit 250 ml Essigsäureethylester extrahiert. Die vereinigten organischen Phasen wurden über Magnesiumsulfat getrocknet, filtriert und das Lösungsmittel unter reduziertem Druck entfernt. Es wurden 25.5 g (97% d. Th.) der Titelverbindung erhalten.
GC/MS [Methode 9]: Rₜ = 3.86 min; MS: m/z = 158 (M)⁺,
¹H-NMR (400 MHz, DMSO-d₆): δ [ppm] = 4.55 (d, 1H), 3.57 (s, 3H), 3.39-3.30 (m, 1H), 2.25-2.16 (m, 1H), 1.89-1.77 (m, 4H), 1.40-1.27 (m, 2H), 1.21-1.08 (m, 2H).

### Beispiel 10.1B

### trans-4-Methoxycyclohexancarbonsäuremethylester

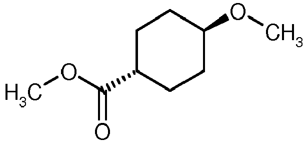

Zu einer Lösung von 24.6 g (149 mmol) *trans*-4-Hydroxycyclohexancarbonsäuremethylester in 1525 ml Dichlormethan wurde bei 0°C (Eisbad) 35.2 g (164 mmol) *N,N,N',N'* Tetramethylnaphthalen-1,8-diamin zugegeben und für 30 min bei RT nachgerührt. Anschließend wurde erneut auf 0°C (Eisbad) abgekühlt und 30.0 g (203 mmol) Trimethyloxoniumtetrafluorborat hinzugegeben. Es wurde über Nacht bei RT nachgerührt. Aufgrund unvollständiger Umsetzung wurde auf 0°C (Eisbad) abgekühlt und 30.0 g (203 mmol) Trimethyloxoniumtetrafluorborat zur Reaktionsmischung gegeben und für weitere 24 h bei RT gerührt. Die Reaktion wurde durch Zugabe 1845 ml Wasser beendet, mit 1100 ml Dichlormethan verdünnt und die Phasen getrennt. Die wässrige Phase wurde dreimal mit Dichlormethan extrahiert und die vereinigten organischen Phasen über Magnesiumsulfat getrocknet, filtriert und das Lösungsmittel unter reduziertem Druck entfernt. Der Rückstand wurde mittels Flashchromatographie (Cyclohexan/Essigsäureethylester-Gradient) gereinigt und 18.4 g (72% d. Th.) der Titelverbindung erhalten.
GC/MS [Methode 9]: Rₜ = 3.54 min; MS: m/z = 172 (M)⁺,
¹H-NMR (400 MHz, DMSO-d₆): δ [ppm] = 3.58 (s, 3H), 3.22 (s, 3H), 3.12-3.03 (m, 1H), 2.32-2.23 (m, 1H), 2.01-1.84 (m, 4H), 1.41-1.29 (m, 2H), 1.19-1.07 (m, 2H).

### Beispiel 10.1C

### (trans-4-Methoxycyclohexyl)methanol

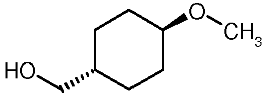

Zu 42.8 ml (103 mmol) Lithiumaluminiumhydrid (2.4M in THF) in 307 ml *tert*.-Butylmethylether wurde bei RT eine Lösung von 16.1 g (93.5 mmol) *trans*-4-Methoxycyclohexancarbonsäuremethylester in 307 ml *tert*.-Butylmethylether langsam getropft. Nach vollständiger Zugabe wurde auf 40°C erwärmt und 6 h nachgerührt. Es wurde auf RT abgekühlt und die Reaktion durch Zugabe von 37.4 ml Wasser und 37.4 ml 10%iger wässriger Kaliumhydroxid-Lösung beendet. Die organische Phase wurde abdekantiert, über Natriumsulfat getrocknet, filtriert und das Lösungsmittel unter reduziertem Druck entfernt. Es wurden 13.0 g (96% d. Th.) der Titelverbindung erhalten.
GC/MS [Methode 9]: Rₜ = 3.09 min; MS: m/z = 144 (M)⁺,
¹H-NMR (400 MHz, DMSO-d₆): δ [ppm] = 4.36 (t, 1H), 3.22 (s, 3H), 3.21-3.17 (m, 2H), 3.06-2.97 (m, 1H), 2.20-1.94 (m, 2H), 1.76-1.69 (m, 2H), 1.33-1.23 (m, 1H), 1.10-0.98 (m, 2H), 0.93-0.81 (m, 2H).

### Beispiel 10.1D

### (trans-4-Methoxycyclohexyl)methyltrifluormethansulfonat

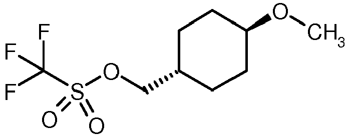

2.30 g (15.9 mmol) (*trans*-4-Methoxycyclohexyl)methanol wurden in 32 ml Dichlormethan vorgelegt und bei 0°C (Eisbad) nacheinander mit 2.79 ml (23.9 mmol) 2,6-Dimethylpyridin und 4.05 ml (23.9 mmol) Trifluormethansulfonsäureanhydrid versetzt. Es wurde für 1h bei 0°C nachgerührt. Die Reaktion wurde mit 300 ml Diethylether verdünnt und viermal mit 100 ml einer Mischung aus 1N wässriger Salzsäure und gesättigter wässriger Natriumchlorid-Lösung (1:3) gewaschen. Die organische Phase wurde abschließend mit gesättigter wässriger Natriumhydrogencarbonat-Lösung gewaschen, über Natriumsulfat getrocknet, filtriert und das Lösungsmittel unter reduziertem Druck entfernt. Es wurden 2.49 g (17% d. Th., 30% Reinheit) der Titelverbindung erhalten.
GC/MS [Methode 9]: Rₜ = 3.76 min; MS: m/z = 276 (M)⁺.

### Beispiel 10.1E

### N-(Diphenylmethyliden)-3-(trans-4-methoxycyclohexyl)alanin-tert.-butylester (Racemat)

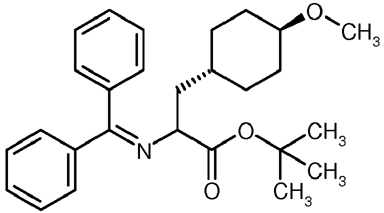

Eine Lösung von 1.42 g (4.81 mmol) *tert*.-Butyl-*N*-(diphenylmethylen)glycinat in 40 ml Tetrahydrofuran wurde unter Argon bei -78°C tropfenweise mit 11.1 ml (1.0M in THF, 11.1 mmol, 2.3 eq.) Bis-(trimethylsilyl)-lithiumamid versetzt, 15 min gerührt und anschließend tropfenweise mit 2.85 g (70% Reinheit, 7.21 mmol, 1.5 eq.) (*trans*-4-Methoxycyclohexyl)methyltrifluor-methansulfonat versetzt. Das Reaktionsgemisch wurde weitere 15 min bei -78°C gerührt, auf RT kommen gelassen, über Nacht nachgerührt und anschließend mit 50 ml Wasser gequencht. Nach Zugabe von Essigsäureethylester und Phasentrennung wurde die wässrige Phase zweimal mit Essigsäureethylester extrahiert. Die vereinigten, organischen Phasen wurden mit gesättigter, wässriger Natriumchlorid-Lösung gewaschen und im Vakuum eingeengt. Der Rückstand wurde mittels Flash-Chromatographie (Kieselgel-50, Cyclohexan-Essigsäureethylester-Gemisch) aufgereinigt. Es wurden 0.54 g (18% d. Th., 68% Reinheit) der Titelverbindung erhalten.
LC/MS [Methode 1]: Rₜ = 1.36 min; MS (ESIpos): m/z = 422 (M+H)⁺.

### Beispiel 10.1F

### 3-(trans-4-Methoxycyclohexyl)alanin-tert.-butylester (Racemat)

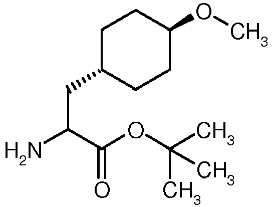

Eine Lösung von 1.70 g (70% Reinheit, 2.82 mmol) *N*-(Diphenylmethyliden)-3-(*trans*-4-methoxycyclohexyl)alanin-*tert*.-butylester (Racemat) in Tetrahydrofuran wurde mit 50 ml einer wässrigen Zitronensäure-Lösung (1M) versetzt und 90 min bei RT gerührt. Anschließend wurde Tetrahydrofuran im Vakuum entfernt. Die resultierende wässrige Lösung wurde vorsichtig mit festem Natriumbicarbonat neutralisiert und zweimal mit Dichlormethan extrahiert. Die vereinigten, organischen Phasen wurden getrocknet (Natriumsulfat), filtriert und am Rotationsverdampfer im Vakuum (>150 mbar bei <25°C Wasserbadtemperatur) eingeengt. Der Rückstand wurde mittels Flash-Chromatographie (Kieselgel-50, Dichlormethan-Methanol-Gemisch) aufgereinigt. Es wurden 739 mg (quant.) der Titelverbindung erhalten.
GC/MS [Methode 9]: Rₜ = 5.50 min; MS: m/z = 157 (M-CO₂*t*Bu)⁺,
¹H-NMR (400 MHz, DMSO-d₆): δ [ppm] = 3.21 (s, 3H), 3.20-3.13 (m, 1H), 3.08-2.99 (m, 1H), 2.02-1.92 (m, 2H), 1.77-1.68 (m, 2H), 1.62 (br. s, 2H), 1.42-1.21 (m, 3H), 1.41 (s, 9H), 1.10-0.78 (m, 4H).

### Beispiel 10.1G

### 1-[1-tert.-Butoxy-3-(trans-4-methoxycyclohexyl)-1-oxopropan-2-yl]-4-hydroxy-6-oxo-1,6-dihydropyridin-3-carbonsäureethylester (Racemat)

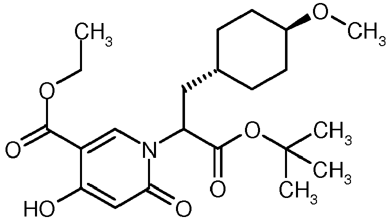

189 µl (1.04 mmol) 3-Oxopentandicarbonsäurediethylester und 239 µl (1.46 mmol, 1.4 eq.) Diethoxymethylacetat wurden 2.5 h bei 100°C gerührt, nach Abkühlen auf RT dreimal mit Toluol koevaporiert und im Hochvakuum getrocknet. Der Rückstand wurde in 1ml Ethanol aufgenommen, unter Eiskühlung mit einer Lösung von 282 mg (1.10 mmol, 1.05 eq.) *3-*(*trans-4-*Methoxycyclohexyl)alanin-*tert*.-butylester (Racemat) in 1ml Ethanol versetzt und 1h bei RT gerührt. Anschließend wurde das Reaktionsgemisch mit 390 µl (1.04 mmol, 1.0 eq.) einer Natriumethylat-Lösung (21%ig in Ethanol, zuvor über ca. 15 g aktiviertem Molsieb 3Å getrocknet) versetzt, 1h bei RT gerührt und mit gesättigter, wässriger Ammoniumchlorid-Lösung gequencht. Nach Phasentrennung wurde die wässrige Phase dreimal mit Essigsäureethylester extrahiert. Die vereinigten, organischen Phasen wurden getrocknet (Magnesiumsulfat), filtriert und im Vakuum eingeengt. Der Rückstand wurde mittels Flash-Chromatographie (Kieselgel-50, Cyclohexan-Essigsäureethylester-Gemisch) aufgereinigt. Es wurden 150 mg (34% d. Th.) der Titelverbindung erhalten.
LC/MS [Methode 1]: Rₜ = 1.05 min; MS (ESIpos): m/z = 424 (M+H)⁺,
¹H-NMR (400 MHz, DMSO-d₆): δ [ppm] = 10.89 (s, 1H), 8.31 (s, 1H), 5.70 (s, 1H), 5.26-5.18 (m, 1H), 4.32-4.26 (m, 2H), 3.19 (s, 3H), 3.06-2.97 (m, 1H), 2.00-1.75 (m, 5H), 1.65-1.57 (m, 1H), 1.37 (s, 9H), 1.29 (t, 3H), 1.05-0.83 (m, 5H).

### Beispiel 10.1H

### 1-[1-tert.-Butoxy-3-(trans-4-methoxycyclohexyl)-1-oxopropan-2-yl]-6-oxo-4-{[(trifluormethyl)-sulfonyl] oxy} -1,6-dihydropyridin-3-carbonsäureethylester (Racemat)

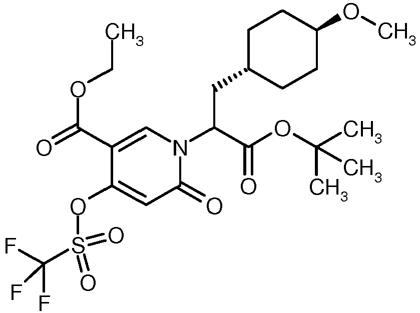

Eine Lösung von 274 mg (647 µmol) 1-[1-*tert*.-Butoxy-3-(*trans*-4-methoxycyclohexyl)-1-oxopropan-2-yl]-4-hydroxy-6-oxo-l,6-dihydropyridin-3-carbonsäureethylester (Racemat) in 5 ml Dichlormethan wurde unter Argon bei -78°C mit 180 µl (1.29 mmol, 2.0 eq.) Triethylamin und anschließend portionsweise mit 321 mg (776 µmol, 1.2 eq.) *N*-(4-*tert*.-Butylphenyl)-1,1,1-trifluor-*N*-[(trifluormethyl)sulfonyl]-methansulfonamid versetzt. Das Reaktionsgemisch wurde auf RT kommen gelassen, über Nacht bei RT gerührt und anschließend im Vakuum eingeengt. Der Rückstand wurde mittels Flash-Chromatographie (Kieselgel-50, Cyclohexan-Essigsäureethylester-Gemisch) aufgereinigt. Es wurden 298 mg (82% d. Th.) der Titelverbindung erhalten.
LC/MS [Methode 1]: Rₜ = 1.27 min; MS (ESIpos): m/z = 556 (M+H)⁺,
¹H-NMR (400 MHz, DMSO-d₆): δ [ppm] = 8.65 (s, 1H), 6.68 (s, 1H), 5.30-5.23 (m, 1H), 4.32 (q, 2H), 3.20 (s, 3H), 3.07-2.97 (m, 1H), 2.01-1.90 (m, 4H), 1.84-1.76 (m, 1H), 1.67-1.59 (m, 1H), 1.37 (s, 9H), 1.30 (t, 3H), 1.12-0.88 (m, 5H).

### Beispiel 10.1I

### 4-[2-({1-[(tert.-Butoxycarbonyl)amino]cyclopropyl}methyl)-5-chlorphenyl]-1-[1-tert.-butoxy-3-(trans-4-methoxycyclohexyl)-1-oxopropan-2-yl]-6-oxo-1,6-dihydropyridin-3-carbonsäureethylester (Stereoisomerengemisch)

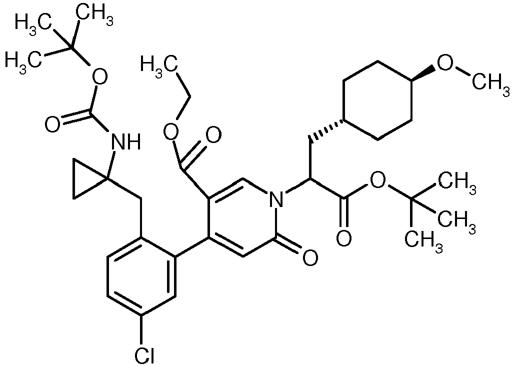

Ein Gemisch aus 292 mg (520 µmol) 1-[1-*tert*.-Butoxy-3-(*trans*-4-methoxycyclohexyl)-1-oxopropan-2-yl]-6-oxo-4-{[(trifluormethyl)sulfonyl]oxy}-1,6-dihydropyridin-3-carbonsäureethylester (Racemat), 209 mg (520 µmol) [2-({1-[(*tert*.-Butoxycarbonyl)anünolcyclo-propyl}methyl)-5-chlorphenyl]borsäure und 216 mg (1.56 mmol, 3.0 eq.) Kaliumcarbonat wurde im Hochvakuum getrocknet, unter Argon gesetzt, mit 8 ml Dioxan versetzt und 10 min mit Argon durchströmt. Anschließend wurde das Reaktionsgemisch mit 42 mg (52 µmol, 0.1 eq.) [1,1-Bis-(diphenylphosphino)-ferrocen]-palladium(II)chlorid-Monodichlormethan-Addukt versetzt und 1h bei 80°C Ölbadtemperatur gerührt. Nach Abkühlen auf RT wurde das Reaktionsgemisch über Celite filtriert. Nach Waschen mit Dioxan wurden die vereinigten Filtrate im Vakuum eingeengt. Der Rückstand wurde in Wasser aufgenommen und zweimal mit Essigsäureethylester extrahiert. Die vereinigten, organischen Phasen wurden mit gesättigter, wässriger Natriumchlorid-Lösung gewaschen, getrocknet (Magnesiumsulfat), filtriert und im Vakuum eingeengt. Der Rückstand wurde mittels Flash-Chromatographie (Kieselgel-50, Cyclohexan-Essigsäureethylester-Gemisch) aufgereinigt. Es wurden 286 mg (79% d. Th.) der Titelverbindung erhalten.
LC/MS [Methode 1]: Rₜ = 1.41 min; MS (ESIpos): m/z = 687 (M+H)⁺.

### Beispiel 10.1J

### 2-[4-{2-[(1-Aminocyclopropyl)methyl]-5-chlorphenyl}-5-(ethoxycarbonyl)-2-oxopyridin-1(2H)-yl]-3-(trans-4-methoxycyclohexyl)propansäure (Stereosiomerengemisch)

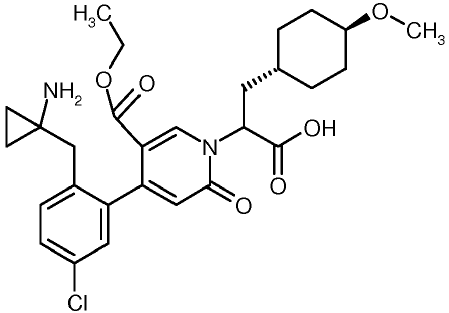

Eine Lösung von 286 mg (412 µmol) 4-[2-({1-[(*tert*.-Patoxycarbonyl)amino]cyclopropyl}methyl)-5-chlorphenyl]-1-[1-*tert*.-butoxy-3-(*trans*-4-methoxycyclohexyl)-1-oxopropan-2-yl]-6-oxo-1,6-dihydropyridin-3-carbonsäureethylester (Stereoisomerengemisch) in 4 ml Dichlormethan wurde unter Argon mit 635 µl (8.24 mmol, 20 eq.) Trifluoressigsäure versetzt, über Nacht bei RT gerührt. Umsatz nicht vollständig, es wurden weitere 159 µl (2.06 mmol, 5 eq.) Trifluoressigsäure hinzugegeben und der Ansatz nach weiteren 12h bei RT im Vakuum eingeengt. Der Rückstand wurde mehrmals mit Toluol koevaporiert, im Hochvakuum getrocknet und ohne weitere Reinigung umgesetzt. Es wurden 320 mg (quant.) der titelverbindung erhalten.
LC/MS [Methode 1]: Rₜ = 0.76 min; MS (ESIpos): m/z = 531 (M+H)⁺.

### Beispiel 10.1K

### 2-(11'-Chlor-2',5'-dioxo-2',5',6',8'-tetrahydro-3'H-spiro[cyclopropan-1,7'-pyrido[3,4-e][3]benzazocin]-3'-yl)-3-(trans-4-methoxycyclohexyl)propansäure (Stereoisomerengemisch)

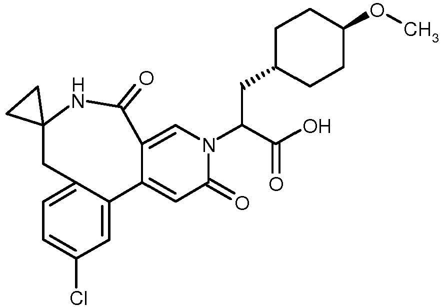

Eine Lösung von 219 mg (412µmol) 2-[4-{2-[(1-Aminocyclopropyl)methyl]-5-chlorphenyl}-5-(ethoxycarbonyl)-2-oxopyridin-1(2*H*)-yl]-3-(*trans*-4-methoxycyclohexyl)propansäure (Stereosiomerengemisch) in 5 ml Tetrahydrofuran wurde unter Argon zu aktiviertem Molsieb 3Å (über Nacht bei 200°C im Trockenschrank getrocknet) gegeben und 2 h bei RT gerührt. Anschließend wurde das Reaktionsgemisch unter Argon bei RT mit 3.1 ml (8.2 mmol, 20 eq.) einer Natriumethylat-Lösung (21%ig in Ethanol, zuvor 2 h über ca. 15 g aktiviertem Molsieb 3Å getrocknet) versetzt und 60 min bei RT gerührt. Nach Verdünnen mit Essigsäureethylester wurde das Reaktionsgemisch mit gesättigter, wässriger Ammoniumchlorid-Lösung versetzt und anschließend mit wässriger Salzsäure-Lösung (1N) auf pH 3 gebracht. Nach Phasentrennung wurde die wässrige Phase dreimal mit Essigsäureethylester extrahiert. Die vereinigten, organischen Phasen wurden zweimal mit 0.5N wässriger Salzsäure, 20 ml gesättigter wässriger Natriumchlorid-Lösung gewaschen, getrocknet (Magnesiumsulfat), filtriert und im Vakuum eingeengt. Es wurden 132 mg (66% d. Th.) der Titelverbindung erhalten.
LC/MS [Methode 1]: Rₜ = 0.83/0.84 min; MS (ESIpos): m/z = 485 (M+H)⁺.
¹H-NMR (400 MHz, DMSO-d₆): δ [ppm] = 13.25/13.05 (2x s, 1H), 8.10/8.09 (2x s, 1H), 7.77 (d, 1H), 7.45 (dd, 1H), 7.33-7.27 (m, 2H), 6.32/6.29 (2x s, 1H), 5.57-5.50/5.26-5.18 (2x m, 1H), 3.23-3.15 (m, 1H), 3.21/3.19 (2x s, 3H), 3.08-2.97 (m, 1H), 2.73-2.65 (m, 1H), 2.28-2.15 (m, 1H), 2.02-1.54 (m, 5H), 1.08-0.61 (m, 9H).

### Beispiel 10.1L

### 4-{[2-(11'-Chlor-2',5'-dioxo-2',5',6',8'-tetrahydro-3'H-spiro[cyclopropan-1,7'-pyrido[3,4-e][3]benzazocin]-3'-yl)-3-(trans-4-methoxycyclohexyl)propanoyl]amino}benzosäureethylester (Stereoisomerengemisch)

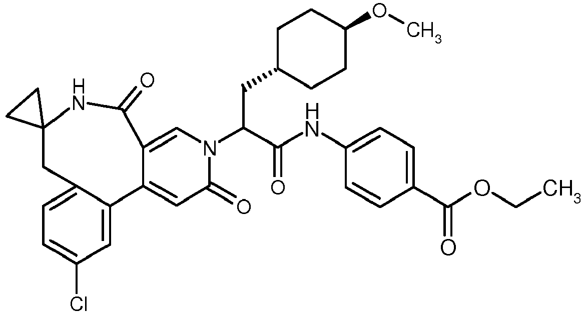

Eine Lösung aus 50.0 mg (103 µmol) 2-(11'-Chlor-2',5'-dioxo-2',5',6',8'-tetrahydro-3'*H-*spiro[cyclopropan-1,7'-pyrido[3,4-*e*][3]benzazocin]-3'-yl)-3-(*trans*-4-methoxycyclohexyl)-propansäure (Stereoisomerengemisch), 17.0 mg (103 µmol, 1.0 eq.) 4-Aminobenzoesäureethylester, 10 mg (72 µmol) Oxyma und 16.1 µl (103 µmol) *N,N'*-Diisopropylcarbodiimid in DMF wurde über Nacht bei 40°C gerührt. Anschließend wurde das Lösungsmittel unter reduziertem Druck entfernt und der Rückstand mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Es wurden 42.8 mg (66% d. Th.) der Titelverbindung erhalten.
LC/MS [Methode 1]: Rₜ = 1.09 min; MS (ESIpos): m/z = 632 (M+H)⁺,
¹H-NMR (400 MHz, DMSO-d₆): δ [ppm] = 10.88/10.82 (2x s, 1H), 8.16/8.10 (2x s, 1H), 7.97-7.68 (m, 5H), 7.48-7.42 (m, 1H), 7.33-7.27 (m, 2H), 6.33/6.32 (2x s, 1H), 5.93/5.83 (2x dd, 1H), 4.29 (q, 2H), 3.25-3.15 (m, 1H), 3.22/3.20 (2x s, 3H), 3.09-2.99 (m, 1H), 2.74-2.61 (m, 1H), 2.34-1.70 (m, 6H), 1.31 (t, 3H), 1.16-0.61 (m, 9H).

### Beispiel 11.1A

### 1-(1-tert.-Butoxy-1-oxo-3-phenylpropan-2-yl)-4-hydroxy-6-oxo-1,6-dihydropyridin-3-carbonsäureethylester (Racemat)

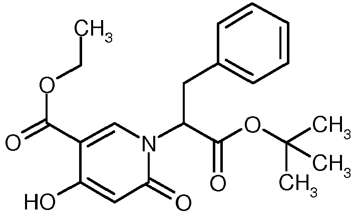

2.1ml (11.1 mmol) 3-Oxopentandicarbonsäurediethylester und 2.6 ml (15.5 mmol, 1.4eq.) Diethoxymethylacetat wurden 2.5 h bei 100°C gerührt, nach Abkühlen auf RT dreimal mit Toluol koevaporiert und im Hochvakuum getrocknet. Der Rückstand wurde in 80 ml Ethanol aufgenommen, unter Eiskühlung mit 3.0 g (11.6 mmol, 1.05 eq.) *tert*.-Butyl-*L*-phenylalaninat-Hydrochlorid (in 20 ml Ethanol und 1.93 ml *N,N-*Diisopropylethylamin) versetzt und 1 h bei RT gerührt. Anschließend wurde das Reaktionsgemisch portionsweise mit insgesamt 10.4 ml (27.7 mmol, 2.5 eq.) einer Natriumethylat-Lösung (21%ig in Ethanol, zuvor über ca. 15 g aktiviertem Molsieb 3Å getrocknet) versetzt und bei RT bis zur nahezu vollständigen Umsetzung gerührt. Anschließend wurde das Reaktionsgemisch mit gesättigter, wässriger Ammoniumchlorid-Lösung gequencht. Der ausfallende Niederschlag wurde filtriert, mit Essigsäureethylester gewaschen und verworfen. Nach Phasentrennung der vereinigten Filtrate wurde die wässrige Phase dreimal mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt. Der Rückstand wurde mittels Flash-Chromatographie (Kieselgel-50, Cyclohexan-Essigsäureethylester-Gemisch) aufgereinigt. Ausbeute: 3.2 g (90% Reinheit, 67% d. Th.)
LC/MS [Methode 1]: Rₜ = 1.15 min; MS (ESIneg): m/z = 388 (M+H)⁺.

### Beispiel 11.1B

### 1-(1-tert.-Butoxy-1-oxo-3-phenylpropan-2-yl)-6-oxo-4-1[(trifluormethyl)sulfonyl]oxy}-1,6-dihydropyridin-3-carbonsäureethylester (Racemat)

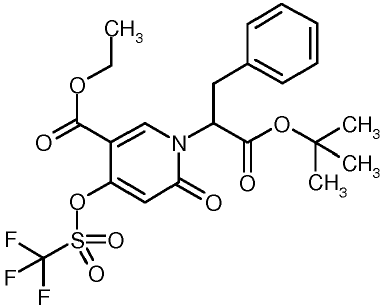

Eine Lösung von 1.00 g (2.19 mmol) 1-(1-*tert*.-Butoxy-1-oxo-3-phenylpropan-2-yl)-4-hydroxy-6-oxo-1,6-dihydropyridin-3-carbonsäureethylester (Racemat) in 17 ml Dichlormethan wurde unter Argon bei -78°C mit 0.61 ml (4.39 mmol, 2.0 eq.) Triethylamin und anschließend portionsweise mit 1.45 g (3.51 mmol, 1.6 eq.) *N*-(4-*tert*.-Butylphenyl)-1,1,1-trifluor-*N*-[(trifluormethyl)sulfonyl]-methansulfonamid versetzt. Das Reaktionsgemisch wurde auf RT kommen gelassen, über Nacht bei RT gerührt und anschließend im Vakuum eingeengt. Der Rückstand wurde mittels Flash-Chromatographie (Kieselgel-50, Cyclohexan-Essigsäureethylester-Gemisch) aufgereinigt. Ausbeute: 554 mg (89% Reinheit, 43% d. Th.)
LC/MS [Methode 1]: Rₜ = 1.30 min; MS (ESIpos): m/z = 520 (M+H)⁺.

### Beispiel 11.1C

### 4-[2-({1-[(tert.-Butoxycarbonyl)amino]cyclopropyl}methyl)-5-chlorphenyl]-1-(1-tert.-butoxy-1-oxo-3-phenylpropan-2-yl)-6-oxo-1,6-dihydropyridin-3-carbonsäureethylester (Stereoisomerengemisch)

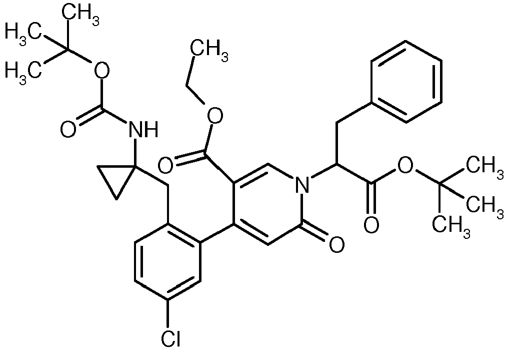

Ein Gemisch aus 554 mg (89% Reinheit, 0.95 mmol) 1-(1-*tert*.-Butoxy-1-oxo-3-phenylpropan-2-yl)-6-oxo-4-{[(trifluormethyl)sulfonyl]oxy}-1,6-dihydropyridin-3-carbonsäureethylester (Racemat), 439 mg (1.09 mmol, 1.15 eq.) [2-({1-[(*tert*.-Butoxycarbonyl)amino]cyclopropyl}-methyl)-5-chlorphenyl]borsäure und 394 mg (2.85 mmol, 3.0 eq.) Kaliumcarbonat wurde im Hochvakuum getrocknet, unter Argon gesetzt, mit 10 ml Dioxan versetzt und 10 min mit Argon durchströmt. Anschließend wurde das Reaktionsgemisch mit 78 mg (0.10 mmol, 0.1 eq.) [1,1-Bis-(diphenylphosphino)-ferrocen]-palladium(II)chlorid-Monodichlormethan-Addukt versetzt und 1h bei 80°C Ölbadtemperatur gerührt. Nach Abkühlen auf RT wurde das Reaktionsgemisch über Celite filtriert. Nach Waschen mit Dioxan wurden die vereinigten Filtrate im Vakuum eingeengt. Der Rückstand wurde in Wasser aufgenommen und zweimal mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt.

Analog wurde ein zweiter Ansatz mit 2.96 g (88% Reinheit, 5.01 mmol) 1-(1-*tert*.-Butoxy-1-oxo-3-phenylpropan-2-yl)-6-oxo-4-{[(trifluormethyl)sulfonyl]oxy}-1,6-dihydropyridin-3-carbonsäureethylester (Racemat) durchgeführt, beide Rohprodukte wurden vereinigt und mittels Flash-Chromatographie (Kieselgel-50, Cyclohexan-Essigsäureethylester-Gemisch) aufgereinigt. Ausbeute: 3.41 g (81% Reinheit, 71% d. Th. bezogen auf beide Ansätze)
LC/MS [Methode 1]: Rₜ = 1.41 min; MS (ESIpos): m/z = 651 (M+H)⁺.

### Beispiel 11.1D

### 2-[4-{2-[(1-Aminocyclopropyl)methyl]-5-chlorphenyl}-5-(ethoxycarbonyl)-2-oxopyridin-1(2H)-yl]-3-phenylpropansäure (Stereoisomerengemisch)

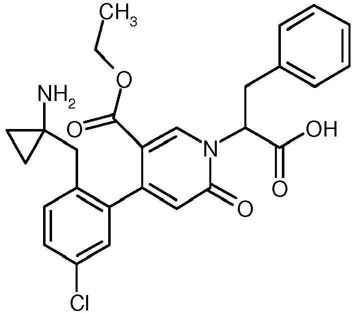

Eine Lösung von 3.41g (angenommene Reinheit von 75%, 3.93 mmol) 4-[2-({1-[(*tert.-*Butoxycarbonyl)amino]cyclopropyl}methyl)-5-chlorphenyl]-1-(1-*tert*.-butoxy-1-oxo-3-phenyl-propan-2-yl)-6-oxo-1,6-dihydropyridin-3-carbonsäureethylester (Stereoisomerengemisch) in 40 ml Dichlormethan wurde unter Argon bei Eisbadkühlung tropfenweise mit 6.0 ml (78.5 mmol, 20 eq.) Trifluoressigsäure versetzt, 5 min gerührt, auf RT kommen gelassen, 6 h bei RT gerührt und anschließend im Vakuum eingeengt. Da die Umsetzung nicht vollständig erfolgt war, wurde der Rückstand nochmals in 40 ml Dichlormethan gelöst, unter Argon bei Eisbadkühlung tropfenweise mit 6.0 ml (78.5 mmol, 20 eq.) Trifluoressigsäure versetzt, 5 min gerührt, auf RT kommen gelassen und weitere 6 h bei RT gerührt. Anschließend wurde das Reaktionsgemisch im Vakuum eingeengt und der Rückstand zweimal mit Toluol koevaporiert, im Hochvakuum getrocknet und ohne weitere Reinigung umgesetzt. Ausbeute: 3.36 g (70% Reinheit, quant.)
LC/MS [Methode 1]: Rₜ = 0.79 min; MS (ESIpos): m/z = 495 (M+H)⁺.

### Beispiel 11.1E

### 2-(11'-Chlor-2',5'-dioxo-2',5',6',8'-tetrahydro-3'H-spiro[cyclopropan-1,7'-pyrido[3,4-e] [3]benzazocin]-3'-yl)-3-phenylpropansäure (Stereoisomerengemisch)

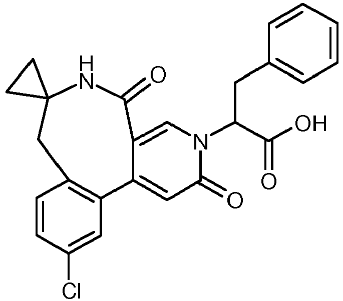

Eine Lösung von 1.5 g (70% Reinheit, 2.1 mmol) 2-[4-{2-[(1-Aminocyclopropyl)methyl]-5-chlorphenyl}-5-(ethoxycarbonyl)-2-oxopyridin-1(2*H*)-yl]-3-phenylpropansäure (Stereoisomerengemisch) in 30 ml Tetrahydrofuran wurde unter Argon zu ca. 4 g aktiviertem Molsieb 3Å (über Nacht bei 200°C im Trockenschrank getrocknet) gegeben und 2 h bei RT gerührt. Anschließend wurde das Reaktionsgemisch unter Argon bei RT mit 12.5 ml (42 mmol, 20 eq.) einer Natriumethylat-Lösung (21%ig in Ethanol, zuvor 2h über ca. 15 g aktiviertem Molsieb 3Å getrocknet) versetzt und 2 h bei RT gerührt. Das Reaktionsgemisch wurde filtriert, mit gesättigter, wässriger Ammoniumchlorid-Lösung versetzt und anschließend mit wässriger Salzsäure-Lösung (1N) auf pH 3 gebracht. Nach Phasentrennung wurde die wässrige Phase zweimal mit Essigsäureethylester extrahiert. Die vereinigten, organischen Phasen wurden getrocknet (Natriumsulfat), filtriert und im Vakuum eingeengt. Der Rückstand ohne weitere Reinigung umgesetzt. Ausbeute: 643 mg (63% Reinheit, 43% d. Th.)
LC/MS [Methode 1]: Rₜ = 0.86 min; MS (ESIpos): m/z = 449 (M+H)⁺.

### Beispiel 11.1F

### 4-{[2-(11'-Chlor-2',5'-dioxo-2',5',6',8'-tetrahydro-3'H-spiro[cyclopropan-1,7'-pyrido[3,4-e][3]benzazocin]-3'-yl)-3-phenylpropanoyl]amino}benzoesäure-tert.-butylester (Stereoisomerengemisch)

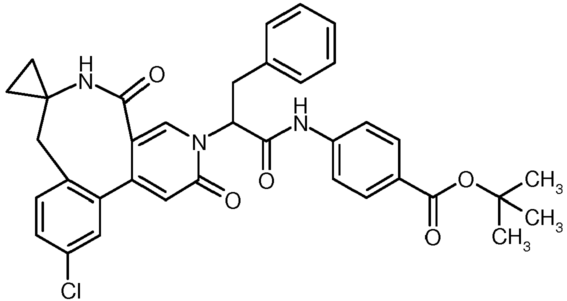

Gemäß der allgemeinen Methode 3A wurden 214 mg (63% Reinheit, 0.30 mmol) 2-(11'-Chlor-2',5'-dioxo-2',5',6',8'-tetrahydro-3'*H*-spiro[cyclopropan-1,7'-pyrido[3,4-e][3]benzazocin]-3'-yl)-3-phenylpropansäure (Stereoisomerengemisch) mit 87 mg (0.45 mmol, 1.5 eq.) 4-Aminobenzoesäure-*tert*.-butylester umgesetzt. Nach wässriger Aufarbeitung wurde das Rohprodukt mittels präparativer RP-HPLC (Reprosil C18, Wasser-Acetonitril-Gradient) aufgereinigt. Ausbeute: 88 mg (47% d. Th.)
LC/MS [Methode 1]: Rₜ = 1.21 min; MS (ESIpos): m/z = 624 (M+H)⁺.

### Beispiel 12.1A

### 1-(1-tert.-Butoxy-4-methoxy-1-oxobutan-2-yl)-6-oxo-4-(4,4,5,5-tetramethyl-1,3,2-dioxaborolan-2-yl)-1,6-dihydropyridin-3-carbonsäureethylester (Racemat)

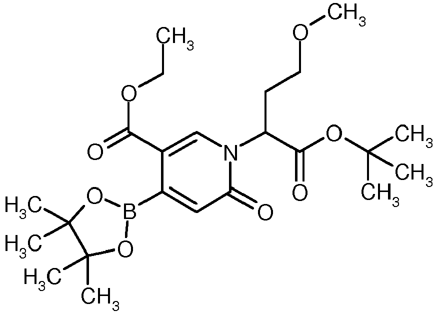

1.00 g (85% Reinheit, 1.74 mmol) 1-(1-*tert*.-Butoxy-4-methoxy-1-oxobutan-2-yl)-6-oxo-4-{[(trifluormethyl)sulfonyl]oxy}-1,6-dihydropyridin-3-carbonsäureethylester (Racemat), 487 mg (1.92 mmol, 1.1 eq.) Bis(pinacolato)diboron und 513 mg (5.23 mmol, 3 eq.) Kaliumacetat wurden unter Argon in 18 ml Dioxan vorgelegt, mit 42.7 mg (0.05 mmol, 0.03 eq.) [1,1-Bis-(diphenylphosphino)-ferrocen]-dichlorpalladium-Monodichlormethan-Addukt versetzt und 7 h bei 80°C gerührt. Das Reaktionsgemisch wurde auf RT gekühlt, über Kieselgur filtriert und mit Dioxan nachgewaschen. Das Filtrat wurde eingeengt und bei 40°C im Hochvakuum getrocknet. Ausbeute: 980 mg (80% Reinheit, 97% d. Th.).
¹H-NMR (400 MHz, DMSO-*d*₆): δ [ppm] = 8.31 (s, 1H), 6.39 (s, 1H), 5.12 (dd, 1H), 4.34-4.20 (m, 2H), 3.38-3.33 (m, 1H), 3.16-3.04 (m, 4H), 2.34-2.17 (m, 2H), 1.36 (s, 9H), 1.33-1.25 (m, 15H).

### Beispiel 12.1B

### 4-(2-{2-[(tert.-Butoxycarbonyl)amino]-3-fluorpropyl}-5-chlorphenyl)-1-(1-tert.-butoxy-4-methoxy-1-oxobutan-2-yl)-6-oxo-1,6-dihydropyridin-3-carbonsäureethylester (Stereoisomerengemisch)

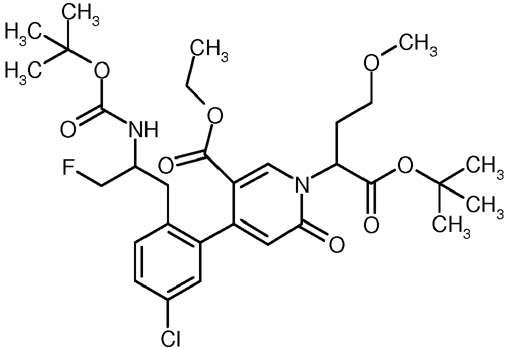

Ein Gemisch aus 399 mg (75% Reinheit, 0.82 mmol) *tert*.-Butyl-[1-(2-brom-4-chlorphenyl)-3-fluorpropan-2-yl]carbamat (Racemat), 950 mg (80% Reinheit, 1.63 mmol, 2 eq.) 1-(1-*tert*.-Butoxy-4-methoxy-1-oxobutan-2-yl)-6-oxo-4-(4,4,5,5-tetramethyl-1,3,2-dioxaborolan-2-yl)-1,6-dihydropyridin-3-carbonsäureethylester (Racemat) und 677 mg (4.90 mmol, 6 eq.) Kaliumcarbonat wurde im Hochvakuum getrocknet, unter Argon gesetzt, mit 18 ml Dioxan versetzt und 5 min mit Argon durchströmt. Anschließend wurde das Reaktionsgemisch mit 133 mg (0.16 mmol, 0.2 eq.) [1,1 -Bis-(diphenylphosphino)-ferrocen] -dichlorpalladium-Monodichlormethan-Addukt versetzt und 6 h bei 80°C Ölbadtemperatur gerührt. Das Reaktionsgemisch wurde über Kieselgur filtriert, mit Dichlormethan/Acetonitril gewaschen und das Filtrat eingeengt. Das Rohprodukt wurde mittels Flash-Chromatographie (Silica Kartusche, Cyclohexan-Essigsäureethylester-Gemisch) aufgereinigt. Ausbeute: 380 mg (60% Reinheit, 44% d. Th.)
LC/MS [Methode 12]: Rₜ = 2.44 min; MS (ESIpos): m/z = 625 (M+H)⁺.

### Beispiel 12.1C

### 2-{4-[2-(2-Amino-3-fluorpropyl)-5-chlorphenyl]-5-(ethoxycarbonyl)-2-oxopyridin-1(2H)-yl}-4-methoxybutansäure (Stereoisomerengemisch)

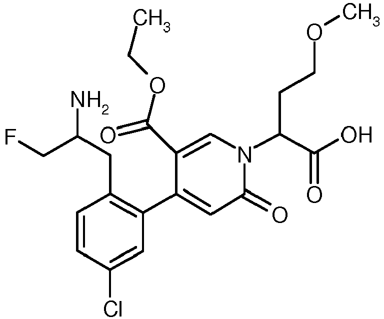

Eine Lösung von 380 mg (60% Reinheit, 0.36 mmol) 4-(2-{2-[(*tert*.-Butoxycarbonyl)amino]-3-fluorpropyl}-5-chlorphenyl)-1-(1-*tert*.-butoxy-4-methoxy-1-oxobutan-2-yl)-6-oxo-1,6-dihydropyridin-3-carbonsäureethylester (Stereoisomerengemisch) in 10 ml einer Lösung von Chlorwasserstoff in Dioxan (4M) wurde 6 h bei RT gerührt. Anschließend wurde das Reaktionsgemisch im Vakuum eingeengt. Das Rohprodukt wurde mittels präparativer HPLC (Wasser-Acetonitril-0.1% Ameisensäure-Gradient) aufgereinigt. Ausbeute: 161 mg (90% Reinheit, 85% d. Th.)
LC/MS [Methode 11]: Rₜ = 1.25 min; MS (ESIpos): m/z = 469 (M+H)⁺.

### Beispiel 12.1D

### 2-[11-Chlor-7-(fluormethyl)-2,5-dioxo-5,6,7,8-tetrahydropyrido[3,4-e][3]benzazocin-3(2H)-yl]-4-methoxybutansäure (Stereoisomerengemisch)

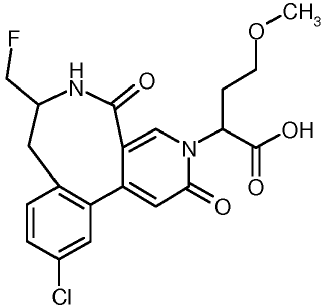

Eine Lösung von 161 mg (90% Reinheit, 0.31 mmol) 2-{4-[2-(2-Amino-3-fluorpropyl)-5-chlorphenyl]-5-(ethoxycarbonyl)-2-oxopyridin-1(2*H*)-yl}-4-methoxybutansäure (Stereoisomerengemisch) in 4.3 ml Tetrahydrofuran wurde mit 600 mg Molsieb 4Å versetzt und 2h bei RT gerührt. Anschließend wurde das Reaktionsgemisch bei RT mit 2.3 ml (6.18 mmol, 20 eq.) einer Natriumethylat-Lösung (21%ig in Ethanol, zuvor 2h über Molsieb 4Å getrocknet) versetzt und 90 min bei RT gerührt. Das Reaktionsgemisch wurde mit ca. 0.7 ml einer wässrigen Salzsäure-Lösung (6N) bis pH 5-6 versetzt und im Vakuum eingeengt. Der Rückstand wurde mittels präparativer HPLC (Wasser-Acetonitril-0.1% Ameisensäure-Gradient) aufgereinigt. Ausbeute: 40 mg (71% Reinheit, 22% d. Th.)
LC/MS [Methode 1]: Rₜ = 0.66 min; MS (ESIpos): m/z = 423 (M+H)⁺.

### Beispiel 12.1E

### 4-(12-[11-Chlor-7-(fluormethyl)-2,5-dioxo-5,6,7,8-tetrahydropyrido[3,4-e][3]benzazocin-3(2H)-yl]-4-methoxybutanoyl}amino)benzoesäuremethylester (Stereoisomerengemisch)

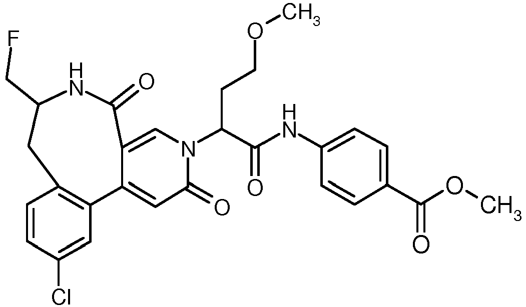

Nach der allgemeinen Methode 3A wurden 28 mg (66 µmol) 2-[11-Chlor-7-(fluormethyl)-2,5-dioxo-5,6,7,8-tetrahydropyrido[3,4-*e*][3]benzazocin-3(2*H*)-yl]-4-methoxybutansäure (Stereoisomerengemisch) mit 15 mg (99 µmol, 1.5 eq.) 4-Aminobenzoesäuremethylester umgesetzt. Das Rohprodukt wurde mittels präparativer HPLC (Wasser-Acetonitril-0.1% Ameisensäure-Gradient) aufgereinigt. Ausbeute: 19 mg (52% d. Th.)
LC/MS [Methode 1]: Rₜ = 0.88 min; MS (ESIpos): m/z = 556 (M+H)⁺.

### Ausführungsbeispiele

### Allgemeine Methode 1: Verseifung eines tert.-Butylesters oder eines Boc-geschützten Amins mittels TFA

Eine Lösung des entsprechenden *tert*.-Butylestersderivates oder eines Boc-geschützten Amins (1.0 eq.) in Dichlormethan (ca. 25 ml/mmol) wurde bei 0°C bis RT mit TFA (10-20 eq.) versetzt und bei RT für 1 bis 8 h gerührt. Anschließend wurde das Reaktionsgemisch im Vakuum eingeengt. Der Rückstand wurde mehrmals mit Dichlormethan und/oder Toluol koevaporiert. Die Aufreinigung des Rohproduktes erfolgte anschließend mittels präparativer RP-HPLC (Acetonitril-Wasser-Gradient oder Wasser-Methanol-Gradient).

### Allgemeine Methode 2: Verseifung eines Methyl- oder Ethylesters mit Lithiumhydroxid

Eine Lösung des entsprechenden Esters (1.0 eq.) in einem Gemisch aus Tetrahydrofuran/Wasser (3:1, ca. 7-15 ml/mmol) wurde bei RT mit Lithiumhydroxid (2-4 eq.) versetzt und bei RT gerührt. Anschließend wurde das Reaktionsgemisch mit wässriger Salzsäure-Lösung (1N) auf pH 1 gestellt. Nach Zugabe von Wasser/Essigsäureethylester wurde die wässrige Phase dreimal mit Essigsäureethylester extrahiert. Die vereinigten, organischen Phasen wurden getrocknet (Natrium- oder Magnesiumsulfat), filtriert und im Vakuum eingeengt. Das Rohprodukt wurde anschließend entweder mittels Normalphasen-Chromatographie (Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 3: Amidkupplung mit HATU/DIEA

Eine Lösung der entsprechenden Carbonsäure (1.0 eq.) in Dimethylformamid (ca. 7-70 ml/mmol) wurde unter Argon bei RT mit dem entsprechenden Amin (1.1-1.2 eq.), *N,N*-Diisopropylethylamin (DIEA) (2.2-3.0 eq.) und einer Lösung aus HATU (1.2 eq.) in wenig Dimethylformamid versetzt. Das Reaktionsgemisch wurde bei RT gerührt. Nach Zugabe von Wasser/Essigsäureethylester und Phasentrennung wurde die organische Phase mit Wasser und mit gesättigter, wässriger Natriumchlorid-Lösung gewaschen, getrocknet (Natrium- oder Magnesiumsulfat), filtriert und im Vakuum eingeengt. Das Rohprodukt wurde anschließend entweder mittels Normalphasen-Chromatographie (Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 4: Amid-Kupplung mit T3P/DIEA

Eine Lösung der Carbonsäure und des entsprechenden Amins (1.1-1.5 eq.) in Dimethylformamid (0.15-0.05 mmol) wurde unter Argon bei 0°C oder RT tropfenweise mit *N,N*-Diisopropylethylamin (3 eq.) und Propylphosphonsäureanhydrid (T3P, 50%ig in Dimethylformamid oder in Essigsäureethylester, 3 eq.) versetzt. Das Reaktionsgemisch wurde bei RT gerührt und anschließend im Vakuum eingeengt. Nach Zugabe von Wasser/Essigsäureethylester und Phasentrennung wurde die wässrige Phase zweimal mit Essigsäureethylester extrahiert. Die vereinigten, organischen Phasen wurden getrocknet (Natriumsulfat oder Magnesiumsulfat), filtriert und im Vakuum eingeengt. Das Rohprodukt wurde anschließend entweder mittels Flash-Chromatographie (Cyclohexan-Essigsäureethylester-Gemische oder Dichlormethan-Methanol-Gemische) oder präparativer HPLC (Reprosil C18, Wasser-Acetonitril-Gradient oder Wasser-Methanol-Gradient) aufgereinigt.

### Allgemeine Methode 5: Amid-Kupplung mit T3P/Pyridin

Eine Lösung der entsprechenden Carbonsäure (1 eq.) und des entsprechenden Amins (1.1-1.5 eq.) in Pyridin (ca. 0.1M) wurde auf 60 bis 90°C erwärmt und tropfenweise mit T3P (50%ig in Dimethylformamid oder in Essigsäureethylester, 1.5-4 eq.) versetzt. Alternativ wurde T3P (50%ig in Dimethylformamid oder in Essigsäureethylester, 1.5-4 eq.) bei RT hinzugefügt und danach bei RT gerührt oder auf 50 bis 90°C erhitzt. Nach 1 bis 20 h wurde das Reaktionsgemisch auf RT gekühlt und mit Wasser und Essigsäureethylester versetzt. Die wässrige Phase wurde mit Essigsäureethylester extrahiert. Die vereinigten organischen Phasen wurden mit wässriger PufferLösung (pH=5), mit gesättigter, wässriger Natriumhydrogencarbonat-Lösung und mit gesättigter, wässriger Natriumchlorid-Lösung gewaschen, über Natriumsulfat getrocknet und im Vakuum eingeengt. Das Rohprodukt wurde gegebenenfalls anschließend entweder mittels Normalphasen-Chromatographie (Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 6: Verseifung eines Methyl- oder Ethylesters mit Cäsiumcarbonat

Zu einer Lösung des entsprechenden Methyl- oder Ethylesters (1 eq.) in einer Mischung aus Methanol/Wasser (4/1, 0.05-0.2M) wurde Cäsiumcarbonat (2 eq.) gegeben und die resultierende Suspension 3 h bis 8 h bei RT bis 60°C gerührt. Das Reaktionsgemisch wurde gegebenenfalls auf RT abgekühlt und mit wässriger Salzsäure (1N) auf pH 3 eingestellt. Methanol wurde bei 30°C im Vakuum entfernt. Die wässrige Phase wurde mit Essigsäureethylester extrahiert. Die vereinigten, organischen Phasen wurden getrocknet (Natriumsulfat oder Magnesiumsulfat), filtriert und unter reduziertem Druck eingeengt. Der Rückstand wurde mittels präparativer HPLC aufgereinigt.

### Allgemeine Methode 7: Amid-Kupplung mit OXIMA/DIC

Zu einer entgasten Lösung der entsprechenden Carbonsäure (1 eq.), Anilin (1 eq.) und Hydroxyiminocyanoessigsäureethylester (Oxima) (0.7 eq.) in Dimethylformamid (0.06-0. 1M) wurde tropfenweise *N,N'*-Diisopropylcarbodiimid (DIC) (1 eq.) gegeben und die resultierende Reaktionslösung für 8 h bis 24 h bei RT bis 40°C gerührt. Das Lösungsmittel wurde unter reduziertem Druck entfernt. Der Rückstand wurde entweder mit Wasser versetzt und das gewünschte Produkt filtriert oder mittels Normalphasen-Chromatographie (Cyclohexan/ Essigsäureethylester-Gradient) oder präparativer RP-HPLC (Wasser-Acetonitril-Gradient oder Wasser-Methanol-Gradient) aufgereinigt.

### Beispiel 1

### 4-{[2-(10-Chlor-2,5-dioxo-2,5,6,7-tetrahydro-3H-pyrido[3,4-d][2] benzazepin-3-yl)butanoyl]-amino}benzoesäure (Stereoisomerengemisch)

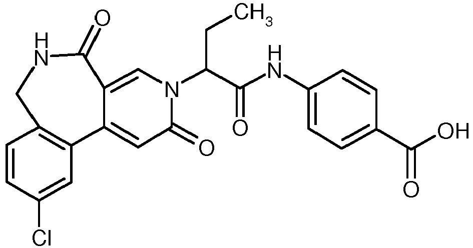

Nach der allgemeinen Methode 1 wurden 42 mg (80 µmol) 4-{[2-(10-Chlor-2,5-dioxo-2,5,6,7-tetrahydro-3*H*-pyrido[3,4-*d*][2]benzazepin-3-yl)butanoy1]amino}benzoesäure-*tert*.-butylester (Stereoisomerengemisch) mit Trifluoressigsäure verseift. Das Lösungsmittel wurde unter reduziertem Druck entfernt, der Rückstand dreimal mit Dichlormethan und dreimal mit Toluol koevaporiert und der Rückstand wurde mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 31 mg (80% d. Th.)
LC/MS [Methode 1]: Rₜ = 0.77 min; MS (ESIpos): m/z = 466 (M+H)⁺,
¹H-NMR (400 MHz, DMSO-d₆): δ [ppm] = 12.8 (br. s, 1H), 10.9 (br. s, 1H), 8.54 (br. s, 1H), 8.28 (s, 1H), 7.91 (d, 2H), 7.77-7.71 (m, 3H), 7.54 (dd, 1H), 7.43 (d, 1H), 6.65 (s, 1H), 5.67-5.60 (m, 1H), 4.23-3.89 (2x br. m, 2H), 2.27-2.14 (m, 1H), 2.11-1.98 (m, 1H), 0.93 (t, 3H).

### Beispiel 2

### 2-(10-Chlor-2,5-dioxo-2,5,6,7-tetrahydro-3H-pyrido[3,4-d][2]benzazepin-3-yl)-N-[4-(5-oxo-4,5-dihydro-1,2,4-oxadiazol-3-yl)phenyl]butanamid (Stereoisomerengemisch)

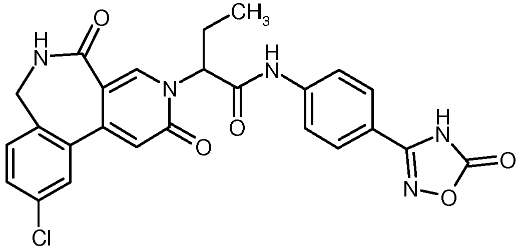

75.0 mg (216 µmol) 2-(10-Chlor-2,5-dioxo-2,5,6,7-tetrahydro-3*H*-pyrido[3,4-*d*][2]benzazepin-3-yl)butansäure (Stereoisomerengemisch) und 57.5mg (324µmol) 3-(4-Aminophenyl)-1,2,4-oxadiazol-5(4*H*)-on [Zerban, G. et al., WO 2007/071742, Beispiel 1] wurden in 5 ml Pyridin vorgelegt. Anschließend wurden 631 µl (1.30 mmol) Propylphosphonsäureanhydrid (T3P, 50%ig in Essigsäureethylester, 4 eq.) portionsweise zugetropft und die Reaktionslösung für 6 Tage bei 60°C gerührt. Die Reaktion wurde auf Raumtemperatur abgekühlt und mit 50 ml Wasser versetzt. Es wurde dreimal mit 30 ml Essigsäureethylester extrahiert. Die vereinigten, organischen Phasen wurden dreimal mit pH 5 Puffer gewaschen und das Lösungsmittel unter reduziertem Druck entfernt. Der Rückstand wurde mittels präparativer HPLC [Säule: Chromatorex C18, 10µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 11.4 mg (10% d. Th.)
LC/MS [Methode 1]: Rₜ = 0.08 min; MS (ESIpos): m/z = 506 (M+H)⁺,
¹H-NMR (400 MHz, DMSO-d₆): δ [ppm] = 12.9 (br. s, 1H), 10.9 (s, 1H), 8.55 (br. s, 1H), 8.28 (s, 1H), 7.83-7.73 (m, 5H), 7.54 (dd, 1H), 7.43 (d, 1H), 6.66 (s, 1H), 5.68-5.60 (m, 1H), 4.29-3.90 (2x br. m, 2H), 2.26-2.16 (m, 1H), 2.12-1.98 (m, 1H), 0.93 (t, 3H).

### Beispiel 3

### 4-{[2-(10'-Chlor-2',5'-dioxo-5',6'-dihydrospiro[cyclopropan-1,7'-pyrido[3,4-d][2]benzazepin]-3'(2'H)-yl)butanoyl]amino}benzoesäure (Stereoisomerengemisch)

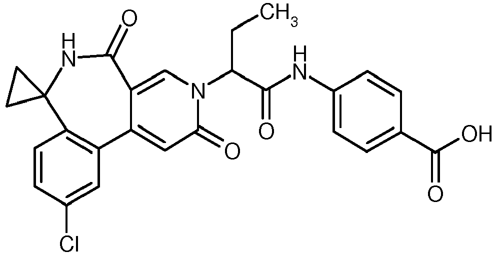

Nach der allgemeinen Methode 1 wurden 45 mg (82 µmol) 4-{[2-(10'-Chlor-2',5'-dioxo-5',6'-dihydrospiro[cyclopropan-1,7'-pyrido[3,4-*d*][2]benzazepin]-3'(2'*H*-yl)butanoyl]amino}benzoesäure-*tert*.-butylester (Stereoisomerengemisch) mit Trifluoressigsäure verseift. Das Lösungsmittel wurde unter reduziertem Druck entfernt und der Rückstand wurde mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 32 mg (78% d. Th.)
LC/MS [Methode 1]: Rₜ = 0.82 min; MS (ESIpos): m/z = 492 (M+H)⁺,
¹H-NMR (400 MHz, DMSO-d₆): δ [ppm] = 12.8 (br. s, 1H), 10.9 (2x s, 1H), 8.88/8.84 (2x s, 1H), 8.21 (s, 1H), 7.94-7.89 (m, 2H), 7.77-7.71 (m, 3H), 7.52 (dd, 1H), 7.39 (d, 1H), 6.66/6.65 (2x s, 1H), 5.68-5.61 (m, 1H), 2.27-2.16 (m, 1H), 2.12-1.99 (m, 1H), 1.53-1.45 (m, 1H), 1.21-1.11 (m, 1H), 0.98-0.81 (m, 4H), 0.63-0.53 (m, 1H).

### Beispiel 4

### 4-{[(11-Chlor-2,5-dioxo-5,6,7,8-tetrahydropyrido[3,4-e][3]benzazocin-3(2H)-yl)acetyl]amino}-benzoesäure

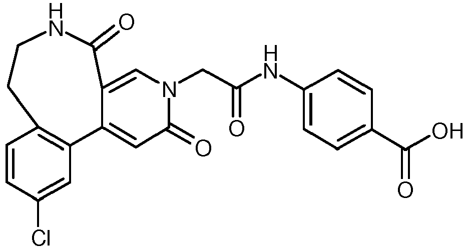

Nach der allgemeinen Methode 1 wurden 5.7 mg (11 µmol) 4-{[(11-Chlor-2,5-dioxo-5,6,7,8-tetrahydropyrido[3,4-*e*][3]benzazocin-3(2*H*)-yl)acetyl}-benzoesäure-*tert*.-butylester mit Trifluoressigsäure verseift. Das Rohprodukt wurde mittels präparativer HPLC (Methode 10) aufgereinigt. Ausbeute: 0.9 mg (17% d. Th.)
LC/MS [Methode 10]: Rₜ = 0.85 min; MS (ESIpos): m/z = 452 (M+H)⁺,
¹H-NMR (400 MHz, DMSO-d₆): δ [ppm] = 12.7 (br. s, 1H), 10.7 (s, 1H), 7.94-7.89 (m, 3H), 7.76-7.69 (m, 3H), 7.46 (dd, 1H), 7.32 (d, 1H), 7.24 (d, 1H), 6.31 (s, 1H), 4.87 (s, 2H), 3.49-3.42 (m, 1H), 2.99-2.92 (m, 2H).

### Beispiel 5

### 2-(11-Chlor-2,5-dioxo-5,6,7,8-tetrahydropyrido[3,4-e][3]benzazocin-3(2H)-yl)-N-[4-(5-oxo-4,5-dihydro-1,2,4-oxadiazol-3-yl)phenyl]acetamid

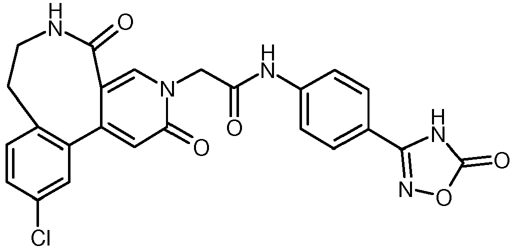

Nach der allgemeinen Methode 3 wurden 10 mg (30 µmol) (11-Chlor-2,5-dioxo-5,6,7,8-tetrahydropyrido[3,4-*e*][3]benzazocin-3(2*H*)-yl)essigsäure mit 6.4 mg (36 µmol, 1.2 eq.) 3-(4-Aminophenyl)-1,2,4-oxadiazol-5(4*H*)-on [Zerban, G. et al., WO 2007/071742, Beispiel 1] umgesetzt. Das Rohprodukt wurde mittels präparativer HPLC (Methode 10) aufgereinigt. Ausbeute: 2.4 mg (15% d. Th.)
LC/MS [Methode 10]: Rₜ = 0.87 min; MS (ESIpos): m/z = 492 (M+H)⁺,
¹H-NMR (400 MHz, DMSO-d₆): δ [ppm] = 12.9 (br. s, 1H), 10.7 (s, 1H), 7.91 (s, 1H), 7.78 (s, 4H), 7.73 (t, 1H), 7.46 (dd, 1H), 7.32 (d, 1H), 7.24 (d, 1H), 6.31 (s, 1H), 4.87 (s, 2H), 3.49-3.42 (m, 1H), 3.01-2.91 (m, 2H).

### Beispiel 6

### 4-{[2-(11-Chlor-2,5-dioxo-5,6,7,8-tetrahydropyrido[3,4-e][3]benzazocin-3(2H)-yl)butanoyl]-amino}benzoesäure(Stereoisomerengemisch)

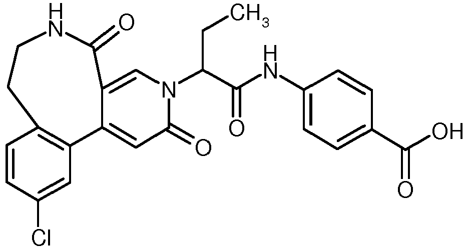

Nach der allgemeinen Methode 1 wurden 155 mg (289 µmol) 4-{[2-(11-Chlor-2,5-dioxo-5,6,7,8-tetrahydropyrido[3,4-*e*][3]benzazocin-3(2*H*)-yl)butanoyl]amino}benzoesäure-*tert*.-butylester (Stereoisomerengemisch) mit Trifluoressigsäure verseift und 11.6 mg des Rohproduktes wurden mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 9.5 mg (7% d. Th.)
LC/MS [Methode 1]: Rₜ = 0.78 min; MS (ESIpos): m/z = 480 (M+H)⁺,
¹H-NMR (400 MHz, DMSO-d₆): δ [ppm] = 12.8 (br. s, 1H), 10.8 (s, 1H), 7.93-7.89 (m, 2H), 7.85/7.79 (2x s, 1H), 7.80-7.71 (m, 3H), 7.47-7.43 (m, 1H), 7.31 (d, 1H), 7.28-7.26 (m, 1H), 6.33 (2x s, 1H), 5.63-5.56 (m, 1H), 3.51-3.24 (m, 2H), 2.99-2.90 (m, 2H), 2.23-2.01 (m, 2H), 0.92/0.90 (2x t, 3H).

### Beispiel 7

### 2-(11-Chlor-2,5-dioxo-5,6,7,8-tetrahydropyrido[3,4-e][3]benzazocin-3(2H)-yl)-N-[4-(5-oxo-4,5-dihydro-1,2,4-oxadiazol-3-yl)phenyl]butanamid(Stereoisomerengemisch)

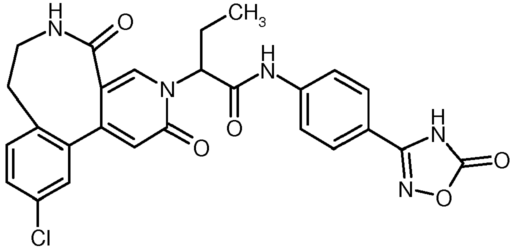

14 mg (39 µmol) 2-(11-Chlor-2,5-dioxo-5,6,7,8-tetrahydropyrido[3,4-*e*][3]benzazocin-3(2*H*)-yl)butansäure (Stereoisomerengemisch) wurden in 0.7 ml Dimethylformamid gelöst und mit 6.9 mg (39 µmol) 3-(4-Aminophenyl)-1,2,4-oxadiazol-5(4*H*)-on [Zerban, G. et al., WO 2007/071742, Beispiel 1] und 5.5 mg (39 µmol) Oxyma versetzt. Anschließend wurden 6.2 µl (39 µmol) Di-*iso*-propylcarbodiimid zugetropft und die Reaktionslösung über Nacht bei 40°C geschüttelt. Das Lösungsmittel wurde unter reduziertem Druck entfernt und der Rückstand mittels präparativer HPLC (Methode 10) gereinigt. Ausbeute: 3.3 mg (16% d. Th.)
LC/MS [Methode 1]: Rₜ = 0.81 min; MS (ESIpos): m/z = 520 (M+H)⁺,
¹H-NMR (400 MHz, DMSO-d₆): δ [ppm] = 12.8 (br. s, 1H), 10.9 (s, 1H), 7.87-7.75 (m, 6H), 7.48-7.44 (m, 1H), 7.32 (d, 1H), 7.29-7.26 (m, 1H), 6.34 (2x s, 1H), 5.63-5.56 (m, 1H), 3.51-3.23 (m, 2H), 3.00-2.90 (m, 2H), 2.25-2.03 (m, 2H), 0.92/0.91 (2x t, 3H).

### Beispiel 8

### 4-1[2-(11-Chlor-2,5-dioxo-5,6,7,8-tetrahydropyrido[3,4-e][3]benzazocin-3(2H)-yl)-4-methylpentanoyl]amino}benzoesäure(Stereoisomerengemisch)

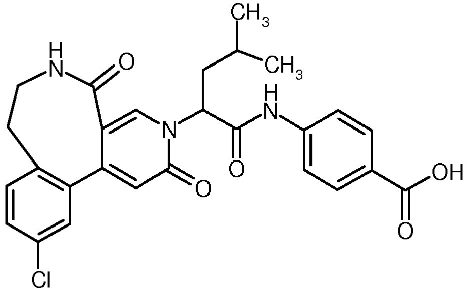

Nach der allgemeinen Methode 1 wurden 12 mg (21 µmol) 4-{[2-(11-Chlor-2,5-dioxo-5,6,7,8-tetrahydropyrido[3,4-*e*][3]benzazocin-3(2*H*)-yl)-4-methylpentanoyl]amino}benzoesäure-*tert*.-butylester (Stereoisomerengemisch) mit Trifluoressigsäure verseift. Das Lösungsmittel wurde unter reduziertem Druck entfernt, der Rückstand dreimal mit Dichlormethan und dreimal mit Toluol koevaporiert und abschließend aus Acetonitril/Wasser lyophilisiert. Ausbeute: 8.6 mg (79% d. Th.)
LC/MS [Methode 1]: Rₜ = 0.88 min; MS (ESIpos): m/z = 508 (M+H)⁺,
¹H-NMR (400 MHz, DMSO-d₆): δ [ppm] = 12.8 (br. s, 1H), 10.9 (s, 1H), 7.94-7.70 (m, 6H), 7.47-7.42 (m, 1H), 7.33-7.26 (m, 2H), 6.34/6.33 (2x s, 1H), 5.87-5.80 (m, 1H), 3.47-3.23 (m, 2H), 3.00-2.87 (m, 2H), 2.17-2.06 (m, 1H), 1.94-1.84 (m, 1H), 1.51-1.39 (m, 1H), 0.97-0.91 (m, 6H).

### Beispiel 9

### 2-(11-Chlor-2,5-dioxo-5,6,7,8-tetrahydropyrido[3,4-e][3]benzazocin-3(2H)-yl)-4-methyl-N-[4-(5-oxo-4,5-dihydro-1,2,4-oxadiazol-3-yl)phenyl]pentanamid(Stereoisomerengemisch)

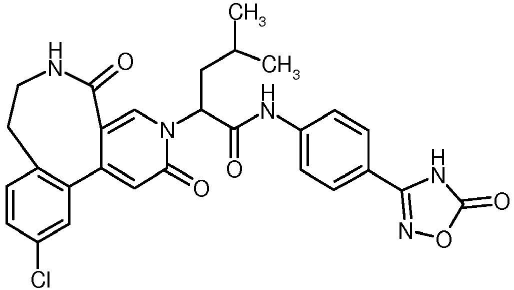

15 mg (39 µmol) 2-(11-Chlor-2,5-dioxo-5,6,7,8-tetrahydropyrido[3,4-*e*][3]benzazocin-3(2*H*)-yl)-4-methylpentansäure (Stereoisomerengemisch) wurden in 0.4 ml Dimethylformamid gelöst und mit 6.8 mg (39 µmol) 3-(4-Aminophenyl)-1,2,4-oxadiazol-5(4*H*)-on [Zerban, G. et al., 1. WO 2007/071742, Beispiel 1] und 5.5 mg (39 µmol) Oxyma versetzt. Anschließend wurden 6.0 µl (39 µmol) Di-*iso*-propylcarbodiimid zugetropft und die Reaktionslösung über Nacht bei 40°C geschüttelt. Es wurden weitere 14 mg (78 µmol) 3-(4-Aminophenyl)-1,2,4-oxadiazol-5(4*H*)-on und 12 µl (78 µmol) Di-*iso*-propylcarbodiimid hinzugegeben und bis zum vollständigen Umsatz bei 40°C geschüttelt. Das Lösungsmittel wurde unter reduziertem Druck entfernt und der Rückstand mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 6.6 mg (31% d. Th.)
LC/MS [Methode 1]: Rₜ = 0.91 min; MS (ESIpos): m/z = 548 (M+H)⁺,
¹H-NMR (400 MHz, DMSO-d₆): δ [ppm] = 12.9 (br. s, 1H), 10.9 (2x s, 1H), 7.90-7.74 (m, 6H), 7.48-7.43 (m, 1H), 7.33-7.26 (m, 2H), 6.35/6.34 (2x s, 1H), 5.87-5.80 (m, 1H), 3.46-3.23 (m, 2H), 2.99-2.87 (m, 2H), 2.18-2.08 (m, 1H), 1.94-1.84 (m, 1H), 1.51-1.39 (m, 1H), 0.97-0.91 (m, 6H).

### Beispiel 10

### 4-{[2-(11'-Chlor-2',5'-dioxo-2',5',6',8'-tetrahydro-3'H-spiro[cyclopropan-1,7'-pyrido[3,4-e][3]benzazocin]-3'-yl)butanoyl]amino}benzoesäure(Stereoisomerengemisch)

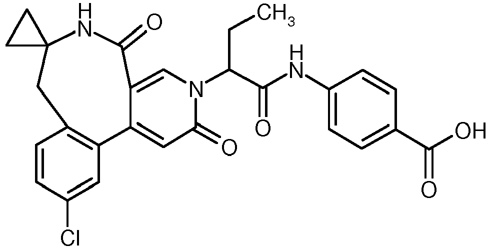

Nach der allgemeinen Methode 1 wurden 87.0 mg (155 µmol) 4-{[2-(11'-Chlor-2',5'-dioxo-2',5',6',8'-tetrahydro-3'*H*-spiro[cyclopropan-1,7'-pyrido[3,4-*e*][3]benzazocin]-3'-yl)butanoyl]-amino}benzoesäure-*tert*.-butylester (Stereoisomerengemisch) mit Trifluoressigsäure verseift. Das Lösungsmittel wurde unter reduziertem Druck entfernt, der Rückstand mehrmals mit Dichlormethan koevaporiert und mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 61.5 mg (79% d. Th.)
LC/MS [Methode 1]: Rₜ = 0.82/0.83 min; MS (ESIpos): m/z = 506 (M+H)⁺,
¹H-NMR (400 MHz, DMSO-d₆): δ [ppm] = 12.8 (br. s, 1H), 10.8 (2x s, 1H), 8.15-8.09 (2x s, 1H), 7.94-7.69 (m, 5H), 7.45 (dd, 1H), 7.33-7.28 (m, 2H), 6.33/6.32 (2x s, 1H), 5.72/5.62 (2x dd, 1H), 3.28-3.17 (m, 1H), 2.74-2.63 (m, 1H), 2.23-2.03 (m, 2H), 1.03-0.87 (m, 4H), 0.80-0.64 (m, 3H).

### Beispiel 11

### 2-(11'-Chlor-2',5'-dioxo-2',5',6',8'-tetrahydro-3'H-spiro[cyclopropan-1,7'-pyrido[3,4-e][3]benzazocin]-3'-yl)-N-[4-(5-oxo-4,5-dihydro-1,2,4-oxadiazol-3-yl)phenyl]butanamid (Stereoisomerengemisch)

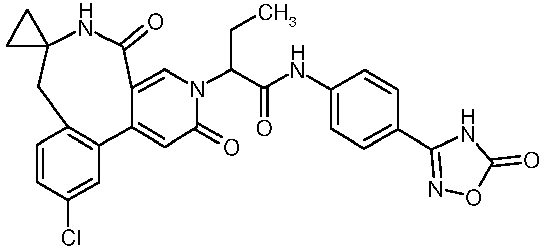

8.0 mg (19 µmol) 2-(11'-Chlor-2',5'-dioxo-2',5',6',8-tetrahydro-3'*H*-spiro[cyclopropan-1,7'-pyrido[3,4-*e*][3]benzazocin]-3'-yl)butansäure (Stereoisomerengemisch) wurden in 0.4 ml Dimethylformamid gelöst und mit 3.3 mg (19 µmol) 3-(4-Aminophenyl)-1,2,4-oxadiazol-5(4*H*)-on [Zerban, G. et al., WO 2007/071742, Beispiel 1] und 0.3 mg (2 µmol) Oxyma versetzt. Anschließend wurden 2.9 µl (19 µmol) Di-*iso*-propylcarbodiimid zugetropft und die Reaktionslösung über Nacht bei 40°C geschüttelt. Das Lösungsmittel wurde unter reduziertem Druck entfernt und der Rückstand mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] und abschließend durch weitere präparative HPLC (Methode 10) gereinigt. Ausbeute: 1.1 mg (11% d. Th.)
LC/MS [Methode 10]: Rₜ = 0.96/0.98 min; MS (ESIpos): m/z = 546 (M+H)⁺,
¹H-NMR (400 MHz, DMSO-d₆): δ [ppm] = 12.9 (br. s, 1H), 10.8 (2x s, 1H), 8.15/8.09 (2x s, 1H), 7.86-7.70 (m, 5H), 7.45 (dd, 1H), 7.33-7.27 (m, 2H), 6.33/6.32 (2x s, 1H), 5.72/5.62 (2x dd, 1H), 3.27-3.17 (m, 1H), 2.73-2.64 (m, 1H), 2.24-2.04 (m, 2H), 1.03-0.87 (m, 4H), 0.80-0.65 (m, 3H).

### Beispiel 12

### 4-{[2-(11'-Chlor-2',5'-dioxo-2',5',6',8'-tetrahydro-3'H-spiro[cyclopropan-1,7'-pyrido[3,4-e][3]benzazocin]-3'-yl)-4-methoxybutanoyl]amino}benzoesäure(Stereoisomerengemisch)

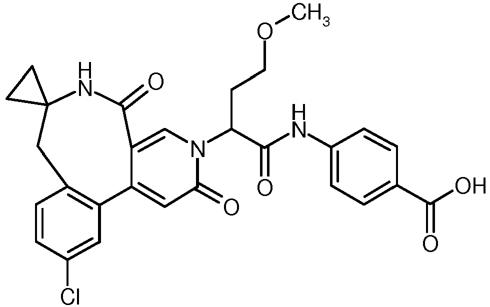

Nach der allgemeinen Methode 1 wurden 27 mg (45 µmol) 4-{[2-(11'-Chlor-2',5'-dioxo-2',5',6',8'-tetrahydro-3'H-spiro[cyclopropan-1,7'-pyrido[3,4-*e*][3]benzazocin]-3'-yl)-4-methoxybutanoyl]-amino}benzoesäure-*tert*.-butylester(Stereoisomerengemisch) mit Trifluoressigsäure verseift. Das Rohprodukt wurde mittels präparativer RP-HPLC (Reprosil C18, Wasser-Acetonitril-Gradient) aufgereinigt. Ausbeute: 13 mg (55% d. Th.)
LC/MS [Methode 2]: Rₜ = 2.27 min/2.31 min; MS (ESIpos): m/z = 536 (M+H)⁺,
¹H-NMR (400 MHz, DMSO-d₆): δ [ppm] = 12.75 (br. s, 1H), 10.82/10.75 (2x s, 1H), 8.14/8.07 (2x s, 1H), 7.94-7.71 (2x m, 5H), 7.45 (dd, 1H), 7.31 (d, 1H), 7.30-7.26 (m, 1H), 6.31 (s, 1H), 5.86/5.76 (t/dd, 1H), 3.45-3.36 (m, 1H), 3.3-3.14 (m, 2H), 3.23/3.20 (2x s, 3H), 2.75-2.65 (m, 1H), 2.44-2.30 (m, 2H), 1.04-0.93 (m, 1H), 0.82-0.72 (m, 2H), 0.72-0.64 (m, 1H).

### Beispiel 13

### 4-{[2-(11'-Chlor-2',5'-dioxo-2',5',6',8'-tetrahydro-3'H-spiro[cyclopropan-1,7'-pyrido[3,4-e][3]benzazocin]-3'-yl)-4-methoxybutanoyl]amino}-2-fluorbenzamid (Stereoisomerengemisch)

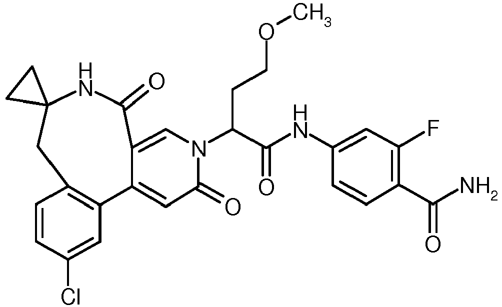

Nach der allgemeinen Methode 5 wurden 63 mg (0.15 mmol) 2-(11'-Chlor-2',5'-dioxo-2',5',6',8'-tetrahydro-3'*H*-spiro[cyclopropan-1,7'-pyrido[3,4-*e*] [3]benzazocin]-3'-yl)-4-methoxybutansäure (Stereoisomerengemisch) mit 35 mg (0.23 mmol, 1.5 eq.) 4-Amino-2-fluorbenzamid umgesetzt. Nach wässriger Aufarbeitung wurde das Rohprodukt mittels präparativer RP-HPLC (Reprosil C18, Wasser-Acetonitril-Gradient) aufgereinigt. Ausbeute: 21 mg (24% d. Th.)
LC/MS [Methode 2]: Rₜ = 2.15 min/2.18 min; MS (ESIpos): m/z = 553 (M+H)⁺,
¹H-NMR (400 MHz, DMSO-d₆): δ [ppm] = 10.90/10.80 (2x s, 1H), 8.14/8.08 (2x s, 1H), 7.87/7.72 (2x s, 1H), 7.71-7.59 (m, 2H), 7.57-7.50 (m, 2H), 7.45 (dd, 1H), 7.42 (dd, 1H), 7.31 (d, 1H), 7.29-7.25 (m, 1H), 6.32 (s, 1H), 5.84/5.73 (t/dd, 1H), 3.45-3.36 (m, 1H), 3.3-3.25 (m, 2H), 3.23/3.20 (2x s, 3H), 2.69 (dd, 1H), 2.45-2.27 (m, 2H), 1.04-0.92 (m, 1H), 0.82-0.63 (m, 3H).

### Beispiel 14

### 2-(11'-Chlor-2',5'-dioxo-2',5',6',8'-tetrahydro-3'H-spiro[cyclopropan-1,7'-pyrido[3,4-e][3]benzazocin]-3'-yl)-4-methoxy-N-(2-oxo-2,3-dihydro-1H-benzimidazol-5-yl)butanamid (Stereoisomerengemisch)

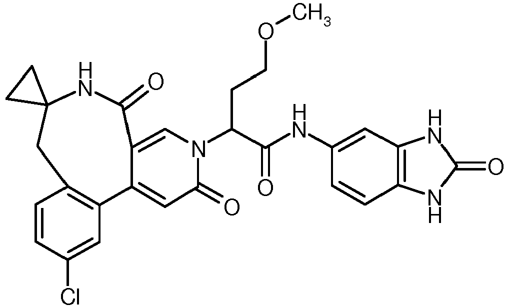

Nach der allgemeinen Methode 3 wurden 30 mg (72 µmol) 2-(11'-Chlor-2',5'-dioxo-2',5',6',8'-tetrahydro-3'*H*-spiro[cyclopropan-1,7'-pyrido[3,4-*e*] [3]benzazocin]-3'-yl)-4-methoxybutansäure (Stereoisomerengemisch) mit 12 mg (79 µmol, 1.1 eq.) 5-Amino-1,3-dihydro-2*H*-benzimidazol-2-on umgesetzt. Das Rohprodukt wurde mittels präparativer RP-HPLC (Reprosil C18, Wasser-Acetonitril-Gradient) aufgereinigt. Ausbeute: 21 mg (53% d. Th.)
LC/MS [Methode 1]: Rₜ = 0.68 min/0.70 min; MS (ESIpos): m/z = 548 (M+H)⁺,
¹H-NMR (400 MHz, DMSO-d₆): δ [ppm] = 10.59/10.55/10.51 (3x s, 2H), 10.37/10.33 (2x s, 1H), 8.13/8.06 (2x s, 1H), 7.89/7.71 (2x s, 1H), 7.48-7.40 (m, 2H), 7.34-7.25 (m, 2H), 7.10/7.07 (2x dd, 1H), 6.85 (d, 1H), 6.30 (s, 1H), 5.82/5.76 (t/dd, 1H), 3.43-3.35 (m, 1H), 3.3-3.15 (m, 2H), 3.23/3.21 (2x s, 3H), 2.69 (dd, 1H), 2.43-2.22 (m, 2H), 1.01-0.92 (m, 1H), 0.80-0.61 (m, 3H).

### Beispiel 15

### 2-(11'-Chlor-2',5'-dioxo-2',5',6',8'-tetrahydro-3'H-spiro[cyclopropan-1,7'-pyrido[3,4-e][3]benzazocin]-3'-yl)-4-methoxy-N-(2-methyl-2H-indazol-5-yl)butanamid(Stereoisomerengemisch)

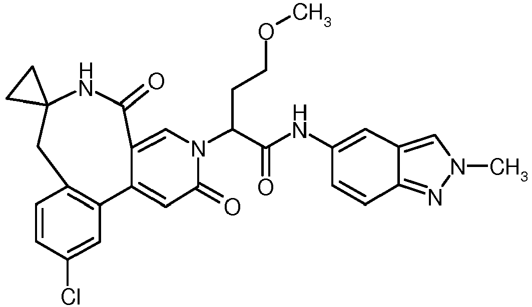

Nach der allgemeinen Methode 3 wurden 30 mg (72 µmol) 2-(11'-Chlor-2',5'-dioxo-2',5',6',8'-tetrahydro-3'*H*-spiro[cyclopropan-1,7'-pyrido[3,4-*e*][3]benzazocin]-3'-yl)-4-methoxybutansäure (Stereoisomerengemisch) mit 12 mg (79 µmol, 1.1 eq.) 2-Methyl-2*H*-indazol-5-amin umgesetzt. Das Rohprodukt wurde mittels präparativer RP-HPLC (Reprosil C18, Wasser-Acetonitril-Gradient) aufgereinigt. Ausbeute: 26 mg (66% d. Th.)
LC/MS [Methode 1]: Rₜ = 0.82 min/0.83 min; MS (ESIpos): m/z = 546 (M+H)⁺,
¹H-NMR (400 MHz, DMSO-d₆): δ [ppm] = 10.45/10.41 (2x s, 1H), 8.26 (d, 1H), 8.14 (s, 1H), 8.08 (d, 1H), 7.91/7.74 (2x s, 1H), 7.55 (d, 1H), 7.45 (dt, 1H), 7.34-7.26 (m, 3H), 6.31 (s, 1H), 5.88/5.80 (t/dd, 1H), 4.13 (s, 3H), 3.45-3.36 (m, 1H), 3.3-3.16 (m, 2H), 3.24/3.21 (2x s, 3H), 2.69 (dd, 1H), 2.45-2.25 (m, 2H), 1.04-0.92 (m, 1H), 0.82-0.62 (m, 3H).

### Beispiel 16

### 2-(11'-Chlor-2',5'-dioxo-2',5',6',8'-tetrahydro-3'H-spiro[cyclopropan-1,7'-pyrido[3,4-e][3]benzazocin]-3'-yl)-4-methoxy-N-(pyrazolo[1,5-a]pyridin-5-yl)butanamid(Stereoisomerengemisch)

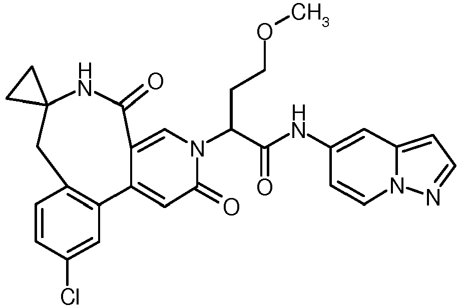

Nach der allgemeinen Methode 4 wurden 83 mg (0.2 mmol) 2-(11'-Chlor-2',5'-dioxo-2',5',6',8'-tetrahydro-3'*H*-spiro[cyclopropan-1,7'-pyrido[3,4-*e*][3]benzazocin]-3'-yl)-4-methoxybutansäure (Stereoisomerengemisch) mit 35 mg (0.26 mmol, 1.3 eq.) Pyrazolo[1,5-a]pyridin-5-amin [B.C. Baguley et al. Bioorganic and Medicinal Chemistry, 2012, 20, 69-85] umgesetzt. Nach wässriger Aufarbeitung wurde das Rohprodukt mittels präparativer RP-HPLC (Reprosil C18, Wasser-Acetonitril-Gradient) aufgereinigt. Ausbeute: 82 mg (77% d. Th.)
LC/MS [Methode 1]: Rₜ = 0.80 min; MS (ESIpos): m/z = 532 (M+H)⁺,
¹H-NMR (400 MHz, DMSO-d₆): δ [ppm] = 10.78/10.70 (2x s, 1H), 8.62 (d, 1H), 8.17-8.05 (m, 2H), 7.94-7.88 (m, 1H), 7.74 (s, 1H), 7.45 (dd, 1H), 7.34-7.25 (m, 2H), 6.99 (dd, 1H), 6.51 (dd, 1H), 6.32 (s, 1H), 5.87/5.76 (t/dd, 1H), 3.46-3.77 (m, 1H), 3.3-3.15 (m, 2H), 3.24/3.21 (2x s, 3H), 2.69 (dd, 1H), 2.45-2.28 (m, 2H), 1.02-0.93 (m, 1H), 0.82-0.63 (m, 3H).

### Beispiel 17

### 2-(11'-Chlor-2',5'-dioxo-2',5',6',8'-tetrahydro-3'H-spiro[cyclopropan-1,7'-pyrido[3,4-e][3]benzazocin]-3'-yl)-N-(imidazo[1,2-a]pyridin-6-yl)-4-methoxybutanamid(Stereoisomerengemisch)

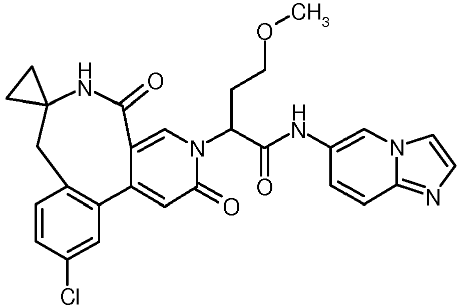

Nach der allgemeinen Methode 3 wurden 30 mg (72 µmol) 2-(11'-Chlor-2',5'-dioxo-2',5',6',8'-tetrahydro-3'*H*-spiro[cyclopropan-1,7'-pyrido[3,4-*e*][3]benzazocin]-3'-yl)-4-methoxybutansäure (Stereoisomerengemisch) mit 11 mg (79 µmol, 1.1 eq.) Imidazo[1,2-*a*]pyridin-6-amin umgesetzt. Das Rohprodukt wurde mittels präparativer RP-HPLC (Reprosil C18, Wasser-Acetonitril-Gradient) aufgereinigt. Ausbeute: 21 mg (54% d. Th.)
LC/MS [Methode 1]: Rₜ = 0.63 min/0.64 min; MS (ESIpos): m/z = 532 (M+H)⁺,
¹H-NMR (400 MHz, DMSO-d₆): δ [ppm] = 11.11/10.95 (2x s, 1H), 9.60/9.51 (2x s, 1H), 8.40 (d, 1H), 8.18-8.06 (m, 2H), 7.98-7.89 (m, 1.5H), 7.89-7.82 (m, 1H), 7.76 (s, 0.5H), 7.49-7.43 (m, 1H), 7.32 (d, 1H), 7.26 (dd, 1H), 6.33 (s, 1H), 5.91/5.71 (t/dd, 1H), 3.49-3.40 (m, 2H), 3.3-3.14 (m, 1H), 3.24/3.21 (2x s, 3H), 2.70 (dd, 1H), 2.47-2.37 (m, 2H), 1.04-0.92 (m, 1H), 0.83-0.65 (m, 3H).

### Beispiel 18

### 2-(11'-Chlor-2',5'-dioxo-2',5',6',8'-tetrahydro-3'H-spiro[cyclopropan-1,7'-pyrido[3,4-e][3]benzazocin]-3'-yl)-N-(1H-indazol-5-yl)-4-methoxybutanamid(Stereoisomerengemisch)

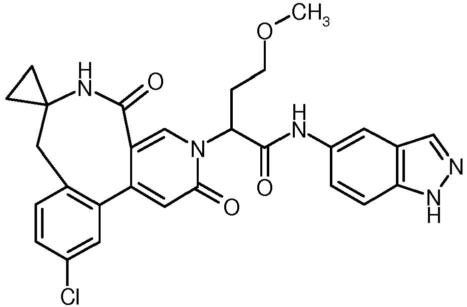

Nach der allgemeinen Methode 3 wurden 30 mg (72 µmol) 2-(11'-Chlor-2',5'-dioxo-2',5',6',8'-tetrahydro-3'*H*-spiro[cyclopropan-1,7'-pyrido[3,4-*e*] [3]benzazocin]-3'-yl)-4-methoxybutansäure (Stereoisomerengemisch) mit 11 mg (79 µmol, 1.1 eq.) 1*H*-Indazol-5-amin umgesetzt. Das Rohprodukt wurde mittels präparativer RP-HPLC (Reprosil C18, Wasser-Acetonitril-Gradient) aufgereinigt. Ausbeute: 26 mg (68% d. Th.)
LC/MS [Methode 1]: Rₜ = 0.77 min/0.78 min; MS (ESIpos): m/z = 532 (M+H)⁺,
¹H-NMR (400 MHz, DMSO-d₆): δ [ppm] = 13.00 (s, 1H), 10.52/10.47 (2x s, 1H), 8.14/8.10/8.07 (3x s, 2H), 8.02 (d, 1H), 7.91/7.74 (2x s, 1H), 7.53-7.42 (m, 3H), 7.31 (d, 1H), 7.29-7.26 (m, 1H), 6.32 (s, 1H), 5.89/5.81 (t/dd, 1H), 3.45-3.37 (m, 1H), 3.3-3.15 (m, 2H), 3.25/3.22 (2x s, 3H), 2.69 (dd, 1H), 2.46-2.25 (m, 2H), 1.04-0.93 (m, 1H), 0.82-0.61 (m, 3H).

### Beispiel 19

### 2-(11'-Chlor-2',5'-dioxo-2',5',6',8'-tetrahydro-3'H-spiro[cyclopropan-1,7'-pyrido[3,4-e][3]benzazocin]-3'-yl)-4-methoxy-N-(2-methyl-3-oxo-2,3-dihydro-1H-indazol-6-yl)butanamid (Stereoisomerengemisch)

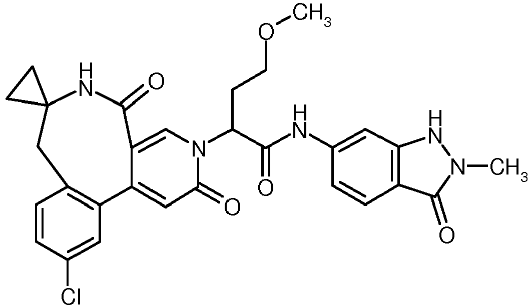

Nach der allgemeinen Methode 1 wurden 81 mg (122 µmol) 6-{[2-(11'-Chlor-2',5'-dioxo-2',5',6',8'-tetrahydro-3'*H*-spiro[cyclopropan-1,7'-pyrido[3,4-*e*][3]benzazocin]-3'-yl)-4-methoxybutanoyl]-amino}-2-methyl-3-oxo-2,3-dihydro-1*H*-indazol-1-carbonsäure-*tert*.-butylester (Stereoisomerengemisch) mit Trifluoressigsäure umgesetzt. Das Rohprodukt wurde mittels präparativer RP-HPLC (Reprosil C18, Wasser-Acetonitril-Gradient) aufgereinigt. Ausbeute: 35 mg (51% d. Th.)
LC/MS [Methode 1]: Rₜ = 0.70 min/0.72 min; MS (ESIpos): m/z = 562 (M+H)⁺,
¹H-NMR (400 MHz, DMSO-d₆): δ [ppm] = 10.74/10.70 (2x s, 1H), 10.23/10.21 (2x s, 1H), 8.15/8.09 (2x s, 1H), 7.89/7.73 (2x s, 1H), 7.76 (d, 1H), 7.56 (d, 1H), 7.45 (dd, 1H), 7.31 (d, 1H), 7.30-7.26 (m, 1H), 7.20-7.15 (m, 1H), 6.31 (s, 1H), 5.87/5.79 (t/dd, 1H), 3.45-3.37 (m, 1H), 3.3-3.15 (m, 2H), 3.23/3.21 (m, 3H), 2.69 (dd, 1H), 2.46-2.29 (m, 2H), 1.05-0.93 (m, 1H), 0.82-0.63 (m, 3H).

### Beispiel 20

### 5-{[2-(11'-Chlor-2',5'-dioxo-2',5',6',8'-tetrahydro-3'H-spiro[cyclopropan-1,7'-pyrido[3,4-e][3]benzazocin]-3'-yl)-4-methoxybutanoyl]amino}pyridin-2-carbonsäure (Stereoisomerengemisch)

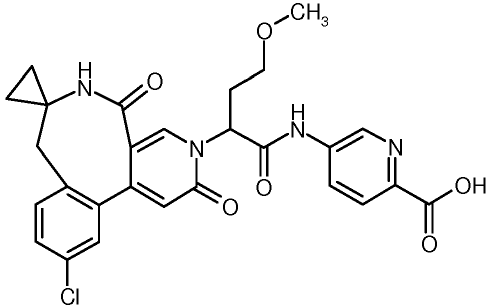

Nach der allgemeinen Methode 2 wurden 135 mg (89% Reinheit, 218 µmol) 5-{[2-(11'-Chlor-2',5'-dioxo-2',5',6',8'-tetrahydro-3'*H*-spiro[cyclopropan-1,7'-pyrido[3,4-*e*][3]benzazocin]-3'-yl)-4-methoxybutanoyl] amino }pyridin-2-carbonsäuremethylester (Stereoisomerengemisch) in Gegenwart von Lithiumhydroxid verseift. Nach wässriger Aufarbeitung wurde das Rohprodukt mittels präparativer RP-HPLC (Reprosil C18, Wasser-Acetonitril-Gradient) aufgereinigt. Ausbeute: 41 mg (35% d. Th.)
LC/MS [Methode 1]: Rₜ = 0.70 min; MS (ESIpos): m/z = 537 (M+H)⁺,
¹H-NMR (400 MHz, DMSO-d₆): δ [ppm] = 13.0 (br. s, 1H), 11.06/10.94 (2x s, 1H), 8.92/8.86 (2x d, 1H), 8.27-8.20 (m, 1H), 8.14/8.09 (2x s, 1H), 8.05 (d, 1H), 7.88/7.74 (2x s, 1H), 7.45 (dd, 1H), 7.34-7.25 (m, 2H), 6.32 (s, 1H), 5.88/5.74 (t/dd, 1H), 3.45-3.38 (m, 1H), 3.3-3.15 (m, 2H), 3.24/3.21 (2x s, 3H), 2.69 (dd, 1H), 2.47-2.31 (m, 2H), 1.03-0.92 (m, 1H), 0.82-0.64 (m, 3H).

### Beispiel 21

### 5-{[2-(11'-Chlor-2',5'-dioxo-2',5',6',8'-tetrahydro-3'H-spiro[cyclopropan-1,7'-pyrido[3,4-e][3]benzazocin]-3'-yl)-4-methoxybutanoyl]amino}pyridin-2-carboxamid(Stereoisomerengemisch)

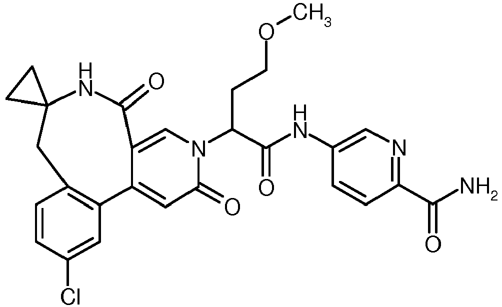

Nach der allgemeinen Methode 4 wurden 41 mg (76 µmol) 5-{[2-(11'-Chlor-2',5'-dioxo-2',5',6',8'-tetrahydro-3'*H*-spiro[cyclopropan-1,7'-pyrido[3,4-*e*][3]benzazocin]-3'-yl)-4-methoxybutanoyl]-amino}pyridin-2-carbonsäure(Stereoisomerengemisch) mit 7 mg (89 µmol, 1.2 eq.) Ammoniumacetat umgesetzt. Das Rohprodukt wurde mittels präparativer RP-HPLC (Reprosil C18, Wasser-Acetonitril-Gradient) aufgereinigt. Ausbeute: 2 mg (6% d. Th.)
LC/MS [Methode 2]: Rₜ = 2.03 min/2.07 min; MS (ESIpos): m/z = 536 (M+H)⁺,
¹H-NMR (400 MHz, DMSO-d₆): δ [ppm] = 11.08-10.85 (m, 1H), 8.94-8.79 (m, 1H), 8.28-8.17 (m 1H), 8.11 (d, 1H), 8.07-7.97 (m, 2H), 7.89/7.73 (2x s, 1H), 7.53 (s, 1H), 7.46 (d, 1H), 7.37-7.24 (m, 2H), 6.32 (s, 1H), 5.93-5.70 (2x m, 1H), 3.47-3.38 (m, 1H), 3.3-3.13 (m, 2H), 3.24/3.21 (2x s, 3H), 2.69 (dd, 1H), 2.46-2.29 (m, 2H), 1.04-0.93 (m, 1H), 0.83-0.63 (m, 3H).

### Beispiel 22

### 5-{[2-(11'-Chlor-2',5'-dioxo-2',5',6',8'-tetrahydro-3'H-spiro[cyclopropan-1,7'-pyrido[3,4-e][3]benzazocin]-3'-yl)-4-methoxybutanoyl]amino}thiophen-2-carbonsäure (Stereoisomerengemisch)

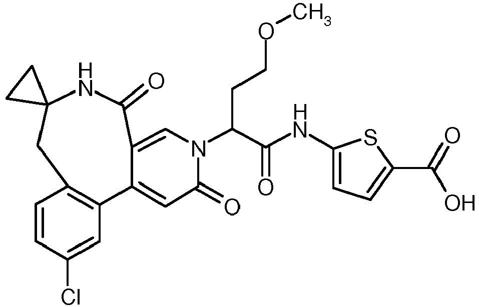

Gemäß der allgemeinen Methode 2 wurden 66 mg (0.12 mmol) 5-{[2-(11'-Chlor-2',5'-dioxo-2',5',6',8'-tetrahydro-3'*H*-spiro[cyclopropan-1,7'-pyrido[3,4-*e*][3]benzazocin]-3'-yl)-4-methoxybutanoyl] amino} thiophen-2-carbonsäuremethylester (Stereoisomerengemisch) mit Lithiumhydroxid verseift. Nach wässriger Aufarbeitung wurde das Rohprodukt mittels präparativer RP-HPLC (Reprosil C18, Wasser-Acetonitril-Gradient) aufgereinigt. Ausbeute: 10 mg (15% d. Th.)
LC/MS [Methode 2]: Rₜ = 2.31 min/2.35 min; MS (ESIpos): m/z = 542 (M+H)⁺,
¹H-NMR (400 MHz, DMSO-d₆): δ [ppm] = 12.6 (br. s, 1H), 12.13/11.96 (2x s, 1H), 8.14/8.09 (2x s, 1H), 7.89/7.74 (2x s, 1H), 7.56-7.49 (m, 1H), 7.49-7.41 (m, 1H), 7.35-7.24 (m, 2H), 6.84-6.76 (m, 1H), 6.31 (s, 1H), 5.86/5.68 (2x dd, 1H), 3.45-3.35 (m, 1H), 3.3-3.14 (m, 2H), 3.23/3.20 (2x s, 3H), 2.70 (dd, 1H), 2.47-2.29 (m, 2H), 1.04-0.93 (m, 1H), 0.82-0.64 (m, 3H).

### Beispiel 23

### 5-{[2-(11'-Chlor-2',5'-dioxo-2',5',6',8'-tetrahydro-3'H-spiro[cyclopropan-1,7'-pyrido[3,4-e][3]benzazocin]-3'-yl)-4-methoxybutanoyl]amino}thiophen-3-carbonsäure (Stereoisomerengemisch)

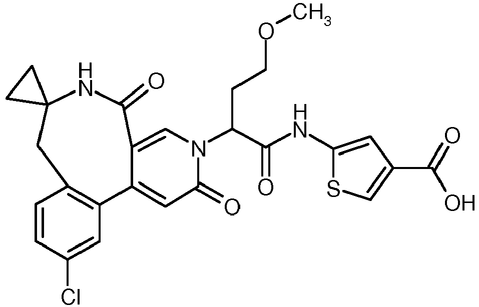

Gemäß der allgemeinen Methode 2 wurden 116 mg (0.21 mmol) 5-{[2-(11'-Chlor-2',5'-dioxo-2',5',6',8'-tetrahydro-3'*H*-spiro[cyclopropan-1,7'-pyrido[3,4-*e*][3]benzazocin]-3'-yl)-4-methoxybutanoyl] amino} thiophen-3-carbonsäuremethylester (Stereoisomerengemisch) mit Lithiumhydroxid verseift (Rühren bei 80°C Ölbadtemperatur für 50 h). Nach wässriger Aufarbeitung wurde das Rohprodukt mittels präparativer RP-HPLC (Reprosil C18, Wasser-Acetonitril-Gradient) aufgereinigt. Ausbeute: 9 mg (8% d. Th.)
LC/MS [Methode 1]: Rₜ = 0.81 min; MS (ESIpos): m/z = 542 (M+H)⁺,
¹H-NMR (400 MHz, DMSO-d₆): δ [ppm] = 12.5 (br. s, 1H), 11.87/11.68 (2x s, 1H), 8.13/8.09 (2x s, 1H), 7.88/7.73 (2x s, 1H), 7.70 (d, 1H), 7.49-7.41 (m, 1H), 7.35-7.25 (m, 2H), 7.04 (s, 1H), 6.31 (s, 1H), 5.85/5.66 (2x dd, 1H), 3.45-3.35 (m, 1H), 3.3-3.14 (m, 2H), 3.24/3.21 (2x s, 3H), 2.70 (dd, 1H), 2.46-2.28 (m, 2H), 1.04-0.92 (m, 1H), 0.82-0.64 (m, 3H).

### Beispiel 24

### 4-{[2-(11'-Chlor-2',5'-dioxo-2',5',6',8'-tetrahydro-3'H-spiro[cyclopropan-1,7'-pyrido[3,4-e] [3]benzazocin] -3'-yl)-4-methoxybutanoyl] amino}thiophen-2-carbonsäure (Stereoisomerengemisch)

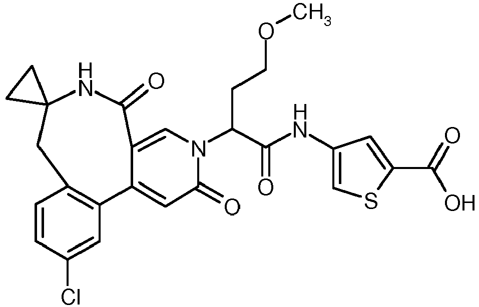

Gemäß der allgemeinen Methode 2 wurden 83 mg (0.15 mmol) 4-{[2-(11'-Chlor-2',5'-dioxo-2',5',6',8'-tetrahydro-3'*H*-spiro[cyclopropan-1,7'-pyrido[3,4-e][3]benzazocin]-3'-yl)-4-methoxybutanoyl] amino} thiophen-2-carbonsäuremethylester (Stereoisomerengemisch) mit Lithiumhydroxid verseift (Rühren bei 80°C Ölbadtemperatur für 3 h). Nach wässriger Aufarbeitung wurde das Rohprodukt mittels präparativer RP-HPLC (Reprosil C18, Wasser-Acetonitril-Gradient) aufgereinigt. Ausbeute: 55 mg (68% d. Th.)
LC/MS [Methode 2]: Rₜ = 2.33/2.36 min; MS (ESIpos): m/z = 542 (M+H)⁺,
¹H-NMR (400 MHz, DMSO-d₆): δ [ppm] = 13.2 (br. s, 1H), 11.00/10.88 (2x s, 1H), 8.13/8.09 (2x s, 1H), 7.87/7.72 (2x s, 1H), 7.85-7.77 (2x dd, 2H), 7.45 (dt, 1H), 7.31 (d, 1H), 7.28 (t, 1H), 6.31 (s, 1H), 5.80/5.66 (2x dd, 1H), 3.43-3.35 (m, 1H), 3.3-3.16 (m, 2H), 3.24/3.21 (2x s, 3H), 2.69 (dd, 1H), 2.46-2.25 (m, 2H), 1.02-0.92 (m, 1H), 0.81-0.63 (m, 3H).

### Beispiel 25

### 4-{[2-(11'-Chlor-2',5'-dioxo-2',5',6',8'-tetrahydro-3'H-spiro[cyclopropan-1,7'-pyrido[3,4-e][3]benzazocin]-3'-yl)-3-(trans-4-methoxycyclohexyl)propanoyl]amino}benzoesäure (Stereoisomerengemisch)

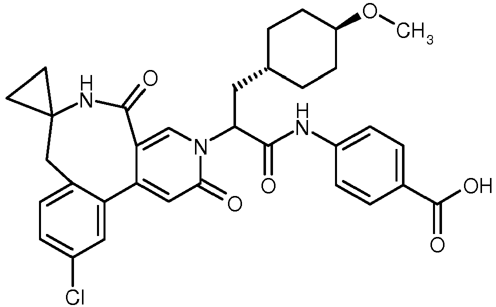

Nach der allgemeinen Methode 6 wurden 41 mg (65 µmol) 4-{[2-(11'-Chlor-2',5'-dioxo-2',5',6',8'-tetrahydro-3'*H*-spiro[cyclopropan-1,7'-pyrido[3,4-*e*][3]benzazocin]-3'-yl)-3-(*trans*-4-methoxycyclo-hexyl)propanoyl]amino}benzosäureethylester (Stereoisomerengemisch) mit 42.3 mg (130 µmol, 2 eq.) Cäsiumcarbonat verseift. Das Rohprodukt wurde mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] aufgereinigt. Ausbeute: 6.1 mg (15% d. Th.)
LC/MS [Methode 2]: Rₜ = 2.69 min/2.76 min; MS (ESIpos): m/z = 604 (M+H)⁺,
¹H-NMR (400 MHz, DMSO-d₆): δ [ppm] = 10.80/10.75 (2x s, 1H), 8.16/8.09 (2x s, 1H), 7.92-7.62 (m, 5H), 7.48-7.43 (m, 1H), 7.33-7.27 (m, 2H), 6.33/6.32 (2x s, 1H), 5.93/5.83 (2x dd, 1H), 3.22/3.20 (2x s, 3H), 3.09-3.00 (m, 1H), 2.68 (dd, 1H), 2.34-1.70 (m, 6H), 1.16-0.61 (m, 9H).

### Beispiel 26

### 4-{[2-(11'-Chlor-2',5'-dioxo-2',5',6',8'-tetrahydro-3'H-spiro[cyclopropan-1,7'-pyrido[3,4-e][3]benzazocin]-3'-yl)-3-(trans-4-methoxycyclohexyl)propanoyl]amino}-2-fluorbenzoesäureamid (Stereoisomerengemisch)

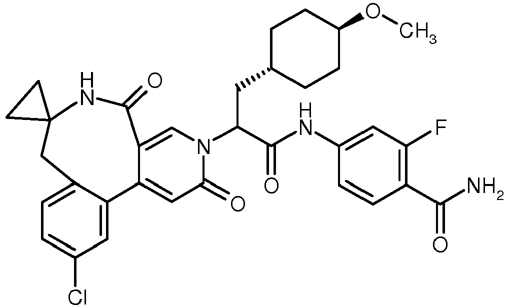

Gemäß allgemeiner Methode 7 wurden 50.0 mg (103 µmol) 2-(11'-Chlor-2',5'-dioxo-2',5',6',8'-tetrahydro-3'*H*-spiro[cyclopropan-1,7'-pyrido[3,4-*e*][3]benzazocin]-3'-yl)-3-(*trans*-4-methoxy-cyclohexyl)propansäure (Stereoisomerengemisch), 15.9 mg (103 µmol) 4-Amino-2-fluorbenzamid, 10 mg (72 µmol) Oxima und 16.0 µl (103 µmol) DIC in 1 ml Dimethylformamid zur Reaktion gebracht. Das Lösungsmittel wurde unter reduziertem Druck entfernt und der Rückstand mittels präparativer HPLC [Säule: Chromatorex C18, 10 µm, 125 mm x 30 mm, Laufmittel: Acetonitril/ 0.1% Ameisensäure-Gradient (0 bis 3 min 10% Acetonitril, bis 35 min 90% Acetonitril und weitere 3 min 90% Acetonitril)] gereinigt. Ausbeute: 30.4 mg (47% d. Th.)
LC/MS [Methode 1]: Rₜ = 0.87 min, 0.88 min; MS (ESIpos): m/z = 621 (M+H)⁺
¹H-NMR (400 MHz, DMSO-d₆): δ [ppm] = 10.92/10.85 (2x s, 1H), 8.17/8.10 (2x s, 1H), 7.87-7.27 (m, 9H), 6.34/6.33 (2x s, 1H), 5.91/5.79 (2x dd, 1H), 3.26-3.16 (m, 1H), 3.22/3.20 (2x s, 3H), 3.10-3.00 (m, 1H), 2.67 (dd, 1H), 2.34-1.71 (m, 6H), 1.16-0.60 (m, 9H).

### Beispiel 27

### 2-(11'-Chlor-2',5'-dioxo-2',5',6',8'-tetrahydro-3'H-spiro[cyclopropan-1,7'-pyrido[3,4-e][3]benzazocin]-3'-yl)-3-(trans-4-methoxycyclohexyl)-N-(2-oxo-2,3-dihydro-1H-benzimidazol-5-yl)-propanamid (Stereoisomerengemisch)

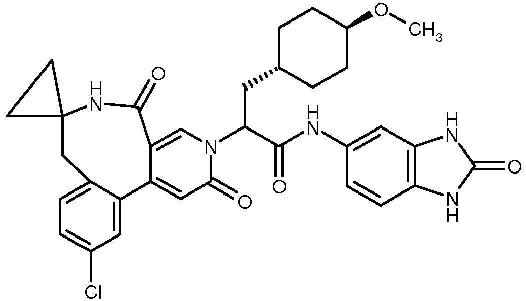

Gemäß allgemeiner Methode 7 wurden 30 mg (62 µmol) 2-(11'-Chlor-2',5'-dioxo-2',5',6',8'-tetrahydro-3'*H*-spiro[cyclopropan-1,7'-pyrido[3,4-*e*][3]benzazocin]-3'-yl)-3-(*trans*-4-meth-oxycyclohexyl)propansäure (Stereoisomerengemisch), 9.2 mg (62 µmol) 5-Amino-1,3-dihydro-2*H-*benzimidazol-2-on, 6.2 mg (43 µmol) Oxima und 9.6 µl (62 µmol) DIC in 1 ml Dimethylformamid zur Reaktion gebracht. Das Lösungsmittel wurde unter reduziertem Druck entfernt und der Rückstand mittels präparativer HPLC [Säule: Chromatorex C18, 10 µm, 125 mm x 30 mm, Laufmittel: Acetonitril/0.1% Ameisensäure-Gradient (0 bis 3 min 10% Acetonitril, bis 35 min 90% Acetonitril und weitere 3 min 90% Acetonitril)] gereinigt. Ausbeute: 30.0 mg (78% d. Th.)
LC/MS [Methode 1]: Rₜ = 0.81 min, 0.84 min; MS (ESIpos): m/z = 616 (M+H)⁺
¹H-NMR (400 MHz, DMSO-d₆): δ [ppm] = 10.60/10.55 (2x s, 1H), 10.51 (s, 1H), 10.39/10.37 (2x s, 1H), 8.15/8.8.08 (2x s, 1H), 7.88/7.68 (2x s, 1H), 7.47-7.26 (m, 4H), 7.10-7.02 (m, 1H), 6.88-6.81 (m, 1H), 6.32/6.31 (2x s, 1H), 5.87/5.81 (2x dd, 1H), 3.25-3.15 (m, 1H), 3.22/3.20 (2x s, 3H), 3.10-2.99 (m, 1H), 2.74-2.60 (m, 1H), 2.25-1.71 (m, 6H), 1.16-0.58 (m, 9H).

### Beispiel 28

### 4-{[2-(11'-Chlor-2',5'-dioxo-2',5',6',8'-tetrahydro-3'H-spiro[cyclopropan-1,7'-pyrido[3,4-e][3]benzazocin]-3'-yl)-3-phenylpropanoyl]amino}benzoesäure(Stereoisomerengemisch)

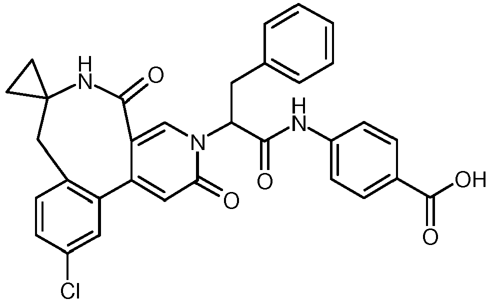

Nach der allgemeinen Methode 1 wurden 88 mg (14 µmol) 4-{[2-(11'-Chlor-2',5'-dioxo-2',5',6',8'-tetrahydro-3'*H*-spiro[cyclopropan-1,7'-pyrido[3,4-e][3]benzazocin]-3'-yl)-3-phenylpropanoyl]-amino}benzoesäure-*tert*.-butylester (Stereoisomerengemisch) mit Trifluoressigsäure verseift. Das Rohprodukt wurde mittels präparativer RP-HPLC (Reprosil C18, Wasser-Acetonitril-Gradient) aufgereinigt. Ausbeute: 27 mg (33% d. Th.)
LC/MS [Methode 2]: Rₜ = 2.83 min/2.90 min; MS (ESIpos): m/z = 568 (M+H)⁺,
¹H-NMR (400 MHz, DMSO-d₆): δ [ppm] = 12.75 (br. s, 1H), 10.96/10.78 (2x s, 1H), 8.09/8.00 (2x s, 1H), 7.98-7.83 (m, 3H), 7.80-7.70 (m, 2H), 7.46-7.39 (m, 1H), 7.37-7.10 (m, 7H), 6.28/6.03 (2x dd, 1H), 6.22/6.18 (2x s, 1H), 3.64-3.46 (m, 2H), 3.20/3.03 (2x d, 1H), 2.62 (d, 0.4H), 0.97-0.83 (m, 1H), 0.76-0.56 (m, 2H), 0.36-0.21 (m, 1H).

### Beispiel 29

### 2-(11'-Chlor-2',5'-dioxo-2',5',6',8'-tetrahydro-3'H-spiro[cyclopropan-1,7'-pyrido[3,4-e][3]benzazocin]-3'-yl)-N-(2-oxo-2,3-dihydro-1H-benzimidazol-5-yl)-3-phenylpropanamid (Stereoisomerengemisch)

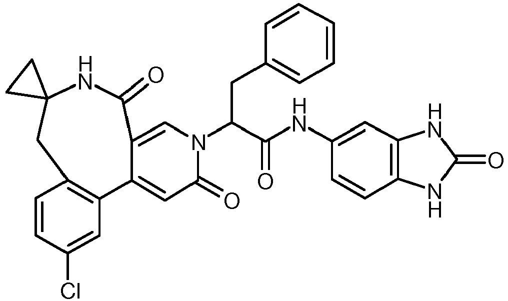

Nach der allgemeinen Methode 5 wurden 30 mg (63% Reinheit, 42 µmol) 2-(11'-Chlor-2',5'-dioxo-2',5',6',8'-tetrahydro-3'*H*-spiro[cyclopropan-1,7'-pyrido[3,4-e][3]benzazocin]-3'-yl)-3-phenylpropansäure (Stereoisomerengemisch) mit 9 mg (63 µmol, 1.5 eq.) 5-Amino-1,3-dihydro-2*H-*benzimidazol-2-on umgesetzt. Das Rohprodukt wurde mittels präparativer RP-HPLC (Reprosil C18, Wasser-Acetonitril-Gradient) aufgereinigt. Ausbeute: 16 mg (64% d. Th.)
LC/MS [Methode 11]: Rₜ = 1.49 min/1.53 min; MS (ESIpos): m/z = 580 (M+H)⁺,
¹H-NMR (400 MHz, DMSO-d₆): δ [ppm] = 10.62/10.57/10.53/10.50/10.36 (6x s, 3H), 8.10/8.00/7.95/7.84 (4x s, 2H), 7.47-7.39 (m, 2H), 7.36-7.15 (m, 7H), 7.10/7.05 (2x dd, 1H), 6.88/6.87 (2x d, 1H), 6.22/6.02 (2x dd, 1H), 6.21/6.17 (2x s, 1H), 3.55-3.43 (m 2H), 3.20/3.03 (2x d, 1H), 2.62/2.54 (2x d, 1H), 0.96-0.84 (m, 1H), 0.75-0.56 (m, 1.5H), 0.38-0.22 (m, 1.5H).

### Beispiel 30

### 2-(11'-Chlor-2',5'-dioxo-2',5',6',8'-tetrahydro-3'H-spiro[cyclopropan-1,7'-pyrido[3,4-e][3]benzazocin]-3'-yl)-N-(2-methyl-2H-indazol-5-yl)-3-phenylpropanamid (Stereoisomerengemisch)

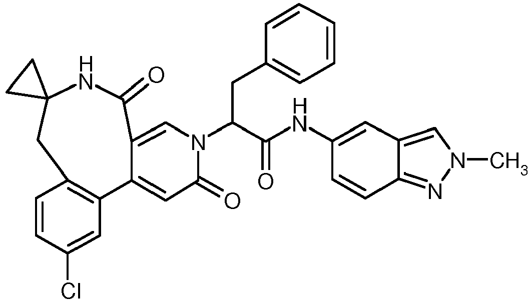

Nach der allgemeinen Methode 5 wurden 118 mg (63% Reinheit, 0.17 mmol) 2-(11'-Chlor-2',5'-dioxo-2',5',6',8'-tetrahydro-3'*H*-spiro[cyclopropan-1,7'-pyrido[3,4-e][3]benzazocin]-3'-yl)-3-phenylpropansäure (Stereoisomerengemisch) mit 37 mg (25 mmol, 1.5 eq.) 2-Methyl-2*H*-indazol-5-amin umgesetzt. Das Rohprodukt wurde mittels präparativer RP-HPLC (Reprosil C18, Wasser-Acetonitril-Gradient) aufgereinigt. Ausbeute: 33 mg (35% d. Th.)
LC/MS [Methode 1]: Rₜ = 0.93 min/0.95 min; MS (ESIpos): m/z = 578 (M+H)⁺,
¹H-NMR (400 MHz, DMSO-d₆): δ [ppm] = 10.59/10.43 (2x s, 1H), 8.28 (s, 1H), 8.17/8.09/8.02/7.95/7.86 (5x s, 3H), 7.61-7.55 (m, 1H), 7.46-7.40 (m, 1H), 7.38-7.10 (m, 8H), 6.28/6.06 (2x dd, 1H), 6.22/6.18 (2x s, 1H), 4.14 (s, 3H), 3.59-3.45 (m, 2H), 3.20/3.04 (2x d, 1H), 2.62/2.54 (2x d, 1H), 0.97-0.85 (m, 1H), 0.77-0.57 (m, 2H), 0.39-0.23 (m, 1H).

### Beispiel 31

### 2-[11-Chlor-7-(fluormethyl)-2,5-dioxo-5,6,7,8-tetrahydropyrido[3,4-e][3]benzazocin-3(2H)-yl]-4-methoxy-N-(2-methyl-2H-indazol-5-yl)butanamid(Stereoisomerengemisch)

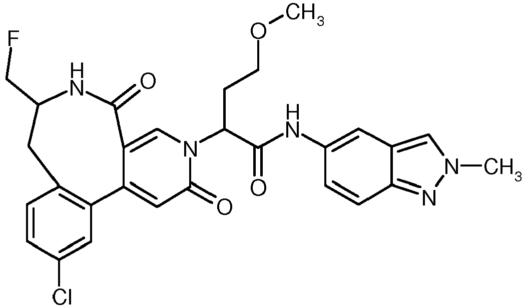

Nach der allgemeinen Methode 5 wurden 10 mg (24 µmol) 2-[ll-Chlor-7-(fluormethyl)-2,5-dioxo-5,6,7,8-tetrahydropyrido[3,4-*e*][3]benzazocin-3(2*H*)-yl]-4-methoxybutansäure (Stereoisomerengemisch) mit 6.3 mg (43 µmol, 1.8 eq.) 2-Methyl-2*H*-indazol-5-amin umgesetzt. Das Rohprodukt wurde mittels präparativer HPLC (Wasser-Acetonitril-0.1% Ameisensäure-Gradient) aufgereinigt. Ausbeute: 5 mg (38% d. Th.)
LC/MS [Methode 1]: Rₜ = 0.76 min; MS (ESIpos): m/z = 552 (M+H)⁺,
¹H-NMR (400 MHz, DMSO-*d₆*): δ [ppm] = 10.50-10.31 (m, 1H), 8.28-8.21 (m, 1H), 8.15-8.06 (m, 1H), 8.01-7.70 (m, 2H), 7.59-7.39 (m, 3H), 7.33-7.21 (m, 2H), 6.40-6.21 (m, 1H), 5.82-5.66 (m, 1H), 4.57-4.28 (m, 2H), 4.13 (s, 3H), 4.12-3.98 und 3.68-3.50 (2x m, 1H), 3.46-3.37 (m, 1H), 3.24-3.16 (m, 4H), 3.13-2.94 (m, 1H), 2.73-2.57 (m, 1H), 2.44-2.24 (m, 3H).

### Beispiel 32

### 4-({2-[11-Chlor-7-(fluormethyl)-2,5-dioxo-5,6,7,8-tetrahydropyrido[3,4-e][3]benzazocin-3(2H)-yl]-4-methoxybutanoyl}amino)-2-fluorbenzamid (Stereoisomerengemisch)

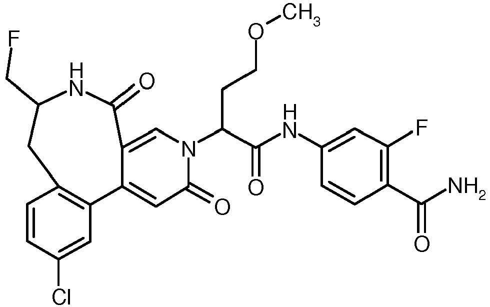

Nach der allgemeinen Methode 5 wurden 15 mg (35 µmol) 2-[ll-Chlor-7-(fluormethyl)-2,5-dioxo-5,6,7,8-tetrahydropyrido[3,4-*e*][3]benzazocin-3(2*H*)-yl]-4-methoxybutansäure (Stereoisomerengemisch) mit 8.5 mg (53 µmol, 1.5 eq.) 4-Amino-2-fluorbenzamid umgesetzt. Das Rohprodukt wurde mittels präparativer HPLC (Wasser-Acetonitril-0.1% Ameisensäure-Gradient) aufgereinigt. Ausbeute: 7 mg (35% d. Th.)
LC/MS [Methode 1]: Rₜ = 0.73 min; MS (ESIpos): m/z = 559 (M+H)⁺,
¹H-NMR (400 MHz, DMSO-*d₆*): δ [ppm] = 10.79-10.69 (m, 1H), 8.02-7.22 (m, 10H), 6.39-6.23 (m, 1H), 5.77-5.58 (m, 1H), 4.56-4.27 (m, 2H), 4.14-3.96 und 3.69-3.52 (2x m, 1H), 3.45-3.36 (m, 1H), 3.23-3.15 (m, 3H), 3.14-2.95 (m, 1H), 2.72-2.57 (m, 1H), 2.46-2.26 (m, 2H).

### Beispiel 33

### 4-({2-[11-Chlor-7-(fluormethyl)-2,5-dioxo-5,6,7,8-tetrahydropyrido[3,4-e][3]benzazocin-3(2H)-yl]-4-methoxybutanoyl}amino)benzoesäure (Stereoisomerengemisch)

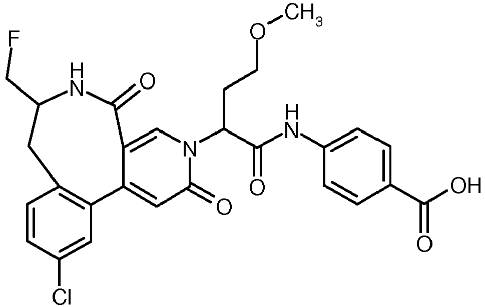

Nach der allgemeinen Methode 2 wurden 19 mg (34 µmol) 4-({2-[11-Chlor-7-(fluormethyl)-2,5-dioxo-5,6,7,8-tetrahydropyrido[3,4-*e*][3]benzazocin-3(2*H*)-yl]-4-methoxybutanoyl}amino)benzoesäuremethylester (Stereoisomerengemisch) umgesetzt. Das Rohprodukt wurde mittels präparativer HPLC (Wasser-Acetonitril-0.1% Ameisensäure-Gradient) aufgereinigt. Ausbeute: 8 mg (43% d. Th.)
LC/MS [Methode 1]: Rₜ = 0.76 min; MS (ESIpos): m/z = 542 (M+H)⁺,
¹H-NMR (400 MHz, DMSO-*d*₆): δ [ppm] = 12.77 (br. s., 1H), 10.78-10.62 (m, 1H), 8.00-7.69 (m, 6H), 7.53-7.41 (m, 2H), 7.34-7.19 (m, 1H), 6.39-6.21 (m, 1H), 5.81-5.69 (m, 1H), 4.56-4.28 (m, 2H), 4.13-3.96 und 3.72-3.50 (2x m, 1H), 3.53-3.37 (m, 2H), 3.23-2.94 (m, 5H), 2.72-2.57 (m, 1H), 2.44-2.24 (m, 2H).

### B) Bewertung der physiologischen Wirksamkeit

Die Eignung der erfindungsgemäßen Verbindungen zur Behandlung von thromboembolischen Erkrankungen kann in folgenden Assaysystemen gezeigt werden:

### a) Testbeschreibungen (in vitro)

### a.1) Messung der FXIa-Hemmung

Zur Bestimmung der Faktor XIa-Hemmung der erfindungsgemäßen Substanzen wird ein biochemisches Testsystem verwendet, in dem die Umsetzung eines peptidischen Faktor XIa-Substrates zur Ermittlung der enzymatischen Aktivität von humanem Faktor XIa benutzt wird. Dabei spaltet Faktor XIa von dem peptischen Faktor XIa-Substrat das C-terminale Aminomethylcoumarin (AMC) ab, dessen Fluoreszenz gemessen wird. Die Bestimmungen werden in Mikrotiterplatten durchgeführt.

Prüfsubstanzen werden in Dimethylsulfoxid aufgelöst und seriell in Dimethylsulfoxid verdünnt (3000 µM bis 0.0078 µM; resultierende Endkonzentrationen im Test: 50 µM bis 0.00013 µM). Jeweils 1 µl der verdünnten Substanzlösungen werden in die Vertiefungen von weißen Mikrotiterplatten der Firma Greiner (384 Vertiefungen) vorgelegt. Anschließend werden nacheinander 20 µl Assaypuffer (50 mM Tris/HCl pH 7.4; 100 mM Natriumchlorid; 5 mM Calciumchlorid; 0,1% bovines Serumalbumin) und 20 µl Faktor XIa der Firma Kordia (0.45 nM in Assaypuffer) hinzugefügt. Nach 15 min Inkubation wird die Enzymreaktion durch Zugabe von 20 µl des in Assaypuffer gelösten Faktor XIa Substrates Boc-Glu(OBzl)-Ala-Arg-AMC (10 µM in Assaypuffer) der Firma Bachem gestartet, für 30 min bei Raumtemperatur (22°C) inkubiert und anschließend eine Fluoreszensmessung durchgeführt (Anregung: 360 nM, Emission: 460 nM). Die gemessenen Emissionen der Testansätze mit Prüfsubstanz werden mit denen von Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet. Wirkdaten aus diesem Test sind in der folgenden Tabelle A aufgeführt:

**Tabelle A**

| **Beispiel-Nr.** | **IC₅₀ [nM]** | | **Beispiel-Nr.** | **IC₅₀ [nM]** |
|---|---|---|---|---|
| 1 | 420 | | 2 | 280 |
| 3 | 180 | | 4 | 720 |
| 5 | 350 | | 6 | 28 |
| 7 | 44 | | 8 | 36 |
| 9 | 140 | | 10 | 7.7 |
| 11 | 16 | | 12 | 1.9 |
| 13 | 27 | | 14 | 18 |
| 15 | 8.2 | | 16 | 11 |
| 17 | 44 | | 18 | 21 |
| 19 | 8.4 | | 20 | 47 |
| 21 | 34 | | 22 | 4.0 |
| 23 | 100 | | 24 | 35 |
| 25 | 2.5 | | 26 | 34 |
| 27 | 18 | | 28 | 1.8 |
| 29 | 4.5 | | 30 | 4.5 |
| 31 | 2.8 | | 32 | 5.3 |
| 33 | 1.2 | | | |

### a.2) Bestimmung der Selektivität

Zum Nachweis der Selektivität der Substanzen bezüglich FXIa-Hemmung werden die Prüfsubstanzen auf ihre Hemmung anderer humaner Serinproteasen wie Faktor Xa, Trypsin und Plasmin hin untersucht. Zur Bestimmung der enzymatischen Aktivität von Faktor Xa (1.3 nmol/l von Kordia), Trypsin (83 mU/ml von Sigma) und Plasmin (0.1 µg/ml von Kordia) werden diese Enzyme gelöst (50 mmol/l Tris-Puffer [C,C,C-Tris(hydroxymethyl)-aminomethan], 100 mmol/l NaCl, 0.1% BSA [bovines Serumalbumin], 5 mmol/l Calciumchlorid, pH 7.4) und für 15 min mit Prüfsubstanz in verschiedenen Konzentrationen in Dimethylsulfoxid sowie mit Dimethylsulfoxid ohne Prüfsubstanz inkubiert. Anschließend wird die enzymatische Reaktion durch Zugabe der entsprechenden Substrate gestartet (5 µmol/l Boc-Ile-Glu-Gly-Arg-AMC von Bachem für Faktor Xa und Trypsin, 5 50 µmol/l MeOSuc-Ala-Phe-Lys-AMC von Bachem für Plasmin). Nach einer Inkubationszeit von 30 min bei 22°C wird die Fluoreszenz gemessen (Anregung: 360 nm, Emission: 460 nm). Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

### a.3) Thrombin Generation Assay (Thrombogram)

Die Wirkung der Prüfsubstanzen auf das Thrombogram (Thrombin Generation Assay nach Hemker) wird in vitro in Humanplasma (Octaplas® von der Firma Octapharma) bestimmt.

Im Thrombin Generation Assay nach Hemker wird die Aktivität von Thrombin in gerinnendem Plasma durch die Messung der fluoreszenten Spaltprodukte des Substrats I-1140 (Z-Gly-Gly-Arg-AMC, Bachem) bestimmt. Die Reaktionen werden in Gegenwart variierender Konzentrationen an Prüfsubstanz oder dem entsprechenden Lösungsmittel durchgeführt. Um die Reaktion zu starten werden Reagenzien der Firma Thrombinoscope verwendet (30 pM or 0.1 pM recombinant tissue factor, 24 µM phospholipids in HEPES). Außerdem wird ein Thrombin Calibrator der Firma Thrombinoscope verwendet, dessen amidolytische Aktivität zur Berechnung der Thrombinaktivität in einer Probe mit unbekannter Menge an Thrombin benötigt wird. Die Testdurchführung erfolgt nach Herstellerangaben (Thrombionsocpe BV): 4 µl der Prüfsubstanz oder des Lösungsmittels, 76 µl Plasma und 20 µl PPP-Reagenz oder Thrombin Calibrator werden 5 min bei 37°C inkubiert. Nach Zugabe von 20 µl 2.5 mM Thrombinsubstrat in 20 mM Hepes, 60 mg/ml BSA, 102 mM Calciumchlorid wird die Thrombin Generierung 120 min alle 20 s gemessen. Die Messung wird mit einem Fluorometer (Fluoroskan Ascent) der Firma Thermo Electron durchgeführt, der mit einem 390/460 nM Filterpaar und einem Dispenser ausgestattet ist.

Durch die Verwendung der "thrombinoscope software" wird das Thrombogramm berechnet und graphisch dargestellt. Die folgenden Parameter werden berechnet: lag time, time to Peak, Peak, ETP (endogenous thrombin potential) und start tail.

### a.4) Bestimmung der antikoagulatorischen Wirkung

Die antikoagulatorische Wirkung der Prüfsubstanzen wird in vitro in Human- und Rattenplasma bestimmt. Dazu wird Blut unter Verwendung einer 0.11 molaren Natriumcitrat-Lösung als Vorlage in einem Mischungsverhältnis Natriumcitrat/Blut 1/9 abgenommen. Das Blut wird unmittelbar nach der Abnahme gut gemischt und 15 Minuten bei ca. 4000 g zentrifugiert. Der Überstand wird abpipettiert.

Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Neoplastin® von der Firma Boehringer Mannheim oder Hemoliance® RecombiPlastin von der Firma Instrumentation Laboratory) bestimmt. Die Testverbindungen werden 3 Minuten bei 37°C mit dem Plasma inkubiert. Anschließend wird durch Zugabe von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt.

Die aktivierte partielle Thromboplastinzeit (APTT) wird in Gegenwart variierender Konzentrationen an Prüfsubstanz oder dem entsprechenden Lösungsmittel mit einem handelsüblichen Testkit (PTT Reagent von der Firma Roche) bestimmt. Die Testverbindungen werden 3 Minuten bei 37°C mit dem Plasma und dem PTT Reagenz (Cephalin, Kaolin) inkubiert. Anschließend wird durch Zugabe von 25 mM Calciumchlorid die Gerinnung ausgelöst und der Zeitpunkt des Gerinnungseintritts bestimmt. Es wird die Konzentration an Prüfsubstanz ermittelt, die eine 50%ige Verlängerung beziehungsweise ein Verdoppelung der APTT bewirkt.

### a.5) Bestimmung der Plasma-Kallikrein Aktivität

Zur Bestimmung der Plasma Kallikrein-Hemmung der erfindungsgemäßen Substanzen wird ein biochemisches Testsystem verwendet, in dem die Umsetzung eines peptidischen Plasma Kallikrein-Substrates zur Ermittlung der enzymatischen Aktivität von humanem Plasma Kallikrein benutzt wird. Dabei spaltet Plasma Kallikrein von dem peptischen Plasma Kallikrein-Substrat das C-terminale Aminomethylcoumarin (AMC) ab, dessen Fluoreszenz gemessen wird. Die Bestimmungen werden in Mikrotiterplatten durchgeführt.

Prüfsubstanzen werden in Dimethylsulfoxid aufgelöst und seriell in Dimethylsulfoxid verdünnt (3000 µM bis 0.0078 µM; resultierende Endkonzentrationen im Test: 50 µM bis 0.00013 µM). Jeweils 1 µl der verdünnten Substanzlösungen werden in die Vertiefungen von weißen Mikrotiterplatten der Firma Greiner (384 Vertiefungen) vorgelegt. Anschließend werden nacheinander 20 µl Assaypuffer (50 mM Tris/HCl pH 7,4; 100 mM Natriumchlorid-Lösung; 5 mM Calciumchlorid-Lösung; 0.1% bovines Serumalbumin) und 20 µl Plasma-Kallikrein der Firma Kordia (0.6 nM in Assaypuffer) hinzugefügt. Nach 15 min Inkubation wird die Enzymreaktion durch Zugabe von 20 µl des in Assaypuffer gelösten Substrates H-Pro-Phe-Arg-AMC (10 µM in Assaypuffer) der Firma Bachem gestartet, für 30 min bei Raumtemperatur (22°C) inkubiert und anschließend eine Fluoreszensmessung durchgeführt (Anregung: 360 nM, Emission: 460 nM). Die gemessenen Emissionen der Testansätze mit Prüfsubstanz werden mit denen von Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

**Tabelle B**

| **Beispiel-Nr.** | **IC₅₀ [nM]** | | **Beispiel-Nr.** | **IC₅₀ [nM]** |
|---|---|---|---|---|
| 1 | >10000 | | 2 | 4900 |
| 3 | >50000 | | 4 | 18000 |
| 5 | 8300 | | 6 | 1900 |
| 7 | 3300 | | 8 | 680 |
| 9 | 3800 | | 10 | 2100 |
| 11 | 1700 | | 12 | 390 |
| 13 | 780 | | 14 | 230 |
| 15 | 240 | | 16 | 210 |
| 17 | 1200 | | 18 | 330 |
| 19 | 230 | | 20 | 4500 |
| 21 | 910 | | 22 | 340 |
| 23 | 3900 | | 24 | 800 |
| 25 | 370 | | 26 | 740 |
| 27 | 250 | | 28 | 55 |
| 29 | 42 | | 30 | 45 |
| 31 | 42 | | 32 | 100 |
| 33 | 97 | | | |

### a.6) Bestimmung der Endothel Integrität

Die Aktivität der erfindungsgemäßen Verbindungen werden mittels eines in-vitro Permeabilitäts-Assays auf "human umbilical venous cells" (HUVEC) charakterisiert. Mittels des EOS Apparatus (EC IS: Electric Cell-substrate Impedance Sensing; Applied Biophysics Inc; Troy, NY) können Unterschiede des transendothelialen elektrischen Widerstandes (TEER) über einer endothelialen Zell-Monoschicht, die über Gold-Elektroden plattiert wurde, kontinuierlich gemessen werden. HUVECs werden auf einer 96-well sensor Elektrodenplatte (96W1 E, Ibidi GmbH, Martinsried) ausgesäht. Eine Hyperpermeabilität der entstandenen konfluenten Zell-Monoschicht wird mittels Stimulation mit Kininogen, Prekallikrein und Faktor XII (je 100 nM) induziert. Die erfindungsgemäßen Verbindungen werden vor der Zugabe der oben angegebenen Substanzen zugegeben. Die üblichen Konzentrationen der Verbindungen sind 1 x 10⁻¹⁰ bis 1 x 10⁻⁶ M.

### a.7) Bestimmung der in-vitro Permeabilität von Endothelzellen

In einem weiteren Hyperpermeabilitäts-Modell wird die Aktivität der Substanzen auf die Modulation der makromolekularen Permeabilität bestimmt. HUVECs werden auf einer Fibronektin-überzogenen Transwell Filter Membran (24-well plates, 6.5 mm-Einsatz mit 0.4 µM Polykarbonat Membran; Costar #3413) ausgesäht. Die Filtermembran trennt den oberen von dem unteren Zellkulturraum mit der konfluenten Endothelzellschicht auf dem Boden des oberen Zellkulturraumes. Dem Medium des oberen Raumes wird 250 g/ml 40 kDa FITC-Dextan (Invitrogen, D1844) zugesetzt. Die Hyperpermeabilität der Monolayer-Schicht wird mittels Stimulation mit Kininogen, Prekallikrein und Faktor XII (je 100 nM) induziert. Medium Proben werden alle 30 min aus der unteren Kammer entnommen und die relative Fluoreszenz, als Parameter für die Veränderungen der makromolekularen Permeabilität in Abhängigkeit von der Zeit, mit einem Fluorimeter bestimmt. Die erfindungsgemäßen Verbindungen werden vor der Zugabe der oben angegebenen Substanzen zugegeben. Die üblichen Konzentationen der Verbindungen sind 1 x 10⁻¹⁰ bis 1 x 10⁻⁶ M.

### b) Bestimmung der antithrombotischen Wirkung (in vivo)

### b.1) Arterielles Thrombose-Modell (Eisen(II)chlorid-induzierte Thrombose) in Kombination mit Ohrblutungszeit im Kaninchen

Die antithrombotische Aktivität der FXIa-Inhibitoren wird in einem arteriellen Thrombose-Modell getestet. Dabei wird die Thrombusbildung durch chemische Beschädigung eines Bereichs der Arteria carotis im Kaninchen ausgelöst. Simultan wird die Ohrblutungszeit bestimmt.

Männliche Kaninchen (Crl:KBL (NZW)BR, Charles River) unter normaler Diät mit einem Gewicht von 2.2 - 2.5 kg Körpergewicht werden durch intramuskuläre Applikation von Xylazin und Ketamin (Rompun, Bayer, 5 mg/kg und Ketavet, Pharmacia & Upjohn GmbH, 40 mg/kg Körpergewicht) anästhesiert. Die Anästhesie wird weiterhin durch intravenöse Gabe derselben Präparate (bolus: Dauerinfusion) über die rechte Ohrvene unterstützt.

Nach Freipräparation der rechten Arteria carotis wird der Gefäßschaden dadurch erzeugt, dass ein Stück Filterpapier (10 mm x 10 mm) auf einem Streifen Parafilm® (25 mm x 12 mm) um die A. carotis gewickelt wird, ohne den Blutfluß dadurch zu beeinträchtigen. Das Filterpapier enthält 100 µL einer 13%igen Lösung von Eisen(II)chlorid (Sigma) in Wasser. Nach 5 min wird das Filterpapier entfernt und das Gefäß zweimal mit wässriger 0.9%iger Natriumchlorid-Lösung gespült. 30 min nach der Verletzung wird die Arteria carotis im Bereich der Schädigung herauspräpariert und eventuell vorhandenes thrombotisches Material entnommen und gewogen.

Die Prüfsubstanzen werden entweder intravenös über die Vena femoralis den anästhesierten oder oral mittels Schlundsonde den wachen Tieren jeweils 5 min beziehungsweise 2 h vor Schädigung verabreicht.

Die Ohrblutungszeit wird 2 min nach der Schädigung der Arteria carotis bestimmt. Hierzu wird das linke Ohr rasiert und ein definierter Schnitt von 3 mm Länge (Klinge Art.Nummer 10-150-10, Martin, Tuttlingen, Germany) parallel zur Ohrlängsachse gesetzt. Dabei wird darauf geachtet, kein sichtbares Gefäß zu verletzen. Eventuell austretendes Blut wird in 15 Sekunden-Intervallen mit genau gewogenen Filterpapierstücken aufgenommen, ohne die Wunde direkt zu berühren. Die Blutungszeit wird berechnet als die Zeitspanne vom Setzen des Schnitts bis zu dem Zeitpunkt, an dem am Filterpapier kein Blut mehr nachweisbar ist. Das ausgetretene Blutvolumen wird nach Wiegen der Filterpapierstücke berechnet.

### c) Bestimmung der Wirkung auf die Extravasation/Ödembildung und/oder Neovaskularisierung im Auge (in vivo)

### c.1) Testung der Wirksamkeit von Substanzen im Laser-induzierten choroidalen Neovaskularisierungs- Modell

Diese Studie dient dem Zweck, die Wirksamkeit einer Testsubstanz auf die Reduktion der Extravasation/Ödembildung und/oder der choroidalen Neovaskularisierung im Rattenmodell der Laser-induzierten choroidalen Neovaskularisierung zu untersuchen.

Dafür werden pigmentierte Ratten vom Stamm Brown-Norway, die keine Anzeichen ophthalmologischer Erkrankungen aufweisen, ausgewählt und nach dem Zufallsprinzip Behandlungsgruppen zugeordnet. Am Tag 0 werden die Tiere mittels intraperitonealer Injektion narkotisiert (15 mg/kg Xylazin und 80 mg/kg Ketamin). Nach Instillation eines Tropfens einer 0.5%igen Tropicamid-Lösung zur Weitstellung der Pupillen wird die choroidale Neovaskularisierung mittels eines 532 nm Argon Laser Photokoagulators an sechs definierten Stellen um den Nervus opticus herum ausgelöst (50-75 µm Durchmesser, 150 mW Intensität, 100 ms Dauer). Die Testsubstanz und das entsprechende Vehikel (z.B. PBS, isotone Kochsalzlösung) werden entweder systemisch oral beziehungsweise intraperitonal appliziert oder lokal am Auge durch mehrfache Gabe als Augentropfen beziehungsweise intravitreale Injektion verabreicht. Das Körpergewicht aller Tiere wird vor Beginn der Studie, und anschließend täglich während der Studie bestimmt.

An Tag 21 wird eine Angiographie mittels einer Fluoreszenz-Funduskammera (z.B. Kowe, HRA) durchgeführt. In Narkose und nach erneuter Pupillenerweiterung wird ein 10%iger Natrium-Fluorescein-Farbstoff subkutan (s.c.) injiziert. 2-10 min später werden Bilder des Augenhintergrundes aufgenommen. Die Stärke der Extravasation/des Ödems, dargestellt durch die Leckage von Fluorescein, wird von zwei bis drei verblindeten Beobachtern beurteilt und in Schweregrade von 0 (keine Extravasation) bis 3 (starke Einfärbung über die eigentliche Läsion hinaus) eingeteilt.

Nach Abtöten der Tiere an Tag 23 werden die Augen entnommen und in 4%iger Paraformaldehyd-Lösung für eine Stunde bei Raumtemperatur fixiert. Nach einem Waschdurchgang wird die Retina vorsichtig herausgeschält, und der Sklera-Choroidea-Komplex wird mit einem FITC-Isolektin B4 Antikörper angefärbt und anschließend flach auf einen Objetträger aufgebracht. Die so erhaltenen Präparate werden mittels eines Fluoreszenz-Mikroskops (Apotom, Zeiss) bei einer Anregungs-Wellenlänge von 488 nm ausgewertet. Die Fläche beziehungsweise das Volumen der choroidalen Neovaskularisierung (in µm² bzw. µm³) wird durch morphometrische Analyse mittels Axiovision 4.6 Software berechnet.

### c.2) Testung der Wirksamkeit von Substanzen im Sauerstoff-induzierten Retinopathie Modell

Es wurde gezeigt, dass eine durch Sauerstoff induzierte Retinopathie ein wertvolles Tiermodell für die Untersuchung der pathologischen retinalen Angiogenese darstellt. Dieses Modell basiert auf der Beobachtung, dass eine Hyperoxie während der frühen postnatalen Entwicklung in der Retina zum Anhalten oder zur Verlangsamung des Wachstums von normalen retinalen Blutgefäßen führt. Sobald die Tiere nach einer 7-tägigen Hyperoxiephase zur normoxischen Raumluft zurückkehren, ist dieses gleichbedeutend einer relativen Hypoxie, da in der Retina die normalen Gefäße fehlen, welche erforderlich sind, um eine ausreichende Versorgung des neuralen Gewebes unter normoxischen Bedingungen zu gewährleisten. Die dadurch erzeugte ischämische Situation führt zur abnormen Neovaskularisation, die einige Ähnlichkeiten mit der pathophysiologischen Neovaskularisation in Augenerkrankungen wie der feuchten AMD aufzeigt. Darüber hinaus ist die hervorgerufene Neovaskularisierung sehr reproduzierbar, quantifizierbar und ein wichtiger Parameter für die Untersuchung der Krankheitsmechanismen und möglichen Behandlungen für verschiedenste Formen von Netzhauterkrankungen.

Das Ziel dieser Studie ist es, die Wirksamkeit von täglich systemisch verabreichten Dosen der Testverbindung auf das Wachstum der retinalen Gefäße im Sauerstoff-induzierten Retinopathie-Modell zu untersuchen. Neugeborene von C57B1 / 6 Mäusen und ihre Mütter werden am postnatalen Tag 7 (PD7) einer Hyperoxie (70% Sauerstoff) für 5 Tage ausgesetzt. Ab PD12, werden die Mäuse unter normoxischen Bedingungen (Raumluft, 21% Sauerstoff) bis zum PD17 gehalten. Von Tag 12 bis Tag 17 werden die Mäuse täglich mit der Testsubstanz oder dem entsprechenden Vehikel behandelt. Am Tag 17 werden alle Mäuse mit Isofluran narkotisiert und anschließend durch Genickbruch abgetötet. Die Augen werden entnommen und in 4% Formalin fixiert. Nach dem Waschen in Phosphat-gepufferter Kochsalzlösung wird die Netzhaut präpariert, ein Flachpräperat davon erzeugt und dieses mit Isolectin B4 Antikörper gefärbt. Die Quantifizierung der neugewachsenen Gefäße wird unter Verwendung eines Zeiss ApoTome durchgeführt.

### C) Ausführungsbeispiele für pharmazeutische Zusammensetzungen

Die erfindungsgemäßen Substanzen können folgendermaßen in pharmazeutische Zubereitungen überführt werden:

### Tablette:

### Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

### Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96%), 400 mg Rhodigel (Xanthan gum) (Fa. FMC, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6 h gerührt.

### Lösung oder Suspension zur topischen Anwendung am Auge (Augentropfen):

Eine sterile pharmazeutische Zubereitung zur topischen Anwendung am Auge kann durch Rekonstitution eines Lyophilisats der erfindungsgemäßen Verbindung in steriler Kochsalz-Lösung hergestellt werden. Als Konservierungsmittel für eine solche Lösung oder Suspension ist beispielsweise Benzalkoniumchlorid, Thiomersal oder Phenylquecksilbernitrat in einem Konzentrationsbereich von 0.001 bis 1 Gewichtsprozent geeignet.

### Lösung oder Suspension zur topischen Anwendung am Auge (Augentropfen):

Eine sterile pharmazeutische Zubereitung zur topischen Anwendung am Auge kann durch Rekonstitution eines Lyophilisats der erfindungsgemäßen Verbindung in steriler Kochsalz-Lösung hergestellt werden. Als Konservierungsmittel für eine solche Lösung oder Suspension ist beispielsweise Benzalkoniumchlorid, Thiomersal oder Phenylquecksilbernitrat in einem Konzentrationsbereich von 0.001 bis 1 Gewichtsprozent geeignet.

## Patentansprüche

1. Verbindung der Formel 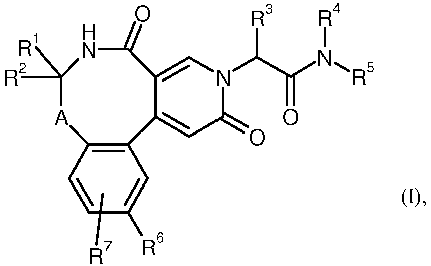 in welcher
A für eine Bindung oder -CH₂- steht,
R¹ für Wasserstoff oder Methyl steht,
wobei Methyl substituiert sein kann mit einem Substituenten Fluor,
R² für Wasserstoff oder Methyl steht,
oder
R¹ und R² bilden zusammen mit dem Kohlenstoffatom, an das sie gebunden sind, einen Cyclopropyl-Ring,
R³ für Wasserstoff, C₁-C₅-Alkyl, C₁-C₄-Alkoxy, Difluormethyl, Trifluormethyl, 1,1-Difluorethyl, 3,3,3-Trifluor-2-hydroxyprop-1-yl, 3,3,3-Trifluor-2-methoxyprop-1-yl, 3,3,3-Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, C₃-C₆-Cycloalkyl, 4- bis 6-gliedriges Oxo- Heterocyclyl, 1,4-Dioxanyl, Oxazolyl, Phenyl und Pyridyl,
worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy,
R⁴ für Wasserstoff steht,
R⁵ für eine Gruppe der Formel 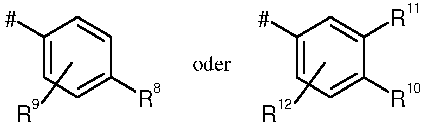 oder 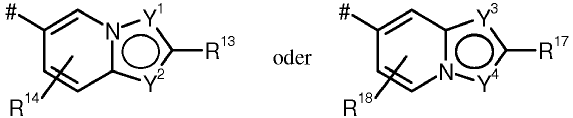 oder 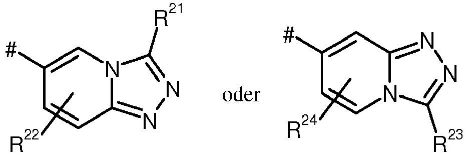 oder 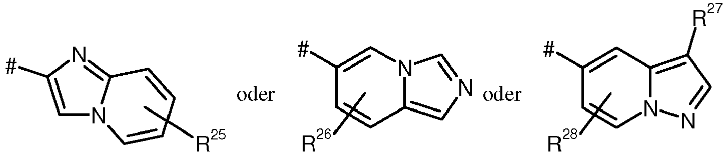 oder 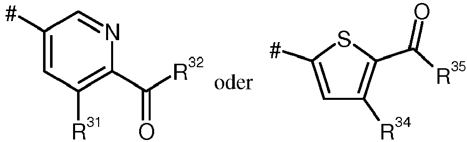 oder 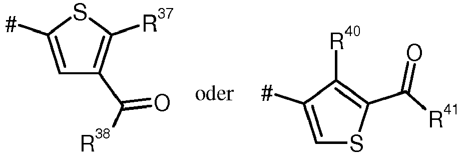 steht,
wobei # die Anknüpfstelle an das Stickstoffatom ist,
R⁸ für Hydroxycarbonyl, Aminocarbonyl oder 5-gliedriges Heterocyclyl steht,
wobei Heterocyclyl substituiert sein kann mit 1 bis 2 Substituenten unabhängig voneinander ausgewählt aus der Gruppe bestehend aus Oxo, Hydroxy, Thioxo, Sulfanyl, Methyl, Difluormethyl, Trifluormethyl, 2-Hydroxycarbonyl-1,1,2,2-tetrafluorethyl und 2-Methoxycarbonyl-1,1,2,2-tetrafluorethyl,
worin Methyl substituiert sein kann mit einem Substituenten Methoxy,
R⁹ für Wasserstoff, Chlor, Fluor oder Methyl steht,
R¹⁰ und R¹¹ zusammen mit den Kohlenstoffatomen an die sie gebunden sind einen 5-gliedrigen Heterocyclus bilden,
wobei der Heterocyclus substituiert sein kann mit 1 bis 2 Substituenten unabhängig voneinander ausgewählt aus der Gruppe bestehend aus Oxo, Chlor, Hydroxy, Hydroxycarbonyl, Methyl, Difluormethyl, Trifluormethyl, 1,1,2,2,2-Pentafluorethyl, 2-Hydroxycarbonyl-1,1,2,2-tetrafluorethyl und 2-Methoxy-carbonyl-1,1,2,2-tetrafluorethyl,
R¹² für Wasserstoff, Chlor, Fluor, Methyl oder Methoxy steht,
Y¹ für ein Stickstoffatom oder C-R¹⁵ steht,
worin
R¹⁵ für Wasserstoff, Chlor, Hydroxy, Methoxy oder C₁-C₃-Alkoxycarbonyl steht,
Y² für ein Stickstoffatom oder C-R¹⁶ steht,
worin
R¹⁶ für Wasserstoff, Chlor, Hydroxy oder Methoxy steht,
R¹³ für Wasserstoff, Hydroxycarbonyl, Hydroxycarbonylmethyl oder Phenyl steht,
worin Phenyl substituiert sein kann mit 1 bis 2 Substituenten Fluor,
R¹⁴ für Wasserstoff, Chlor, Fluor oder Methyl steht,
Y³ für ein Stickstoffatom oder C-R¹⁹ steht,
worin
R¹⁹ für Wasserstoff, Chlor, Hydroxy oder Methoxy steht,
Y⁴ für ein Stickstoffatom oder C-R²⁰ steht,
worin
R²⁰ für Wasserstoff, Chlor, Hydroxy oder Methoxy steht,
R¹⁷ für Wasserstoff, Hydroxycarbonyl, Hydroxycarbonylmethyl, C₁-C₃-Alkoxycarbonyl oder Aminocarbonyl steht,
R¹⁸ für Wasserstoff, Chlor, Fluor oder Methyl steht,
R²¹ für Wasserstoff, Chlor, Hydroxy, C₁-C₄-Alkyl, Methoxy, C₁-C₃-Alkylaminomethyl oder Morpholinylmethyl steht,
R²² für Wasserstoff, Chlor, Fluor oder Methyl steht,
R²³ für Wasserstoff, Chlor, Hydroxy oder Methoxy steht,
R²⁴ für Wasserstoff, Chlor, Fluor oder Methyl steht,
R²⁵ für Wasserstoff, Hydroxycarbonyl oder Hydroxycarbonylmethyl steht,
R²⁶ für Wasserstoff, Chlor, Fluor oder Methyl steht,
R²⁷ für Hydroxycarbonyl, Aminocarbonyl, C₁-C₃-Alkoxycarbonyl oder C₁-C₃-Alkylaminocarbonyl steht,
wobei Alkylaminocarbonyl substituiert sein kann mit einem Substituenten ausgewählt aus der Gruppe bestehend aus Hydroxy, Trifluormethyl, Methoxy und Trifluormethoxy,
R²⁸ für Wasserstoff, Chlor, Fluor oder Methyl steht,
R³¹ für Wasserstoff oder Fluor steht,
R³² für Hydroxy oder -NHR³³ steht,
worin
R³³ für Wasserstoff, Methyl oder Ethyl steht,
R³⁴ für Wasserstoff oder Fluor steht,
R³⁵ für Hydroxy oder -NHR³⁶ steht,
worin
R³⁶ für Wasserstoff, Methyl oder Ethyl steht,
R³⁷ für Wasserstoff oder Fluor steht,
R³⁸ für Hydroxy oder -NHR³⁹ steht,
worin
R³⁹ für Wasserstoff, Methyl oder Ethyl steht,
R⁴⁰ für Wasserstoff oder Fluor steht,
R⁴¹ für Hydroxy oder -NHR⁴² steht,
worin
R⁴² für Wasserstoff, Methyl oder Ethyl steht,
R⁶ für Brom, Chlor, Fluor, Methyl, Difluormethyl, Trifluormethyl, Methoxy, Difluormethoxy oder Trifluormethoxy steht,
R⁷ für Wasserstoff, Chlor oder Fluor steht,
oder eines ihrer Salze, ihrer Solvate oder der Solvate ihrer Salze.

2. Verbindung nach Anspruch 1, **dadurch gekennzeichnet, dass**
A für eine Bindung oder -CH₂- steht,
R¹ für Wasserstoff oder Methyl steht,
R² für Wasserstoff oder Methyl steht,
oder
R¹ und R² bilden zusammen mit dem Kohlenstoffatom, an das sie gebunden sind, einen Cyclopropyl-Ring,
R³ für Wasserstoff, Methyl, Ethyl, n-Propyl, 2-Methyl-prop-1-yl, n-Butyl oder Ethoxy steht,
wobei Methyl substituiert sein kann mit einem Substituenten ausgewählt aus der Gruppe bestehend aus Difluormethyl, Trifluormethyl, Cyclopropyl, Cyclobutyl, Cyclohexyl, Oxetanyl, Tetrahydrofuranyl, Tetrahydro-2H-pyranyl und 1,4-Dioxanyl,
worin Cyclopropyl, Cyclobutyl, Cyclohexyl und Oxetanyl substituiert sein können mit 1 bis 2 Substituenten unabhängig voneinander ausgewählt aus der Gruppe bestehend aus Fluor, Hydroxy, Methyl, Ethyl und Methoxy,
und
wobei Ethyl, n-Propyl und n-Butyl substituiert sein können mit einem Substituenten ausgewählt aus der Gruppe bestehend aus Fluor, Methoxy und Trifluormethoxy,
R⁴ für Wasserstoff steht,
R⁵ für eine Gruppe der Formel 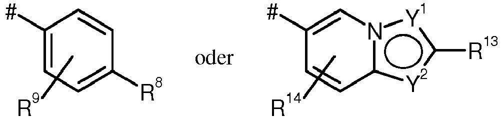 oder 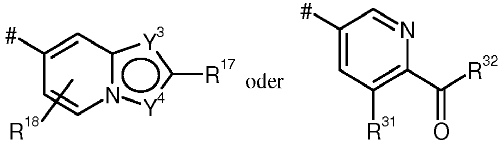 oder 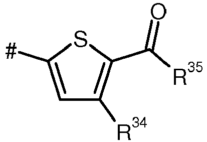 steht,
wobei # die Anknüpfstelle an das Stickstoffatom ist,
R⁸ für Hydroxycarbonyl, Aminocarbonyl, Oxazolyl, Oxadiazolyl, Thiadiazolyl, Pyrazolyl, Imidazolyl, Triazolyl, Tetrazolyl oder Dihydrooxazolyl steht,
wobei Oxazolyl, Oxadiazolyl, Thiadiazolyl, Pyrazolyl, Imidazolyl, Triazolyl und Dihydrooxazolyl substituiert sein können mit 1 bis 2 Substituenten unabhängig voneinander ausgewählt aus der Gruppe bestehend aus Oxo, Hydroxy, Thioxo, Sulfanyl, Methyl, Trifluormethyl und 2-Hydroxycarbonyl-1,1,2,2-tetrafluorethyl,
worin Methyl substituiert sein kann mit einem Substituenten Methoxy,
R⁹ für Wasserstoff, Chlor, Fluor oder Methyl steht,
Y¹ für ein Stickstoffatom oder C-R¹⁵ steht,
worin
R¹⁵ für Wasserstoff, Chlor, Hydroxy oder Methoxy steht,
Y² für ein Stickstoffatom oder C-R¹⁶ steht,
worin
R¹⁶ für Wasserstoff, Chlor, Hydroxy oder Methoxy steht,
R¹³ für Wasserstoff oder Hydroxycarbonyl steht,
R¹⁴ für Wasserstoff oder Fluor steht,
Y³ für ein Stickstoffatom oder C-R¹⁹ steht,
worin
R¹⁹ für Wasserstoff, Chlor, Hydroxy oder Methoxy steht,
Y⁴ für ein Stickstoffatom oder C-R²⁰ steht,
worin
R²⁰ für Wasserstoff, Chlor, Hydroxy oder Methoxy steht,
R¹⁷ für Wasserstoff oder Hydroxycarbonyl steht,
R¹⁸ für Wasserstoff oder Fluor steht,
R³¹ für Wasserstoff steht,
R³² für Hydroxy oder -NHR³³ steht,
worin
R³³ für Wasserstoff steht,
R³⁴ für Wasserstoff steht,
R³⁵ für Hydroxy steht,
oder
R⁵ für 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl, 1H-Indazol-5-yl oder 2H-Indazol-5-yl steht,
wobei der 5-gliedrige Heterocyclus in 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl, 1H-Indazol-5-yl und 2H-Indazol-5-yl substituiert sein kann mit 1 bis 2 Substituenten unabhängig voneinander ausgewählt aus der Gruppe bestehend aus Oxo, Chlor, Hydroxycarbonyl, Methyl und Trifluormethyl,
und
wobei der Benzylring in 2,3-Dihydro-1H-indazol-6-yl, 1H-Benzimidazol-6-yl, Indol-6-yl, 2,3-Dihydro-1H-indazol-5-yl, 2,3-Dihydro-1H-benzimidazol-5-yl, Indol-5-yl, 1H-Indazol-6-yl und 1H-Indazol-5-yl substituiert sein kann mit einem Substituenten ausgewählt aus der Gruppe bestehend aus Fluor und Methoxy,
R⁶ für Chlor steht,
R⁷ für Wasserstoff steht,
oder eines ihrer Salze, ihrer Solvate oder der Solvate ihrer Salze.

3. Verbindung nach einem der Ansprüche 1 oder 2, **dadurch gekennzeichnet, dass**
A für eine Bindung oder -CH₂- steht,
R¹ für Wasserstoff steht,
R² für Wasserstoff steht,
oder
R¹ und R² bilden zusammen mit dem Kohlenstoffatom, an das sie gebunden sind, einen Cyclopropyl-Ring,
R³ für Wasserstoff, Ethyl oder 2-Methyl-prop-1-yl steht,
wobei Ethyl substituiert sein kann mit einem Substituenten Methoxy,
R⁴ für Wasserstoff steht,
R⁵ für eine Gruppe der Formel 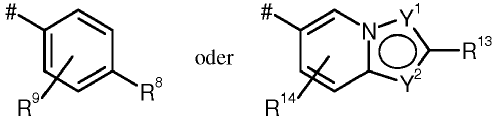 oder 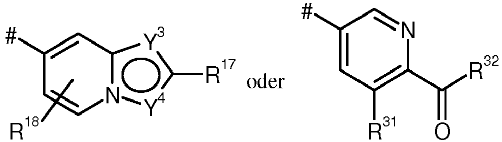 oder 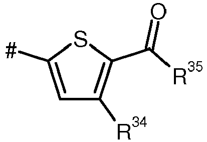 steht,
wobei # die Anknüpfstelle an das Stickstoffatom ist,
R⁸ für Hydroxycarbonyl, Aminocarbonyl oder Oxadiazolyl steht,
wobei Oxadiazolyl substituiert sein kann mit einem Substituenten Oxo,
R⁹ für Wasserstoff oder Fluor steht,
Y¹ für C-R¹⁵ steht,
worin
R¹⁵ für Wasserstoff steht,
Y² für ein Stickstoffatom steht,
R¹³ für Wasserstoff steht,
R¹⁴ für Wasserstoff steht,
Y³ für C-R¹⁹ steht,
worin
R¹⁹ für Wasserstoff steht,
Y⁴ für ein Stickstoffatom steht,
R¹⁷ für Wasserstoff steht,
R¹⁸ für Wasserstoff steht,
R³¹ für Wasserstoff steht,
R³² für Hydroxy oder -NHR³³ steht,
worin
R³³ für Wasserstoff steht,
R³⁴ für Wasserstoff steht,
R³⁵ für Hydroxy steht,
oder
R⁵ für 2,3-Dihydro-1H-indazol-6-yl, 2,3-Dihydro-1H-benzimidazol-5-yl, 1H-Indazol-5-yl oder 2H-Indazol-5-yl steht,
wobei der 5-gliedrige Heterocyclus in 2,3-Dihydro-1H-indazol-6-yl, 2,3-Dihydro-1H-benzimidazol-5-yl, 1H-Indazol-5-yl und 2H-Indazol-5-yl substituiert sein kann mit 1 bis 2 Substituenten unabhängig voneinander ausgewählt aus der Gruppe bestehend aus Oxo und Methyl,
R⁶ für Chlor steht,
R⁷ für Wasserstoff steht,
oder eines ihrer Salze, ihrer Solvate oder der Solvate ihrer Salze.

4. Verfahren zur Herstellung einer Verbindung der Formel (I) oder eines ihrer Salze, ihrer Solvate oder der Solvate ihrer Salze nach Anspruch 1, **dadurch gekennzeichnet, dass** entweder
[A] eine Verbindung der Formel 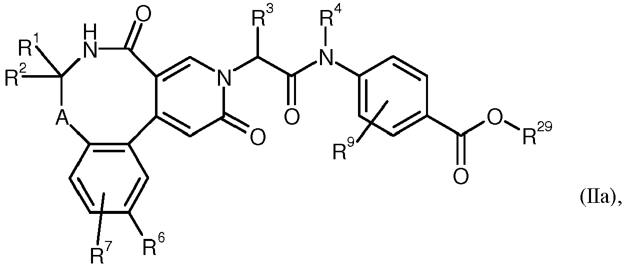 in welcher
A, R¹, R², R³, R⁴, R⁶, R⁷ und R⁹ die in Anspruch 1 angegebene Bedeutung haben, und
R²⁹ für tert-Butyl steht,
mit einer Säure zu einer Verbindung der Formel 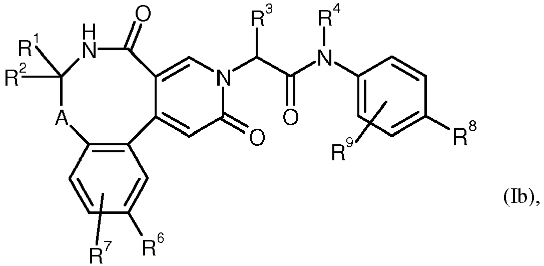 in welcher
A, R¹, R², R³, R⁴, R⁶, R⁷ und R⁹ die in Anspruch 1 angegebene Bedeutung haben, und
R⁸ für Hydroxycarbonyl steht,
umgesetzt wird,
oder
[B] eine Verbindung der Formel 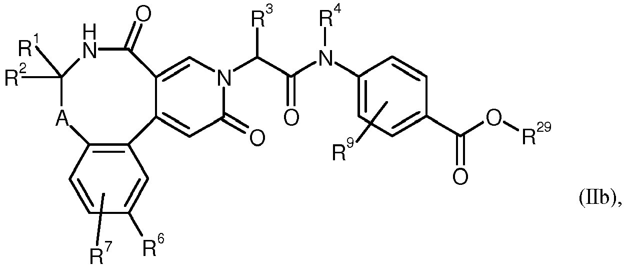 in welcher
A, R¹, R², R³, R⁴, R⁶, R⁷ und R⁹ die in Anspruch 1 angegebene Bedeutung haben, und
R²⁹ für Methyl oder Ethyl steht,
mit einer Base zu einer Verbindung der Formel 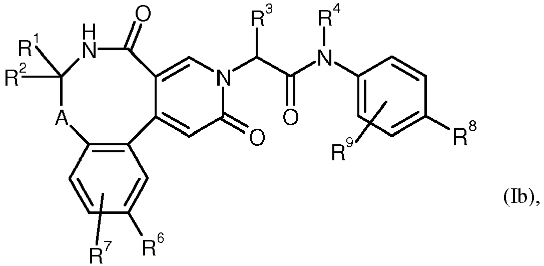 in welcher
A, R¹, R², R³, R⁴, R⁶, R⁷ und R⁹ die in Anspruch 1 angegebene Bedeutung haben, und
R⁸ für Hydroxycarbonyl steht,
umgesetzt wird,
oder
[C] eine Verbindung der Formel 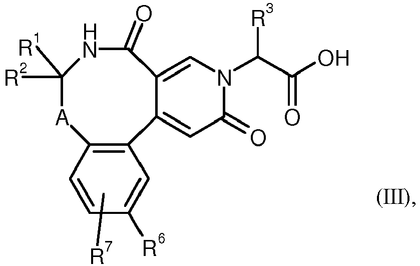 in welcher
A, R¹, R², R³, R⁶ und R⁷ die in Anspruch 1 angegebene Bedeutung haben,
mit einer Verbindung der Formel 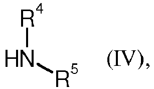 in welcher
R⁴ und R⁵ die in Anspruch 1 angegebene Bedeutung haben,
in Gegenwart eines Dehydratisierungsreagenzes zu einer Verbindung der Formel (I) umgesetzt wird.

5. Verbindung nach einem der Ansprüche 1 bis 3 zur Verwendung in der Behandlung und/oder Prophylaxe von Krankheiten.

6. Verwendung einer Verbindung nach einem der Ansprüche 1 bis 3 zur Herstellung eines Arzneimittels zur Behandlung und/oder Prophylaxe von Krankheiten.

7. Verwendung einer Verbindung nach einem der Ansprüche 1 bis 3 zur Herstellung eines Arzneimittels zur Behandlung und/oder Prophylaxe von thrombotischen beziehungsweise thromboembolischen Erkrankungen.

8. Verwendung einer Verbindung nach einem der Ansprüche 1 bis 3 zur Herstellung eines Arzneimittels zur Behandlung und/oder Prophylaxe von ophthalmologischen Erkrankungen.

9. Verwendung einer Verbindung nach einem der Ansprüche 1 bis 3 zur Herstellung eines Arzneimittels zur Behandlung und/oder Prophylaxe von hereditärem Angioödem oder entzündlichen Erkrankungen des Darms, wie Morbus Crohn oder Colitis ulcerosa.

10. Arzneimittel enthaltend eine Verbindung nach einem der Ansprüche 1 bis 3 in Kombination mit einem inerten, nichttoxischen, pharmazeutisch geeigneten Hilfsstoff.

11. Arzneimittel nach Anspruch 10 zur Verwendung in der Behandlung und/oder Prophylaxe von thrombotischen beziehungsweise thromboembolischen Erkrankungen.

12. Arzneimittel nach Anspruch 10 zur Verwendung in der Behandlung und/oder Prophylaxe von ophthalmologischen Erkrankungen.

13. Arzneimittel nach Anspruch 10 zur Verwendung in der Behandlung und/oder Prophylaxe von hereditärem Angioödem oder entzündlichen Erkrankungen des Darms, wie Morbus Crohn oder Colitis ulcerosa.

## Claims

1. Compound of the formula 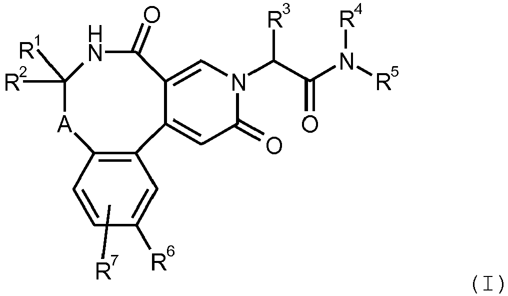 in which
A is a bond or -CH₂-,
R¹ is hydrogen or methyl,
where methyl may be substituted by a fluorine substituent,
R² is hydrogen or methyl,
or
R¹ and R² together with the carbon atom to which they are bonded form a cyclopropyl ring,
R³ is hydrogen, C₁-C₅-alkyl, C₁-C₄-alkoxy, difluoromethyl, trifluoromethyl, 1,1-difluoroethyl, 3,3,3-trifluoro-2-hydroxyprop-1-yl, 3,3,3-trifluoro-2-methoxyprop-1-yl, 3,3,3-trifluoro-2-ethoxyprop-1-yl, prop-2-yn-1-yl, cyclopropyloxy or cyclobutyloxy,
where alkyl may be substituted by a substituent selected from the group consisting of fluorine, cyano, hydroxyl, difluoromethyl, trifluoromethyl, methoxy, ethoxy, difluoromethoxy, trifluoromethoxy, C₃-C₆-cycloalkyl, 4- to 6-membered oxoheterocyclyl, 1,4-dioxanyl, oxazolyl, phenyl and pyridyl,
in which cycloalkyl may be substituted by 1 to 2 substituents selected independently from the group consisting of fluorine, hydroxyl, methyl, ethyl, methoxy, ethoxy, difluoromethyl, trifluoromethyl, difluoromethoxy and trifluoromethoxy,
R⁴ is hydrogen,
R⁵ is a group of the formula 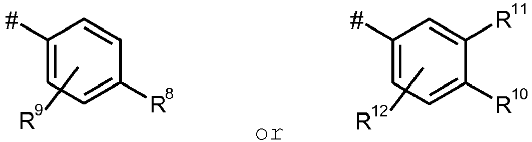 or 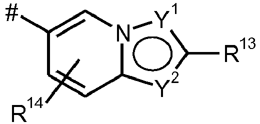
or 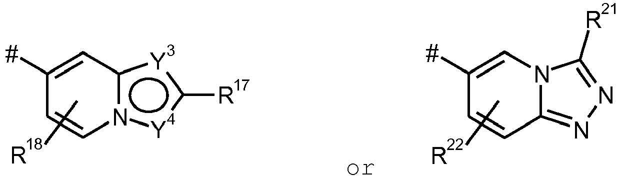 or 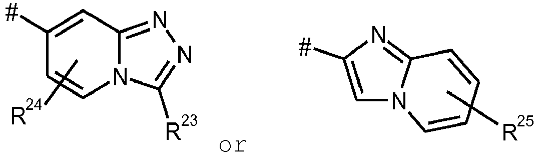 or 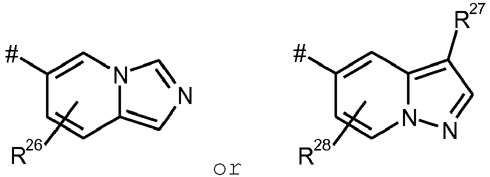 or 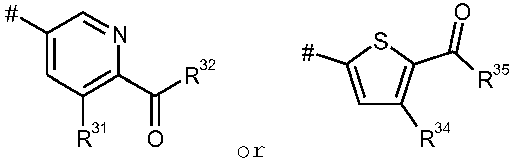 or 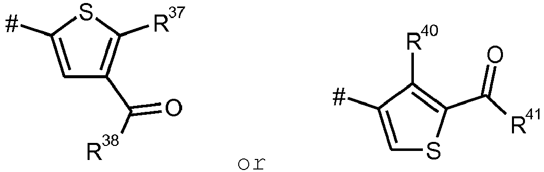 where # is the attachment point to the nitrogen atom,
R⁸ is hydroxycarbonyl, aminocarbonyl or 5-membered heterocyclyl,
where heterocyclyl may be substituted by 1 to 2 substituents selected independently from the group consisting of oxo, hydroxyl, thioxo, sulphanyl, methyl, difluoromethyl, trifluoromethyl, 2-hydroxycarbonyl-1,1,2,2-tetrafluoroethyl and 2-methoxycarbonyl-1,1,2,2-tetrafluoroethyl,
in which methyl may be substituted by a methoxy substituent,
R⁹ is hydrogen, chlorine, fluorine or methyl,
R¹⁰ and R¹¹ together with the carbon atoms to which they are bonded form a 5-membered heterocycle,
where the heterocycle may be substituted by 1 to 2 substituents selected independently from the group consisting of oxo, chlorine, hydroxyl, hydroxycarbonyl, methyl, difluoromethyl, trifluoromethyl, 1,1,2,2,2-pentafluoroethyl, 2-hydroxycarbonyl-1,1,2,2-tetrafluoroethyl and 2-methoxycarbonyl-1,1,2,2-tetrafluoroethyl,
R¹² is hydrogen, chlorine, fluorine, methyl or methoxy,
Y¹ is a nitrogen atom or C-R¹⁵
in which
R¹⁵ is hydrogen, chlorine, hydroxyl, methoxy or C₁-C₃-alkoxycarbonyl,
Y² is a nitrogen atom or C-R¹⁶
in which
R¹⁶ is hydrogen, chlorine, hydroxyl or methoxy,
R¹³ is hydrogen, hydroxycarbonyl, hydroxycarbonylmethyl or phenyl,
where phenyl may be substituted by 1 to 2 fluorine substituents,
R¹⁴ is hydrogen, chlorine, fluorine or methyl,
Y³ is a nitrogen atom or C-R¹⁹
in which
R¹⁹ is hydrogen, chlorine, hydroxyl or methoxy,
Y⁴ is a nitrogen atom or C-R²⁰
in which
R²⁰ is hydrogen, chlorine, hydroxyl or methoxy,
R¹⁷ is hydrogen, hydroxycarbonyl, hydroxycarbonylmethyl, C₁-C₃-alkoxycarbonyl or aminocarbonyl,
R¹⁸ is hydrogen, chlorine, fluorine or methyl,
R²¹ is hydrogen, chlorine, hydroxy, C₁-C₄-alkyl, methoxy, C₁-C₃-alkylaminomethyl or morpholinylmethyl,
R²² is hydrogen, chlorine, fluorine or methyl,
R²³ is hydrogen, chlorine, hydroxyl or methoxy,
R²⁴ is hydrogen, chlorine, fluorine or methyl,
R²⁵ is hydrogen, hydroxycarbonyl or hydroxycarbonylmethyl,
R²⁶ is hydrogen, chlorine, fluorine or methyl,
R²⁷ is hydroxycarbonyl, aminocarbonyl, C₁-C₃-alkoxycarbonyl or C₁-C₃-alkylaminocarbonyl,
where alkylaminocarbonyl may be substituted by a substituent selected from the group consisting of hydroxyl, trifluoromethyl, methoxy and trifluoromethoxy,
R²⁸ is hydrogen, chlorine, fluorine or methyl,
R³¹ is hydrogen or fluorine,
R³² is hydroxyl or -NHR³³,
in which
R³³ is hydrogen, methyl or ethyl,
R³⁴ is hydrogen or fluorine,
R³⁵ is hydroxyl or -NHR³⁶,
in which
R³⁶ is hydrogen, methyl or ethyl,
R³⁷ is hydrogen or fluorine,
R³⁸ is hydroxyl or -NHR³⁹,
in which
R³⁹ is hydrogen, methyl or ethyl,
R⁴⁰ is hydrogen or fluorine,
R⁴¹ is hydroxyl or -NHR⁴²,
in which
R⁴² is hydrogen, methyl or ethyl,
R⁶ is bromine, chlorine, fluorine, methyl, difluoromethyl, trifluoromethyl, methoxy, difluoromethoxy or trifluoromethoxy,
R⁷ is hydrogen, chlorine or fluorine,
or one of the salts thereof, solvates thereof or solvates of the salts thereof.

2. Compound according to Claim 1, **characterized in that**
A is a bond or -CH₂-,
R¹ is hydrogen or methyl,
R² is hydrogen or methyl,
or
R¹ and R² together with the carbon atom to which they are bonded form a cyclopropyl ring,
R³ is hydrogen, methyl, ethyl, n-propyl, 2-methylprop-1-yl, n-butyl or ethoxy,
where methyl may be substituted by a substituent selected from the group consisting of difluoromethyl, trifluoromethyl, cyclopropyl, cyclobutyl, cyclohexyl, oxetanyl, tetrahydrofuranyl, tetrahydro-2H-pyranyl and 1,4-dioxanyl,
where cyclopropyl, cyclobutyl, cyclohexyl and oxetanyl may be substituted by 1 to 2 substituents selected independently from the group consisting of fluorine, hydroxyl, methyl, ethyl and methoxy,
and
where ethyl, n-propyl and n-butyl may be substituted by a substituent selected from the group consisting of fluorine, methoxy and trifluoromethoxy,
R⁴ is hydrogen,
R⁵ is a group of the formula 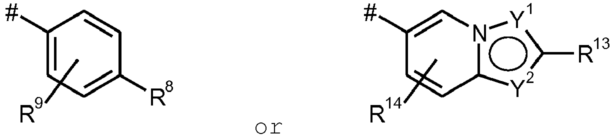 or 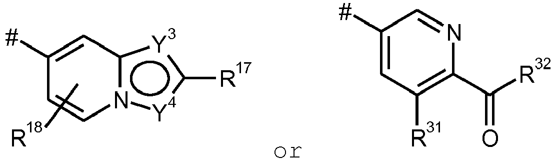 or 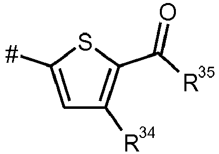
where # is the attachment point to the nitrogen atom,
R⁸ is hydroxycarbonyl, aminocarbonyl, oxazolyl, oxadiazolyl, thiadiazolyl, pyrazolyl, imidazolyl, triazolyl, tetrazolyl or dihydrooxazolyl,
where oxazolyl, oxadiazolyl, thiadiazolyl, pyrazolyl, imidazolyl, triazolyl and dihydrooxazolyl may be substituted by 1 to 2 substituents selected independently from the group consisting of oxo, hydroxyl, thioxo, sulphanyl, methyl, trifluoromethyl and 2-hydroxycarbonyl-1,1,2,2-tetrafluoroethyl,
in which methyl may be substituted by a methoxy substituent,
R⁹ is hydrogen, chlorine, fluorine or methyl,
Y¹ is a nitrogen atom or C-R¹⁵
in which
R¹⁵ is hydrogen, chlorine, hydroxyl or methoxy,
Y² is a nitrogen atom or C-R¹⁶
in which
R¹⁶ is hydrogen, chlorine, hydroxyl or methoxy,
R¹³ is hydrogen or hydroxycarbonyl,
R¹⁴ is hydrogen or fluorine,
Y³ is a nitrogen atom or C-R¹⁹
in which
R¹⁹ is hydrogen, chlorine, hydroxyl or methoxy,
Y⁴ is a nitrogen atom or C-R²⁰
in which
R²⁰ is hydrogen, chlorine, hydroxyl or methoxy,
R¹⁷ is hydrogen or hydroxycarbonyl,
R¹⁸ is hydrogen or fluorine,
R³¹ is hydrogen,
R³² is hydroxyl or -NHR³³, in which
R³³ is hydrogen,
R³⁴ is hydrogen,
R³⁵ is hydroxyl,
or
R⁵ is 2,3-dihydro-1H-indazol-6-yl, 1H-benzimidazol-6-yl, indol-6-yl, 2,3-dihydro-1H-indazol-5-yl, 2,3-dihydro-1H-benzimidazol-5-yl, indol-5-yl, 1H-indazol-6-yl, 1H-indazol-5-yl or 2H-indazol-5-yl,
where the 5-membered heterocycle in 2,3-dihydro-1H-indazol-6-yl, 1H-benzimidazol-6-yl, indol-6-yl, 2,3-dihydro-1H-indazol-5-yl, 2,3-dihydro-1H-benzimidazol-5-yl, indol-5-yl, 1H-indazol-6-yl, 1H-indazol-5-yl and 2H-indazol-5-yl may be substituted by 1 to 2 substituents independently selected from the group consisting of oxo, chlorine, hydroxycarbonyl, methyl and trifluoromethyl,
and
where the benzyl ring in 2,3-dihydro-1H-indazol-6-yl, 1H-benzimidazol-6-yl, indol-6-yl, 2,3-dihydro-1H-indazol-5-yl, 2,3-dihydro-1H-benzimidazol-5-yl, indol-5-yl, 1H-indazol-6-yl and 1H-indazol-5-yl may be substituted by a substituent selected from the group consisting of fluorine and methoxy.
R⁶ is chlorine,
R⁷ is hydrogen,
or one of the salts thereof, solvates thereof or solvates of the salts thereof.

3. Compound according to either of Claims 1 and 2, **characterized in that**
A is a bond or -CH₂-,
R¹ is hydrogen,
R² is hydrogen,
or
R¹ and R² together with the carbon atom to which they are bonded form a cyclopropyl ring,
R³ is hydrogen, ethyl or 2-methylprop-1-yl,
where ethyl may be substituted by a methoxy substituent,
R⁴ is hydrogen,
R⁵ is a group of the formula 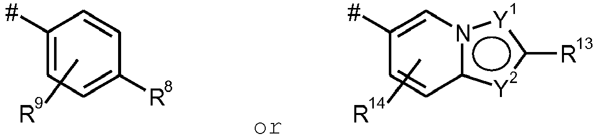 or 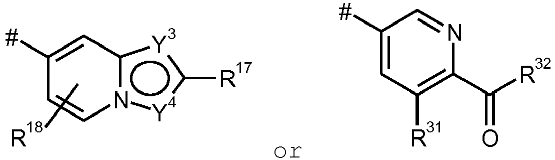 or 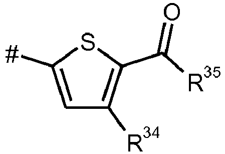 where # is the attachment point to the nitrogen atom,
R⁸ is hydroxycarbonyl, aminocarbonyl or oxadiazolyl,
where oxadiazolyl may be substituted by an oxo substituent,
R⁹ is hydrogen or fluorine,
Y¹ is C-R¹⁵,
in which
R¹⁵ is hydrogen,
Y² is a nitrogen atom,
R¹³ is hydrogen,
R¹⁴ is hydrogen,
Y³ is C-R¹⁹,
in which
R¹⁹ is hydrogen,
Y⁴ is a nitrogen atom,
R¹⁷ is hydrogen,
R¹⁸ is hydrogen,
R³¹ is hydrogen,
R³² is hydroxyl or -NHR³³,
in which
R³³ is hydrogen,
R³⁴ is hydrogen,
R³⁵ is hydroxyl,
or
R⁵ is 2,3-dihydro-1H-indazol-6-yl, 2,3-dihydro-1H-benzimidazol-5-yl, 1H-indazol-5-yl or 2H-indazol-5-yl,
where the 5-membered heterocycle in 2,3-dihydro-1H-indazol-6-yl, 2,3-dihydro-1H-benzimidazol-5-yl, 1H-indazol-5-yl and 2H-indazol-5-yl may be substituted by 1 to 2 substituents selected independently from the group consisting of oxo and methyl,
R⁶ is chlorine,
R⁷ is hydrogen,
or one of the salts thereof, solvates thereof or solvates of the salts thereof.

4. Process for preparing a compound of the formula (I) or one of the salts thereof, solvates thereof or solvates of the salts thereof according to Claim 1, **characterized in that** either
[A] a compound of the formula 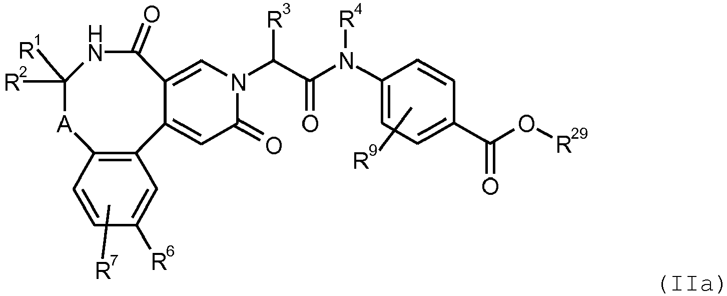 in which
A, R¹, R², R³, R⁴, R⁶, R⁷ and R⁹ have the definition given in Claim 1, and
R²⁹ is tert-butyl
is reacted with an acid to give a compound of the formula 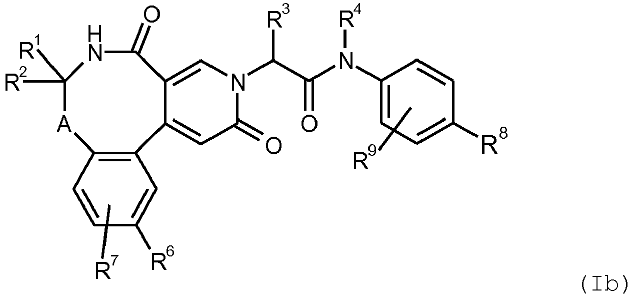 in which
A, R¹, R², R³, R⁴, R⁶, R⁷ and R⁹ have the definition given in Claim 1, and
R⁸ is hydroxycarbonyl,
or
[B] a compound of the formula 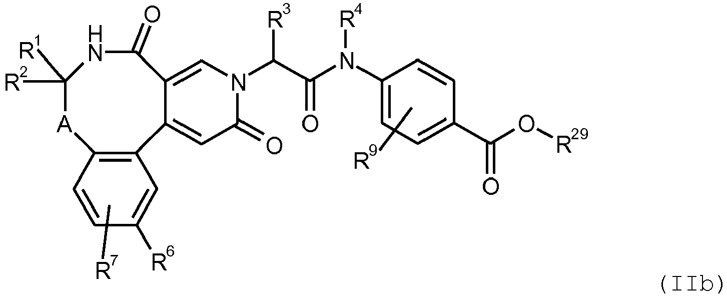 in which
A, R¹, R², R³, R⁴, R⁶, R⁷ and R⁹ have the definition given in Claim 1, and
R²⁹ is methyl or ethyl
is reacted with a base to give a compound of the formula 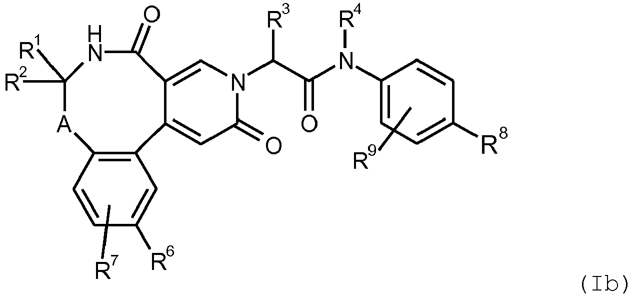 in which
A, R¹, R², R³, R⁴, R⁶, R⁷ and R⁹ have the definition given in Claim 1, and
R⁸ is hydroxycarbonyl,
or
[C] a compound of the formula 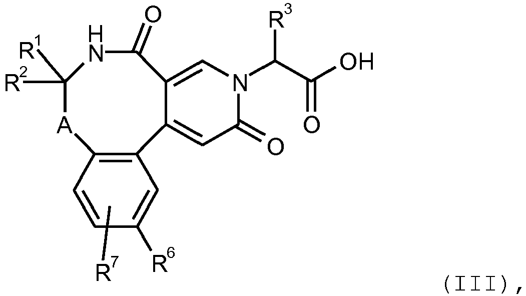 in which
A, R¹, R², R³, R⁶ and R⁷ have the definition given in Claim 1
is reacted with a compound of the formula 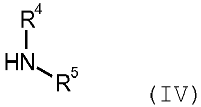 in which
R⁴ and R⁵ have the definition given in Claim 1
in the presence of a dehydrating reagent to give a compound of the formula (I).

5. Compound according to any of Claims 1 to 3 for use in the treatment and/or prophylaxis of diseases.

6. Use of a compound according to any of Claims 1 to 3 for production of a medicament for treatment and/or prophylaxis of diseases.

7. Use of a compound according to any of Claims 1 to 3 for production of a medicament for treatment and/or prophylaxis of thrombotic or thromboembolic disorders.

8. Use of a compound according to any of Claims 1 to 3 for production of a medicament for treatment and/or prophylaxis of ophthalmic disorders.

9. Use of a compound according to any of Claims 1 to 3 for production of a medicament for treatment and/or prophylaxis of hereditary angiooedema or inflammatory disorders of the intestine, such as Crohn's disease or ulcerative colitis.

10. Medicament comprising a compound according to any of Claims 1 to 3 in combination with an inert, nontoxic, pharmaceutically suitable excipient.

11. Medicament according to Claim 10 for use in the treatment and/or prophylaxis of thrombotic or thromboembolic disorders.

12. Medicament according to Claim 10 for use in the treatment and/or prophylaxis of ophthalmic disorders.

13. Medicament according to Claim 10 for use in the treatment and/or prophylaxis of hereditary angiooedema or inflammatory disorders of the intestine, such as Crohn's disease or ulcerative colitis.

## Revendications

1. Composé de formule 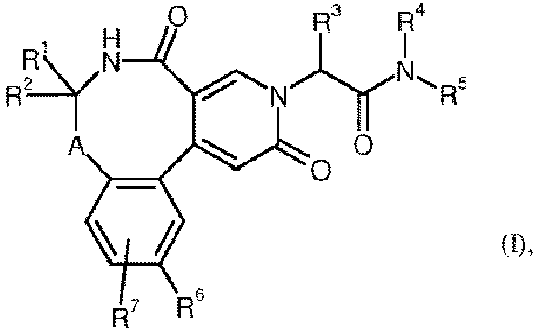 dans laquelle
A représente une liaison ou -CH₂-,
R¹ représente hydrogène ou méthyle,
le méthyle pouvant être substitué avec un substituant fluor,
R² représente hydrogène ou méthyle,
ou
R¹ et R² forment ensemble avec l'atome de carbone auquel ils sont reliés un cycle cyclopropyle,
R³ représente hydrogène, alkyle en C₁-C₅, alcoxy en C₁-C₄, difluorométhyle, trifluorométhyle, 1,1-difluoroéthyle, 3,3,3-trifluoro-2-hydroxyprop-1-yle, 3,3,3-trifluoro-2-méthoxyprop-1-yle, 3,3,3-trifluoro-2-éthoxyprop-1-yle, prop-2-yn-1-yle, cyclopropyloxy ou cyclobutyloxy,
l'alkyle pouvant être substitué avec un substituant choisi dans le groupe constitué par fluor, cyano, hydroxy, difluorométhyle, trifluorométhyle, méthoxy, éthoxy, difluorométhoxy, trifluorométhoxy, cycloalkyle en C₃-C₆, oxo-hétérocyclyle de 4 à 6 chaînons, 1,4-dioxanyle, oxazolyle, phényle et pyridyle,
le cycloalkyle pouvant être substitué avec 1 à 2 substituants choisis indépendamment l'un de l'autre dans le groupe constitué par fluor, hydroxy, méthyle, éthyle, méthoxy, éthoxy, difluorométhyle, trifluorométhyle, difluorométhoxy et trifluorométhoxy,
R⁴ représente hydrogène,
R⁵ représente un groupe de formule 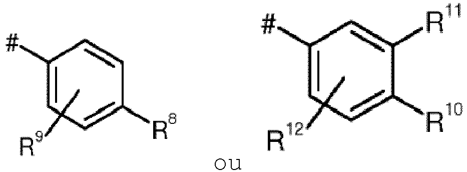 ou 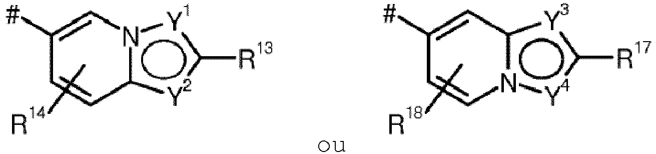 ou 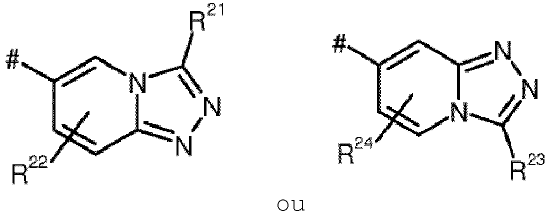 ou 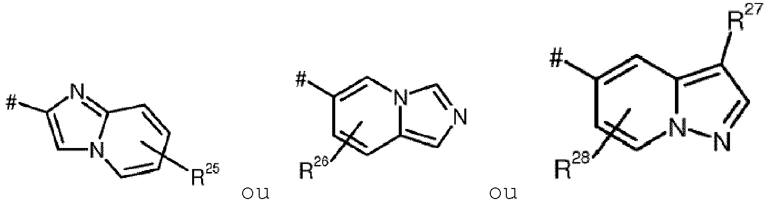 ou 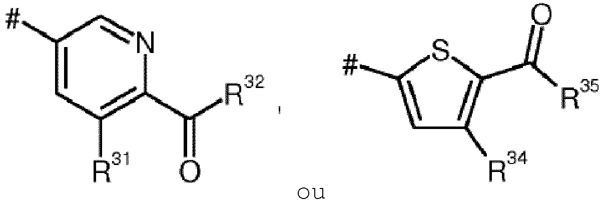 ou 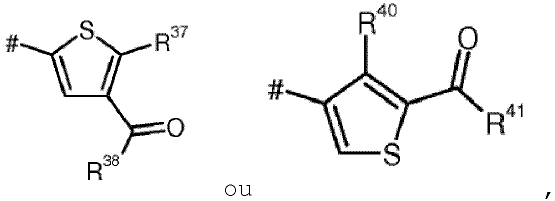
# étant l'emplacement de liaison à l'atome d'azote,
R⁸ représentant hydroxycarbonyle, aminocarbonyle ou hétérocyclyle à 5 chaînons,
l'hétérocyclyle pouvant être substitué avec 1 à 2 substituants choisis indépendamment l'un de l'autre dans le groupe constitué par oxo, hydroxy, thioxo, sulfanyle, méthyle, difluorométhyle, trifluorométhyle, 2-hydroxycarbonyl-1,1,2,2-tétrafluoroéthyle et 2-méthoxycarbonyl-1,1,2,2-tétrafluoroéthyle,
le méthyle pouvant être substitué avec un substituant méthoxy,
R⁹ représentant hydrogène, fluor ou méthyle,
R¹⁰ et R¹¹ formant ensemble avec les atomes de carbone auxquels ils sont reliés un hétérocycle à 5 chaînons,
l'hétérocycle pouvant être substitué avec 1 à 2 substituants choisis indépendamment l'un de l'autre dans le groupe constitué par oxo, chlore, hydroxy, hydroxycarbonyle, méthyle, difluorométhyle, trifluorométhyle, 1,1,2,2,2-pentafluoroéthyle, 2-hydroxycarbonyl-1,1,2,2-tétrafluoroéthyle et 2-méthoxy-carbonyl-1,1,2,2-tétrafluoroéthyle,
R¹² représentant hydrogène, chlore, fluor, méthyle ou méthoxy,
Y¹ représentant un atome d'azote ou C-R¹⁵,
R¹⁵ représentant hydrogène, chlore, hydroxy, méthoxy ou alcoxycarbonyle en C₁-C₃,
Y² représentant un atome d'azote ou C-R¹⁶,
R¹⁶ représentant hydrogène, chlore, hydroxy ou méthoxy,
R¹³ représentant hydrogène, hydroxycarbonyle, hydroxycarbonylméthyle ou phényle,
le phényle pouvant être substitué avec 1 à 2 substituants fluor,
R¹⁴ représentant hydrogène, chlore, fluor ou méthyle,
Y³ représentant un atome d'azote ou C-R¹⁹,
R¹⁹ représentant hydrogène, chlore, hydroxy ou méthoxy,
Y⁴ représentant un atome d'azote ou C-R²⁰,
R²⁰ représentant hydrogène, chlore, hydroxy ou méthoxy,
R¹⁷ représentant hydrogène, hydroxycarbonyle, hydroxycarbonylméthyle, alcoxycarbonyle en C₁-C₃ ou aminocarbonyle,
R¹⁸ représentant hydrogène, chlore, fluor ou méthyle,
R²¹ représentant hydrogène, chlore, hydroxy, alkyle en C₁-C₄, méthoxy, alkylaminométhyle en C₁-C₃ ou morpholinylméthyle,
R²² représentant hydrogène, chlore, fluor ou méthyle,
R²³ représentant hydrogène, chlore, hydroxy ou méthoxy,
R²⁴ représentant hydrogène, chlore, fluor ou méthyle,
R²⁵ représentant hydrogène, hydroxycarbonyle ou hydroxycarbonylméthyle,
R²⁶ représentant hydrogène, chlore, fluor ou méthyle,
R²⁷ représentant hydroxycarbonyle, aminocarbonyle, alcoxycarbonyle en C₁-C₃ ou alkylaminocarbonyle en C₁-C₃,
l'alkylaminocarbonyle pouvant être substitué avec un substituant choisi dans le groupe constitué par hydroxy, trifluorométhyle, méthoxy et trifluorométhoxy,
R²⁸ représentant hydrogène, chlore, fluor ou méthyle,
R³¹ représentant hydrogène ou fluor,
R³² représentant hydroxy ou -NHR³³,
R³³ représentant hydrogène, méthyle ou éthyle,
R³⁴ représentant hydrogène ou fluor,
R³⁵ représentant hydroxy ou -NHR³⁶,
R³⁶ représentant hydrogène, méthyle ou éthyle,
R³⁷ représentant hydrogène ou fluor,
R³⁸ représentant hydroxy ou -NHR³⁹,
R³⁹ représentant hydrogène, méthyle ou éthyle,
R⁴⁰ représentant hydrogène ou fluor,
R⁴¹ représentant hydroxy ou -NHR⁴²,
R⁴² représentant hydrogène, méthyle ou éthyle,
R⁶ représente brome, chlore, fluor, méthyle, difluorométhyle, trifluorométhyle, méthoxy, difluorométhoxy ou trifluorométhoxy,
R⁷ représente hydrogène, chlore ou fluor,
ou un de ses sels, de ses solvates ou des solvates de ses sels.

2. Composé selon la revendication 1, **caractérisé en ce que**
A représente une liaison ou -CH₂-,
R¹ représente hydrogène ou méthyle,
R² représente hydrogène ou méthyle,
ou
R¹ et R² forment ensemble avec l'atome de carbone auquel ils sont reliés un cycle cyclopropyle,
R³ représente hydrogène, méthyle, éthyle, n-propyle, 2-méthyl-prop-1-yle, n-butyle ou éthoxy,
le méthyle pouvant être substitué avec un substituant choisi dans le groupe constitué par difluorométhyle, trifluorométhyle, cyclopropyle, cyclobutyle, cyclohexyle, oxétanyle, tétrahydrofuranyle, tétrahydro-2H-pyranyle et 1,4-dioxanyle,
le cyclopropyle, le cyclobutyle, le cyclohexyle et l'oxétanyle pouvant être substitués avec 1 à 2 substituants choisis indépendamment l'un de l'autre dans le groupe constitué par fluor, hydroxy, méthyle, éthyle et méthoxy,
et
l'éthyle, le n-propyle et le n-butyle pouvant être substitués avec un substituant choisi dans le groupe constitué par fluor, méthoxy et trifluorométhoxy,
R⁴ représente hydrogène,
R⁵ représente un groupe de formule 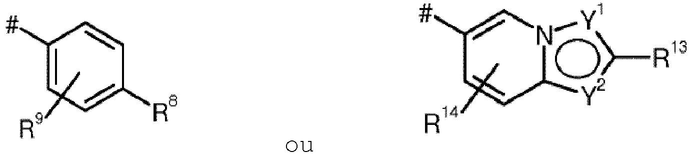 ou 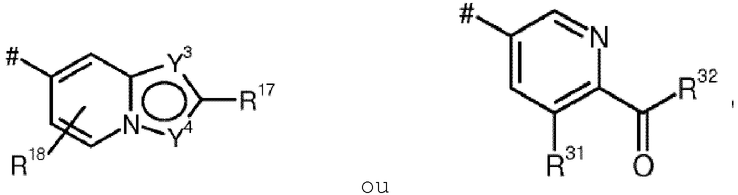 ou 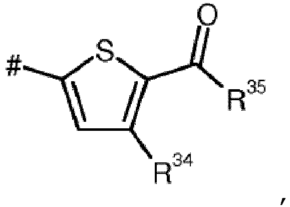
# étant l'emplacement de liaison à l'atome d'azote,
R⁸ représentant hydroxycarbonyle, aminocarbonyle, oxazolyle, oxadiazolyle, thiadiazolyle, pyrazolyle, imidazolyle, triazolyle, tétrazolyle ou dihydrooxazolyle,
l'oxazolyle, l'oxadiazolyle, le thiadiazolyle, le pyrazolyle, l'imidazolyle, le triazolyle et le dihydrooxazolyle pouvant être substitués avec 1 à 2 substituants choisis indépendamment l'un de l'autre dans le groupe constitué par oxo, hydroxy, thioxo, sulfanyle, méthyle, trifluorométhyle et 2-hydroxycarbonyl-1,1,2,2-tétrafluoroéthyle,
le méthyle pouvant être substitué avec un substituant méthoxy,
R⁹ représentant hydrogène, chlore, fluor ou méthyle,
Y¹ représentant un atome d'azote ou C-R¹⁵,
R¹⁵ représentant hydrogène, chlore, hydroxy ou méthoxy,
Y² représentant un atome d'azote ou C-R¹⁶,
R¹⁶ représentant hydrogène, chlore, hydroxy ou méthoxy,
R¹³ représentant hydrogène ou hydroxycarbonyle,
R¹⁴ représentant hydrogène ou fluor,
Y³ représentant un atome d'azote ou C-R¹⁹,
R¹⁹ représentant hydrogène, chlore, hydroxy ou méthoxy,
Y⁴ représentant un atome d'azote ou C-R²⁰,
R²⁰ représentant hydrogène, chlore, hydroxy ou méthoxy,
R¹⁷ représentant hydrogène ou hydroxycarbonyle,
R¹⁸ représentant hydrogène ou fluor,
R³¹ représentant hydrogène,
R³² représentant hydroxy ou -NHR³³,
R³³ représentant hydrogène,
R³⁴ représentant hydrogène,
R³⁵ représentant hydroxy,
ou
R⁵ représente 2,3-dihydro-1H-indazol-6-yle, 1H-benzimidazol-6-yle, indol-6-yle, 2,3-dihydro-1H-indazol-5-yle, 2,3-dihydro-1H-benzimidazol-5-yle, indol-5-yle, 1H-indazol-6-yle, 1H-indazol-5-yle ou 2H-indazol-5-yle,
l'hétérocycle à 5 chaînons dans le 2,3-dihydro-1H-indazol-6-yle, le 1H-benzimidazol-6-yle, l'indol-6-yle, le 2,3-dihydro-1H-indazol-5-yle, le 2,3-dihydro-1H-benzimidazol-5-yle, l'indol-5-yle, le 1H-indazol-6-yle, le 1H-indazol-5-yle et le 2H-indazol-5-yle pouvant être substitué avec 1 à 2 substituants choisis indépendamment l'un de l'autre dans le groupe constitué par oxo, chlore, hydroxycarbonyle, méthyle et trifluorométhyle,
et
le cycle benzyle dans le 2,3-dihydro-1H-indazol-6-yle, le 1H-benzimidazol-6-yle, l'indol-6-yle, le 2,3-dihydro-1H-indazol-5-yle, le 2,3-dihydro-1H-benzimidazol-5-yle, l'indol-5-yle, le 1H-indazol-6-yle et le 1H-indazol-5-yle pouvant être substitué avec un substituant choisi dans le groupe constitué par fluor et méthoxy,
R⁶ représente chlore,
R⁷ représente hydrogène,
ou un de ses sels, de ses solvates ou des solvates de ses sels.

3. Composé selon l'une quelconque des revendications 1 ou 2, **caractérisé en ce que**
A représente une liaison ou -CH₂-,
R¹ représente hydrogène,
R² représente hydrogène,
ou
R¹ et R² forment ensemble avec l'atome de carbone auquel ils sont reliés un cycle cyclopropyle,
R³ représente hydrogène, éthyle ou 2-méthyl-prop-1-yle,
l'éthyle pouvant être substitué avec un substituant méthoxy,
R⁴ représente hydrogène,
R⁵ représente un groupe de formule 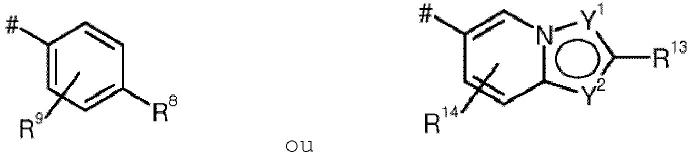 ou 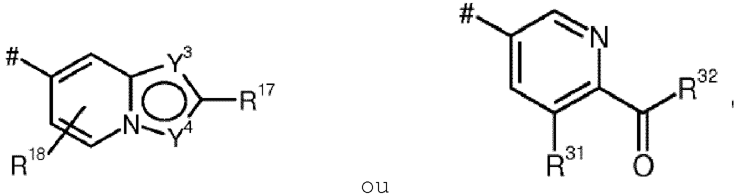 ou 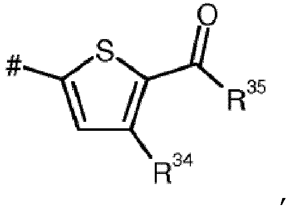
# étant l'emplacement de liaison à l'atome d'azote,
R⁸ représentant hydroxycarbonyle, aminocarbonyle ou oxadiazolyle,
l'oxadiazolyle pouvant être substitué avec un substituant oxo,
R⁹ représentant hydrogène ou fluor,
Y¹ représentant C-R¹⁵,
R¹⁵ représentant hydrogène,
Y² représentant un atome d'azote,
R¹³ représentant hydrogène,
R¹⁴ représentant hydrogène,
Y³ représentant C-R¹⁹,
R¹⁹ représentant hydrogène,
Y⁴ représentant un atome d'azote,
R¹⁷ représentant hydrogène,
R¹⁸ représentant hydrogène,
R³¹ représentant hydrogène,
R³² représentant hydroxy ou -NHR³³,
R³³ représentant hydrogène,
R³⁴ représentant hydrogène,
R³⁵ représentant hydroxy,
ou
R⁵ représente 2,3-dihydro-1H-indazol-6-yle, 2,3-dihydro-1H-benzimidazol-5-yle, 1H-indazol-5-yle ou 2H-indazol-5-yle,
l'hétérocycle à 5 chaînons dans le 2,3-dihydro-1H-indazol-6-yle, le 2,3-dihydro-1H-benzimidazol-5-yle, le 1H-indazol-5-yle et le 2H-indazol-5-yle pouvant être substitué avec 1 à 2 substituants choisis indépendamment l'un de l'autre dans le groupe constitué par oxo et méthyle,
R⁶ représente chlore,
R⁷ représente hydrogène,
ou un de ses sels, de ses solvates ou des solvates de ses sels.

4. Procédé de fabrication d'un composé de formule (I) ou d'un de ses sels, de ses solvates ou des solvates de ses sels selon la revendication 1, **caractérisé en ce que** soit
[A] un composé de formule 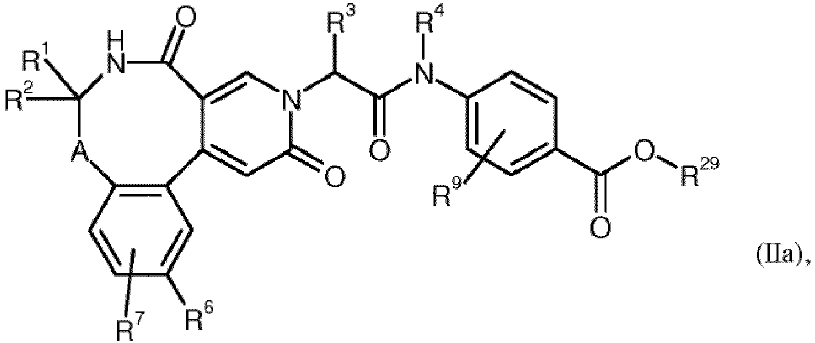 dans laquelle
A, R¹, R², R³, R⁴, R⁶, R⁷ et R⁹ ont la signification indiquée dans la revendication 1, et
R²⁹ représente tert-butyle,
est mis en réaction avec un acide pour former un composé de formule 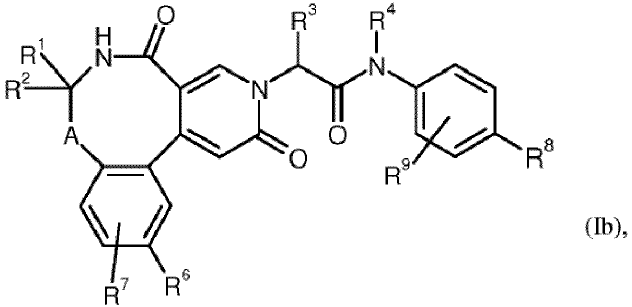 dans laquelle
A, R¹, R², R³, R⁴, R⁶, R⁷ et R⁹ ont la signification indiquée dans la revendication 1, et
R⁸ représente hydroxycarbonyle,
soit
[B] un composé de formule 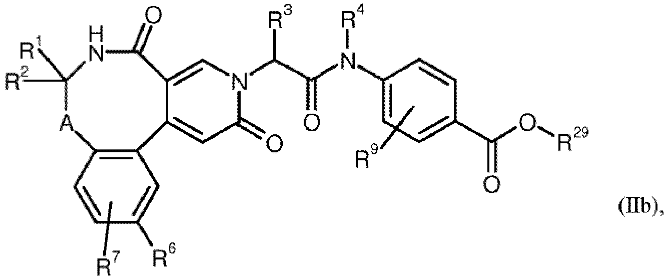 dans laquelle
A, R¹, R², R³, R⁴, R⁶, R⁷ et R⁹ ont la signification indiquée dans la revendication 1, et
R²⁹ représente méthyle ou éthyle,
est mis en réaction avec une base pour former un composé de formule 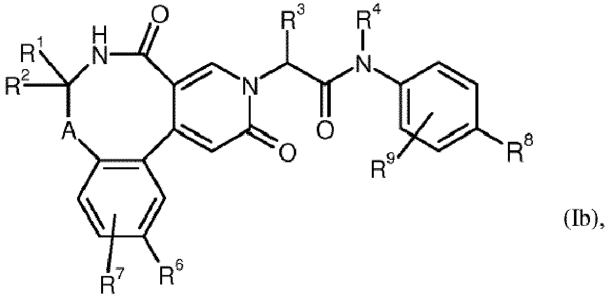 dans laquelle
A, R¹, R², R³, R⁴, R⁶, R⁷ et R⁹ ont la signification indiquée dans la revendication 1, et
R⁸ représente hydroxycarbonyle,
soit
[C] un composé de formule 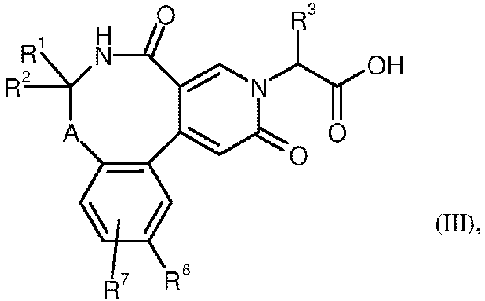 dans laquelle
A, R¹, R², R³, R⁶ et R⁷ ont la signification indiquée dans la revendication 1,
est mis en réaction avec un composé de formule 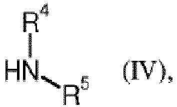 dans laquelle
R⁴ et R⁵ ont la signification indiquée dans la revendication 1,
en présence d'un réactif de déshydratation pour former un composé de formule (I).

5. Composé selon l'une quelconque des revendications 1 à 3, destiné à une utilisation pour le traitement et/ou la prophylaxie de maladies.

6. Utilisation d'un composé selon l'une quelconque des revendications 1 à 3 pour la fabrication d'un médicament pour le traitement et/ou la prophylaxie de maladies.

7. Utilisation d'un composé selon l'une quelconque des revendications 1 à 3 pour la fabrication d'un médicament pour le traitement et/ou la prophylaxie de maladies thrombotiques ou thromboemboliques.

8. Utilisation d'un composé selon l'une quelconque des revendications 1 à 3 pour la fabrication d'un médicament pour le traitement et/ou la prophylaxie de maladies ophtalmiques.

9. Utilisation d'un composé selon l'une quelconque des revendications 1 à 3 pour la fabrication d'un médicament pour le traitement et/ou la prophylaxie d'angio-oedèmes héréditaires ou de maladies inflammatoires de l'intestin, telles que la maladie de Crohn ou la colite ulcéreuse.

10. Médicament contenant un composé selon l'une quelconque des revendications 1 à 3 en combinaison avec un adjuvant inerte, non toxique, pharmaceutiquement approprié.

11. Médicament selon la revendication 10, destiné à une utilisation pour le traitement et/ou la prophylaxie de maladies thrombotiques ou thromboemboliques.

12. Médicament selon la revendication 10, destiné à une utilisation pour le traitement et/ou la prophylaxie de maladies ophtalmiques.

13. Médicament selon la revendication 10, destiné à une utilisation pour le traitement et/ou la prophylaxie d'angio-oedèmes héréditaires ou de maladies inflammatoires de l'intestin, telles que la maladie de Crohn ou la colite ulcéreuse.
